# EUROPEAN PATENT SPECIFICATION

(11) **EP 3 430 141 B1**
(45) Date of publication and mention of the grant of the patent: **30.12.2020**
(21) Application number: 17710874.3
(22) Date of filing: 14.03.2017
(51) Int. Cl.: C12N 15/113, A61K 31/712, A61K 31/7125, A61K 47/50, C07H 21/00, A61P 37/04, A61P 33/02, A61P 31/00

(54) **OLIGONUCLEOTIDES FOR REDUCTION OF PD-L1 EXPRESSION**
OLIGONUKLEOTIDE ZUR REDUKTION DER PD-L1-EXPRESSION
OLIGONUCLÉOTIDES DESTINÉS À LA RÉDUCTION DE L'EXPRESSION DE PD-L1

(30) Priority: 14.03.2016 EP 16160149
(43) Date of publication of application: 23.01.2019
(60) Divisional application: 20201780.2
(73) Proprietor: F. Hoffmann-La Roche AG, 4070 Basel (CH)
(72) Inventor: PEDERSEN, Lykke, 2970 Horsholm (DK); JAVANBAKHT, Hassan, 4070 Basel (CH); JACKEROTT, Malene, 2970 Horsholm (DK); OTTOSEN, Søren, 2970 Horsholm (DK); LUANGSAY, Souphalone, 4070 Basel (CH)
(74) Representative: D Young & Co LLP
(86) International application number: PCT/EP2017/055925
(87) International publication number: WO 2017/157899

(56) References cited:
- EP-A1- 1 537 878
- WO-A1-2011/127180
- WO-A1-2014/076195
- WO-A1-2014/118267
- WO-A1-2014/118272
- WO-A1-2017/055423
- WO-A2-2006/042237

## Description

### FIELD OF INVENTION

The present invention relates to oligonucleotides (oligomers) that are complementary to programmed death ligand-1 (PD-L1), leading to reduction of the expression of PD-L1 the liver. The present invention also relates to a method of alleviating the T cell exhaustion caused by an infection of the liver or cancer in the liver. Relevant infections are chronic HBV, HCV and HDV and parasite infections like malaria and toxoplasmosis (e.g. caused by protozoa of the *Plasmodium,* in particular of the species *P. vivax, P. malariae* and *P. falciparum).*

### BACKGROUND

The costimulatory pathway consisting of the programmed death-1 (PD-1) receptor and its ligand, PD-L1 (or B7-H1 or CD274) is known to contribute directly to T cell exhaustion resulting in lack of viral control during chronic infections of the liver. The PD-1 pathway also plays a role in autoimmunity as mice disrupted in this pathway develop autoimmune diseases.

It has been shown that antibodies that block the interaction between PD-1 and PD-L1 enhance T cell responses, in particular the response of CD8+ cytotoxic T cells (see Barber et al 2006 Nature Vol 439 p682 and Maier et al 2007 J. Immunol. Vol 178 p 2714).

WO 2006/042237 describes a method of diagnosing cancer by assessing PD-L1 (B7-H1) expression in tumors and suggests delivering an agent, which interferes with the PD-1/PD-L1 interaction, to a patient. Interfering agents can be antibodies, antibody fragments, siRNA or antisense oligonucleotides. There are no specific examples of such interfering agents nor is there any mentioning of chronic liver infections.

RNA interference mediated inhibition of PD-L1 using double stranded RNA (dsRNA, RNAi or siRNA) molecules have also been disclosed in for example WO 2005/007855, WO 2007/084865 and US 8,507,663. None of these describes targeted delivery to the liver.

Dolina et al. 2013 Molecular Therapy-Nucleic Acids, 2 e72 describes in vivo delivery of PD-L1 targeting siRNA molecules to Kupffer cells thereby enhancing NK and CD8+ T cell clearance in MCMV infected mice. This paper concludes that PD-L1 targeting siRNA molecules delivered to hepatocytes are not effective in relation to enhancing CD8+ T cell effector function.

The siRNA approach is significantly different from the single stranded antisense oligonucleotide approach since the biodistribution and the mode of actions is quite different. As described in Xu et al 2003 Biochem. Biophys. Res .Comm. Vol 306 page 712-717, antisense oligonucleotides and siRNAs have different preferences for target sites in the mRNA.

WO2016/138278 describes inhibition of immune checkpoints including PD-L1, using two or more single stranded antisense oligonucleotides that are linked at their 5' ends. The application does not mention HBV or targeted delivery to the liver.

### OBJECTIVE OF THE INVENTION

The present invention identifies novel oligonucleotides and oligonucleotide conjugates which reduce PD-L1 mRNA very efficiently in liver cells, both in parenchymal cells (e.g. hepatocytes) and in non-parenchymal cells such as Kupffer cells and liver sinusoidal endothelial cells (LSECs). By reducing or silencing PD-L1, the oligonucleotides and oligonucleotide conjugates decrease PD-1-mediated inhibition and thereby promote immunostimulation of exhausted T cells. Alleviation of the T cell exhaustion in a chronic pathogenic infection of the liver will result in regained immune control and reduced levels of viral antigens in the blood during a chronic pathogenic infection of the liver. Natural killer (NK) cells and natural killer T (NKT) cells may also be activated by the oligonucleotides and oligonucleotide conjugates of the present invention.

The oligonucleotide conjugates secures local reduction of PD-L1 in liver cells and therefore reduces the risk of autoimmune side effects, such as pneumonitis, non-viral hepatitis and colitis associated with systemic depletion of PD-L1.

### SUMMARY OF INVENTION

In one aspect the present invention is an antisense oligonucleotide of formula CCtatttaacatcAGAC, wherein capital letters represent beta-D-oxy LNA nucleosides, lowercase letters represent DNA nucleosides, all LNA C are 5-methyl cytosine and all internucleoside linkages are phosphorothioate internucleoside linkages.

In another aspect the present invention is an antisense oligonucleotide conjugate comprising the oligonucleotide of the present invention and a conjugate moiety covalently attached to said oligonucleotide.

In another aspect the present invention is a pharmaceutical composition comprising the antisense oligonucleotide of the present invention or the antisense oligonucleotide conjugate of the present invention and a pharmaceutically acceptable diluent, solvent, carrier, salt and/or adjuvant.

In another aspect the present invention is an *in vitro* method for modulating PD-L1 expression in a target cell which is expressing PD-L1, said method comprising administering the antisense oligonucleotide of the present invention, the antisense oligonucleotide conjugate of the present invention or the pharmaceutical composition of any one of the present invention in an effective amount to said cell.

In another aspect the present invention is the antisense oligonucleotide of the present invention, the antisense oligonucleotide conjugate of the present invention or the pharmaceutical composition of the present invention for use in restoration of immune response against a virus.

In another aspect the present invention is the antisense oligonucleotide of the present invention, the antisense oligonucleotide conjugate of the present invention or the pharmaceutical composition of the present invention for use in restoration of immune response against a parasite.

In another aspect the present invention is the antisense oligonucleotide of the present invention, the antisense oligonucleotide conjugate of the present invention or the pharmaceutical composition of the present invention for use as a medicament.

In another aspect the present invention is the antisense oligonucleotide of the present invention, the antisense oligonucleotide conjugate of the present invention or the pharmaceutical composition of the present invention for use in the treatment of HBV infection.

The present invention relates to oligonucleotides or conjugates thereof targeting a nucleic acid capable of modulating the expression of PD-L1 and to treat or prevent diseases related to the functioning of the PD-L1. The oligonucleotides or oligonucleotide conjugates may in particular be used to treat diseases where the immune response against an infectious agent has been exhausted.

Accordingly, described herein are oligonucleotides which comprise a contiguous nucleotide sequence of 10 to 30 nucleotides in length with at least 90% complementarity to a PD-L1 target nucleic acid. The oligonucleotide can be an antisense oligonucleotide, preferably with a gapmer design. Preferably, the oligonucleotide is capable of inhibiting the expression of PD-L1 by cleavage of a target nucleic acid. The cleavage is preferably achieved via nuclease recruitment.

In a further aspect, the oligonucleotide of the present invention is conjugated to at least one asialoglycoprotein receptor targeting conjugate moiety, such as a conjugate moiety comprising at least one N-Acetylgalactosamine (GalNAc) moiety. The conjugation moiety and the oligonucleotide may be linked together by a linker, in particular a biocleavable linker.

In a further aspect, the invention provides pharmaceutical compositions comprising the oligonucleotides or oligonucleotide conjugates of the invention and pharmaceutically acceptable diluents, carriers, salts and/or adjuvants.

Described herein are methods for *in vivo* or *in vitro* method for reduction of PD-L1 expression in a target cell which is expressing PD-L1, by administering an oligonucleotide or composition of the invention in an effective amount to said cell.

In a further aspect, the invention provides oligonucleotides, oligonucleotide conjugates or pharmaceutical compositions of the present invention for use in restoration of immunity against a virus or parasite.

In a further aspect, the invention provides oligonucleotides, oligonucleotide conjugates or pharmaceutical compositions of the present invention for use as a medicament.

Decribed herein are methods for treating or preventing a disease, disorder or dysfunction by administering a therapeutically or prophylactically effective amount of the oligonucleotide of the invention to a subject suffering from or susceptible to the disease, disorder or dysfunction, in particular diseases selected from viral liver infections or parasite infections.

As described herein the oligonucleotide, oligonucleotide conjugates or pharmaceutical composition of the invention is used in the treatment or prevention of viral liver infections such as HBV, HCV and HDV or a parasite infections such as malaria, toxoplasmosis, leishmaniasis and trypanosomiasis or liver cancer or metastases in the liver.

### BRIEF DESCRIPTION OF FIGURES

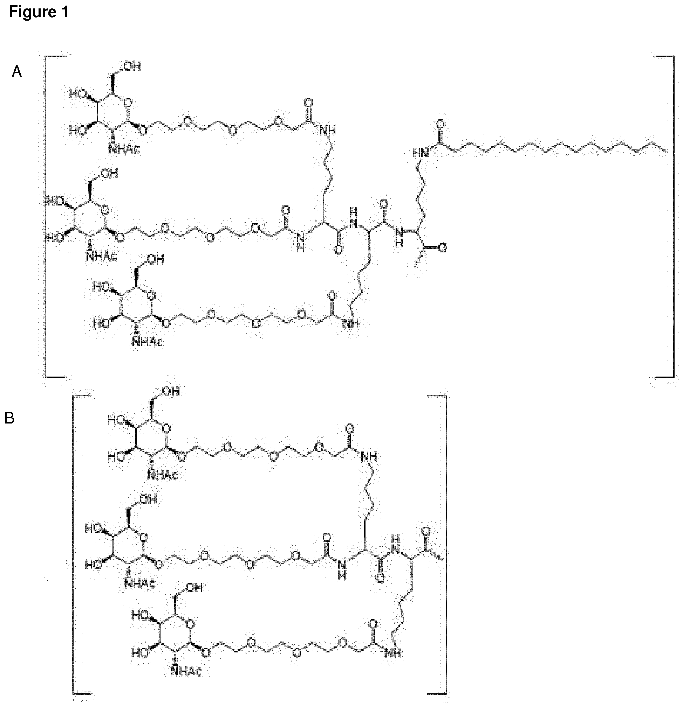

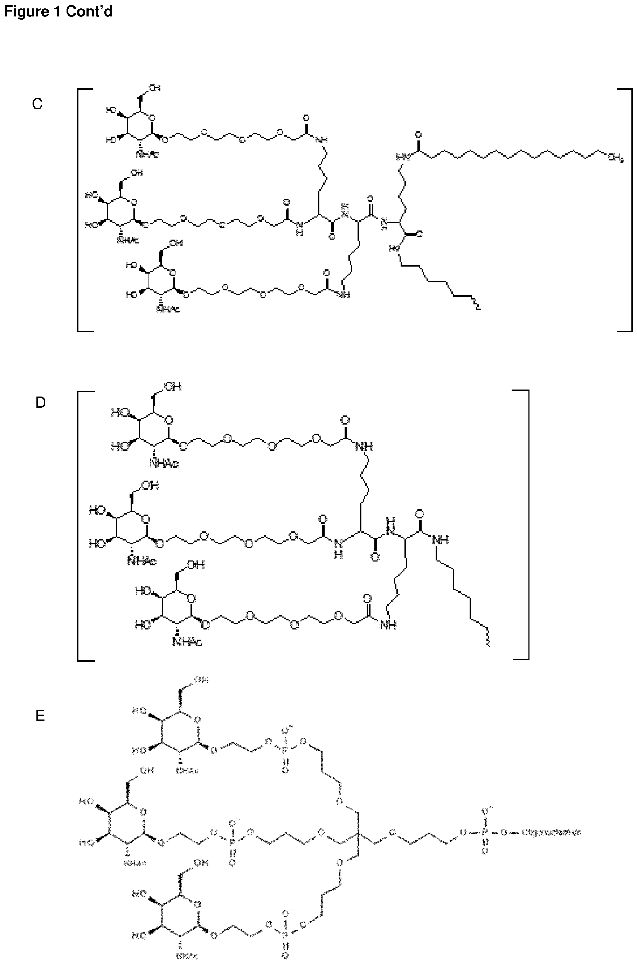

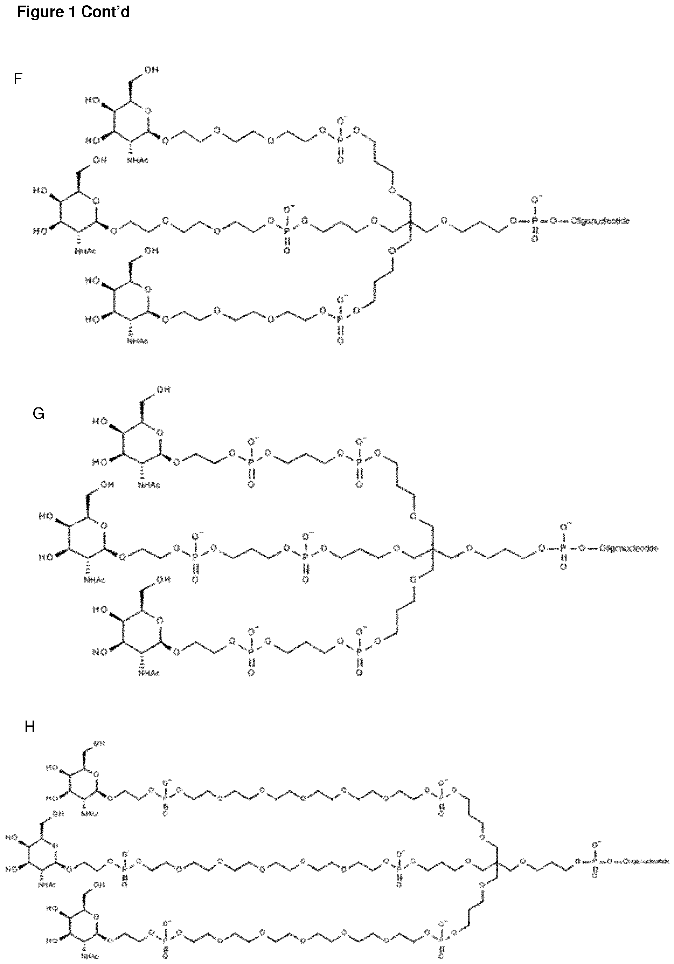

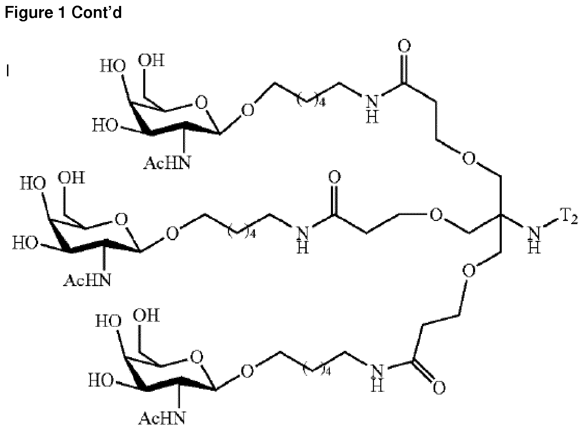

**Figure 1****:** Illustrates exemplary antisense oligonucleotide conjugates, where the oligonucleotide either is represented as a wavy line (A-D) or as "oligonucleotide" (E-H) or as T₂ (I) and the asialoglycoprotein receptor targeting conjugate moieties are trivalent N-acetylgalactosamine moieties. Compounds A to D comprise a di-lysine brancher molecule a PEG3 spacer and three terminal GalNAc carbohydrate moieties. In compound A and B the oligonucleotide is attached directly to the asialoglycoprotein receptor targeting conjugate moiety without a linker. In compound C and D the oligonucleotide is attached directly to the asialoglycoprotein receptor targeting conjugate moiety via a C6 linker. Compounds E-I comprise a trebler brancher molecule and spacers of varying length and structure and three terminal GalNAc carbohydrate moieties.
**Figure 2****:** Graph showing EC50 (A) and PD-L1 knock down as % of saline (B) for the compounds tested in Example 2, in relation to their position on the target nucleic acid. The cell line in which the compound were tested are THP1(•) and Karpas (*).
**Figure 3****:** Structural formula of the trivalent GalNAc cluster (GN2). GN2 is useful as conjugation moiety in the present invention. The wavy line illustrates the site of conjugation of the cluster to e.g. a C6 amino linker or directly to the oligonucleotide.
**Figure 4****:** Structural formula of CMP ID NO 766_2.
**Figure 5****:** Structural formula of CMP ID NO 767_2.
**Figure 6****:** Structural formula of CMP ID NO 768_2.
**Figure 7****:** Structural formula of CMP ID NO 769_2.
**Figure 8****:** Structural formula of CMP ID NO 770_2.

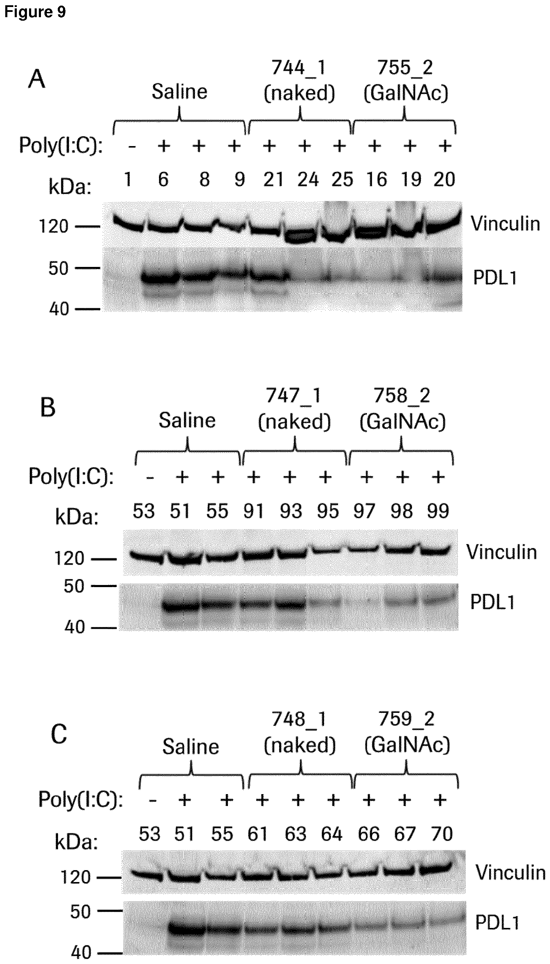

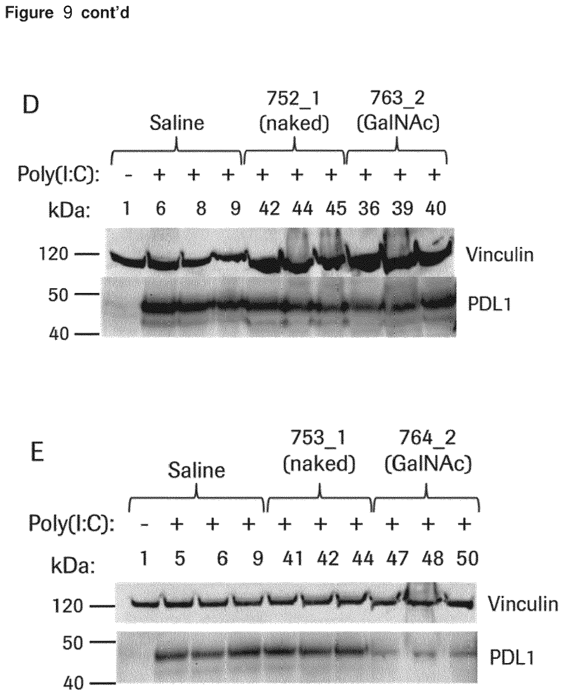

**Figure 9****:** Western blot detecting PD-L1 protein expression in liver from poly(IC) induced animals following treatment with saline and the indicated CMP ID NO's. Each blot shows a naked oligonucleotide versus a GalNAc conjugated version of the same oligonucleotide, blot A) CMP ID NO 744_1 and 755_2, B) CMP ID NO 747_1 and 758_2, C) CMP ID NO 748_1 and 759_2, D) CMP ID NO 752_1 and 763_2 and E) CMP ID NO 753_1 and 764_2. The upper band is the vinculin loading control, the lower band is the PD-L1 protein. The first lane in each blot show saline treated mice without Poly(IC) induction. These mice express very little PD-L protein.

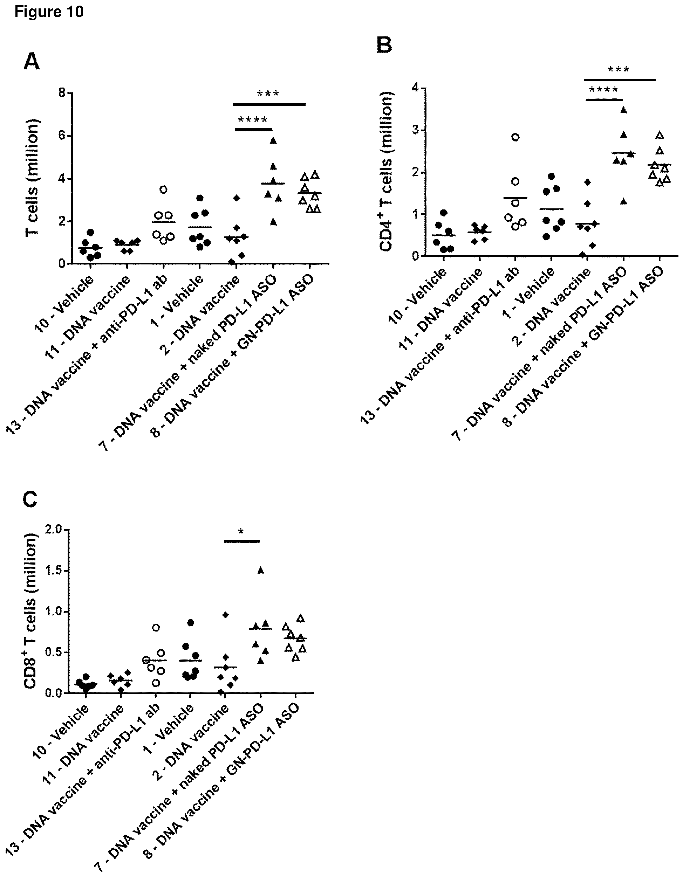

**Figure 10****:** Population of mononuclear cells in the liver after treatment with ● vehicle (group 10 and 1), ◆ DNA vaccine (group 11 and 2), ○ anti-PD-L1 antibody (group 12), ▲naked PD-L1 ASO + DNA vaccine (group 7) or △ GalNAc conjugated PD-L1 ASO + DNA vaccine (group 8), for each group the individual animals are represented and the average is indicated by the vertical line for each group (see table 18). Statistical significance between the DNA vaccine group and the three treatment groups has been assessed and if present it is indicated by * between the groups (* = P< 0.05, *** = P< 0.001 and **** = P< 0.0001). A) represents the number of T cells in the liver following treatment. B) represents the fraction of CD4+ T cells and C) represents the fraction of CD8+ T cells.

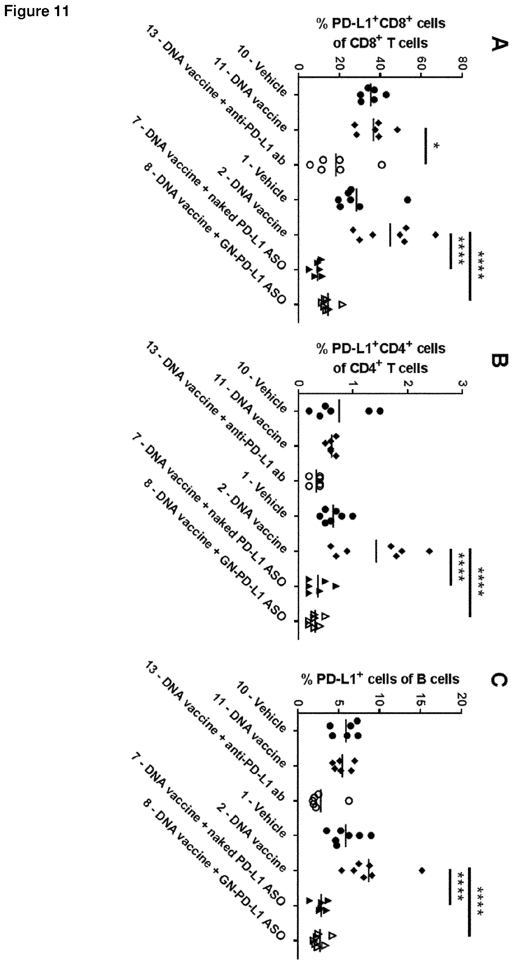

**Figure 11****:** Modulation of PD-L1 positive cells in the liver after treatment with ● vehicle (group 10 and 1), ◆ DNA vaccine (group 11 and 2), ○ anti-PD-L1 antibody (group 12), ▲naked PD-L1 ASO + DNA vaccine (group 7) or △ GalNAc conjugated PD-L1 ASO + DNA vaccine (group 8), for each group the individual animals are represented and the average is indicated by the vertical line for each group (see table 19). Statistical significance between the DNA vaccine group and the three treatment groups has been assessed and if present it is indicated by * between the groups (* = P< 0.05 and **** = P< 0.0001). A) represents the pertentage of CD8+ T cells which express PD-L1 in the liver following treatment. B) represents the pertentage of CD4+ T cells which express PD-L1 in the liver following treatment and C) represents the pertentage of B cells which express PD-L1 in the liver following treatment.

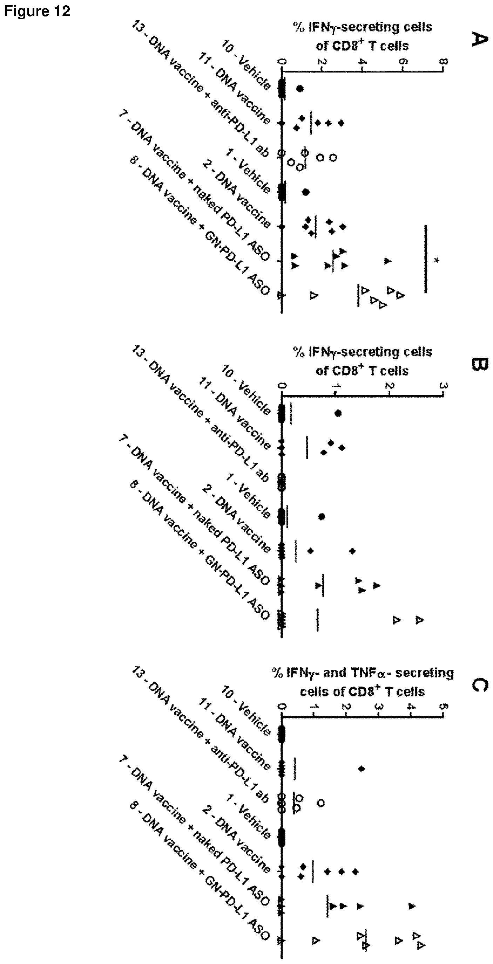

**Figure 12****:** HBV antigen specific CD8+ cytokine secreting cells in the liver after treatment with ● vehicle (group 10 and 1), ◆ DNA vaccine (group 11 and 2), ○ anti-PD-L1 antibody (group 12), ▲naked PD-L1 ASO + DNA vaccine (group 7) or △ GalNAc conjugated PD-L1 ASO + DNA vaccine (group 8), for each group the individual animals are represented and the average is indicated by the vertical line for each group (see table 20). Statistical significance between the DNA vaccine group and the three treatment groups has been assessed and if present it is indicated by * between the groups (* = P< 0.05). A) represents the pertentage of IFN-γ secreting CD8+ T cells in the liver which are specific towards HBV PreS2+S antigen following treatment. B) represents the pertentage of IFN-γ secreting CD8+ T cells in the liver which are specific towards HBV core antigen following treatment and C) represents the pertentage of IFN-γ and TNF-α secreting CD8+ T cells in the liver which are specific towards HBV PreS2+S antigen following treatment.
**Figure 13****:** HBV-DNA, HBsAg and HBeAg in AAV/HBV mice following treatment with GalNAc conjugated PD-L1 antisense CMP NO: 759_2 (▼) compared to vehicle (■). The vertical line indicates the end of treatment.

### DEFINITIONS

### Oligonucleotide

The term "oligonucleotide" as used herein is defined as it is generally understood by the skilled person as a molecule comprising two or more covalently linked nucleosides. Such covalently bound nucleosides may also be referred to as nucleic acid molecules or oligomers. Oligonucleotides are commonly made in the laboratory by solid-phase chemical synthesis followed by purification. When referring to a sequence of the oligonucleotide, reference is made to the sequence or order of nucleobase moieties, or modifications thereof, of the covalently linked nucleotides or nucleosides. The oligonucleotide of the invention is man-made, and is chemically synthesized, and is typically purified or isolated. The oligonucleotide of the invention may comprise one or more modified nucleosides or nucleotides.

### Antisense oligonucleotides

The term "Antisense oligonucleotide" as used herein is defined as oligonucleotides capable of modulating expression of a target gene by hybridizing to a target nucleic acid, in particular to a contiguous sequence on a target nucleic acid. The antisense oligonucleotides are not essentially double stranded and are therefore not siRNAs. Preferably, the antisense oligonucleotides of the present invention are single stranded.

### Contiguous Nucleotide Sequence

The term "contiguous nucleotide sequence" refers to the region of the oligonucleotide which is complementary to the target nucleic acid. The term is used interchangeably herein with the term "contiguous nucleobase sequence" and the term "oligonucleotide motif sequence". In some embodiments all the nucleotides of the oligonucleotide constitute the contiguous nucleotide sequence. In some embodiments the oligonucleotide comprises the contiguous nucleotide sequence and may optionally comprise further nucleotide(s), for example a nucleotide linker region which may be used to attach a functional group to the contiguous nucleotide sequence. The nucleotide linker region may or may not be complementary to the target nucleic acid.

### Nucleotides

Nucleotides are the building blocks of oligonucleotides and polynucleotides and for the purposes of the present invention include both naturally occurring and non-naturally occurring nucleotides. In nature, nucleotides, such as DNA and RNA nucleotides comprise a ribose sugar moiety, a nucleobase moiety and one or more phosphate groups (which is absent in nucleosides). Nucleosides and nucleotides may also interchangeably be referred to as "units" or "monomers".

### Modified nucleoside

The term "modified nucleoside" or "nucleoside modification" as used herein refers to nucleosides modified as compared to the equivalent DNA or RNA nucleoside by the introduction of one or more modifications of the sugar moiety or the (nucleo)base moiety. The modified nucleoside comprise a modified sugar moiety. The term modified nucleoside may also be used herein interchangeably with the term "nucleoside analogue" or modified "units" or modified "monomers".

### Modified internucleoside linkage

The term "modified internucleoside linkage" is defined as generally understood by the skilled person as linkages other than phosphodiester (PO) linkages, that covalently couples two nucleosides together. Nucleotides with modified internucleoside linkage are also termed "modified nucleotides". In some embodiments, the modified internucleoside linkage increases the nuclease resistance of the oligonucleotide compared to a phosphodiester linkage. For naturally occurring oligonucleotides, the internucleoside linkage includes phosphate groups creating a phosphodiester bond between adjacent nucleosides. Modified internucleoside linkages are particularly useful in stabilizing oligonucleotides for in vivo use, and may serve to protect against nuclease cleavage at regions of DNA or RNA nucleosides in the oligonucleotide of the invention, for example within the gap region of a gapmer oligonucleotide, as well as in regions of modified nucleosides.

In an embodiment, the oligonucleotide comprises one or more internucleoside linkages modified from the natural phosphodiester to a linkage that is for example more resistant to nuclease attack. Nuclease resistance may be determined by incubating the oligonucleotide in blood serum or by using a nuclease resistance assay (e.g. snake venom phosphodiesterase (SVPD)), both are well known in the art. Internucleoside linkages which are capable of enhancing the nuclease resistance of an oligonucleotide are referred to as nuclease resistant internucleoside linkages. In some embodiments at least 50% of the internucleoside linkages in the oligonucleotide, or contiguous nucleotide sequence thereof, are modified, such as at least 60%, such as at least 70%, such as at least 80 or such as at least 90% of the internucleoside linkages in the oligonucleotide, or contiguous nucleotide sequence thereof, are modified. In some embodiments all of the internucleoside linkages of the oligonucleotide, or contiguous nucleotide sequence thereof, are modified. It will be recognized that, in some embodiments the nucleosides which link the oligonucleotide of the invention to a non-nucleotide functional group, such as a conjugate, may be phosphodiester. In some embodiments all of the internucleoside linkages of the oligonucleotide, or contiguous nucleotide sequence thereof, are nuclease resistant internucleoside linkages.

Modified internucleoside linkages may be selected from the group comprising phosphorothioate, diphosphorothioate and boranophosphate. In some embodiments, the modified internucleoside linkages are compatible with the RNaseH recruitment of the oligonucleotide of the invention, for example phosphorothioate, diphosphorothioate or boranophosphate.

In some embodiments the internucleoside linkage comprises sulphur (S), such as a phosphorothioate internucleoside linkage.

A phosphorothioate internucleoside linkage is particularly useful due to nuclease resistance, beneficial pharmakokinetics and ease of manufacture. In some embodiments at least 50% of the internucleoside linkages in the oligonucleotide, or contiguous nucleotide sequence thereof, are phosphorothioate, such as at least 60%, such as at least 70%, such as at least 80 or such as at least 90% of the internucleoside linkages in the oligonucleotide, or contiguous nucleotide sequence thereof, are phosphorothioate. In some embodiments all of the internucleoside linkages of the oligonucleotide, or contiguous nucleotide sequence thereof, are phosphorothioate.

In some embodiments, the oligonucleotide comprises one or more neutral internucleoside linkage, particularly a internucleoside linkage selected from phosphotriester, methylphosphonate, MMI, amide-3, formacetal or thioformacetal.

Further internucleoside linkages are disclosed in WO2009/124238. In an embodiment the internucleoside linkage is selected from linkers disclosed in WO2007/031091. Particularly, the internucleoside linkage may be selected from -O-P(O)₂-O-, -O-P(O,S)-O-, -O-P(S)₂-O-, -S-P(O)₂-O-, -S-P(O,S)-O-, -S-P(S)₂-O-, -O-P(O)₂-S-, -O-P(O,S)-S-, -S-P(O)₂-S-, -O-PO(R^{H})-O-, 0-PO(OCH₃)-0-, -O-PO(NR^{H})-O-, -O-PO(OCH₂CH₂S-R)-O-, -O-PO(BH₃)-O-, -O-PO(NHR^{H})-O-, -OP(O)₂-NR^{H}-, -NR^{H}-P(O)₂-O-, -NR^{H}-CO-O-, -NR^{H}-CO-NR^{H}-, and/or the internucleoside linker may be selected form the group consisting of: -O-CO-O-, -O-CO-NR^{H}-, -NR^{H}-CO-CH₂-, -O-CH₂-CO-NR^{H}-, -O-CH₂-CH₂-NR^{H}-, -CO-NR^{H}-CH₂-, -CH₂-NR^{H}CO-, -O-CH₂-CH₂-S-, -S-CH₂-CH₂-O-, -S-CH₂-CH₂-S-, -CH₂-SO₂-CH₂-, -CH₂-CO-NR^{H}-, -O-CH₂-CH₂-NR^{H}-CO-, -CH₂-NCH₃-O-CH₂-, where R^{H} is selected from hydrogen and C1 -4-alkyl.

Nuclease resistant linkages, such as phosphothioate linkages, are particularly useful in oligonucleotide regions capable of recruiting nuclease when forming a duplex with the target nucleic acid, such as region G for gapmers, or the non-modified nucleoside region of headmers and tailmers. Phosphorothioate linkages may, however, also be useful in non-nuclease recruiting regions and/or affinity enhancing regions such as regions F and F' for gapmers, or the modified nucleoside region of headmers and tailmers.

Each of the design regions may however comprise internucleoside linkages other than phosphorothioate, such as phosphodiester linkages, in particularly in regions where modified nucleosides, such as LNA, protect the linkage against nuclease degradation. Inclusion of phosphodiester linkages, such as one or two linkages, particularly between or adjacent to modified nucleoside units (typically in the non-nuclease recruiting regions) can modify the bioavailability and/or bio-distribution of an oligonucleotide - see WO2008/113832.

As described herein all the internucleoside linkages in the oligonucleotide are phosphorothioate and/or boranophosphate linkages. Preferably, all the internucleoside linkages in the oligonucleotide are phosphorothioate linkages.

### Nucleobase

The term nucleobase includes the purine (e.g. adenine and guanine) and pyrimidine (e.g. uracil, thymine and cytosine) moiety present in nucleosides and nucleotides which form hydrogen bonds in nucleic acid hybridization. In the context of the present invention the term nucleobase also encompasses modified nucleobases which may differ from naturally occurring nucleobases, but are functional during nucleic acid hybridization. In this context "nucleobase" refers to both naturally occurring nucleobases such as adenine, guanine, cytosine, thymidine, uracil, xanthine and hypoxanthine, as well as non-naturally occurring variants. Such variants are for example described in Hirao et al (2012) Accounts of Chemical Research vol 45 page 2055 and Bergstrom (2009) Current Protocols in Nucleic Acid Chemistry Suppl. 37 1.4.1.

In a some embodiments the nucleobase moiety is modified by changing the purine or pyrimidine into a modified purine or pyrimidine, such as substituted purine or substituted pyrimidine, such as a nucleobased selected from isocytosine, pseudoisocytosine, 5-methyl cytosine, 5-thiozolo-cytosine, 5-propynyl-cytosine, 5-propynyl-uracil, 5-bromouracil 5-thiazolo-uracil, 2-thio-uracil, 2'thio-thymine, inosine, diaminopurine, 6-aminopurine, 2-aminopurine, 2,6-diaminopurine and 2-chloro-6-aminopurine.

The nucleobase moieties may be indicated by the letter code for each corresponding nucleobase, e.g. A, T, G, C or U, wherein each letter may optionally include modified nucleobases of equivalent function. For example, in the exemplified oligonucleotides, the nucleobase moieties are selected from A, T, G, C, and 5-methyl cytosine. Optionally, for LNA gapmers, 5-methyl cytosine LNA nucleosides may be used.

### Modified oligonucleotide

The term modified oligonucleotide describes an oligonucleotide comprising one or more sugar-modified nucleosides and/or modified internucleoside linkages. The term chimeric" oligonucleotide is a term that has been used in the literature to describe oligonucleotides with modified nucleosides.

### Complementarity

The term "complementarity" describes the capacity for Watson-Crick base-pairing of nucleosides/nucleotides. Watson-Crick base pairs are guanine (G)-cytosine (C) and adenine (A) - thymine (T)/uracil (U). It will be understood that oligonucleotides may comprise nucleosides with modified nucleobases, for example 5-methyl cytosine is often used in place of cytosine, and as such the term complementarity encompasses Watson Crick base-paring between non-modified and modified nucleobases (see for example Hirao et al (2012) Accounts of Chemical Research vol 45 page 2055 and Bergstrom (2009) Current Protocols in Nucleic Acid Chemistry Suppl. 37 1.4.1).

The term "% complementary" as used herein, refers to the number of nucleotides in percent of a contiguous nucleotide sequence in a nucleic acid molecule (e.g. oligonucleotide) which, at a given position, are complementary to (*i.e.* form Watson Crick base pairs with) a contiguous nucleotide sequence, at a given position of a separate nucleic acid molecule (e.g. the target nucleic acid). The percentage is calculated by counting the number of aligned bases that form pairs between the two sequences (when aligned with the target sequence 5'-3' and the oligonucleotide sequence from 3'-5'), dividing by the total number of nucleotides in the oligonucleotide and multiplying by 100. In such a comparison a nucleobase/nucleotide which does not align (form a base pair) is termed a mismatch.

The term "fully complementary", refers to 100% complementarity.

The following is an example of an oligonucleotide (SEQ ID NO: 5) that is fully complementary to the target nucleic acid (SEQ ID NO: 772).
5'gcagtagagccaatta3' (SEQ ID NO:772)
3'cgtcatctcggttaat5' (SEQ ID NO: 5)

### Identity

The term "Identity" as used herein, refers to the number of nucleotides in percent of a contiguous nucleotide sequence in a nucleic acid molecule (e.g. oligonucleotide) which, at a given position, are identical to (*i.e.* in their ability to form Watson Crick base pairs with the complementary nucleoside) a contiguous nucleotide sequence, at a given position of a separate nucleic acid molecule (e.g. the target nucleic acid). The percentage is calculated by counting the number of aligned bases that are identical between the two sequences, including gaps, dividing by the total number of nucleotides in the oligonucleotide and multiplying by 100. $Percent Identity = \left(Matches\times 100\right) / Length of aligned region \left(with gaps\right) .$

### Hybridization

The term "hybridizing" or "hybridizes" as used herein is to be understood as two nucleic acid strands (e.g. an oligonucleotide and a target nucleic acid) forming hydrogen bonds between base pairs on opposite strands thereby forming a duplex. The affinity of the binding between two nucleic acid strands is the strength of the hybridization. It is often described in terms of the melting temperature (Tₘ) defined as the temperature at which half of the oligonucleotides are duplexed with the target nucleic acid. At physiological conditions Tₘ is not strictly proportional to the affinity (Mergny and Lacroix, 2003, Oligonucleotides 13:515-537). The standard state Gibbs free energy ΔG° is a more accurate representation of binding affinity and is related to the dissociation constant (K_{d}) of the reaction by ΔG°=-RTIn(K_{d}), where R is the gas constant and T is the absolute temperature. Therefore, a very low ΔG° of the reaction between an oligonucleotide and the target nucleic acid reflects a strong hybridization between the oligonucleotide and target nucleic acid. ΔG° is the energy associated with a reaction where aqueous concentrations are 1M, the pH is 7, and the temperature is 37°C. The hybridization of oligonucleotides to a target nucleic acid is a spontaneous reaction and for spontaneous reactions ΔG° is less than zero. ΔG° can be measured experimentally, for example, by use of the isothermal titration calorimetry (ITC) method as described in Hansen et al., 1965, Chem. Comm. 36-38 and Holdgate et al., 2005, Drug Discov Today*.* The skilled person will know that commercial equipment is available for ΔG° measurements. ΔG° can also be estimated numerically by using the nearest neighbor model as described by SantaLucia, 1998, Proc Natl Acad Sci USA. 95: 1460-1465 using appropriately derived thermodynamic parameters described by Sugimoto et al., 1995, Biochemistry 34:11211-11216 and McTigue et al., 2004, Biochemistry 43:5388-5405. In order to have the possibility of modulating its intended nucleic acid target by hybridization, oligonucleotides of the present invention hybridize to a target nucleic acid with estimated ΔG° values below -10 kcal for oligonucleotides that are 10-30 nucleotides in length. In some embodiments the degree or strength of hybridization is measured by the standard state Gibbs free energy ΔG°. The oligonucleotides may hybridize to a target nucleic acid with estimated ΔG° values below the range of -10 kcal, such as below -15 kcal, such as below -20 kcal and such as below -25 kcal for oligonucleotides that are 8-30 nucleotides in length. In some embodiments the oligonucleotides hybridize to a target nucleic acid with an estimated ΔG° value of -10 to -60 kcal, such as -12 to -40, such as from -15 to -30 kcal or-16 to -27 kcal such as -18 to -25 kcal.

### Target nucleic acid

According to the present invention, the target nucleic acid is a nucleic acid which encodes mammalian PD-L1 and may for example be a gene, a RNA, a mRNA, and pre-mRNA, a mature mRNA or a cDNA sequence. The target may therefore be referred to as a PD-L1 target nucleic acid. The oligonucleotide as described herein may for example target exon regions of a mammalian PD-L1, or may for example target intron region in the PD-L1 pre-mRNA (see Table 1).

**Table 1: human PD-L1 Exons and Introns**

| Exonic regions in the human PD-L1 premRNA (SEQ ID NO 1) | | | Intronic regions in the human PD-L1 premRNA (SEQ ID NO 1) | | |
|---|---|---|---|---|---|
| **ID** | **start** | **end** | **ID** | **start** | **end** |
| e1 | 1 | 94 | i1 | 95 | 5597 |
| e2 | 5598 | 5663 | i2 | 5664 | 6576 |
| e3 | 6577 | 6918 | i3 | 6919 | 12331 |
| e4 | 12332 | 12736 | i4 | 12737 | 14996 |
| e5 | 14997 | 15410 | i5 | 15411 | 16267 |
| e6 | 16268 | 16327 | i6 | 16328 | 17337 |
| e7 | 17338 | 20064 | | | |

Suitably, the target nucleic acid encodes a PD-L1 protein, in particular mammalian PD-L1, such as human PD-L1 (See for example tables 2 and 3, which provide reference to the mRNA and pre-mRNA sequences for human, monkey, and mouse PD-L1). In the context of the present invention pre-mRNA is also considered as a nucleic acid that encodes a protein.

In some embodiments, the target nucleic acid is selected from the group consisting of SEQ ID NO: 1, 2 and 3 or naturally occurring variants thereof (e.g. sequences encoding a mammalian PD-L1 protein).

If employing the oligonucleotide of the invention in research or diagnostics the target nucleic acid may be a cDNA or a synthetic nucleic acid derived from DNA or RNA.

For *in vivo* or *in vitro* application, the oligonucleotide of the invention is typically capable of inhibiting the expression of the PD-L1 target nucleic acid in a cell which is expressing the PD-L1 target nucleic acid. The contiguous sequence of nucleobases of the oligonucleotide of the invention is typically complementary to the PD-L1 target nucleic acid, as measured across the length of the oligonucleotide, optionally with the exception of one or two mismatches, and optionally excluding nucleotide based linker regions which may link the oligonucleotide to an optional functional group such as a conjugate, or other non-complementary terminal nucleotides (e.g. region D' or D"). The target nucleic acid may, in some embodiments, be a RNA or DNA, such as a messenger RNA, such as a mature mRNA or a pre-mRNA. In some embodiments the target nucleic acid is a RNA or DNA which encodes mammalian PD-L1 protein, such as human PD-L1, e.g. the human PD-L1 premRNA sequence, such as that disclosed as SEQ ID NO 1 or the human mRNA sequence with NCBI reference number NM_014143. Further information on exemplary target nucleic acids is provided in tables 2 and 3.

**Table 2: Genome and assembly information for PD-L1 across species.**

| **Species** | **Chr.** | **Strand** | **Genomic coordinates** | | **Assembly** | **NCBI reference sequence* accession number for mRNA** |
|---|---|---|---|---|---|---|
| | | | **Start** | **End** | | |
| Human | 9 | fwd | 5450503 | 5470566 | GRCh38:CM000671.2 | NM_014143 |
| Cynomol gus monkey | 15 | | 73560846 | 73581371 | GCF_000364345.1 | XM_005581779 |
| Mouse | 19 | fwd | 29367455 | 29388095 | GRCm38:CM001012.2 | NM_021893 |

| | | | | | | |
|---|---|---|---|---|---|---|
| Fwd = forward strand. The genome coordinates provide the pre-mRNA sequence (genomic sequence). The NCBI reference provides the mRNA sequence (cDNA sequence). *The National Center for Biotechnology Information reference sequence database is a comprehensive, integrated, non-redundant, well-annotated set of reference sequences including genomic, transcript, and protein. It is hosted at www.ncbi.nlm.nih.gov/refseq. | | | | | | |

**Table 3: Sequence details for PD-L1 across species.**

| **Species** | **RNA type** | **Length (nt)** | **SEQ ID NO** |
|---|---|---|---|
| Human | premRNA | 20064 | 1 |
| Monkey Cyno | premRNA GCF ref | 20261 | 2 |
| Monkey Cyno | premRNA Internal | 20340 | 3 |
| Mouse | premRNA | 20641 | 4 |

### Target Sequence

The term "target sequence" as used herein refers to a sequence of nucleotides present in the target nucleic acid which comprises the nucleobase sequence which is complementary to the oligonucleotide of the invention. In some embodiments, the target sequence consists of a region on the target nucleic acid which is complementary to the contiguous nucleotide sequence of the oligonucleotide of the invention. In some embodiments the target sequence is longer than the complementary sequence of a single oligonucleotide, and may, for example represent a preferred region of the target nucleic acid which may be targeted by several oligonucleotides of the invention.

The target sequence may be a sub-sequence of the target nucleic acid.

In some embodiments the sub-sequence is a sequence selected from the group consisting of a1-a149 (see tables 4). In some embodiments the sub-sequence is a sequence selected from the group consisting of a human PD-L1 mRNA exon, such as a PD-L1 human mRNA exon selected from the group consisting of e1, e2, e3, e4, e5, e6, and e7 (see table 1 above).

In some embodiments the sub-sequence is a sequence selected from the group consisting of a human PD-L1 mRNA intron, such as a PD-L1 human mRNA intron selected from the group consisting of i1, i2, i3, i4, i5 and i6 (see table 1 above).

The oligonucleotide as described herein comprises a contiguous nucleotide sequence which is complementary to or hybridizes to the target nucleic acid, such as a sub-sequence of the target nucleic acid, such as a target sequence described herein.

The oligonucleotide as described herein comprises a contiguous nucleotide sequence of at least 8 nucleotides which is complementary to or hybridizes to a target sequence present in the target nucleic acid molecule. The contiguous nucleotide sequence (and therefore the target sequence) comprises of at least 8 contiguous nucleotides, such as 9, 10, 11, 12, 13, 14, 15, 16, 17, 18, 19, 20, 21, 22, 23, 24, 25, 26, 27, 28, 29 or 30 contiguous nucleotides, such as from 12-25, such as from 14-18 contiguous nucleotides.

### Target Cell

The term a "target cell" as used herein refers to a cell which is expressing the target nucleic acid. As described herein the target cell may be *in vivo* or *in vitro.* In some embodiments the target cell is a mammalian cell such as a rodent cell, such as a mouse cell or a rat cell, or a primate cell such as a monkey cell or a human cell.

In preferred embodiments the target cell expresses PD-L1 mRNA, such as the PD-L1 pre-mRNA or PD-L1 mature mRNA. The poly A tail of PD-L1 mRNA is typically disregarded for antisense oligonucleotide targeting.

### Naturally occurring variant

The term "naturally occurring variant" refers to variants of PD-L1 gene or transcripts which originate from the same genetic loci as the target nucleic acid, but may differ for example, by virtue of degeneracy of the genetic code causing a multiplicity of codons encoding the same amino acid, or due to alternative splicing of pre-mRNA, or the presence of polymorphisms, such as single nucleotide polymorphisms, and allelic variants. Based on the presence of the sufficient complementary sequence to the oligonucleotide, the oligonucleotide of the invention may therefore target the target nucleic acid and naturally occurring variants thereof.

In some embodiments, the naturally occurring variants have at least 95% such as at least 98% or at least 99% homology to a mammalian PD-L1 target nucleic acid, such as a target nucleic acid selected form the group consisting of SEQ ID NO 1, 2 and 3.

Numerous single nucleotide polymorphisms are known in the PD-L1 gene, for example those disclosed in the following table (human premRNA start/reference sequence is SEQ ID NO 2)

| **Variant name** | **Variant alleles** | **minor allele** | **Minor allele frequency** | **Start on SEQ ID NO: 1** |
|---|---|---|---|---|
| rs73397192 | G/A | A | 0,10 | 2591 |
| rs12342381 | A/G | G | 0,12 | 308 |
| rs16923173 | G/A | A | 0,13 | 14760 |
| rs2890658 | C/A | A | 0,16 | 14628 |
| rs2890657 | G/C | C | 0,21 | 2058 |
| rs3780395 | A/G | A | 0,21 | 14050 |
| rs147367592 | AG/- | - | 0,21 | 13425 |
| rs7023227 | T/C | T | 0,22 | 6048 |
| rs2297137 | G/A | A | 0,23 | 15230 |
| rs1329946 | G/A | A | 0,23 | 2910 |
| rs5896124 | -/G | G | 0,23 | 2420 |
| rs61061063 | T/C | C | 0,23 | 11709 |
| rs1411263 | T/C | C | 0,23 | 8601 |
| rs59906468 | A/G | G | 0,23 | 15583 |
| rs6476976 | T/C | T | 0,24 | 21012 |
| rs35744625 | C/A | A | 0,24 | 3557 |
| rs17804441 | T/C | C | 0,24 | 7231 |
| rs148602745 | C/T | T | 0,25 | 22548 |
| rs4742099 | G/A | A | 0,25 | 20311 |
| rs10815228 | T/C | C | 0,25 | 21877 |
| rs58817806 | A/G | G | 0,26 | 20769 |
| rs822342 | T/C | T | 0,27 | 3471 |
| rs10481593 | G/A | A | 0,27 | 7593 |
| rs822339 | A/G | A | 0,28 | 2670 |
| rs860290 | A/C | A | 0,28 | 2696 |
| rs822340 | A/G | A | 0,28 | 2758 |
| rs822341 | T/C | T | 0.28 | 2894 |
| rs12002985 | C/G | C | 0.28 | 6085 |
| rs822338 | C/T | C | 0.28 | 1055 |
| rs866066 | C/T | T | 0.28 | 451 |
| rs6651524 | A/T | T | 0.28 | 8073 |
| rs6415794 | A/T | A | 0.28 | 8200 |
| rs4143815 | G/C | C | 0.28 | 17755 |
| rs111423622 | G/A | A | 0.28 | 24096 |
| rs6651525 | C/A | A | 0.29 | 8345 |
| rs4742098 | A/G | G | 0.29 | 19995 |
| rs10975123 | C/T | T | 0.30 | 10877 |
| rs2282055 | T/G | G | 0.30 | 5230 |
| rs4742100 | A/C | C | 0.30 | 20452 |
| rs60520638 | -/TC | TC | 0.30 | 9502 |
| rs17742278 | T/C | C | 0.30 | 6021 |
| rs7048841 | T/C | T | 0.30 | 10299 |
| rs10815229 | T/G | G | 0.31 | 22143 |
| rs10122089 | C/T | C | 0.32 | 13278 |
| rs1970000 | C/A | C | 0.32 | 14534 |
| rs112071324 | AGAGAG/- | AGAGAG | 0.33 | 16701 |
| rs2297136 | G/A | G | 0.33 | 17453 |
| rs10815226 | A/T | T | 0.33 | 9203 |
| rs10123377 | A/G | A | 0.36 | 10892 |
| rs10123444 | A/G | A | 0.36 | 11139 |
| rs7042084 | G/T | G | 0.36 | 7533 |
| rs10114060 | G/A | A | 0.36 | 11227 |
| rs7028894 | G/A | G | 0.36 | 10408 |
| rs4742097 | C/T | C | 0.37 | 5130 |
| rs1536926 | G/T | G | 0.37 | 13486 |
| rs1411262 | C/T | T | 0.39 | 8917 |
| rs7041009 | G/A | A | 0.45 | 12741 |

### Modulation of expression

The term "modulation of expression" as used herein is to be understood as an overall term for an oligonucleotide's ability to alter the amount of PD-L1 when compared to the amount of PD-L1 before administration of the oligonucleotide. Alternatively modulation of expression may be determined by reference to a control experiment. It is generally understood that the control is an individual or target cell treated with a saline composition or an individual or target cell treated with a non-targeting oligonucleotide (mock). It may however also be an individual treated with the standard of care.

One type of modulation is an oligonucleotide's ability to inhibit, down-regulate, reduce, suppress, remove, stop, block, prevent, lessen, lower, avoid or terminate expression of PD-L1, e.g. by degradation of mRNA or blockage of transcription. Another type of modulation is an oligonucleotide's ability to restore, increase or enhance expression of PD-L1, e.g. by repair of splice sites or prevention of splicing or removal or blockage of inhibitory mechanisms such as microRNA repression.

### High affinity modified nucleosides

A high affinity modified nucleoside is a modified nucleotide which, when incorporated into the oligonucleotide enhances the affinity of the oligonucleotide for its complementary target, for example as measured by the melting temperature (T^{m}). A high affinity modified nucleoside of the present invention preferably result in an increase in melting temperature between +0.5 to +12°C, more preferably between +1.5 to +10°C and most preferably between+3 to +8°C per modified nucleoside. Numerous high affinity modified nucleosides are known in the art and include for example, many 2' substituted nucleosides as well as locked nucleic acids (LNA) (see e.g. Freier & Altmann; Nucl. Acid Res., 1997, 25, 4429-4443 and Uhlmann; Curr. Opinion in Drug Development, 2000, 3(2), 293-213).

### Sugar modifications

The oligomer of the invention may comprise one or more nucleosides which have a modified sugar moiety, *i.e.* a modification of the sugar moiety when compared to the ribose sugar moiety found in DNA and RNA.

Numerous nucleosides with modification of the ribose sugar moiety have been made, primarily with the aim of improving certain properties of oligonucleotides, such as affinity and/or nuclease resistance.

Such modifications include those where the ribose ring structure is modified, *e.g.* by replacement with a hexose ring (HNA), or a bicyclic ring, which typically have a biradicle bridge between the C2 and C4 carbons on the ribose ring (LNA), or an unlinked ribose ring which typically lacks a bond between the C2 and C3 carbons (e.g. UNA). Other sugar modified nucleosides include, for example, bicyclohexose nucleic acids (WO2011/017521) or tricyclic nucleic acids (WO2013/154798). Modified nucleosides also include nucleosides where the sugar moiety is replaced with a non-sugar moiety, for example in the case of peptide nucleic acids (PNA), or morpholino nucleic acids.

Sugar modifications also include modifications made via altering the substituent groups on the ribose ring to groups other than hydrogen, or the 2'-OH group naturally found in DNA and RNA nucleosides. Substituents may, for example be introduced at the 2', 3', 4' or 5' positions. Nucleosides with modified sugar moieties also include 2' modified nucleosides, such as 2' substituted nucleosides. Indeed, much focus has been spent on developing 2' substituted nucleosides, and numerous 2' substituted nucleosides have been found to have beneficial properties when incorporated into oligonucleotides, such as enhanced nucleoside resistance and enhanced affinity.

### 2' modified nucleosides.

A 2' sugar modified nucleoside is a nucleoside which has a substituent other than H or -OH at the 2' position (2' substituted nucleoside) or comprises a 2' linked biradicle, and includes 2' substituted nucleosides and LNA (2' - 4' biradicle bridged) nucleosides. For example, the 2' modified sugar may provide enhanced binding affinity and/or increased nuclease resistance to the oligonucleotide. Examples of 2' substituted modified nucleosides are 2'-O-alkyl-RNA, 2'-O-methyl-RNA, 2'-alkoxy-RNA, 2'-O-methoxyethyl-RNA (MOE), 2'-amino-DNA, 2'-Fluoro-RNA, and 2'-F-ANA nucleoside. For further examples, please see e.g. Freier & Altmann; Nucl. Acid Res., 1997, 25, 4429-4443 and Uhlmann; Curr. Opinion in Drug Development, 2000, 3(2), 293-213, and Deleavey and Damha, Chemistry and Biology 2012, 19, 937. Below are illustrations of some 2' substituted modified nucleosides.

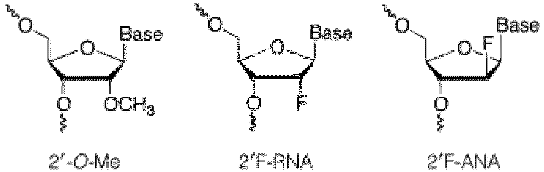

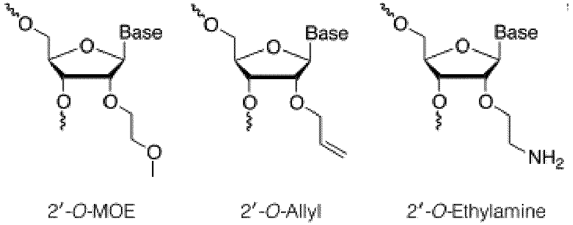

### Locked Nucleic Acid Nucleosides (LNA).

LNA nucleosides are modified nucleosides which comprise a linker group (referred to as a biradicle or a bridge) between C2' and C4' of the ribose sugar ring of a nucleotide. These nucleosides are also termed bridged nucleic acid or bicyclic nucleic acid (BNA) in the literature.

As described herein, the modified nucleoside or the LNA nucleosides of the oligomer of the invention has a general structure of the formula I or II: 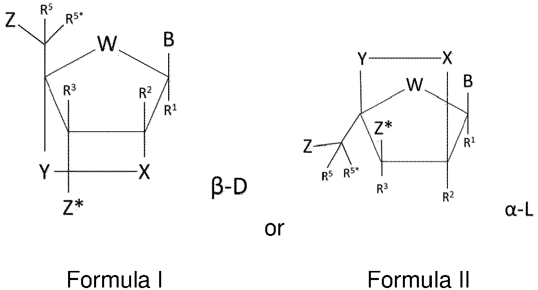
wherein W is selected from -O-, -S-, -N(R^{a})-, -C(R^{a}R^{b})-, such as, in some embodiments -O-; B designates a nucleobase or modified nucleobase moiety;
Z designates an internucleoside linkage to an adjacent nucleoside, or a 5'-terminal group;
Z* designates an internucleoside linkage to an adjacent nucleoside, or a 3'-terminal group;
X designates a group selected from the list consisting of -C(R^{a}R^{b})-, -C(R^{a})=C(R^{b})-,-C(R^{a})=N-, -O-, -Si(R^{a})₂-, -S-, -SO₂-, -N(R^{a})-, and >C=Z

In some embodiments, X is selected from the group consisting of: -O-, -S-, NH-, NR^{a}R^{b}, -CH₂-, CR^{a}R^{b}, -C(=CH₂)-, and -C(=CR^{a}R^{b})-

In some embodiments, X is -O-
Y designates a group selected from the group consisting of -C(R^{a}R^{b})-, -C(R^{a})=C(R^{b})-,-C(R^{a})=N-, -O-, -Si(R^{a})₂-, -S-, -SO₂-, -N(R^{a})-, and >C=Z

In some embodiments, Y is selected from the group consisting of: -CH₂-, -C(R^{a}R^{b})-,-CH₂CH₂-, -C(R^{a}R^{b})-C(R^{a}R^{b})-, -CH₂CH₂CH₂-, -C(R^{a}R^{b})C(R^{a}R^{b})C(R^{a}R^{b})-, -C(R^{a})=C(R^{b})-, and -C(R^{a})=N-

In some embodiments, Y is selected from the group consisting of: -CH₂-, -CHR^{a}-,-CHCH₃-, CR^{a}R^{b}-
or -X-Y- together designate a bivalent linker group (also referred to as a radicle) together designate a bivalent linker group consisting of 1, 2, 3 or 4 groups/atoms selected from the group consisting of -C(R^{a}R^{b})-, -C(R^{a})=C(R^{b})-, -C(R^{a})=N-, -O-, -Si(R^{a})₂-, -S-, -SO₂-, -N(R^{a})-, and >C=Z,

In some embodiments, -X-Y- designates a biradicle selected from the groups consisting of: -X-CH₂-, -X-CR^{a}R^{b}-, -X-CHR^{a-}, -X-C(HCH₃)⁻, -O-Y-, -O-CH₂-, -S-CH₂-, -NH-CH₂-, -O-CHCH₃-, -CH₂-O-CH₂, -O-CH(CH₃CH₃)-, -O-CH₂-CH₂-, OCH₂-CH₂-CH₂-,-O-CH₂OCH₂-,-O-NCH₂-, -C(=CH₂)-CH₂-, -NR^{a}-CH₂-, N-O-CH₂, -S-CR^{a}R^{b}- and -S-CHR^{a}-.

In some embodiments -X-Y- designates -O-CH₂- or -O-CH(CH₃)-.
wherein Z is selected from -O-, -S-, and -N(R^{a})-,
and R^{a} and, when present R^{b}, each is independently selected from hydrogen, optionally substituted C₁₋₆-alkyl, optionally substituted C₂₋₆-alkenyl, optionally substituted C₂₋₆-alkynyl, hydroxy, optionally substituted C₁₋₆-alkoxy, C₂₋₆-alkoxyalkyl, C₂₋₆-alkenyloxy, carboxy, c₁₋₆-alkoxycarbonyl, C₁₋₆-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkylaminocarbonyl, mono- and di(C₁₋₆-alkyl)amino-C₁₋₆-alkyl-aminocarbonyl, C₁₋₆-alkylcarbonylamino, carbamido, C₁₋₆-alkanoyloxy, sulphono, C₁₋₆-alkylsulphonyloxy, nitro, azido, sulphanyl, C₁₋₆-alkylthio, halogen, where aryl and heteroaryl may be optionally substituted and where two geminal substituents R^{a} and R^{b} together may designate optionally substituted methylene (=CH₂), wherein for all chiral centers, asymmetric groups may be found in either *R* or *S* orientation.
wherein R¹, R², R³, R⁵ and R^{5*} are independently selected from the group consisting of: hydrogen, optionally substituted C₁₋₆-alkyl, optionally substituted C₂₋₆-alkenyl, optionally substituted C₂₋₆-alkynyl, hydroxy, C₁₋₆-alkoxy, C₂₋₆-alkoxyalkyl, C₂₋₆-alkenyloxy, carboxy, C₁₋₆-alkoxycarbonyl, C₁₋₆-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkylaminocarbonyl, mono- and di(C₁₋₆-alkyl)amino-C₁₋₆-alkyl-aminocarbonyl, C₁₋₆-alkylcarbonylamino, carbamido, C₁₋₆-alkanoyloxy, sulphono, C₁₋₆-alkylsulphonyloxy, nitro, azido, sulphanyl, C₁₋₆-alkylthio, halogen, where aryl and heteroaryl may be optionally substituted, and where two geminal substituents together may designate oxo, thioxo, imino, or optionally substituted methylene.

In some embodiments R¹, R², R³, R⁵ and R^{5*} are independently selected from C₁₋₆ alkyl, such as methyl, and hydrogen.

In some embodiments R¹, R², R³, R⁵ and R^{5*} are all hydrogen.

In some embodiments R¹, R², R³, are all hydrogen, and either R⁵ and R^{5*} is also hydrogen and the other of R⁵ and R^{5*} is other than hydrogen, such as C₁₋₆ alkyl such as methyl.

In some embodiments, R^{a} is either hydrogen or methyl. In some embodiments, when present, R^{b} is either hydrogen or methyl.

In some embodiments, one or both of R^{a} and R^{b} is hydrogen

In some embodiments, one of R^{a} and R^{b} is hydrogen and the other is other than hydrogen

In some embodiments, one of R^{a} and R^{b} is methyl and the other is hydrogen

In some embodiments, both of R^{a} and R^{b} are methyl.

In some embodiments, the biradicle -X-Y- is -O-CH₂-, W is O, and all of R¹, R², R³, R⁵ and R^{5*} are all hydrogen. Such LNA nucleosides are disclosed in WO99/014226, WO00/66604, WO98/039352 and WO2004/046160 and include what are commonly known as **beta-D-oxy LNA** and **alpha-L-oxy LNA** nucleosides.

In some embodiments, the biradicle -X-Y- is -S-CH₂-, W is O, and all of R¹, R², R³, R⁵ and R^{5*} are all hydrogen. Such **thio LNA** nucleosides are disclosed in WO99/014226 and WO2004/046160.

In some embodiments, the biradicle -X-Y- is -NH-CH₂-, W is O, and all of R¹, R², R³, R⁵ and R^{5*} are all hydrogen. Such **amino LNA** nucleosides are disclosed in WO99/014226 and WO2004/046160.

In some embodiments, the biradicle -X-Y- is -O-CH₂-CH₂- or -O-CH₂-CH₂- CH₂-, W is O, and all of R¹, R², R³, R⁵ and R^{5*} are all hydrogen. Such LNA nucleosides are disclosed in WO00/047599 and Morita et al, Bioorganic & Med.Chem. Lett. 12 73-76 and include what are commonly known as 2'-O-4'C-ethylene bridged nucleic acids (ENA).

In some embodiments, the biradicle -X-Y- is -O-CH₂-, W is O, and all of R¹, R², R³, and one of R⁵ and R^{5*} are hydrogen, and the other of R⁵ and R^{5*} is other than hydrogen such as C₁₋₆ alkyl, such as methyl. Such **5' substituted** LNA nucleosides are disclosed in WO2007/134181.

In some embodiments, the biradicle -X-Y- is -O-CR^{a}R^{b}-, wherein one or both of R^{a} and R^{b} are other than hydrogen, such as methyl, W is O, and all of R¹, R², R³, and one of R⁵ and R^{5*} are hydrogen, and the other of R⁵ and R^{5*} is other than hydrogen such as C₁₋₆ alkyl, such as methyl. Such **bis modified LNA nucleosides** are disclosed in WO2010/077578.

In some embodiments, the biradicle -X-Y- designate the bivalent linker group -O-CH(CH₂OCH₃)- (2' O-methoxyethyl bicyclic nucleic acid - Seth at al., 2010, J. Org. Chem. Vol 75(5) pp. 1569-81). In some embodiments, the biradicle -X-Y- designate the bivalent linker group -O-CH(CH₂CH₃)- (2'O-ethyl bicyclic nucleic acid - Seth at al., 2010, J. Org. Chem. Vol 75(5) pp. 1569-81). In some embodiments, the biradicle -X-Y- is -O-CHR^{a}-, W is O, and all of R¹, R², R³, R⁵ and R^{5*} are all hydrogen. Such **6' substituted** LNA nucleosides are disclosed in WO10036698 and WO07090071.

In some embodiments, the biradicle -X-Y- is -O-CH(CH₂OCH₃)-, W is O, and all of R¹, R², R³, R⁵ and R^{5*} are all hydrogen. Such LNA nucleosides are also known as **cyclic MOEs** in the art (cMOE) and are disclosed in WO07090071.

In some embodiments, the biradicle -X-Y- designate the bivalent linker group -O-CH(CH₃)-. - in either the R- or S- configuration. In some embodiments, the biradicle -X-Y- together designate the bivalent linker group -O-CH₂-O-CH₂- (Seth at al., 2010, J. Org. Chem). In some embodiments, the biradicle -X-Y- is -O-CH(CH₃)-, W is O, and all of R¹, R², R³, R⁵ and R^{5*} are all hydrogen. Such 6' methyl LNA nucleosides are also known as **cET nucleosides** in the art, and may be either (S)cET or (R)cET stereoisomers, as disclosed in WO07090071 (beta-D) and WO2010/036698 (alpha-L).

In some embodiments, the biradicle -X-Y- is -O-CR^{a}R^{b}-, wherein in neither R^{a} or R^{b} is hydrogen, W is O, and all of R¹, R², R³, R⁵ and R^{5*} are all hydrogen. In some embodiments, R^{a} and R^{b} are both methyl. Such **6' di-substituted** LNA nucleosides are disclosed in WO 2009006478.

In some embodiments, the biradicle -X-Y- is -S-CHR^{a}-, W is O, and all of R¹, R², R³, R⁵ and R^{5*} are all hydrogen. Such **6' substituted thio** LNA nucleosides are disclosed in WO11156202. In some 6' substituted thio LNA embodiments R^{a} is methyl.

In some embodiments, the biradicle -X-Y- is -C(=CH2)-C(R^{a}R^{b})-, such as -C(=CH₂)-CH₂- , or-C(=CH₂)-CH(CH₃)-W is O, and all of R¹, R², R³, R⁵ and R^{5*} are all hydrogen. Such **vinyl carbo** LNA nucleosides are disclosed in WO08154401 and WO09067647.

In some embodiments the biradicle -X-Y- is -N(-OR^{a})-, W is O, and all of R¹, R², R³, R⁵ and R^{5*} are all hydrogen. In some embodiments R^{a} is C₁₋₆ alkyl such as methyl. Such LNA nucleosides are also known as N substituted LNAs and are disclosed in WO2008/150729. In some embodiments, the biradicle -X-Y- together designate the bivalent linker group -O-NR^{a}-CH₃-(Seth at al., 2010, J. Org. Chem). In some embodiments the biradicle -X-Y- is -N(R^{a})-, W is O, and all of R¹, R², R³, R⁵ and R^{5*} are all hydrogen. In some embodiments R^{a} is C₁₋₆ alkyl such as methyl.

In some embodiments, one or both of R⁵ and R^{5*} is hydrogen and, when substituted the other of R⁵ and R^{5*} is C₁₋₆ alkyl such as methyl. In such an embodiment, R¹, R², R³, may all be hydrogen, and the biradicle -X-Y- may be selected from -O-CH2- or -O-C(HCR^{a})-, such as -O-C(HCH3)-.

In some embodiments, the biradicle is -CR^{a}R^{b}-O-CR^{a}R^{b}-, such as CH₂-O-CH₂-, W is O and all of R¹, R², R³, R⁵ and R^{5*} are all hydrogen. In some embodiments R^{a} is C₁₋₆ alkyl such as methyl. Such LNA nucleosides are also known as conformationally restricted nucleotides (CRNs) and are disclosed in WO2013036868.In some embodiments, the biradicle is -O-CR^{a}R^{b}-O-CR^{a}R^{b}-, such as O-CH₂-O-CH₂-, W is O and all of R¹, R², R³, R⁵ and R^{5*} are all hydrogen. In some embodiments R^{a} is C₁₋₆ alkyl such as methyl. Such LNA nucleosides are also known as COC nucleotides and are disclosed in Mitsuoka et al., Nucleic Acids Research 2009 37(4), 1225-1238.

Certain examples of LNA nucleosides are presented in Scheme 1.

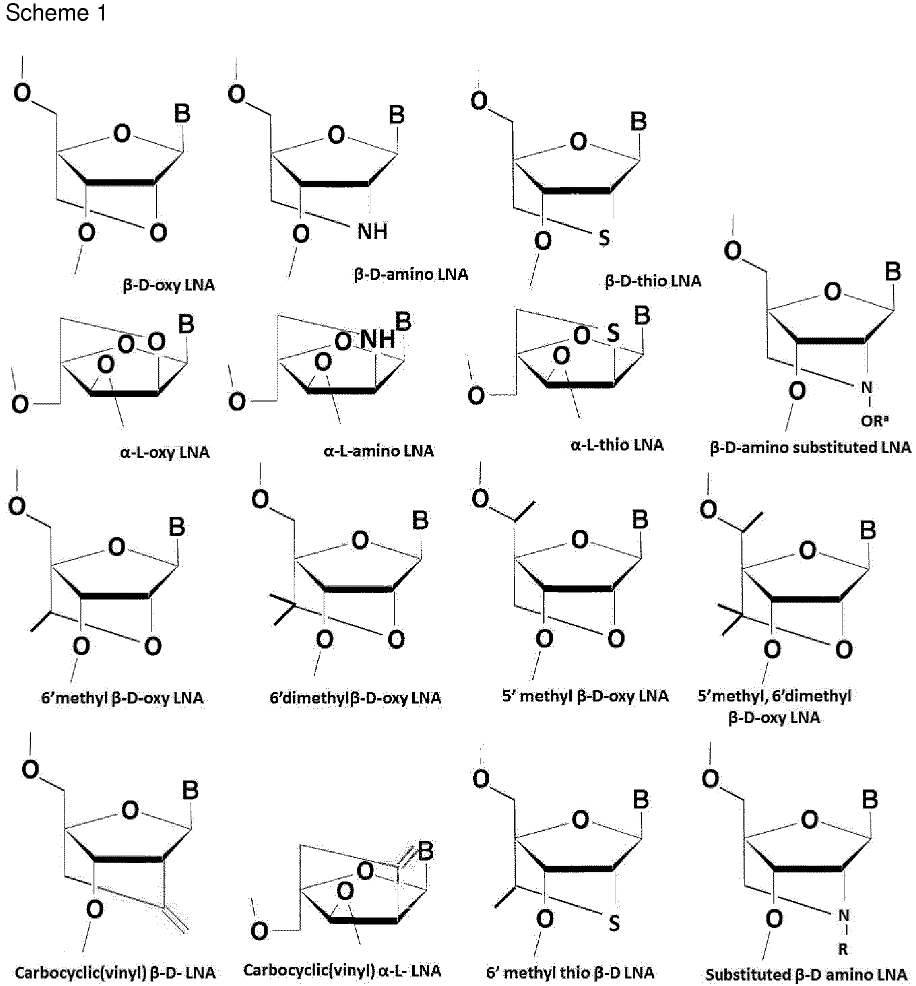

As illustrated in the examples, in some embodiments of the invention the LNA nucleosides in the oligonucleotides are beta-D-oxy-LNA nucleosides.

### Nuclease mediated degradation

Nuclease mediated degradation refers to an oligonucleotide capable of mediating degradation of a complementary nucleotide sequence when forming a duplex with such a sequence.

In some embodiments, the oligonucleotide may function via nuclease mediated degradation of the target nucleic acid, where the oligonucleotides of the invention are capable of recruiting a nuclease, particularly and endonuclease, preferably endoribonuclease (RNase), such as RNase H. Examples of oligonucleotide designs which operate via nuclease mediated mechanisms are oligonucleotides which typically comprise a region of at least 5 or 6 DNA nucleosides and are flanked on one side or both sides by affinity enhancing nucleosides, for example gapmers, headmers and tailmers.

### RNase H Activity and Recruitment

The RNase H activity of an antisense oligonucleotide refers to its ability to recruit RNase H when in a duplex with a complementary RNA molecule. WO01/23613 provides *in vitro* methods for determining RNaseH activity, which may be used to determine the ability to recruit RNaseH. Typically an oligonucleotide is deemed capable of recruiting RNase H if it, when provided with a complementary target nucleic acid sequence, has an initial rate, as measured in pmol/l/min, of at least 5%, such as at least 10% or more than 20% of the of the initial rate determined when using a oligonucleotide having the same base sequence as the modified oligonucleotide being tested, but containing only DNA monomers with phosphorothioate linkages between all monomers in the oligonucleotide, and using the methodology provided by Example 91 - 95 of WO01/23613.

### Gapmer

The term gapmer as used herein refers to an antisense oligonucleotide which comprises a region of RNase H recruiting oligonucleotides (gap) which is flanked 5' and 3' by regions which comprise one or more affinity enhancing modified nucleosides (flanks or wings). Various gapmer designs are described herein and a characterized by their ability to recruit RNaseH. Headmers and tailmers are oligonucleotides capable of recruiting RNase H where one of the flanks is missing, i.e. only one of the ends of the oligonucleotide comprises affinity enhancing modified nucleosides. For headmers the 3' flank is missing (i.e. the 5' flank comprises affinity enhancing modified nucleosides) and for tailmers the 5' flank is missing (i.e. the 3' flank comprises affinity enhancing modified nucleosides).

### LNA Gapmer

The term LNA gapmer is a gapmer oligonucleotide wherein at least one of the affinity enhancing modified nucleosides is an LNA nucleoside.

### Mixed Wing Gapmer

The term mixed wing gapmer or mixed flank gapmer refers to a LNA gapmer wherein at least one of the flank regions comprise at least one LNA nucleoside and at least one non-LNA modified nucleoside, such as at least one 2' substituted modified nucleoside, such as, for example, 2'-O-alkyl-RNA, 2'-O-methyl-RNA, 2'-alkoxy-RNA, 2'-O-methoxyethyl-RNA (MOE), 2'-amino-DNA, 2'-Fluoro-RNA and 2'-F-ANA nucleoside(s). As described herein the mixed wing gapmer has one flank which comprises only LNA nucleosides (e.g. 5' or 3') and the other flank (3' or 5' respectfully) comprises 2' substituted modified nucleoside(s) and optionally LNA nucleosides.

### Gapbreaker

The term "gapbreaker oligonucleotide" is used in relation to a gapmer capable of maintaining RNAseH recruitment even though the gap region is disrupted by a non-RNaseH recruiting nucleoside (a gap-breaker nucleoside, E) such that the gap region comprise less than 5 consecutive DNA nucleosides. Non-RNaseH recruiting nucleosides are for example nucleosides in the 3' endo conformation, such as LNA's where the bridge between C2' and C4' of the ribose sugar ring of a nucleoside is in the beta conformation, such as beta-D-oxy LNA or ScET nucleoside. The ability of gapbreaker oligonucleotide to recruit RNaseH is typically sequence or even compound specific - see Rukov et al. 2015 Nucl. Acids Res. Vol. 43 pp. 8476-8487, which discloses "gapbreaker" oligonucleotides which recruit RNaseH which in some instances provide a more specific cleavage of the target RNA.

The oligonucleotide of the invention described herein may be a gapbreaker oligonucleotide. As described herein the gapbreaker oligonucleotide may comprise a 5'-flank (F), a gap (G) and a 3'-flank (F'), wherein the gap is disrupted by a non-RNaseH recruiting nucleoside (a gap-breaker nucleoside, E) such that the gap contain at least 3 or 4 consecutive DNA nucleosides. As described herein the gapbreaker nucleoside (E) may be an LNA nucleoside where the bridge between C2' and C4' of the ribose sugar ring of a nucleoside is in the beta conformation and is placed within the gap region such that the gap-breaker LNA nucleoside is flanked 5' and 3' by at least 3 (5') and 3 (3') or at least 3 (5') and 4 (3') or at least 4(5') and 3(3') DNA nucleosides, and wherein the oligonucleotide is capable of recruiting RNaseH.

The gapbreaker oligonucleotide can be represented by the following formulae:
F-G-E-G-F'; in particular F₁₋₇-G₃₋₄-E₁-G₃₋₄-F'₁₋₇
D'-F-G-F', in particular D'₁₋₃-F₁₋₇-G₃₋₄-E₁-G₃₋₄-F'₁₋₇
F-G-F'-D", in particular F₁₋₇-G₃₋₄-E₁-G₃₋₄-F'₁₋₇-D"₁₋₃
D'-F-G-F'-D", in particular D'₁₋₃-F₁₋₇-G₃₋₄-E₁-G₃₋₄-F'₁₋₇-D"₁₋₃

Where region D' and D" are as described in the section "Gapmer design".

As described herein the gapbreaker nucleoside (E) is a beta-D-oxy LNA or ScET or another beta-LNA nucleosides shown in Scheme 1).

### Conjugate

The term conjugate as used herein refers to an oligonucleotide which is covalently linked to a non-nucleotide moiety (conjugate moiety or region C or third region), also termed a oligonucleotide conjugate.

Conjugation of the oligonucleotides of the invention to one or more non-nucleotide moieties may improve the pharmacology of the oligonucleotide, *e.g.* by affecting the activity, cellular distribution, cellular uptake or stability of the oligonucleotide. In some embodiments the conjugate moiety targets the oligonucleotide to the liver. A the same time the conjugate serve to reduce activity of the oligonucleotide in non-target cell types, tissues or organs, e.g. off target activity or activity in non-target cell types, tissues or organs. In one embodiment of the invention the oligonucleotide conjugate of the invention display improved inhibition of PD-L1 in the target cell when compared to an unconjugated oligonucleotide. In another embodiment the oligonucleotide conjugate of the invention has improved cellular distribution between liver and other organs, such as spleen or kidney (i.e. more conjugated oligonucleotide goes to the liver than the spleen or kidney) when compared to an unconjugated oligonucleotide. In another embodiment the oligonucleotide conjugate of the invention show improved cellular uptake into the liver of the conjugate oligonucleotide when compared to an unconjugated oligonucleotide.

WO 93/07883 and WO2013/033230 provides suitable conjugate moieties. Further suitable conjugate moieties are those capable of binding to the asialoglycoprotein receptor (ASGPr). In particular tri-valent N-acetylgalactosamine conjugate moieties are suitable for binding to the the ASGPr, see for example WO 2014/076196, WO 2014/207232 and WO 2014/179620. The conjugate moiety is essentially the part of the antisense oligonucleotides conjugates which is not composed of nucleic acids.

Oligonucleotide conjugates and their synthesis has also been reported in comprehensive reviews by Manoharan in Antisense Drug Technology, Principles, Strategies, and Applications, S.T. Crooke, ed., Ch. 16, Marcel Dekker, Inc., 2001 and Manoharan, Antisense and Nucleic Acid Drug Development, 2002, 12, 103.

In an embodiment, the non-nucleotide moiety (conjugate moiety) is selected from the group consisting of carbohydrates, cell surface receptor ligands, drug substances, hormones, lipophilic substances, polymers, proteins, peptides, toxins (e.g. bacterial toxins), vitamins, viral proteins (e.g. capsids) or combinations thereof.

### Linkers

A linkage or linker is a connection between two atoms that links one chemical group or segment of interest to another chemical group or segment of interest via one or more covalent bonds. Conjugate moieties can be attached to the oligonucleotide directly or through a linking moiety (e.g. linker or tether). Linkers serve to covalently connect a third region, e.g. a conjugate moiety (Region C), to a first region, e.g. an oligonucleotide or contiguous nucleotide sequence complementary to the target nucleic acid (region A).

In some embodiments of the invention the conjugate or oligonucleotide conjugate of the invention may optionally, comprise a linker region (second region or region B and/or region Y) which is positioned between the oligonucleotide or contiguous nucleotide sequence complementary to the target nucleic acid (region A or first region) and the conjugate moiety (region C or third region).

Region B refers to biocleavable linkers comprising or consisting of a physiologically labile bond that is cleavable under conditions normally encountered or analogous to those encountered within a mammalian body. Conditions under which physiologically labile linkers undergo chemical transformation (e.g., cleavage) include chemical conditions such as pH, temperature, oxidative or reductive conditions or agents, and salt concentration found in or analogous to those encountered in mammalian cells. Mammalian intracellular conditions also include the presence of enzymatic activity normally present in a mammalian cell such as from proteolytic enzymes or hydrolytic enzymes or nucleases. In one embodiment the biocleavable linker is susceptible to S1 nuclease cleavage. In a preferred embodiment the nuclease susceptible linker comprises between 1 and 10 nucleosides, such as 1, 2, 3, 4, 5, 6, 7, 8, 9 or 10 nucleosides, more preferably between 2 and 6 nucleosides and most preferably between 2 and 4 linked nucleosides comprising at least two consecutive phosphodiester linkages, such as at least 3 or 4 or 5 consecutive phosphodiester linkages. Preferably the nucleosides are DNA or RNA. Phosphodiester containing biocleavable linkers are described in more detail in WO 2014/076195.

Region Y refers to linkers that are not necessarily biocleavable but primarily serve to covalently connect a conjugate moiety (region C or third region), to an oligonucleotide or contiguous nucleotide sequence complementary to the target nucleic acid (region A or first region). The region Y linkers may comprise a chain structure or an oligomer of repeating units such as ethylene glycol, amino acid units or amino alkyl groups The oligonucleotide conjugates of the present invention can be constructed of the following regional elements A-C, A-B-C, A-B-Y-C, A-Y-B-C or A-Y-C. In some embodiments the linker (region Y) is an amino alkyl, such as a C2 - C36 amino alkyl group, including, for example C6 to C12 amino alkyl groups. In a preferred embodiment the linker (region Y) is a C6 amino alkyl group.

### Treatment

The term 'treatment' as used herein refers to both treatment of an existing disease (*e.g*. a disease or disorder as herein referred to), or prevention of a disease, *i.e.* prophylaxis. It will therefore be recognized that treatment as referred to herein may, in some embodiments, be prophylactic.

### Restoration of immune response against pathogens

The immune response is divided into the innate and adaptive immune response. The innate immune system provides an immediate, but non-specific response. The adaptive immune response is activated by innate immune response and is highly specific to a particular pathogen. Upon presentation of a pathogen-derived antigen on the surface of antigen-presenting cells, immune cells of the adaptive immune response (i.e. T and B lymphocytes) are activated through their antigen-specific receptors leading to a pathogenic-specifc immune response and development of immunological memory. Chronic viral infections, such as HBV and HCV, are associated with T cell exhaustion characterized by unresponsiveness of the viral-specific T cells. T cell exhaustion is well studied, for a review see for example Yi et al 2010 Immunology129, 474-481. Chronic viral infections are also associated with reduced function of NK cells that are innate immune cells. Enhancing viral immune response is important for clearance of chronic infection. Restoration of immune response against pathogens, mediated by T cells and NK cells, can be assessed by measurement of proliferation, cytokine secretion and cytolytic function (Dolina et al. 2013 Molecular Therapy-Nucleic Acids, 2 e72 and Example 6 herein).

### DETAILED DESCRIPTION OF THE INVENTION

The present invention relates to the use of antisense oligonucleotides and conjugates thereof and pharmaceutical compositions comprising these to restore immune response against pathogens that have infected an animal, in particular a human. The antisense oligonucleotide conjugates of the present invention are particular useful against pathogens that have infected the liver, in particular chronic liver infections like HBV. The conjugates allow targeted distribution of the oligonucleotides and prevents systemic knockdown of the target nucleic acid.

### The Oligonucleotides of the Invention

The invention relates to oligonucleotides capable of modulating expression of PD-L1. The modulation is may achieved by hybridizing to a target nucleic acid encoding PD-L1 or which is involved in the regulation of PD-L1. The target nucleic acid may be a mammalian PD-L1 sequence, such as a sequence selected from the group consisting of SEQ ID NO: 1, SEQ ID NO: 2 and/or SEQ ID NO: 3. The target nucleic acid may be a pre-mRNA, an mRNA or any RNA sequence expressed from a mammalian cell that supports the expression or regulation of PD-L1.

The oligonucleotide of the invention is an antisense oligonucleotide which targets PD-L1.

In one aspect of the invention the oligonucleotide of the invention is conjugated to a conjugate moiety, in particular an asialoglycoprotein receptor targeting conjugate moiety.

In some embodiments the antisense oligonucleotide of the invention is capable of modulating the expression of the target by inhibiting or down-regulating it. Preferably, such modulation produces an inhibition of expression of at least 20% compared to the normal expression level of the target, more preferably at least 30%, 40%, 50%, 60%, 70%, 80%, or 90% inhibition compared to the normal expression level of the target. Preferably, such modulation produces an inhibition of expression of at least 20% compared to the expression level when the cell or organism is challenged by an infectious agent, or treated with an agent simulating the challenge by an infectious agent (eg poly I:C or LPS), more preferably at least 30%, 40%, 50%, 60%, 70%, 80%, or 90% inhibition compared to the expression level when the cell or organism is challenged by an infectious agent, or treated with an agent simulating the challenge by an infectious agent (eg poly I:C or LPS). In some embodiments the oligonucleotide of the invention may be capable of inhibiting expression levels of PD-L1 mRNA by at least 60% or 70% *in vitro* using KARPAS-299 or THP1 cells. In some embodiments compounds of the invention may be capable of inhibiting expression levels of PD-L1 protein by at least 50% *in vitro* using KARPAS-299 or THP1 cells. Suitably, the examples provide assays which may be used to measure PD-L1 RNA (e.g. example 1). The target modulation is triggered by the hybridization between a contiguous nucleotide sequence of the oligonucleotide and the target nucleic acid. In some embodiments the oligonucleotide of the invention comprises mismatches between the oligonucleotide and the target nucleic acid. Despite mismatches, hybridization to the target nucleic acid may still be sufficient to show a desired modulation of PD-L1 expression. Reduced binding affinity resulting from mismatches may advantageously be compensated by increased number of nucleotides in the oligonucleotide and/or an increased number of modified nucleosides capable of increasing the binding affinity to the target, such as 2' modified nucleosides, including LNA, present within the oligonucleotide sequence.

In some embodiments the antisense oligonucleotide of the invention is capable of restoring pathogen-specific T cells. In some embodiments, the oligonucleotide of the invention is capable of increasing the pathogen-specific T cells by at least 40%, 50%, 60% or 70% when compared to untreated controls or controls treated with standard of care. In one embodiment the antisense oligonucleotide or conjugate of the invention is capable increasing HBV-specific T cells when compared to untreated controls or controls treated with standard of care. Suitably, the examples provide assays which may be used to measure the HBV-specific T cells (e.g. T cell proliferation, cytokine secretion and cytolytic activity). In another embodiment the the antisense oligonucleotide or conjugate of the invention is capable increasing HCV-specific T cells when compared to untreated controls or controls treated with standard of care. In another embodiment the the antisense oligonucleotide or conjugate of the invention is capable increasing HDV-specific T cells when compared to untreated controls or controls treated with standard of care.

In some embodiments the antisense oligonucleotide of the invention is capable reducing HBsAg levels in an animal or human. In some embodiments, the oligonucleotide of the invention is capable of reducing the HBsAg levels by at least 40%, 50%, 60% or 70%, more preferably by at least 80%, 90% or 95% when compared to the level prior to treatment. Most preferably oligonucleotides of the invention are capable of achieving seroconversion of HBsAg in an animal or human infected with HBV.

Described herein is an antisense oligonucleotide which comprises a contiguous nucleotide sequence of 10 to 30 nucleotides in length with at least 90% complementarity to a PD-L1 target nucleic acid.

As decribed herein, the oligonucleotide comprises a contiguous sequence which is at least 90% complementary, such as at least 91%, such as at least 92%, such as at least 93%, such as at least 94%, such as at least 95%, such as at least 96%, such as at least 97%, such as at least 98%, or 100% complementary with a region of the target nucleic acid.

As described herein, the oligonucleotide of the invention, or contiguous nucleotide sequence thereof is fully complementary (100% complementary) to a region of the target nucleic acid, or in some embodiments may comprise one or two mismatches between the oligonucleotide and the target nucleic acid.

As described herein, the oligonucleotide comprises a contiguous nucleotide sequence of 10 to 30 nucleotides in length with at least 90% complementary, such as fully (or 100%) complementary, to a region target nucleic acid region present in SEQ ID NO: 1 or SEQ ID NO: 2. As described herein the oligonucleotide sequence is 100% complementary to a corresponding target nucleic acid region present SEQ ID NO: 1 and SEQ ID NO: 2. As described herein the oligonucleotide sequence is 100% complementary to a corresponding target nucleic acid region present SEQ ID NO: 1 and SEQ ID NO: 3.

As described herein, the oligonucleotide or oligonucleotide conjugate comprises a contiguous nucleotide sequence of 10 to 30 nucleotides in length with at least 90% complementary, such as 100% complementarity, to a corresponding target nucleic acid region wherein the contiguous nucleotide sequence is complementary to a sub-sequence of the target nucleic acid selected from the group consisting of position 371-3068, 5467-12107 and 15317-19511 on SEQ ID NO: 1. As described herein the sub-sequence of the target nucleic acid is selected from the group consisting of position 371-510, 822-1090, 1992-3068, 5467-5606, 6470-12107, 15317-15720, 15317-18083, 18881-19494 and 1881-19494 on SEQ ID NO: 1. As described herein the sub-sequence of the target nucleic acid is selected from the group consisting of position 7300-7333, 8028-8072, 9812-9859, 11787-11873 and 15690-15735 on SEQ ID NO: 1.

As described herein, the oligonucleotide or oligonucleotide conjugate comprises a contiguous nucleotide sequence of 10 to 30 nucleotides in length with at least 90% complementary, such as 100% complementarity, to a corresponding target nucleic acid region present in SEQ ID NO: 1, wherein the target nucleic acid region is selected from the group consisting of region a1 to a449 in table 4.

**Table 4: Regions of SEQ ID NO 1 which may be targeted using oligonucleotide as described herein**

| **Reg. a** | **Position in SEQ ID NO 1** | | **Length** | **Reg. a** | **Position in SEQ ID NO 1** | | **Length** | **Reg. a** | **Position in SEQ ID NO 1** | | **Length** |
|---|---|---|---|---|---|---|---|---|---|---|---|
| | **from** | **to** | | | **from** | **to** | | | **from** | **to** | |
| a1 | 51 | 82 | 32 | a151 | 6994 | 7020 | 27 | a301 | 13092 | 13115 | 24 |
| a2 | 87 | 116 | 30 | a152 | 7033 | 7048 | 16 | a302 | 13117 | 13134 | 18 |
| a3 | 118 | 133 | 16 | a153 | 7050 | 7066 | 17 | a303 | 13136 | 13169 | 34 |
| a4 | 173 | 206 | 34 | a154 | 7078 | 7094 | 17 | a304 | 13229 | 13249 | 21 |
| a5 | 221 | 287 | 67 | a155 | 7106 | 7122 | 17 | a305 | 13295 | 13328 | 34 |
| a6 | 304 | 350 | 47 | a156 | 7123 | 7144 | 22 | a306 | 13330 | 13372 | 43 |
| a7 | 354 | 387 | 34 | a157 | 7146 | 7166 | 21 | a307 | 13388 | 13406 | 19 |
| a8 | 389 | 423 | 35 | a158 | 7173 | 7193 | 21 | a308 | 13408 | 13426 | 19 |
| a9 | 425 | 440 | 16 | a159 | 7233 | 7291 | 59 | a309 | 13437 | 13453 | 17 |
| a10 | 452 | 468 | 17 | a160 | 7300 | 7333 | 34 | a310 | 13455 | 13471 | 17 |
| a11 | 470 | 484 | 15 | a161 | 7336 | 7351 | 16 | a311 | 13518 | 13547 | 30 |
| a12 | 486 | 500 | 15 | a162 | 7353 | 7373 | 21 | a312 | 13565 | 13597 | 33 |
| a13 | 503 | 529 | 27 | a163 | 7375 | 7412 | 38 | a313 | 13603 | 13620 | 18 |
| a14 | 540 | 574 | 35 | a164 | 7414 | 7429 | 16 | a314 | 13630 | 13663 | 34 |
| a15 | 576 | 649 | 74 | a165 | 7431 | 7451 | 21 | a315 | 13665 | 13679 | 15 |
| a16 | 652 | 698 | 47 | a166 | 7453 | 7472 | 20 | a316 | 13706 | 13725 | 20 |
| a17 | 700 | 750 | 51 | a167 | 7474 | 7497 | 24 | a317 | 13727 | 13774 | 48 |
| a18 | 744 | 758 | 15 | a168 | 7517 | 7532 | 16 | a318 | 13784 | 13821 | 38 |
| a19 | 774 | 801 | 28 | a169 | 7547 | 7601 | 55 | a319 | 13831 | 13878 | 48 |
| a20 | 805 | 820 | 16 | a170 | 7603 | 7617 | 15 | a320 | 13881 | 13940 | 60 |
| a21 | 827 | 891 | 65 | a171 | 7632 | 7647 | 16 | a321 | 13959 | 14013 | 55 |
| a22 | 915 | 943 | 29 | a172 | 7649 | 7666 | 18 | a322 | 14015 | 14031 | 17 |
| a23 | 950 | 982 | 33 | a173 | 7668 | 7729 | 62 | a323 | 14034 | 14049 | 16 |
| a24 | 984 | 1000 | 17 | a174 | 7731 | 7764 | 34 | a324 | 14064 | 14114 | 51 |
| a25 | 1002 | 1054 | 53 | a175 | 7767 | 7817 | 51 | a325 | 14116 | 14226 | 111 |
| a26 | 1060 | 1118 | 59 | a176 | 7838 | 7860 | 23 | a326 | 14229 | 14276 | 48 |
| a27 | 1124 | 1205 | 82 | a177 | 7862 | 7876 | 15 | a327 | 14292 | 14306 | 15 |
| a28 | 1207 | 1255 | 49 | a178 | 7880 | 7944 | 65 | a328 | 14313 | 14384 | 72 |
| a29 | 1334 | 1349 | 16 | a179 | 7964 | 8012 | 49 | a329 | 14386 | 14408 | 23 |
| a30 | 1399 | 1425 | 27 | a180 | 8028 | 8072 | 45 | a330 | 14462 | 14481 | 20 |
| a31 | 1437 | 1458 | 22 | a181 | 8086 | 8100 | 15 | a331 | 14494 | 14519 | 26 |
| a32 | 1460 | 1504 | 45 | a182 | 8102 | 8123 | 22 | a332 | 14557 | 14577 | 21 |
| a33 | 1548 | 1567 | 20 | a183 | 8125 | 8149 | 25 | a333 | 14608 | 14628 | 21 |
| a34 | 1569 | 1586 | 18 | a184 | 8151 | 8199 | 49 | a334 | 14646 | 14668 | 23 |
| a35 | 1608 | 1662 | 55 | a185 | 8218 | 8235 | 18 | a335 | 14680 | 14767 | 88 |
| a36 | 1677 | 1700 | 24 | a186 | 8237 | 8276 | 40 | a336 | 14765 | 14779 | 15 |
| a37 | 1702 | 1721 | 20 | a187 | 8299 | 8344 | 46 | a337 | 14815 | 14844 | 30 |
| a38 | 1723 | 1745 | 23 | a188 | 8346 | 8436 | 91 | a338 | 14848 | 14925 | 78 |
| a39 | 1768 | 1794 | 27 | a189 | 8438 | 8470 | 33 | a339 | 14934 | 14976 | 43 |
| a40 | 1820 | 1835 | 16 | a190 | 8472 | 8499 | 28 | a340 | 14978 | 15009 | 32 |
| a41 | 1842 | 1874 | 33 | a191 | 8505 | 8529 | 25 | a341 | 15013 | 15057 | 45 |
| a42 | 1889 | 1979 | 91 | a192 | 8538 | 8559 | 22 | a342 | 15064 | 15091 | 28 |
| a43 | 1991 | 2011 | 21 | a193 | 8562 | 8579 | 18 | a343 | 15094 | 15140 | 47 |
| a44 | 2013 | 2038 | 26 | a194 | 8581 | 8685 | 105 | a344 | 15149 | 15165 | 17 |
| a45 | 2044 | 2073 | 30 | a195 | 8688 | 8729 | 42 | a345 | 15162 | 15182 | 21 |
| a46 | 2075 | 2155 | 81 | a196 | 8730 | 8751 | 22 | a346 | 15184 | 15198 | 15 |
| a47 | 2205 | 2228 | 24 | a197 | 8777 | 8800 | 24 | a347 | 15200 | 15221 | 22 |
| a48 | 2253 | 2273 | 21 | a198 | 8825 | 8865 | 41 | a348 | 15232 | 15247 | 16 |
| a49 | 2275 | 2303 | 29 | a199 | 8862 | 8894 | 33 | a349 | 15250 | 15271 | 22 |
| a50 | 2302 | 2333 | 32 | a200 | 8896 | 8911 | 16 | a350 | 15290 | 15334 | 45 |
| a51 | 2335 | 2366 | 32 | a201 | 8938 | 8982 | 45 | a351 | 15336 | 15369 | 34 |
| a52 | 2368 | 2392 | 25 | a202 | 8996 | 9045 | 50 | a352 | 15394 | 15416 | 23 |
| a53 | 2394 | 2431 | 38 | a203 | 9048 | 9070 | 23 | a353 | 15433 | 15451 | 19 |
| a54 | 2441 | 2455 | 15 | a204 | 9072 | 9139 | 68 | a354 | 15453 | 15491 | 39 |
| a55 | 2457 | 2494 | 38 | a205 | 9150 | 9168 | 19 | a355 | 15496 | 15511 | 16 |
| a56 | 2531 | 2579 | 49 | a206 | 9170 | 9186 | 17 | a356 | 15520 | 15553 | 34 |
| a57 | 2711 | 2732 | 22 | a207 | 9188 | 9202 | 15 | a357 | 15555 | 15626 | 72 |
| a58 | 2734 | 2757 | 24 | a208 | 9204 | 9236 | 33 | a358 | 15634 | 15652 | 19 |
| a59 | 2772 | 2786 | 15 | a209 | 9252 | 9283 | 32 | a359 | 15655 | 15688 | 34 |
| a60 | 2788 | 2819 | 32 | a210 | 9300 | 9331 | 32 | a360 | 15690 | 15735 | 46 |
| a61 | 2835 | 2851 | 17 | a211 | 9339 | 9354 | 16 | a361 | 15734 | 15764 | 31 |
| a62 | 2851 | 2879 | 29 | a212 | 9370 | 9398 | 29 | a362 | 15766 | 15787 | 22 |
| a63 | 2896 | 2912 | 17 | a213 | 9400 | 9488 | 89 | a363 | 15803 | 15819 | 17 |
| a64 | 2915 | 2940 | 26 | a214 | 9490 | 9537 | 48 | a364 | 15846 | 15899 | 54 |
| a65 | 2944 | 2973 | 30 | a215 | 9611 | 9695 | 85 | a365 | 15901 | 15934 | 34 |
| a66 | 2973 | 2992 | 20 | a216 | 9706 | 9721 | 16 | a366 | 15936 | 15962 | 27 |
| a67 | 2998 | 3016 | 19 | a217 | 9723 | 9746 | 24 | a367 | 15964 | 15985 | 22 |
| a68 | 3018 | 3033 | 16 | a218 | 9748 | 9765 | 18 | a368 | 15987 | 16023 | 37 |
| a69 | 3036 | 3051 | 16 | a219 | 9767 | 9788 | 22 | a369 | 16025 | 16061 | 37 |
| a70 | 3114 | 3139 | 26 | a220 | 9794 | 9808 | 15 | a370 | 16102 | 16122 | 21 |
| a71 | 3152 | 3173 | 22 | a221 | 9812 | 9859 | 48 | a371 | 16134 | 16183 | 50 |
| a72 | 3181 | 3203 | 23 | a222 | 9880 | 9913 | 34 | a372 | 16185 | 16281 | 97 |
| a73 | 3250 | 3271 | 22 | a223 | 9923 | 9955 | 33 | a373 | 16283 | 16298 | 16 |
| a74 | 3305 | 3335 | 31 | a224 | 9966 | 10007 | 42 | a374 | 16305 | 16323 | 19 |
| a75 | 3346 | 3363 | 18 | a225 | 10009 | 10051 | 43 | a375 | 16325 | 16356 | 32 |
| a76 | 3391 | 3446 | 56 | a226 | 10053 | 10088 | 36 | a376 | 16362 | 16404 | 43 |
| a77 | 3448 | 3470 | 23 | a227 | 10098 | 10119 | 22 | a377 | 16406 | 16456 | 51 |
| a78 | 3479 | 3497 | 19 | a228 | 10133 | 10163 | 31 | a378 | 16494 | 16523 | 30 |
| a79 | 3538 | 3554 | 17 | a229 | 10214 | 10240 | 27 | a379 | 16536 | 16562 | 27 |
| a80 | 3576 | 3597 | 22 | a230 | 10257 | 10272 | 16 | a380 | 16564 | 16580 | 17 |
| a81 | 3603 | 3639 | 37 | a231 | 10281 | 10298 | 18 | a381 | 16582 | 16637 | 56 |
| a82 | 3663 | 3679 | 17 | a232 | 10300 | 10318 | 19 | a382 | 16631 | 16649 | 19 |
| a83 | 3727 | 3812 | 86 | a233 | 10339 | 10363 | 25 | a383 | 16655 | 16701 | 47 |
| a84 | 3843 | 3869 | 27 | a234 | 10409 | 10426 | 18 | a384 | 16737 | 16781 | 45 |
| a85 | 3874 | 3904 | 31 | a235 | 10447 | 10497 | 51 | a385 | 16783 | 16804 | 22 |
| a86 | 3926 | 3955 | 30 | a236 | 10499 | 10529 | 31 | a386 | 16832 | 16907 | 76 |
| a87 | 3974 | 3993 | 20 | a237 | 10531 | 10546 | 16 | a387 | 16934 | 16965 | 32 |
| a88 | 3995 | 4042 | 48 | a238 | 10560 | 10580 | 21 | a388 | 16972 | 17035 | 64 |
| a89 | 4053 | 4073 | 21 | a239 | 10582 | 10596 | 15 | a389 | 17039 | 17069 | 31 |
| a90 | 4075 | 4123 | 49 | a240 | 10600 | 10621 | 22 | a390 | 17072 | 17109 | 38 |
| a91 | 4133 | 4157 | 25 | a241 | 10623 | 10664 | 42 | a391 | 17135 | 17150 | 16 |
| a92 | 4158 | 4188 | 31 | a242 | 10666 | 10685 | 20 | a392 | 17167 | 17209 | 43 |
| a93 | 4218 | 4250 | 33 | a243 | 10717 | 10773 | 57 | a393 | 17211 | 17242 | 32 |
| a94 | 4277 | 4336 | 60 | a244 | 10775 | 10792 | 18 | a394 | 17244 | 17299 | 56 |
| a95 | 4353 | 4375 | 23 | a245 | 10794 | 10858 | 65 | a395 | 17304 | 17344 | 41 |
| a96 | 4383 | 4398 | 16 | a246 | 10874 | 10888 | 15 | a396 | 17346 | 17400 | 55 |
| a97 | 4405 | 4446 | 42 | a247 | 10893 | 10972 | 80 | a397 | 17447 | 17466 | 20 |
| a98 | 4448 | 4464 | 17 | a248 | 10974 | 10994 | 21 | a398 | 17474 | 17539 | 66 |
| a99 | 4466 | 4493 | 28 | a249 | 10996 | 11012 | 17 | a399 | 17561 | 17604 | 44 |
| a100 | 4495 | 4558 | 64 | a250 | 11075 | 11097 | 23 | a400 | 17610 | 17663 | 54 |
| a101 | 4571 | 4613 | 43 | a251 | 11099 | 11124 | 26 | a401 | 17681 | 17763 | 83 |
| a102 | 4624 | 4683 | 60 | a252 | 11140 | 11157 | 18 | a402 | 17793 | 17810 | 18 |
| a103 | 4743 | 4759 | 17 | a253 | 11159 | 11192 | 34 | a403 | 17812 | 17852 | 41 |
| a104 | 4761 | 4785 | 25 | a254 | 11195 | 11226 | 32 | a404 | 17854 | 17928 | 75 |
| a105 | 4811 | 4858 | 48 | a255 | 11235 | 11261 | 27 | a405 | 17941 | 18005 | 65 |
| a106 | 4873 | 4932 | 60 | a256 | 11279 | 11337 | 59 | a406 | 18007 | 18035 | 29 |
| a107 | 4934 | 4948 | 15 | a257 | 11344 | 11381 | 38 | a407 | 18041 | 18077 | 37 |
| a108 | 4955 | 4974 | 20 | a258 | 11387 | 11411 | 25 | a408 | 18085 | 18146 | 62 |
| a109 | 4979 | 5010 | 32 | a259 | 11427 | 11494 | 68 | a409 | 18163 | 18177 | 15 |
| a110 | 5012 | 5052 | 41 | a260 | 11496 | 11510 | 15 | a410 | 18179 | 18207 | 29 |
| a111 | 5055 | 5115 | 61 | a261 | 11512 | 11526 | 15 | a411 | 18209 | 18228 | 20 |
| a112 | 5138 | 5166 | 29 | a262 | 11528 | 11551 | 24 | a412 | 18230 | 18266 | 37 |
| a113 | 5168 | 5198 | 31 | a263 | 11570 | 11592 | 23 | a413 | 18268 | 18285 | 18 |
| a114 | 5200 | 5222 | 23 | a264 | 11594 | 11634 | 41 | a414 | 18287 | 18351 | 65 |
| a115 | 5224 | 5284 | 61 | a265 | 11664 | 11684 | 21 | a415 | 18365 | 18395 | 31 |
| a116 | 5286 | 5302 | 17 | a266 | 11699 | 11719 | 21 | a416 | 18402 | 18432 | 31 |
| a117 | 5317 | 5332 | 16 | a267 | 11721 | 11746 | 26 | a417 | 18434 | 18456 | 23 |
| a118 | 5349 | 5436 | 88 | a268 | 11753 | 11771 | 19 | a418 | 18502 | 18530 | 29 |
| a119 | 5460 | 5512 | 53 | a269 | 11787 | 11873 | 87 | a419 | 18545 | 18590 | 46 |
| a120 | 5514 | 5534 | 21 | a270 | 11873 | 11905 | 33 | a420 | 18603 | 18621 | 19 |
| a121 | 5548 | 5563 | 16 | a271 | 11927 | 11942 | 16 | a421 | 18623 | 18645 | 23 |
| a122 | 5565 | 5579 | 15 | a272 | 11946 | 11973 | 28 | a422 | 18651 | 18708 | 58 |
| a123 | 5581 | 5597 | 17 | a273 | 11975 | 11993 | 19 | a423 | 18710 | 18729 | 20 |
| a124 | 5600 | 5639 | 40 | a274 | 12019 | 12114 | 96 | a424 | 18731 | 18758 | 28 |
| a125 | 5644 | 5661 | 18 | a275 | 12116 | 12135 | 20 | a425 | 18760 | 18788 | 29 |
| a126 | 5663 | 5735 | 73 | a276 | 12137 | 12158 | 22 | a426 | 18799 | 18859 | 61 |
| a127 | 5737 | 5770 | 34 | a277 | 12165 | 12192 | 28 | a427 | 18861 | 18926 | 66 |
| a128 | 5778 | 5801 | 24 | a278 | 12194 | 12216 | 23 | a428 | 18928 | 18980 | 53 |
| a129 | 5852 | 5958 | 107 | a279 | 12218 | 12246 | 29 | a429 | 19001 | 19018 | 18 |
| a130 | 6007 | 6041 | 35 | a280 | 12262 | 12277 | 16 | a430 | 19034 | 19054 | 21 |
| a131 | 6049 | 6063 | 15 | a281 | 12283 | 12319 | 37 | a431 | 19070 | 19092 | 23 |
| a132 | 6065 | 6084 | 20 | a282 | 12334 | 12368 | 35 | a432 | 19111 | 19154 | 44 |
| a133 | 6086 | 6101 | 16 | a283 | 12370 | 12395 | 26 | a433 | 19191 | 19213 | 23 |
| a134 | 6119 | 6186 | 68 | a284 | 12397 | 12434 | 38 | a434 | 19215 | 19240 | 26 |
| a135 | 6189 | 6234 | 46 | a285 | 12436 | 12509 | 74 | a435 | 19255 | 19356 | 102 |
| a136 | 6236 | 6278 | 43 | a286 | 12511 | 12543 | 33 | a436 | 19358 | 19446 | 89 |
| a137 | 6291 | 6312 | 22 | a287 | 12545 | 12565 | 21 | a437 | 19450 | 19468 | 19 |
| a138 | 6314 | 6373 | 60 | a288 | 12567 | 12675 | 109 | a438 | 19470 | 19512 | 43 |
| a139 | 6404 | 6447 | 44 | a289 | 12677 | 12706 | 30 | a439 | 19514 | 19541 | 28 |
| a140 | 6449 | 6482 | 34 | a290 | 12708 | 12724 | 17 | a440 | 19543 | 19568 | 26 |
| a141 | 6533 | 6555 | 23 | a291 | 12753 | 12768 | 16 | a441 | 19570 | 19586 | 17 |
| a142 | 6562 | 6622 | 61 | a292 | 12785 | 12809 | 25 | a442 | 19588 | 19619 | 32 |
| a143 | 6624 | 6674 | 51 | a293 | 12830 | 12859 | 30 | a443 | 19683 | 19739 | 57 |
| a144 | 6679 | 6762 | 84 | a294 | 12864 | 12885 | 22 | a444 | 19741 | 19777 | 37 |
| a145 | 6764 | 6780 | 17 | a295 | 12886 | 12916 | 31 | a445 | 19779 | 19820 | 42 |
| a146 | 6782 | 6822 | 41 | a296 | 12922 | 12946 | 25 | a446 | 19822 | 19836 | 15 |
| a147 | 6824 | 6856 | 33 | a297 | 12948 | 12970 | 23 | a447 | 19838 | 19911 | 74 |
| a148 | 6858 | 6898 | 41 | a298 | 12983 | 13003 | 21 | a448 | 19913 | 19966 | 54 |
| a149 | 6906 | 6954 | 49 | a299 | 13018 | 13051 | 34 | a449 | 19968 | 20026 | 59 |
| a150 | 6969 | 6992 | 24 | a300 | 13070 | 13090 | 21 | | | | |

As described herein the oligonucleotide or contiguous nucleotide sequence is complementary to a region of the target nucleic acid, wherein the target nucleic acid region is selected from the group consisting of a7, a26, a43, a119, a142, a159, a160, a163, a169, a178, a179, a180, a189, a201, a202, a204, a214, a221, a224, a226, a243, a254, a258, 269, a274, a350, a360, a364, a365, a370, a372, a381, a383, a386, a389, a400, a427, a435 and a438.

As described herein the oligonucleotide or contiguous nucleotide sequence is complementary to a region of the target nucleic acid, wherein the target nucleic acid region is selected from the group consisting of a160, a180, a221, a269 and a360.

As described herein, the oligonucleotide of the invention comprises or consists of 8 to 35 nucleotides in length, such as from 9 to 30, such as 10 to 22, such as from 11 to 20, such as from 12 to 18, such as from 13 to 17 or 14 to 16 contiguous nucleotides in length. As described herein, the oligonucleotide comprises or consists of 16 to 20 nucleotides in length. It is to be understood that any range given herein includes the range endpoints. Accordingly, if an oligonucleotide is said to include from 10 to 30 nucleotides, both 10 and 30 nucleotides are included.

As described herein, the contiguous nucleotide sequence comprises or consists of 8, 9, 10, 11, 12, 13, 14, 15, 16, 17, 18, 19, 20, 21, 22, 23, 24, 25, 26, 27, 28, 29 or 30 contiguous nucleotides in length. As described herein, the oligonucleotide comprises or consists of 16, 17, 18, 19 or 20 nucleotides in length.

As described herein, the oligonucleotide or contiguous nucleotide sequence comprises or consists of a sequence selected from the group consisting of sequences listed in table 5.

As described herein, the antisense oligonucleotide or contiguous nucleotide sequence comprises or consists of 10 to 30 nucleotides in length with at least 90% identity, preferably 100% identity, to a sequence selected from the group consisting of SEQ ID NO: 5 to 743 (see motif sequences listed in table 5).

As described herein, the antisense oligonucleotide or contiguous nucleotide sequence comprises or consists of 10 to 30 nucleotides in length with at least 90% identity, preferably 100% identity, to a sequence selected from the group consisting of SEQ ID NO: 5 to 743 and 771.

As described herein, the antisense oligonucleotide or contiguous nucleotide sequence comprises or consists of 10 to 30 nucleotides in length with at least 90% identity, preferably 100% identity, to a sequence selected from the group consisting of SEQ ID NO: 6, 8, 9, 13, 41, 42, 58, 77, 92, 111, 128, 151, 164, 166, 169, 171, 222, 233, 245, 246, 250, 251, 252, 256, 272, 273, 287, 292, 303, 314, 318, 320, 324, 336, 342, 343, 344, 345, 346, 349, 359, 360, 374, 408, 409, 415, 417, 424, 429, 430, 458, 464, 466, 474, 490, 493, 512, 519, 519, 529, 533, 534, 547, 566, 567, 578, 582, 601, 619, 620, 636, 637, 638, 640, 645, 650, 651, 652, 653, 658, 659, 660, 665, 678, 679, 680, 682, 683, 684, 687, 694, 706, 716, 728, 733, 734, and 735.

As described herein, the antisense oligonucleotide or contiguous nucleotide sequence comprises or consists of 10 to 30 nucleotides in length with at least 90% identity, preferably 100% identity, to SEQ ID NO: 287.

As described herein, the antisense oligonucleotide or contiguous nucleotide sequence comprises or consists of 10 to 30 nucleotides in length with at least 90% identity, preferably 100% identity, to SEQ ID NO: 342.

As described herein, the antisense oligonucleotide or contiguous nucleotide sequence comprises or consists of 10 to 30 nucleotides in length with at least 90% identity, preferably 100% identity, to SEQ ID NO: 640.

As described herein, the antisense oligonucleotide or contiguous nucleotide sequence comprises or consists of 10 to 30 nucleotides in length with at least 90% identity, preferably 100% identity, to SEQ ID NO: 466.

As described herein, the antisense oligonucleotide or contiguous nucleotide sequence comprises or consists of 10 to 30 nucleotides in length with at least 90% identity, preferably 100% identity, to SEQ ID NO: 566.

As described herein where the oligonucleotide is longer than the contigious nucleotide sequence (which is complementary to the target nucleic acid), the motif sequences in table 5 form the contigious nucleotide sequence part of the antisense oligonucleotides of the invention. As described herein the sequence of the oligonucleotide is equivalent to the contigious nucleotide sequence (e.g. if no biocleavable linkers are added).

It is understood that the contiguous nucleobase sequences (motif sequence) can be modified to for example increase nuclease resistance and/or binding affinity to the target nucleic acid. Modifications are described in the definitions and in the "Oligonucleotide design" section. Table 5 lists preferred designs of each motif sequence.

### Oligonucleotide design

Oligonucleotide design refers to the pattern of nucleoside sugar modifications in the oligonucleotide sequence. The oligonucleotides as described herein comprise sugar-modified nucleosides and may also comprise DNA or RNA nucleosides. In some embodiments, the oligonucleotide described herein comprises sugar-modified nucleosides and DNA nucleosides. Incorporation of modified nucleosides into the oligonucleotide described herein may enhance the affinity of the oligonucleotide for the target nucleic acid. In that case, the modified nucleosides can be referred to as affinity enhancing modified nucleotides, the modified nucleosides may also be termed units.

As described herein, the oligonucleotide comprises at least 1 modified nucleoside, such as at least 2, at least 3, at least 4, at least 5, at least 6, at least 7, at least 8, at least 9, at least 10, at least 11, at least 12, at least 13, at least 14, at least 15 or at least 16 modified nucleosides. As described herein the oligonucleotide comprises from 1 to 10 modified nucleosides, such as from 2 to 8 modified nucleosides, such as from 3 to 7 modified nucleosides, such as from 4 to 6 modified nucleosides, such as 3, 4, 5, 6 or 7 modified nucleosides.

As described herein, the oligonucleotide comprises one or more sugar modified nucleosides, such as 2' sugar modified nucleosides. Preferably the oligonucleotide described herein comprise the one or more 2' sugar modified nucleoside independently selected from the group consisting of 2'-O-alkyl-RNA, 2'-O-methyl-RNA, 2'-alkoxy-RNA, 2'-O-methoxyethyl-RNA, 2'-amino-DNA, 2'-fluoro-DNA, arabino nucleic acid (ANA), 2'-fluoro-ANA and LNA nucleosides. Even more preferably the one or more modified nucleoside is a locked nucleic acid (LNA).

As described herein the oligonucleotide comprises at least one modified internucleoside linkage. As described herein all the internucleoside linkages within the contiguous nucleotide sequence are phosphorothioate or boranophosphate internucleoside linkages. In some embodiments all the internucleotide linkages in the contiguous sequence of the oligonucleotide are phosphorothioate linkages.

As described herein, the oligonucleotide of the invention comprises at least one LNA nucleoside, such as 1, 2, 3, 4, 5, 6, 7, or 8 LNA nucleosides, such as from 2 to 6 LNA nucleosides, such as from 3 to 7 LNA nucleosides, 4 to 6 LNA nucleosides or 3, 4, 5, 6 or 7 LNA nucleosides. As described herein, at least 75% of the modified nucleosides in the oligonucleotide are LNA nucleosides, such as 80%, such as 85%, such as 90% of the modified nucleosides are LNA nucleosides. As described herein all the modified nucleosides in the oligonucleotide are LNA nucleosides. As described herein, the oligonucleotide may comprise both beta-D-oxy-LNA, and one or more of the following LNA nucleosides: thio-LNA, amino-LNA, oxy-LNA, and/or ENA in either the beta-D or alpha-L configurations or combinations thereof. In a further embodiment, all LNA cytosine units are 5-methyl-cytosine. As described herein the oligonucleotide or contiguous nucleotide sequence has at least 1 LNA nucleoside at the 5' end and at least 2 LNA nucleosides at the 3' end of the nucleotide sequence.

As described herein, the oligonucleotide of the invention comprises at least one modified nucleoside which is a 2'-MOE-RNA nucleoside, such as 2, 3, 4, 5, 6, 7, 8, 9 or 10 2'-MOE-RNA nucleosides. As described herein, at least one of said modified nucleoside is 2'-fluoro DNA, such as 2, 3, 4, 5, 6, 7, 8, 9 or 10 2'-fluoro-DNA nucleosides.

The oligonucleotide as described herein comprises at least one LNA nucleoside and at least one 2' substituted modified nucleoside.

As described herein, the oligonucleotide comprise both 2' sugar modified nucleosides and DNA units. Preferably the oligonucleotide comprises both LNA and DNA nucleosides (units). Preferably, the combined total of LNA and DNA units is 8-30, such as 10 - 25, preferably 12-22, such as 12 - 18, even more preferably 11-16. As described herein, the nucleotide sequence of the oligonucleotide, such as the contiguous nucleotide sequence consists of at least one or two LNA nucleosides and the remaining nucleosides are DNA units. As described herein the oligonucleotide comprises only LNA nucleosides and naturally occurring nucleosides (such as RNA or DNA, most preferably DNA nucleosides), optionally with modified internucleoside linkages such as phosphorothioate.

In an embodiment of the invention the oligonucleotide of the invention is capable of recruiting RNase H.

The structural design of the oligonucleotide as described herein may be selected from gapmers, gapbreakers, headmers and tailmers.

### Gapmer design

In a preferred embodiment the oligonucleotide of the invention has a gapmer design or structure also referred herein merely as "Gapmer". In a gapmer structure the oligonucleotide comprises at least three distinct structural regions a 5'-flank, a gap and a 3'-flank, F-G-F' in '5 -> 3' orientation. In this design, flanking regions F and F' (also termed wing regions) comprise a contiguous stretch of modified nucleosides, which are complementary to the PD-L1 target nucleic acid, while the gap region, G, comprises a contiguous stretch of nucleotides which are capable of recruiting a nuclease, preferably an endonuclease such as RNase, for example RNase H, when the oligonucleotide is in duplex with the target nucleic acid. Nucleosides which are capable of recruiting a nuclease, in particular RNase H, can be selected from the group consisting of DNA, alpha-L-oxy-LNA, 2'-Flouro-ANA and UNA. Regions F and F', flanking the 5' and 3' ends of region G, preferably comprise non-nuclease recruiting nucleosides (nucleosides with a 3' endo structure), more preferably one or more affinity enhancing modified nucleosides. As described herein, the 3' flank comprises at least one LNA nucleoside, preferably at least 2 LNA nucleosides. As described herein, the 5' flank comprises at least one LNA nucleoside. As described herein both the 5' and 3' flanking regions comprise a LNA nucleoside. As described herein all the nucleosides in the flanking regions are LNA nucleosides. As described herein the flanking regions may comprise both LNA nucleosides and other nucleosides (mixed flanks), such as DNA nucleosides and/or non-LNA modified nucleosides, such as 2' substituted nucleosides. In this case the gap is defined as a contiguous sequence of at least 5 RNase H recruiting nucleosides (nucleosides with a 2' endo structure, preferably DNA) flanked at the 5' and 3' end by an affinity enhancing modified nucleoside, preferably LNA, such as beta-D-oxy-LNA. Consequently, the nucleosides of the 5' flanking region and the 3' flanking region which are adjacent to the gap region are modified nucleosides, preferably non-nuclease recruiting nucleosides.

### Region F

Region F (5' flank or 5' wing) attached to the '5 end of region G comprises, contains or consists of at least one modified nucleoside such as at least 2, at least 3, at least 4, at least 5, at least 6, at least 7 modified nucleosides. As described herein region F comprises or consists of from 1 to 7 modified nucleosides, such as from 2 to 6 modified nucleosides, such as from 2 to 5 modified nucleosides, such as from 2 to 4 modified nucleosides, such as from 1 to 3 modified nucleosides, such as 1, 2, 3 or 4 modified nucleosides. The F region is defined by having at least on modified nucleoside at the 5' end and at the 3' end of the region.

As described herein, the modified nucleosides in region F have a 3' endo structure.

As described herein, one or more of the modified nucleosides in region F are 2' modified nucleosides. As described herein all the nucleosides in Region F are 2' modified nucleosides.

As described herein region F comprises DNA and/or RNA in addition to the 2' modified nucleosides. Flanks comprising DNA and/or RNA are characterized by having a 2' modified nucleoside in the 5' end and the 3' end (adjacent to the G region) of the F region. As described herein the region F comprise DNA nucleosides, such as from 1 to 3 contiguous DNA nucleosides, such as 1 to 3 or 1 to 2 contiguous DNA nucleosides. The DNA nucleosides in the flanks should preferably not be able to recruit RNase H. As described herein the 2' modified nucleosides and DNA and/or RNA nucleosides in the F region alternate with 1 to 3 2' modified nucleosides and 1 to 3 DNA and/or RNA nucleosides. Such flanks can also be termed alternating flanks. The length of the 5' flank (region F) in oligonucleotides with alternating flanks may be 4 to 10 nucleosides, such as 4 to 8, such as 4 to 6 nucleosides, such as 4, 5, 6 or 7 modified nucleosides. As described herein only the 5' flank of the oligonucleotide is alternating. Specific examples of region F with alternating nucleosides are

2'₁₋₃-N'₁₋₄-2'₁₋₃

2'₁₋₂-N'₁₋₂-2'₁₋₂-N'₁₋₂-2'₁₋₂

Where 2' indicates a modified nucleoside and N' is a RNA or DNA. As described herein all the modified nucleosides in the alternating flanks are LNA and the N' is DNA. In a further embodiment one or more of the 2' modified nucleosides in region F are selected from 2'-O-alkyl-RNA units, 2'-O-methyl-RNA, 2'-amino-DNA units, 2'-fluoro-DNA units, 2'-alkoxy-RNA, MOE units, LNA units, arabino nucleic acid (ANA) units and 2'-fluoro-ANA units.

As described herein, the F region comprises both LNA and a 2' substituted modified nucleoside. These are often termed mixed wing or mixed flank oligonucleotides.

As described herein all the modified nucleosides in region F are LNA nucleosides. In a further embodiment all the nucleosides in Region F are LNA nucleosides. As described herein the LNA nucleosides in region F are independently selected from the group consisting of oxy-LNA, thio-LNA, amino-LNA, cET, and/or ENA, in either the beta-D or alpha-L configurations or combinations thereof. As described herein region F comprise at least 1 beta-D-oxy LNA unit, at the 5' end of the contiguous sequence.

### Region G

Region G (gap region) preferably comprise, contain or consist of at least 4, such as at least 5, such as at least 6, at least 7, at least 8, at least 9, at least 10, at least 11, at least 12, at least 13, at least 14, at least 15 or at least 16 consecutive nucleosides capable of recruiting the aforementioned nuclease, in particular RNaseH. As described herein region G comprise, contain or consist of from 5 to 12, or from 6 to 10 or from 7 to 9, such as 8 consecutive nucleotide units capable of recruiting aforementioned nuclease.

The nucleoside units in region G, which are capable of recruiting nuclease are as described hereinselected from the group consisting of DNA, alpha-L-LNA, C4' alkylated DNA (as described in PCT/EP2009/050349 and Vester et al., Bioorg. Med. Chem. Lett. 18 (2008) 2296 - 2300), arabinose derived nucleosides like ANA and 2'F-ANA (Mangos et al. 2003 J. AM. CHEM. SOC. 125, 654-661), UNA (unlocked nucleic acid) (as described in Fluiter et al., Mol. Biosyst., 2009, 10, 1039). UNA is unlocked nucleic acid, typically where the bond between C2 and C3 of the ribose has been removed, forming an unlocked "sugar" residue.

As described herein at least one nucleoside unit in region G is a DNA nucleoside unit, such as from 1 to 18 DNA units, such as 2, 3, 4, 5, 6, 7, 8, 9, 10, 11,12, 13, 14, 15, 16 or 17 DNA units, preferably from 2 to 17 DNA units, such as from 3 to 16 DNA units, such as from 4 to 15 DNA units. such as from 5 to 14 DNA units, such as from 6 to 13 DNA units, such as from 7 to 12 DNA units, such as from 8 to 11 DNA units, more preferably from units 8 to 17 DNA units, or from 9 to 16 DNA units, 10 to 15 DNA units or 11 to 13 DNA units, such as 8, 9, 10, 11, 12, 13, 14, 154, 16, 17 DNA units. In some embodiments, region G consists of 100% DNA units.

As described herein the region G may consist of a mixture of DNA and other nucleosides capable of mediating RNase H cleavage. Region G may consist of at least 50% DNA, more preferably 60 %, 70% or 80 % DNA, and even more preferred 90% or 95% DNA.

As described herein at least one nucleoside unit in region G is an alpha-L-LNA nucleoside unit, such as at least one alpha-L-LNA, such as 2, 3, 4, 5, 6, 7, 8 or 9 alpha-L-LNA. As described herein, region G comprises the least one alpha-L-LNA is alpha-L-oxy-LNA. As described herein region G comprises a combination of DNA and alpha-L-LNA nucleoside units.

As described herein, nucleosides in region G have a 2' endo structure.

As described herein region G may comprise a gapbreaker nucleoside, leading to a gapbreaker oligonucleotide, which is capable of recruiting RNase H.

### Region F'

Region F' (3' flank or 3' wing) attached to the '3 end of region G comprises, contains or consists of at least one modified nucleoside such as at least 2, at least 3, at least 4, at least 5, at least 6, at least 7 modified nucleosides. As described herein F' comprise or consist of from 1 to 7 modified nucleosides, such as from 2 to 6 modified nucleoside, such as from 2 to 4 modified nucleosides, such as from 1 to 3 modified nucleosides, such as 1, 2, 3 or 4 modified nucleosides. The F' region is defined by having at least on modified nucleoside at the 5' end and at the 3' end of the region.

As described herein, the modified nucleosides in region F' have a 3' endo structure.

As described herein, one or more of the modified nucleosides in region F' are 2' modified nucleosides. As described herein all the nucleosides in Region F' are 2' modified nucleosides.

As described herein, one or more of the modified nucleosides in region F' are 2' modified nucleosides.

As described herein all the nucleosides in Region F' are 2' modified nucleosides. As described herein region F' comprises DNA or RNA in addition to the 2' modified nucleosides. Flanks comprising DNA or RNA are characterized by having a 2' modified nucleoside in the 5' end (adjacent to the G region) and the 3' end of the F' region. As described herein the region F' comprises DNA nucleosides, such as from 1 to 4 contiguous DNA nucleosides, such as 1 to 3 or 1 to 2 contiguous DNA nucleosides. The DNA nucleosides in the flanks should preferably not be able to recruit RNase H. In some embodiments the 2' modified nucleosides and DNA and/or RNA nucleosides in the F' region alternate with 1 to 3 2' modified nucleosides and 1 to 3 DNA and/or RNA nucleosides, such flanks can also be termed alternating flanks. The length of the 3' flank (region F') in oligonucleotides with alternating flanks may be 4 to 10 nucleosides, such as 4 to 8, such as 4 to 6 nucleosides, such as 4, 5, 6 or 7 modified nucleosides. As described herein only the 3' flank of the oligonucleotide is alternating. Specific examples of region F' with alternating nucleosides are

2'₁₋₂-N'₁₋₄-2'₁₋₄

2'₁₋₂-N'₁₋₂-2'₁₋₂-N'₁₋₂-2'₁₋₂

Where 2' indicates a modified nucleoside and N' is a RNA or DNA. As described herein all the modified nucleosides in the alternating flanks are LNA and the N' is DNA. As described herein modified nucleosides in region F' are selected from 2'-O-alkyl-RNA units, 2'-O-methyl-RNA, 2'-amino-DNA units, 2'-fluoro-DNA units, 2'-alkoxy-RNA, MOE units, LNA units, arabino nucleic acid (ANA) units and 2'-fluoro-ANA units.

As described herein the F' region comprises both LNA and a 2' substituted modified nucleoside. These are often termed mixed wing or mixed flank oligonucleotides.

As described herein all the modified nucleosides in region F' are LNA nucleosides. As described herein all the nucleosides in Region F' are LNA nucleosides. As described herein the LNA nucleosides in region F' are independently selected from the group consisting of oxy-LNA, thio-LNA, amino-LNA, cET and/or ENA, in either the beta-D or alpha-L configurations or combinations thereof. As described herein region F' has at least 2 beta-D-oxy LNA unit, at the 3' end of the contiguous sequence.

*Region D' and D"*
Region D' and D" can be attached to the 5' end of region F or the 3' end of region F', respectively. Region D' or D" are optional.

Region D' or D" may independently comprise 0 to 5, such as 1 to 5, such as 2 to 4, such as 0, 1, 2, 3, 4 or 5 additional nucleotides, which may be complementary or non-complementary to the target nucleic acid. In this respect the oligonucleotide of the invention, may in some embodiments comprise a contiguous nucleotide sequence capable of modulating the target which is flanked at the 5' and/or 3' end by additional nucleotides. Such additional nucleotides may serve as a nuclease susceptible biocleavable linker (see definition of linkers). In some embodiments the additional 5' and/or 3' end nucleosides are linked with phosphodiester linkages, and may be DNA or RNA. In another embodiment, the additional 5' and/or 3' end nucleosides are modified nucleosides which may for example be included to enhance nuclease stability or for ease of synthesis. In one embodiment, the oligonucleotide of the invention, comprises a region D' and/or D" at the 5' or 3' end of the contiguous nucleotide sequence. In a further embodiment the D' and/or D" region is composed of 1 to 5 phosphodiester linked DNA or RNA nucleosides which are not complementary to the target nucleic acid.

The gapmer oligonucleotide of the present invention can be represented by the following formulae:
5'-F-G-F'-3'; in particular F₁₋₇-G₄₋₁₂-F'₁₋₇
5'-D'-F-G-F'-3', in particular D'₁₋₃-F₁₋₇-G₄₋₁₂-F'₁₋₇
5'-F-G-F'-D"-3', in particular F₁₋₇-G₄₋₁₂-F'₁₋₇-D"₁₋₃
5'-D'-F-G-F'-D'-3", in particular D'₁₋₃-F₁₋₇-G₄₋₁₂-F'₁₋₇-D"₁₋₃

The preferred number and types of nucleosides in regions F, G and F', D' and D" have been described above. The oligonucleotide conjugates of the present invention have a region C covalently attached to either the 5' or 3' end of the oligonucleotide, in particular the gapmer oligonucleotides presented above.

As described herein the oligonucleotide conjugate of the invention comprises a oligonucleotide with the formula 5'-D'-F-G-F'-3' or 5'-F-G-F'-D"-3', where region F and F' independently comprise 1 - 7 modified nucleosides, G is a region between 6 and 16 nucleosides which are capable of recruiting RNaseH and region D' or D" comprise 1 - 5 phosphodiester linked nucleosides. Preferably region D' or D" is present in the end of the oligonucleotide where conjugation to a conjugate moiety is contemplated.

Examples of oligonucleotides with alternating flanks can be represented by the following formulae:

2'₁₋₃-N'₁₋₄-2'₁₋₃-G₆₋₁₂-2'₁₋₂-N'₁₋₄-2'₁₋₄

2'₁₋₂-N'₁₋₂-2'₁₋₂-N'₁₋₂-2'₁₋₂-G₆₋₁₂-2'₁₋₂-N'₁₋₂-2'₁₋₂-N'₁₋₂-2'₁₋₂

F-G₆₋₁₂-2'₁₋₂-N'₁₋₄-2'₁₋₄

F-G₆₋₁₂-2'₁₋₂-N'₁₋₂-2'₁₋₂-N'₁₋₂-2'₁₋₂

2'₁₋₃-N'₁₋₄-2'₁₋₃-G₆₋₁₂-F'

2'₁₋₂-N'₁₋₂-2'₁₋₂-N₁₋₂-2'₁₋₂-G₆₋₁₂-F'

Where a flank is indicated by F or F' it only contains 2' modified nucleosides, such as LNA nucleosides. The preferred number and types of nucleosides in the alternating regions, and region F, G and F', D' and D" have been described above.

As described herein the oligonucleotide is a gapmer consisting of 16, 17, 18, 19, 20, 21, 22 nucleotides in length, wherein each of regions F and F' independently consists of 1, 2, 3 or 4 modified nucleoside units complementary to the PD-L1 target nucleic acid and region G consists of 8, 9, 10, 11,12,13,14,15,16,17 nucleoside units, capable of recruiting nuclease when in duplex with the PD-L1 target nucleic acid and region D' consists of 2 phosphodiester linked DNAs.

As described herein, the oligonucleotide is a gapmer wherein each of regions F and F' independently consists of 3, 4, 5 or 6 modified nucleoside units, such as nucleoside units containing a 2'-O-methoxyethyl-ribose sugar (2'-MOE) or nucleoside units containing a 2'-fluoro-deoxyribose sugar and/or LNA units, and region G consists of 8, 9, 10, 11, 12, 13, 14, 15, 16 or 17 nucleoside units, such as DNA units or other nuclease recruiting nucleosides such as alpha-L-LNA or a mixture of DNA and nuclease recruiting nucleosides.

As described herein, the oligonucleotide is a gapmer wherein each of regions F and F' region consists of two LNA units each, and region G consists of 12, 13, 14 nucleoside units, preferably DNA units. Specific gapmer designs of this nature include 2-12-2, 2-13-2 and 2-14-2.

As described herein, the oligonucleotide is a gapmer wherein each of regions F and F' independently consists of three LNA units, and region G consists of 8, 9, 10, 11, 12, 13 or 14 nucleoside units, preferably DNA units. Specific gapmer designs of this nature include 3-8-3, 3-9-3 3-10-3, 3-11-3, 3-12-3, 3-13-3 and 3-14-3.

As described herein, the oligonucleotide is a gapmer wherein each of regions F and F' consists of four LNA units each, and region G consists of 8 or 9, 10, 11 or 12 nucleoside units, preferably DNA units. Specific gapmer designs of this nature include 4-8-4, 4-9-4, 4-10-4, 4-11-4 and 4-12-4.

Specific gapmer designs of this nature include F-G-F' designs selected from a group consisting of a gap with 6 nucleosides and independently 1 to 4 modified nucleosides in the wings including 1-6-1, 1-6-2, 2-6-1, 1-6-3, 3-6-1, 1-6-4, 4-6-1, 2-6-2, 2-6-3, 3-6-2 2-6-4, 4-6-2, 3-6-3, 3-6-4 and 4-6-3 gapmers.

Specific gapmer designs of this nature include F-G-F' designs selected from a group consisting of a gap with 7 nucleosides and independently 1 to 4 modified nucleosides in the wings including 1-7-1, 2-7-1, 1-7-2, 1-7-3, 3-7-1, 1-7-4, 4-7-1, 2-7-2, 2-7-3, 3-7-2, 2-7-4, 4-7-2, 3-7-3, 3-7-4, 4-7-3 and 4-7-4 gapmers.

Specific gapmer designs of this nature include F-G-F' designs selected from a group consisting of a gap with 8 nucleosides and independently 1 to 4 modified nucleosides in the wings including 1-8-1, 1-8-2, 1-8-3, 3-8-1, 1-8-4, 4-8-1,2-8-1, 2-8-2, 2-8-3, 3-8-2, 2-8-4, , 4-8-2, 3-8-3, 3-8-4, 4-8-3 and 4-8-4 gapmers.

Specific gapmer designs of this nature include F-G-F' designs selected from a group consisting of a gap with 9 nucleosides and independently 1 to 4 modified nucleosides in the wings including, 1-9-1, 2-9-1, 1-9-2, 1-9-3, 3-9-1, 1-9-4, 4-9-1, 2-9-2, 2-9-3, 3-9-2, 2-9-4, 4-9-2, 3-9-3, 3-9-4, 4-9-3 and 4-9-4 gapmers.

Specific gapmer designs of this nature include F-G-F' designs selected from a group consisting of a gap with 10 nucleosides including, 1-10-1, 2-10-1, 1-10-2, 1-10-3, 3-10-1, 1-10-4, 4-10-1, 2-10-2, 2-10-3, 3-10-2, 2-10-4, 4-10-2, 3-10-3, 3-10-4, 4-10-3 and 4-10-4 gapmers.

Specific gapmer designs of this nature include F-G-F' designs selected from a group consisting of a gap with 11 nucleosides including, 1-11-1, 2-11-1, 1-11-2, 1-11-3, 3-11-1, 1-11-4, 4-11-1, 2-11-2, 2-11-3, 3-11-2, 2-11-4, 4-11-2, 3-11-3, 3-11-4, 4-11-3 and 4-11-4 gapmers.

Specific gapmer designs of this nature include F-G-F' designs selected from a group consisting of a gap with 12 nucleosides including, 1-12-1, 2-12-1, 1-12-2, 1-12-3, 3-12-1, 1-12-4, 4-12-1, 2-12-2, 2-12-3, 3-12-2, 2-12-4, 4-12-2, 3-12-3, 3-12-4, 4-12-3 and 4-12-4 gapmers.

Specific gapmer designs of this nature include F-G-F' designs selected from a group consisting of a gap with 13 nucleosides including, 1-13-1, 2-13-1, 1-13-2, 1-13-3, 3-13-1, 1-13-4, 4-13-1, 2-13-2, 2-13-3, 3-13-2, 2-13-4, 4-13-2, 3-13-3, 3-13-4, 4-13-3 and 4-13-4 gapmers.

Specific gapmer designs of this nature include F-G-F' designs selected from a group consisting of a gap with 14 nucleosides including, 1-14-1, 2-14-1, 1-14-2, 1-14-3, 3-14-1, 1-14-4, 4-14-1, 2-14-2, 2-14-3, 3-14-2, 2-14-4, 4-14-2, 3-14-3, 3-14-4, 4-14-3 and 4-14-4 gapmers.

Specific gapmer designs of this nature include F-G-F' designs selected from a group consisting of a gap with 15 nucleosides including, 1-15-1, 2-15-1, 1-15-2, 1-15-3, 3-15-1, 1-15-4, 4-15-1, 2-15-2, 2-15-3, 3-15-2, 2-15-4, 4-15-2 and 3-15-3 gapmers.

Specific gapmer designs of this nature include F-G-F' designs selected from a group consisting of a gap with 16 nucleosides including, 1-16-1, 2-16-1, 1-16-2, 1-16-3, 3-16-1, 1-16-4, 4-16-1, 2-16-2, 2-16-3, 3-16-2, 2-16-4, 4-16-2 and 3-16-3 gapmers.

Specific gapmer designs of this nature include F-G-F' designs selected from a group consisting of a gap with 17 nucleosides including, 1-17-1, 2-17-1, 1-17-2, 1-17-3, 3-17-1, 1-17-4, 4-17-1, 2-17-2, 2-17-3 and 3-17-2 gapmers.

In all instances the F-G-F' design may further include region D' and/or D", which may have 1, 2 or 3 nucleoside units, such as DNA units, such as 2 phosphodiester linked DNA units. Preferably, the nucleosides in region F and F' are modified nucleosides, while nucleotides in region G are preferably unmodified nucleosides.

In each design, the preferred modified nucleoside is LNA.

As described herein all the internucleoside linkages in the gap in a gapmer are phosphorothioate and/or boranophosphate linkages. As described herein all the internucleoside linkages in the flanks (F and F' region) in a gapmer are phosphorothioate and/or boranophosphate linkages. As described herein all the internucleoside linkages in the D' and D" region in a gapmer are phosphodiester linkages.

For specific gapmers as disclosed herein, when the cytosine (C) residues are annotated as 5-methyl-cytosine, as described herein, one or more of the Cs present in the oligonucleotide may be unmodified C residues.

As described herein, the gapmer is a so-called shortmer as described in WO2008/113832.

Further gapmer designs are disclosed in WO2004/046160, WO2007/146511.

As described herein, the oligonucleotide is selected from the group of oligonucleotide compounds with CMP-ID-NO: 5_1 to 743_1 and 771_1.

As described herein, the oligonucleotide is selected from the group of oligonucleotide compounds with CMP-ID-NO 6_1, 8_1, 9_1, 13_1, 41_1, 42_1, 58_1, 77_1, 92_1, 111_1, 128_1, 151_1, 164_1, 166_1, 169_1, 171_1, 222_1, 233_1, 245_1, 246_1, 250_1, 251_1, 252_1, 256_1, 272_1, 273_1, 287_1, 292_1, 303_1, 314_1, 318_1, 320_1, 324_1, 336_1, 342_1, 343_1, 344_1, 345_1, 346_1, 349_1, 359_1, 360_1, 374_1, 408_1, 409_1, 415_1, 417_1, 424_1, 429_1, 430_1, 458_1, 464_1, 466_1, 474_1, 490_1, 493_1, 512_1, 519_1, 519_1, 529_1, 533_1, 534_1, 547_1, 566_1, 567_1, 578_1, 582_1, 601_1, 619_1, 620_1, 636_1, 637_1, 638_1, 640_1, 645_1, 650_1, 651_1, 652_1, 653_1, 658_1, 659_1, 660_1, 665_1, 678_1, 679_1, 680_1, 682_1, 683_1, 684_1, 687_1, 694_1, 706_1, 716_1, 728_1, 733_1, 734_1, and 735_1.

As described herein, the oligonucleotide is CMP-ID-NO: 287_1.

As described herein, the oligonucleotide is CMP-ID-NO: 342_1.

In another preferred embodiment of the invention, the oligonucleotide is CMP-ID-NO: 640_1.

As described herein, the oligonucleotide is CMP-ID-NO: 466_1.

As described herein, the oligonucleotide is CMP-ID-NO: 566_1.

In a further embodiment of the invention the contiguous nucleotide sequence of the oligonucleotide motifs and oligonucleotide compounds of the invention comprise two to four additional phosphodiester linked nucleosides at the 5' end of the contiguous nucleotide sequence (e.g. region D'). In one embodiment the nucleosides serve as a biocleavable linker (see sectionon biocleavable linkers). In a preferred embodiment a ca (cytidine-adenosine) dinucleotide is linked to the 5' end of contiguous nucleotide sequence (i.e. any one of the motif sequences or oligonucleotide compounds listed in table 5) via a phosphodiester linkage. In a preferred emboduiment the ca di nucleotide is not complementary to the target sequence at the position where the reminder of the contigious nucleotide is complementary.

As described herein the oligonucleotide or contiguous nucleotide sequence is selected from the group consisting of the nucleotide motif sequences with SEQ ID NO: 766, 767, 768, 769 and 770.

As described herein the oligonucleotide is selected from the group consisting of the oligonucleotide compounds with CMP-ID-NO 766_1, 767_1, 768_1, 769_1 and 770_1.

### Carbohydrate conjugate moieties

Carbohydrate conjugate moieties include galactose, lactose, n-acetylgalactosamine, mannose and mannose-6-phosphate. Carbohydrate conjugates may be used to enhance delivery or activity in a range of tissues, such as liver and/or muscle. See, for example, EP1495769, WO99/65925, Yang et al., Bioconjug Chem (2009) 20(2): 213-21. Zatsepin & Oretskaya Chem Biodivers. (2004) 1(10): 1401-17.

In some embodiments the carbohydrate conjugate moiety is multivalent, such as, for example 2, 3 or 4 identical or non-identical carbohydrate moieties may be covalently joined to the oligonucleotide, optionally via a linker or linkers. In some embodiments the invention provides a conjugate comprising the oligonucleotide of the invention and a carbohydrate conjugate moiety. In some embodiments, the conjugate moiety is or may comprise mannose or mannose-6-phosphate. This is particular useful for targeting muscle cells, see for example US 2012/122801.

Conjugate moieties capable of binding to the asialoglycoprotein receptor (ASGPr) are particular useful for targeting hepatocytes in liver. In some embodiments the invention provides a oligonucleotide conjugate comprising the oligonucleotide of the invention and an asialoglycoprotein receptor targeting conjugate moiety. The asialoglycoprotein receptor targeting conjugate moiety comprises one or more carbohydrate moieties capable of binding to the asialoglycoprotein receptor (ASPGr binding carbohydrate moieties) with affinity equal to or greater than that of galactose. The affinities of numerous galactose derivatives for the asialoglycoprotein receptor have been studied (see for example: Jobst, S.T. and Drickamer, K. JB.C. 1996, 271, 6686) or are readily determined using methods typical in the art.

Described herein is an antisense oligonucleotide conjugate comprising a) an oligonucleotide (Region A) comprising a contiguous nucleotide sequence of 10 to 30 nucleotides in length with at least 90% complementarity to a PD-L1 target nucleic acid; and b) at least one asialoglycoprotein receptor targeting conjugate moiety (Region C) covalently attached to the oligonucleotide in a). The oligonucleotide or a contiguous nucleotide sequence can be as described in any of the sections "oligonucleotides of the invention", "oligonucleotide design and "gapmer design".

In some embodiments asialoglycoprotein receptor targeting conjugate moiety comprises at least one ASPGr binding carbohydrate moiety selected from the group consisting of galactose, galactosamine, N-formyl-galactosamine, N-acetylgalactosamine, N-propionyl-galactosamine, N-n-butanoyl-galactosamine and N-isobutanoylgalactosamine. In some embodiments, the asialoglycoprotein receptor targeting conjugate moiety is mono-valent, di-valent, tri-valent or tetra-valent (i.e. containing 1, 2, 3 or 4 terminal carbohydrate moieties capable of binding to the asialoglycoprotein receptor). Preferably, the asialoglycoprotein receptor targeting conjugate moiety is di-valent, even more preferred it is trivalent. In a preferred embodiment the asialoglycoprotein receptor targeting conjugate moiety comprises 1 to 3 N-acetylgalactosamine (GalNAc) moieties (also termed a GalNAc conjugate). In some embodiments the oligonucleotide conjugate comprises a asialoglycoprotein receptor targeting conjugate moiety that is a tri-valent N-acetylgalactosamine (GalNAc) moiety. GalNAc conjugates have been used with phosphodiester, methylphosphonate and PNA antisense oligonucleotides (e.g. US 5,994517 and Hangeland et al., Bioconjug Chem. 1995 Nov-Dec;6(6):695-701, Biessen et al 1999 Biochem J. 340, 783-792 and Maier et al 2003 Bioconjug Chem 14, 18-29) and siRNAs (e.g. WO 2009/126933, WO 2012/089352 & WO 2012/083046) and with LNA and 2'-MOE modified nucleosides WO 2014/076196 WO 2014/207232 and WO 2014/179620.

To generate the asialoglycoprotein receptor targeting conjugate moiety the ASPGr binding carbohydrate moieties (preferably GalNAc) are attached to a brancher molecule through the C-I carbons of the saccharides. The ASPGr binding carbohydrate moieties are preferably linked to the brancher molecule via spacers. A preferred spacer is a flexible hydrophilic spacer (U.S. Patent 5885968; Biessen et al. J. Med. Chern. 1995 Vol. 39 p. 1538-1546). A preferred flexible hydrophilic spacer is a PEG spacer. A preferred PEG spacer is a PEG3 spacer (three ethylene units). The brancher molecule can be any small molecule which permits attachment of two or three terminal ASPGr binding carbohydrate moieties and further permits attachment of the branch point to the oligonucleotide. An exemplary brancher molecule is a di-lysine. A di-lysine molecule contains three amine groups through which three ASPGr binding carbohydrate moieties may be attached and a carboxyl reactive group through which the di-lysine may be attached to the oligonucleotide. Alternative brancher molecules may be a doubler or trebler such as those supplied by Glen Research. In some embodiments the brancher may be selected from the from the group consisting of 1,3-bis-[5-(4,4'-dimethoxytrityloxy)pentylamido]propyl-2-[(2-cyanoethyl)-(N,N-diisopropyl)] phosphoramidite (Glen Research Catalogue Number: 10-1920-xx), tris-2,2,2-[3-(4,4'-dimethoxytrityloxy)propyloxymethyl]ethyl-[(2-cyanoethyl)-(N,N-diisopropyl)]-phosphoramidite (Glen Research Catalogue Number: 10-1922-xx), tris-2,2,2-[3-(4,4'-dimethoxytrityloxy)propyloxymethyl]methyleneoxypropyl-[(2-cyanoethyl)-(N,N-diisopropyl)]-phosphoramidite and 1-[5-(4,4'-dimethoxy-trityloxy)pentylamido]-3-[5-fluorenomethoxy-carbonyloxy-pentylamido]-propyl-2-[(2-cyanoethyl)-(N,N-diisopropyl)]-phosphoramidite (Glen Research Catalogue Number: 10-1925-xx). WO 2014/179620 and PCT application No. PCT/EP2015/073331 describes the generation of various GalNAc conjugate moieties. One or more linkers may be inserted between the brancher molecule and the oligonucleotide. In a preferred embodiment the linker is a biocleavable linker. The linker may be selected from the linkers described in the section "Linkers" and its subsections.

The asialoglycoprotein receptor targeting conjugate moiety, in particular the GalNAc conjugate moiety, may be attached to the 3'- or 5'-end of the oligonucleotide using methods known in the art. In preferred embodiments the asialoglycoprotein receptor targeting conjugate moiety is linked to the 5'-end of the oligonucleotide.

Pharmacokinetic modulators in relation to siRNAs delivery has been described in WO 2012/083046. In some embodiments the carbohydrate conjugate moiety comprises a pharmacokinetic modulator selected from the group consisting of a hydrophobic group having 16 or more carbon atoms, hydrophobic group having 16-20 carbon atoms, palmitoyl, hexadec-8-enoyl, oleyl, (9E,12E)-octadeca-9,12dienoyl, dioctanoyl, and C16-C20 acyl, and cholesterol. In a preferred embodiment the pharmacokinetic modulator containing carbohydrate conjugate moiety is a GalNAc conjugate.

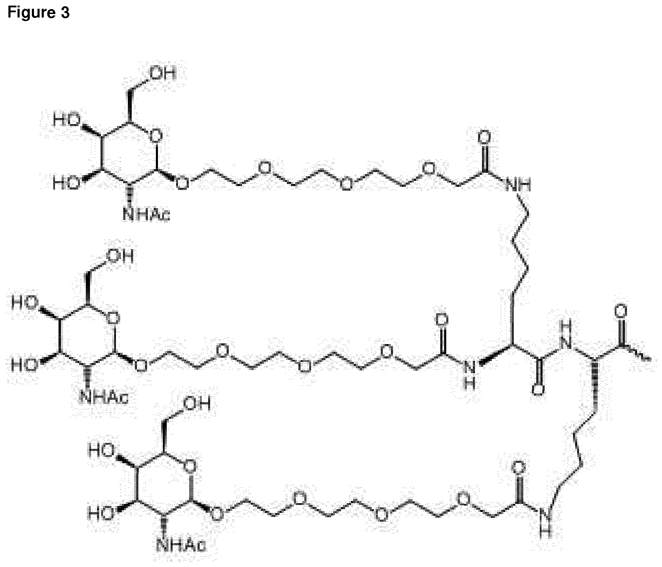

Preferred carbohydrate conjugate moieties comprises one to three terminal ASPGr binding carbohydrate moieties, preferably N-acetylgalactosamine moiety(s). In some embodiments the carbohydrate conjugate moiety comprises three ASPGr binding carbohydrate moieties, preferably N-acetylgalactosamine moieties, linked via a spacer to a brancher molecule. The spacer molecule can be between 8 and 30 atoms long. A preferred carbohydrate conjugate moiety comprises three terminal GalNAc moieties linked via a PEG spacer to a di-lysine brancher molecule. Preferably the PEG spacer is a 3PEG spacer. Suitable asialoglycoprotein receptor targeting conjugate moieties are shown in Figure 1. A preferred asialoglycoprotein receptor targeting conjugate moiety is shown in figure 3.

Other GalNAc conjugate moieties can include, for example, small peptides with GalNAc moieties attached such as Tyr-Glu-Glu-(aminohexyl GalNAc)3 (YEE(ahGalNAc)3; a glycotripeptide that binds to asialoglycoprotein receptor on hepatocytes, see, e.g., Duff, et al., Methods Enzymol, 2000, 313, 297); lysine-based galactose clusters (e.g., L3G4; Biessen, et al., Cardovasc. Med., 1999, 214); and cholane-based galactose clusters (e.g., carbohydrate recognition motif for asialoglycoprotein receptor).

As described herein the antisense oligonucleotide conjugate is selected from the group consisting of the following CPM ID NO: 766_2, 767 2, 768_2, 769_2 and 770_2.

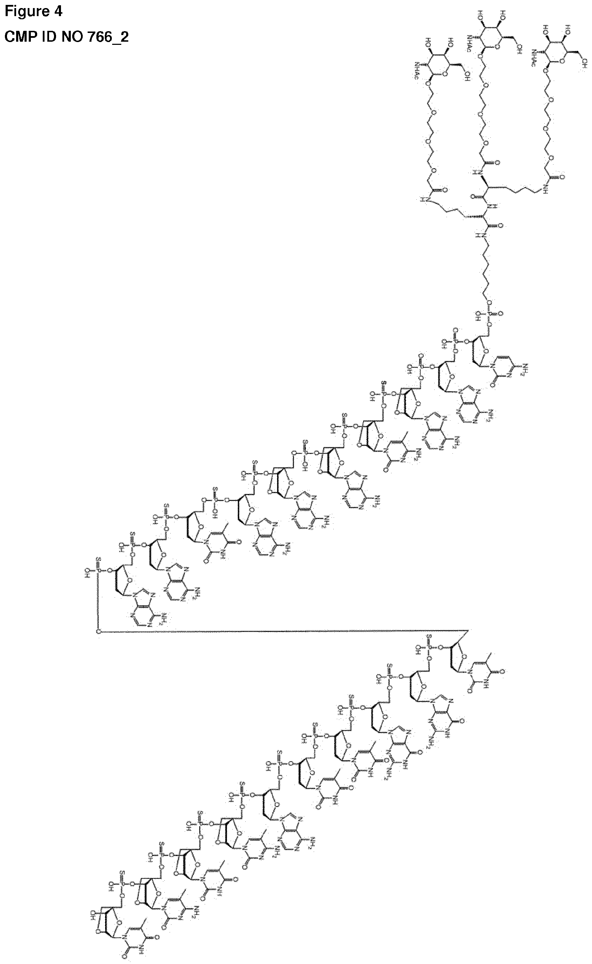

As described herein the antisense oligonucleotide conjugate corresponds to the compound represented in figure 4.

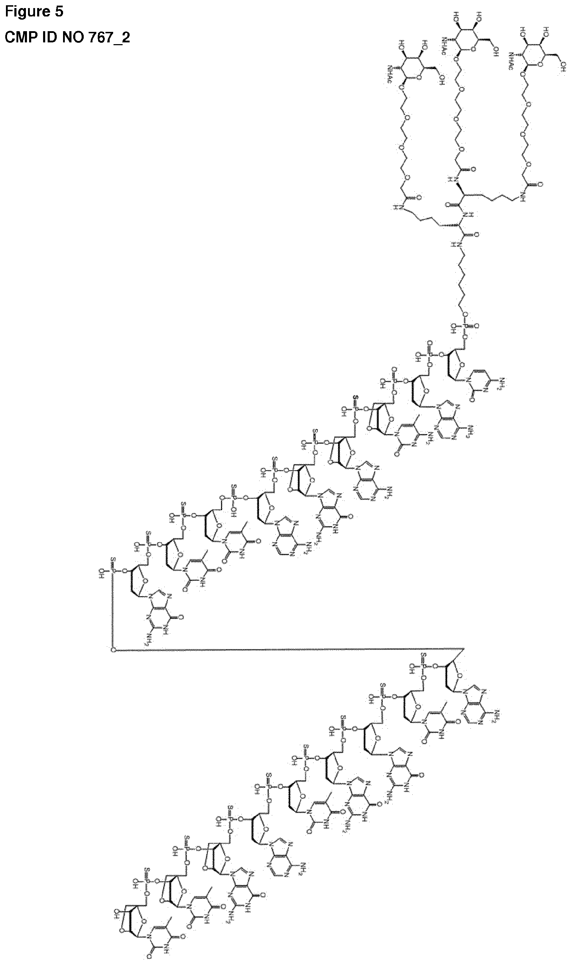

As described herein the antisense oligonucleotide conjugate corresponds to the compound represented in figure 5.

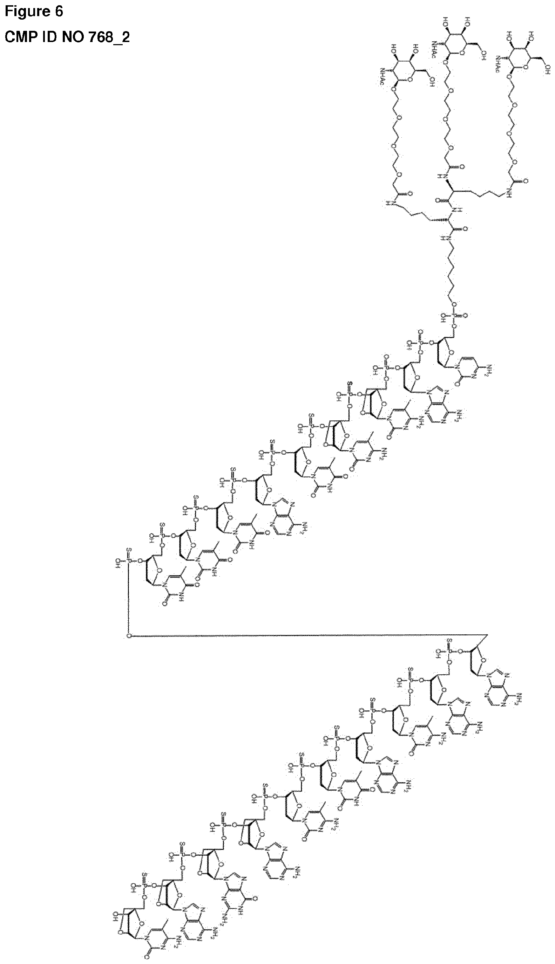

In another preferred embodiment the antisense oligonucleotide conjugate corresponds to the compound represented in figure 6.

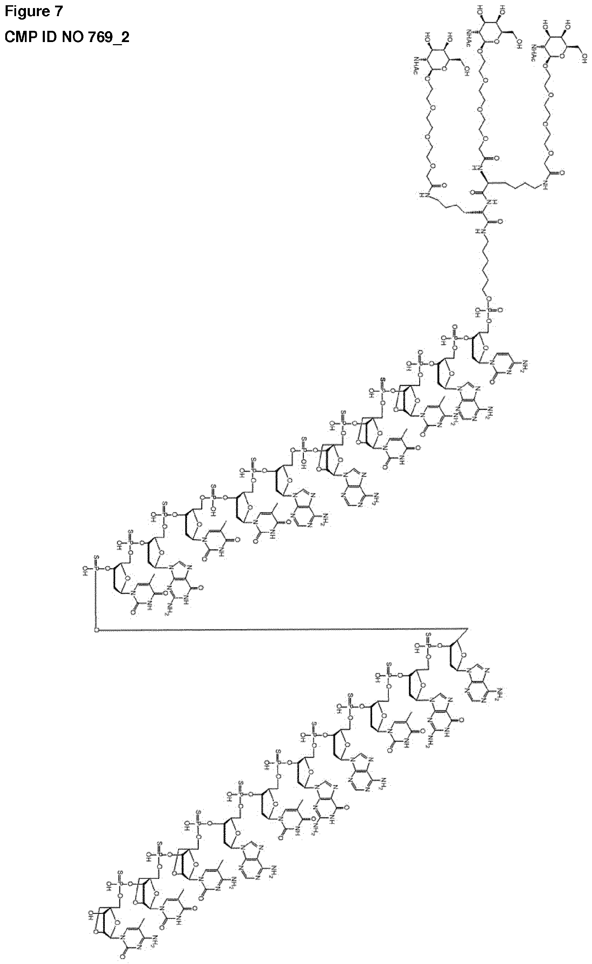

As described herein the antisense oligonucleotide conjugate corresponds to the compound represented in figure 7.

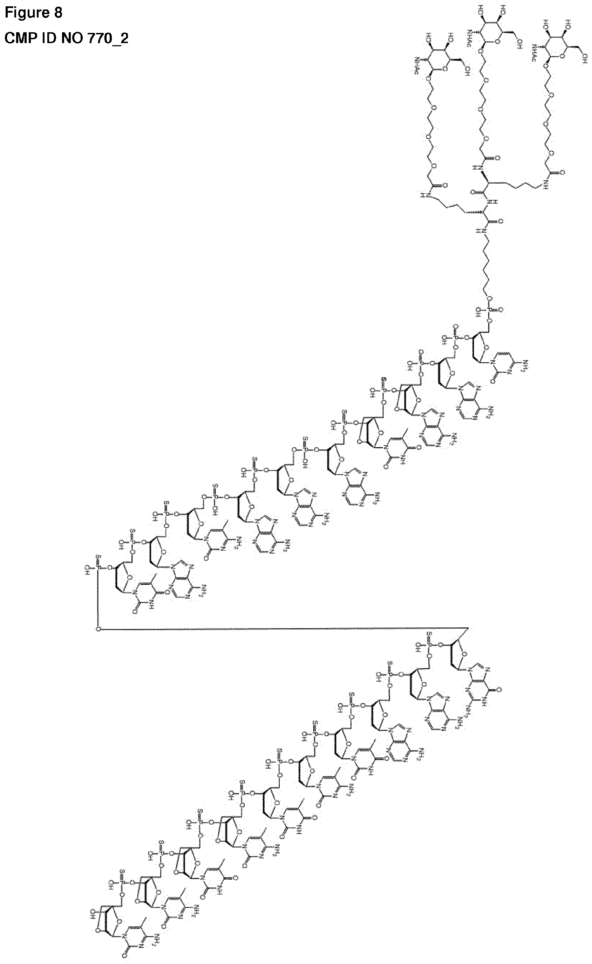

As described herein the antisense oligonucleotide conjugate corresponds to the compound represented in figure 8.

### Linkers

### Biocleavable linkers (Region B)

The use of a conjugate is often associated with enhanced pharmacokinetic or pharmeodynamic dynamic properties. However, the presence of a conjugate moiety may interfere with the activity of the oligonucleotide against its intended target, for example via steric hindrance preventing hybridization or nuclease recruitment (e.g. RNAseH). The use of a physiologically labile bond (biocleavable linker) between the oligonucleotide (region A or first region) and the conjugate moiety (region C or third region), allows for the improved properties due to the presence of the conjugate moiety, whilst ensuring that once at the target tissue, the conjugate group does not prevent effective activity of the oligonucleotide.

Cleavage of the physiologically labile bond occurs spontaneously when a molecule containing the labile bond reaches an appropriate intra-and/or extra-cellular environment. For example, a pH labile bond may be cleaved when the molecule enters an acidified endosome. Thus, a pH labile bond may be considered to be an endosomal cleavable bond. Enzyme cleavable bonds may be cleaved when exposed to enzymes such as those present in an endosome or lysosome or in the cytoplasm. A disulfide bond may be cleaved when the molecule enters the more reducing environment of the cell cytoplasm. Thus, a disulfide may be considered to be a cytoplasmic cleavable bond. As used herein, a pH-labile bond is a labile bond that is selectively broken under acidic conditions (pH<7). Such bonds may also be termed endosomally labile bonds, since cell endosomes and lysosomes have a pH less than 7.

For biocleavable linkers associated with a conjugate moiety for targeted delivery it is preferred that, the cleavage rate seen in the target tissue (for example muscle, liver, kidney or a tumor) is greater than that found in blood serum. Suitable methods for determining the level (%) of cleavage in target tissue versus serum or cleavage by S1 nuclease are described in the "Materials and methods" section. In some embodiments, the biocleavable linker (also referred to as the physiologically labile linker, or nuclease susceptible linker or region B), in a conjugate of the invention, is at least about 20% cleaved, such as at least about 30% cleaved, such as at least about 40% cleaved, such as at least about 50% cleaved, such as at least about 60% cleaved, such as at least about 70% cleaved, such as at least about 75% cleaved when compared against a standard.

As described herein the oligonucleotide conjugate comprises three regions: i) a first region (region A), which comprises 10 - 25 contiguous nucleotides complementary to the target nucleic acid; ii) a second region (region B) which comprises a biocleavable linker and iii) a third region (region C) which comprises a conjugate moiety, such as an asialoglycoprotein receptor targeting conjugate moiety, wherein the third region is covalent linked to the second region which is covalently linked to the first region.

As described herein the oligonucleotide conjugate comprises a biocleavable linker (Region B) between the contiguous nucleotide sequence (region A) and the asialoglycoprotein receptor targeting conjugate moiety (region C).

In some embodiments, the biocleavable linker may be situated either at the 5' end and/or the 3'-end of the contiguous nucleotides complementary to the target nucleic acid (region A). In a preferred embodiment the biocleavable linker is at the 5'-end.

In some embodiments, the cleavable linker is susceptible to nuclease(s) which may for example, be expressed in the target cell. In some embodiments the biocleavable linker is composed of 2 to 5 consecutive phosphodiester linkages. The linker may be a short region (e.g. 1 - 10 as detailed in the definition of linkers) phosphodiester linked nucleosides. In some embodiments, the nucleosides in the biocleavable linker region B is (optionally independently) selected from the group consisting of DNA and RNA or modifications thereof which do not interfere with nuclease cleavage. Modifications of DNA and RNA nucleosides which do not interfere with nuclease cleavage may be non-naturally occurring nucleobases. Certain sugar-modified nucleosides may also allow nuclease cleavage such as an alpha-L-oxy-LNA. In some embodiments, all the nucleosides of region B comprise (optionally independently) either a 2'-OH ribose sugar (RNA) or a 2'-H sugar - i.e. RNA or DNA. In a preferred embodiment, at least two consecutive nucleosides of region B are DNA or RNA nucleosides (such as at least 3 or 4 or 5 consecutive DNA or RNA nucleosides). In an even more preferred embodiment, the nucleosides of region B are DNA nucleosides Preferably region B consists of between 1 to 5, or 1 to 4, such as 2, 3, 4 consecutive phosphodiester linked DNA nucleosides. In preferred embodiments region B is so short that it does not recruit RNAseH. In some embodiments, region B comprises no more than 3 or no more than 4 consecutive phospodiester linked DNA and/or RNA nucleosides (such as DNA nucleosides).

Where region B is composed of phosphodiester linked nucleosides, region A and B may together form the oligonucleotide that is linked to region C. In this context region A can be differentiated from region B in that Region A starts with at least one, preferably at least two, modified nucleosides with increased binding affinity to the target nucleic acid (e.g. LNA or nucleosides with a 2' substituted sugar moiety) and region A on its own is capable of modulation of the expression the target nucleic acid in a relevant cell line. Furthermore, if region A comprises DNA or RNA nucleosides these are linked with nuclease resistant internucleoside linkage, such phosphorothioate or boranophosphate. Region B on the other hand comprises phophodiester linkages between DNA or RNA nucleosides. In some embodiments region B is not complementary to or comprises at least 50% mismatches to the target nucleic acid.

In some embodiments, region B is not complementary to the target nucleic acid sequence or to the contiguous nucleotides complementary to the target nucleic acid in region A.

In some embodiments, region B is complementary with the target nucleic acid sequence. In this respect region A and B together may form a single contiguous sequence which is complementary to the target sequence.

In some aspects of the invention the internucleoside linkage between the first (region A) and the second region (region B) may be considered part of the second region.

In some embodiments, the sequence of bases in region B is selected to provide an optimal endonuclease cleavage site, based upon the predominant endonuclease cleavage enzymes present in the target tissue or cell or sub-cellular compartment. In this respect, by isolating cell extracts from target tissues and non-target tissues, endonuclease cleavage sequences for use in region B may be selected based upon a preferential cleavage activity in the desired target cell (e.g. liver/hepatocytes) as compared to a non-target cell (e.g. kidney). In this respect, the potency of the compound for target down-regulation may be optimized for the desired tissue/cell.

In some embodiments region B comprises a dinucleotide of sequence AA, AT, AC, AG, TA, TT, TC, TG, CA, CT, CC, CG, GA, GT, GC, or GG, wherein C may be 5-methylcytosine, and/or T may be replaced with U. Preferably, the internucleoside linkage is a phosphodiester linkage. In some embodiments region B comprises a trinucleotide of sequence AAA, AAT, AAC, AAG, ATA, ATT, ATC, ATG, ACA, ACT, ACC, ACG, AGA, AGT, AGC, AGG, TAA, TAT, TAC, TAG, TTA, TTT, TTC, TAG, TCA, TCT, TCC, TCG, TGA, TGT, TGC, TGG, CAA, CAT, CAC, CAG, CTA, CTG, CTC, CTT, CCA, CCT, CCC, CCG, CGA, CGT, CGC, CGG, GAA, GAT, GAC, CAG, GTA, GTT, GTC, GTG, GCA, GCT, GCC, GCG, GGA, GGT, GGC, and GGG wherein C may be 5-methylcytosine and/or T may be replaced with U. Preferably, the internucleoside linkages are phosphodiester linkages. In some embodiments region B comprises a trinucleotide of sequence AAAX, AATX, AACX, AAGX, ATAX, ATTX, ATCX, ATGX, ACAX, ACTX, ACCX, ACGX, AGAX, AGTX, AGCX, AGGX, TAAX, TATX, TACX, TAGX, TTAX, TTTX, TTCX, TAGX, TCAX, TCTX, TCCX, TCGX, TGAX, TGTX, TGCX, TGGX, CAAX, CATX, CACX, CAGX, CTAX, CTGX, CTCX, CTTX, CCAX, CCTX, CCCX, CCGX, CGAX, CGTX, CGCX, CGGX, GAAX, GATX, GACX, CAGX, GTAX, GTTX, GTCX, GTGX, GCAX, GCTX, GCCX, GCGX, GGAX, GGTX, GGCX, and GGGX, wherein X may be selected from the group consisting of A, T, U, G, C and analogues thereof, wherein C may be 5-methylcytosine and/or T may be replaced with U. Preferably, the internucleoside linkages are phosphodiester linkages. It will be recognized that when referring to (naturally occurring) nucleobases A, T, U, G, C, these may be substituted with nucleobase analogues which function as the equivalent natural nucleobase (e.g. base pair with the complementary nucleoside).

### Other linkers (Region Y)

The linker can have at least two functionalities, one for attaching to the oligonucleotide and the other for attaching to the conjugate moiety. Example linker functionalities can be electrophilic for reacting with nucleophilic groups on the oligonucleotide or conjugate moiety, or nucleophilic for reacting with electrophilic groups. In some embodiments, linker functionalities include amino, hydroxyl, carboxylic acid, thiol, phosphoramidate, phosphorothioate, phosphate, phosphite, unsaturations (e.g., double or triple bonds). Some example linkers (region Y) include 8-amino-3,6-dioxaoctanoic acid (ADO), succinimidyl 4-(N-maleimidomethyl)cyclohexane-l-carboxylate (SMCC), 6- aminohexanoic acid (AHEX or AHA), 6-aminohexyloxy, 4-aminobutyric acid, 4-aminocyclohexylcarboxylic acid, succinimidyl 4-(N-maleimidomethyl)cyclohexane-l-carboxy-(6-amido-caproate) (LCSMCC), succinimidyl m-maleimido-benzoylate (MBS), succinimidyl N-e-maleimido-caproylate (EMCS), succinimidyl 6-(beta - maleimido-propionamido) hexanoate (SMPH), succinimidyl N-(a-maleimido acetate) (AMAS), succinimidyl 4-(p-maleimidophenyl)butyrate (SMPB), beta -alanine (beta -ALA), phenylglycine (PHG), 4-aminocyclohexanoic acid (ACHC), beta -(cyclopropyl) alanine (beta -CYPR), amino dodecanoic acid (ADC), alylene diols, polyethylene glycols, amino acids. In some embodiments the linker (region Y) is an amino alkyl, such as a C2 - C36 amino alkyl group, including, for example C6 to C12 amino alkyl groups. In a preferred embodiment the linker (region Y) is a C6 amino alkyl group. The amino alkyl group may be added to the oligonucleotide (region A or region A-B) as part of standard oligonucleotide synthesis, for example using a (e.g. protected) amino alkyl phosphoramidite. The linkage group between the amino alkyl and the oligonucleotide may for example be a phosphorothioate or a phosphodiester, or one of the other nucleoside linkage groups referred to herein. The amino alkyl group is covalently linked to the 5' or 3'-end of the oligonucleotide. Commercially available amino alkyl linkers are for example 3'-Amino-Modifier reagent for linkage at the 3'-end of the oligonucleotide and for linkage at the 5 '-end of an oligonucleotide 5'- Amino-Modifier C6 is available. These reagents are available from Glen Research Corporation (Sterling, Va.). These compounds or similar ones were utilized by Krieg, et al, Antisense Research and Development 1991, 1, 161 to link fluorescein to the 5'- terminus of an oligonucleotide. A wide variety of further linker groups are known in the art and can be useful in the attachment of conjugate moieties to oligonucleotides. A review of many of the useful linker groups can be found in, for example, Antisense Research and Applications, S. T. Crooke and B. Lebleu, Eds., CRC Press, Boca Raton, Fla., 1993, p. 303-350. Other compounds such as acridine have been attached to the 3 '-terminal phosphate group of an oligonucleotide via a polymethylene linkage (Asseline, et al., Proc. Natl. Acad. Sci. USA 1984, 81, 3297). Any of the above groups can be used as a single linker (region Y) or in combination with one or more further linkers (region Y-Y' or region Y-B or B-Y).

Linkers and their use in preparation of conjugates of oligonucleotides are provided throughout the art such as in WO 96/11205 and WO 98/52614 and U.S. Pat. Nos. 4,948,882; 5,525,465; 5,541,313; 5,545,730; 5,552,538; 5,580,731; 5,486,603; 5,608,046; 4,587,044; 4,667,025; 5,254,469; 5,245,022; 5,112,963; 5,391,723; 5,510475; 5,512,667; 5,574,142; 5,684,142; 5,770,716; 6,096,875; 6,335,432; and 6,335,437,WO 2012/083046.

### Method of manufacture

Described herein are methods for manufacturing the oligonucleotides of the invention comprising reacting nucleotide units and thereby forming covalently linked contiguous nucleotide units comprised in the oligonucleotide. Preferably, the method uses phophoramidite chemistry (see for example Caruthers et al, 1987, Methods in Enzymology vol. 154, pages 287-313). As described herein the method further comprises reacting the contiguous nucleotide sequence with a conjugating moiety (ligand). Described herein is a method is provided for manufacturing the composition of the invention, comprising mixing the oligonucleotide or conjugated oligonucleotide of the invention with a pharmaceutically acceptable diluent, solvent, carrier, salt and/or adjuvant.

### Pharmaceutical Composition

As described herein, the invention provides pharmaceutical compositions comprising any of the aforementioned oligonucleotides and/or oligonucleotide conjugates and a pharmaceutically acceptable diluent, solvent, carrier, salt and/or adjuvant. A pharmaceutically acceptable diluent includes phosphate-buffered saline (PBS) and pharmaceutically acceptable salts include sodium and potassium salts. In some embodiments the pharmaceutically acceptable diluent is sterile phosphate buffered saline. In some embodiments the oligonucleotide is used in the pharmaceutically acceptable diluent at a concentration of 50 - 300µM solution.

Suitable formulations for use in the present invention are found in Remington's Pharmaceutical Sciences, Mack Publishing Company, Philadelphia, Pa., 17th ed., 1985. For a brief review of methods for drug delivery, see, e.g., Langer (Science 249:1527-1533, 1990). WO 2007/031091 provides further suitable and preferred examples of pharmaceutically acceptable diluents, carriers and adjuvants. Suitable dosages, formulations, administration routes, compositions, dosage forms, combinations with other therapeutic agents, pro-drug formulations are also provided in WO2007/031091.

Oligonucleotides or oligonucleotide conjugates of the invention may be mixed with pharmaceutically acceptable active or inert substances for the preparation of pharmaceutical compositions or formulations. Compositions and methods for the formulation of pharmaceutical compositions are dependent upon a number of criteria, including route of administration, extent of disease, or dose to be administered.

These compositions may be sterilized by conventional sterilization techniques, or may be sterile filtered. The resulting aqueous solutions may be packaged for use as is, or lyophilized, the lyophilized preparation being combined with a sterile aqueous carrier prior to administration. The pH of the preparations typically will be between 3 and 11, more preferably between 5 and 9 or between 6 and 8, and most preferably between 7 and 8, such as 7 to 7.5. The resulting compositions in solid form may be packaged in multiple single dose units, each containing a fixed amount of the above-mentioned agent or agents, such as in a sealed package of tablets or capsules. The composition in solid form can also be packaged in a container for a flexible quantity, such as in a squeezable tube designed for a topically applicable cream or ointment.

In some embodiments, the oligonucleotide or oligonucleotide conjugate of the invention is a prodrug. In particular with respect to oligonucleotide conjugates the conjugate moiety is cleaved of the oligonucleotide once the prodrug is delivered to the site of action, e.g. the target cell.

### Applications

The oligonucleotides or oligonucleotide conjugates of the present invention may be utilized as research reagents for, for example, diagnostics, therapeutics and prophylaxis.

In research, such oligonucleotides or oligonucleotide conjugates may be used to specifically modulate the synthesis of PD-L1 protein in cells (e.g. *in vitro* cell cultures) and experimental animals thereby facilitating functional analysis of the target or an appraisal of its usefulness as a target for therapeutic intervention. Typically the target modulation is achieved by degrading or inhibiting the mRNA producing the protein, thereby prevent protein formation or by degrading or inhibiting a modulator of the gene or mRNA producing the protein.

If employing the oligonucleotide of the invention in research or diagnostics the target nucleic acid may be a cDNA or a synthetic nucleic acid derived from DNA or RNA.

Described herein is an *in vivo* or *in vitro* method for modulating PD-L1 expression in a target cell which is expressing PD-L1, said method comprising administering an oligonucleotide or oligonucleotide conjugate of the invention in an effective amount to said cell.

As described herein, the target cell, is a mammalian cell in particular a human cell. The target cell may be an *in vitro* cell culture or an *in vivo* cell forming part of a tissue in a mammal. As described herein the target cell is present in the liver. Liver target cell can be selected from parenchymal cells (e.g. hepatocytes) and non-parenchymal cells such as Kupffer cells, LSECs, stellate cells (or Ito cells), cholangiocytes and liver-associated leukocytes (including T cells and NK cells). As described herein the target cell is an antigen-presenting cell. Antigen-presenting cells displays foreign antigens complexed with major histocompatibility complex (MHC) class I or class II on their surfaces. In some embodiments the antigen-presenting cell expresses MHC class II (i.e. professional antigen-presenting cells such as dendritic cells, macrophages and B cells).

In diagnostics the oligonucleotides may be used to detect and quantitate PD-L1 expression in cell and tissues by northern blotting, *in-situ* hybridisation or similar techniques.

For therapeutics oligonucleotides or oligonucleotide conjugates of the present invention or pharmaceutical compositions thereof may be administered to an animal or a human, suspected of having a disease or disorder, which can be alleviated or treated by reduction of the expression of PD-L1, in particular by reduction of the expression of PD-L1 in liver target cells.

Described herein are methods for treating or preventing a disease, comprising administering a therapeutically or prophylactically effective amount of an oligonucleotide, an oligonucleotide conjugate or a pharmaceutical composition of the invention to a subject suffering from or susceptible to the disease.

The invention also relates to an oligonucleotide, oligonucleotide conjugate or a pharmaceutical composition according to the invention for use as a medicament.

The oligonucleotide, oligonucleotide conjugate or a pharmaceutical composition according to the invention is typically administered in an effective amount.

Described herein is the use of the oligonucleotide or oligonucleotide conjugate or pharmaceutical composition of the invention as described for the manufacture of a medicament for the treatment of a disease or disorder as referred to herein. As described herein the disease is selected from a) viral liver infections such as HBV, HCV and HDV; b) parasite infections such as malaria, toxoplasmosis, leishmaniasis and trypanosomiasis and c) liver cancer or metastases in the liver.

Described herein are oligonucleotides, oligonucleotide conjugates or pharmaceutical compositions for use in the treatment of diseases or disorders selected from viral or parasitic infections. As described herein the disease is selected from a) viral liver infections such as HBV, HCV and HDV; b) parasite infections such as malaria, toxoplasmosis, leishmaniasis and trypanosomiasis and c) liver cancer or metastases in the liver.

The disease or disorder, as referred to herein, is associated with immune exhaustion. In particular the disease or disorder is associated with exhaustion of virus-specific T-cell responses. As described herein, the disease or disorder may be alleviated or treated by reduction of PD-L1 expression.

The methods of the invention are preferably employed for treatment or prophylaxis against diseases associated with immune exhaustion.

As described herein the oligonucleotide, oligonucleotide conjugate or pharmaceutical compositions of the invention are used in restoration of immune response against a liver cancer or metastases in the liver.

As described herein the oligonucleotide, oligonucleotide conjugate or pharmaceutical compositions of the invention are used in restoration of immune response against a pathogen. As described herein the pathogen can be found in the liver. The pathogens can be a virus or a parasite, in particular those described herein. In a preferred embodiment the pathogen is HBV.

Described herein is the use of an oligonucleotide, oligonucleotide conjugate or a pharmaceutical composition as defined herein for the manufacture of a medicament for the restoration of immunity against a viral or parasite infection as mentioned herein.

Oligonucleotides or oligonucleotide conjugates or pharmaceutical compositions of the present invention can be used in the treatment of viral infections, in particular viral infections in the liver where the PD-1 patheway is affected (see for example Kapoor and Kottilil 2014 Future Virol Vol. 9 pp. 565-585 and Salem and El-Badawy 2015 World J Hepatol Vol. 7 pp. 2449-2458). Viral liver infections can be selected from the group consisting of hepatitis viruses, in particular HBV, HCV and HDV, in particular chronic forms of these infections. In one embodiment the oligonucleotides or oligonucleotide conjugates or pharmaceutical compositions of the present invention are used to treat HBV, in particular chronic HBV. Indicators of chronic HBV infections are high levels of viral load (HBV DNA) and even higher levels of empty HBsAg particles (>100-fold in excess of virions) in the circulation.

Oligonucleotides or oligonucleotide conjugates of the present invention can also be used to treat viral liver infections that occur as co-infections with HIV. Other viral infections which can be treated with the oligonucleotides or oligonucleotide conjugates or pharmaceutical compositions of the present invention are Icmv (Lymphocytic Choriomeningitis Virus), and HIV as a mono infection, HSV-1 and -2, and other herpesviruses. These viruses are not hepatotrophic, however they may be sensitive to PDL1 down regulation.

In some embodiments the restoration of immunity or immune response involves improvement of the T-cell and/or NK cell response and/or alleviation of the T-cell exhaustion, in particular the HBV-specific T-cell response, the HCV-specific T-cell response and or the HDV-specific T-cell response is restored. An improvement of the T cell response can for example be assessed as an increase in T cells in the liver, in particular an increase in CD8+ and/or CD4+ T cells when compared to a control (e.g. the level prior to treatment or the level in a vehicle treated subject) In a further embodiment it is the virus specific CD8₊ T cells that are restored or increased when compared to cotrol), in particular HBV specific CD8₊ T cells or HCV specific CD8₊ T cells or HDV specific CD8₊ T cells are restored or increased when compared to control. In a preferred embodiment CD8₊ T cells specific for HBV s antigen (HBsAg) and/or CD8₊ T cells specific for HBV e antigen (HBeAg) and/or CD8₊ T cells specific for HBV core antigen (HBcAg) are increased in subjects treated with an oligonucleotide, oligonucleotide conjugate or pharmaceutical composition of the present invention compared to control. Preferably the HBV antigen specific CD8₊ T cells produce one or more cytokines, such as interferon-gamma (IFN-γ) or tumor necrosis factor alpha (TNF-α). The increase in CD8₊ T cells described above is in particular observed in the liver. The increase described herein should be statistically significant when compared to a control. Preferably the increase is at least 20%, such as 25%, such as 50% such as 75% when compared to control. In another embodiment natural killer (NK) cells and/or natural killer T (NKT) cells are activated by the oligonucleotides or oligonucleotide conjugates of the present invention.

Oligonucleotides or oligonucleotide conjugates or pharmaceutical compositions of the present invention can be used in the treatment parasite infections, in particular parasite infections where the PD-1 pathway is affected (see for example Bhadra et al. 2012 J Infect Dis vol 206 pp. 125-134; Bhadra et al. 2011 Proc Natl Acad Sci U S A Vol. 108 pp. 9196-9201; Esch et al. J Immunol vol 191 pp 5542-5550; Freeman and Sharpe 2012 Nat Immunol Vol 13 pp. 113-115; Gutierrez et al. 2011 Infect Immun Vol 79 pp. 1873-1881; Joshi et al. 2009 PLoS Pathog Vol 5 e1000431; Liang et al. 2006 Eur J Immunol Vol. 36 pp 58-64; Wykes et al. 2014 Front Microbiol Vol 5 pp 249). Parasite infections can be selected from the group consisting of malaria, toxoplasmosis, leishmaniasis and trypanosomiasis. Malaria infection is caused by protozoa of the genus *Plasmodium,* in particular of the species *P. vivax, P. malariae* and *P. falciparum.* Toxoplasmosis is a parasitic disease caused by *Toxoplasma gondii.* Leishmaniasis is a disease caused by protozoan parasites of the genus *Leishmania.* Trypanosomiasis is caused by the protozoan of the genus *Trypanosoma.* Chaga disease which is the tropical form caused by the species *Trypanosoma cruzi,* and sleeping disease is caused by the species *Trypanosoma brucei.*

In some embodiments the restoration of immunity involves restoration of a parasite-specific T cell and NK cell response, in particular a *Plasmodium-specific* T-cell response, a *Toxoplasma gondii*-specific T-cell and NK cell response, a *Leishmania-specific* T-cell and NK cell response, a *Trypanosoma cruzi*-specific T-cell and NK cell response or a *Trypanosoma brucei*-specific T-cell and NK cell response. In a further embodiment it is the parasite-specific CD8₊ T cell and NK cell response that is restored.

### Administration

The oligonucleotides or pharmaceutical compositions of the present invention may be administered topical (such as, to the skin, inhalation, ophthalmic or otic) or enteral (such as, orally or through the gastrointestinal tract) or parenteral (such as, intravenous, subcutaneous, intra-muscular, intracerebral, intracerebroventricular or intrathecal).

In a preferred embodiment the oligonucleotide or pharmaceutical compositions of the present invention are administered by a parenteral route including intravenous, intraarterial, subcutaneous, intraperitoneal or intramuscular injection or infusion, intrathecal or intracranial, e.g. intracerebral or intraventricular, intravitreal administration. In one embodiment the active oligonucleotide or oligonucleotide conjugate is administered intravenously. In another embodiment the active oligonucleotide or oligonucleotide conjugate is administered subcutaneously.

In some embodiments, the oligonucleotide, oligonucleotide conjugate or pharmaceutical composition of the invention is administered at a dose of 0.1 - 15 mg/kg, such as from 0.1 - 10 mg/kg, such as from 0.2 - 10 mg/kg, such as from 0.25 - 10 mg/kg, such as from 0.1 - 5 mg/kg, such as from 0.2 - 5 mg/kg, such as from 0.25 - 5 mg/kg. The administration can be once a week, every 2^{nd} week, every third week or even once a month.

### Combination therapies

In some embodiments the oligonucleotide, oligonucleotide conjugate or pharmaceutical composition of the invention is for use in a combination treatment with another therapeutic agent. The therapeutic agent can for example be the standard of care for the diseases or disorders described above.

For the treatment of chronic HBV infections a combination of antiviral drugs and immune system modulators is recommended as standard of care. The antiviral drugs effective against HBV are for example nucleos(t)ide analogs. There are five nucleos(t)ide analogs licensed for therapy of HBV namely lamivudine (Epivir), adefovir (Hepsera), tenofovir (Viread), telbivudine (Tyzeka), entecavir (Baraclude) these are effective in suppressing viral replication (HBV DNA) but have no effect on HBsAg levels. Other antiviral drugs include ribavirin and an HBV antibody therapy (monoclonal or polyclonal). The immune system modulators can for example be interferon alpha-2a and PEGylated interferon alpha-2a (Pegasys) or TLR7 agonists (e.g. GS-9620) or therapeutic vaccines. IFN-α treatment show only very modest effect in reducing viral load, but result in some HBsAg decline, albeit very inefficiently (<10% after 48 week therapy).

The oligonucleotide or oligonucleotide conjugates of the present invention may also be combined with other antiviral drugs effective against HBV such as the antisense oligonucleotides described in WO2012/145697 and WO 2014/179629 or the siRNA molecules described in WO 2005/014806, WO 2012/024170, WO 2012/2055362, WO 2013/003520 and WO 2013/159109.

When the oligonucleotides or oligonucleotide conjugates of this invention are administered in combination therapies with other agents, they may be administered sequentially or concurrently to an individual. Alternatively, pharmaceutical compositions according to the present invention may be comprised of a combination of an oligonucleotide or oligonucleotide conjugate of the present invention in association with a pharmaceutically acceptable excipient, as described herein, and another therapeutic or prophylactic agent known in the art.

### EXAMPLES

### Materials and methods

### Motif sequences and oligonucleotide compounds

**Table 5: list of oligonucleotide motif sequences (indicated by SEQ ID NO) targeting the human PD-L1 transcript (SEQ ID NO: 1), designs of these, as well as specific antisense oligonucleotide compounds (indicated by CMP ID NO) designed based on the motif sequence.**

| **SEQ ID NO** | **Motif sequence** | **Design** | **Oligonucleotide Compound** | **CMP ID NO** | **Start ID NO: 1** | **dG** |
|---|---|---|---|---|---|---|
| 5 | taattggctctactgc | 2-11-3 | TAattggctctacTGC | 5_1 | 236 | -20 |
| 6 | tcgcataagaatgact | 4-10-2 | TCGCataagaatgaCT | 6_1 | 371 | -19 |
| 7 | tgaacacacagtcgca | 2-12-2 | TGaacacacagtcgCA | 7_1 | 382 | -19 |
| 8 | ctgaacacacagtcgc | 3-10-3 | CTGaacacacagtCGC | 8_1 | 383 | -22 |
| 9 | tctgaacacacagtcg | 3-11-2 | TCTgaacacacagtCG | 9_1 | 384 | -19 |
| 10 | ttctgaacacacagtc | 3-11-2 | TTCtgaacacacagTC | 10_1 | 385 | -17 |
| 11 | acaagtcatgttacta | 2-11-3 | ACaagtcatgttaCTA | 11_1 | 463 | -16 |
| 12 | acacaagtcatgttac | 2-12-2 | ACacaagtcatgttAC | 12_1 | 465 | -14 |
| 13 | cttacttagatgctgc | 2-11-3 | CTtacttagatgcTGC | 13_1 | 495 | -20 |
| 14 | acttacttagatgctg | 2-11-3 | ACttacttagatgCTG | 14_1 | 496 | -18 |
| 15 | gacttacttagatgct | 3-11-2 | GACttacttagatgCT | 15_1 | 497 | -19 |
| 16 | agacttacttagatgc | 2-11-3 | AGacttacttagaTGC | 16_1 | 498 | -18 |
| 17 | gcaggaagagacttac | 3-10-3 | GCAggaagagactTAC | 17_1 | 506 | -20 |
| 18 | aataaattccgttcagg | 4-9-4 | AATAaattccgttCAGG | 18_1 | 541 | -22 |
| 19 | gcaaataaattccgtt | 3-10-3 | GCAaataaattccGTT | 19_2 | 545 | -18 |
| 19 | gcaaataaattccgtt | 4-8-4 | GCAAataaattcCGTT | 19_1 | 545 | -20 |
| 20 | agcaaataaattccgt | 4-9-3 | AGCAaataaattcCGT | 20_1 | 546 | -20 |
| 21 | cagagcaaataaattcc | 4-10-3 | CAGAgcaaataaatTCC | 21_1 | 548 | -21 |
| 22 | tggacagagcaaataaat | 4-11-3 | TGGAcagagcaaataAAT | 22_1 | 551 | -19 |
| 23 | atggacagagcaaata | 4-8-4 | ATGGacagagcaAATA | 23_1 | 554 | -20 |
| 24 | cagaatggacagagca | 2-11-3 | CAgaatggacagaGCA | 24_1 | 558 | -21 |
| 25 | ttctcagaatggacag | 3-11-2 | TTCtcagaatggacAG | 25_1 | 562 | -17 |
| 26 | ctgaactttgacatag | 4-8-4 | CTGAactttgacATAG | 26_1 | 663 | -20 |
| 27 | aagacaaacccagactga | 2-13-3 | AAgacaaacccagacTGA | 27_1 | 675 | -21 |
| 28 | tataagacaaacccagac | 4-10-4 | TATAagacaaacccAGAC | 28_1 | 678 | -22 |
| 29 | ttataagacaaacccaga | 4-10-4 | TTATaagacaaaccCAGA | 29_1 | 679 | -23 |
| 30 | tgttataagacaaaccc | 4-10-3 | TGTTataagacaaaCCC | 30_1 | 682 | -22 |
| 31 | tagaacaatggtacttt | 4-9-4 | TAGAacaatggtaCTTT | 31_1 | 708 | -20 |
| 32 | gtagaacaatggtact | 4-10-2 | GTAGaacaatggtaCT | 32_1 | 710 | -19 |
| 33 | aggtagaacaatggta | 3-10-3 | AGGtagaacaatgGTA | 33_1 | 712 | -19 |
| 34 | aagaggtagaacaatgg | 4-9-4 | AAGAggtagaacaATGG | 34_1 | 714 | -21 |
| 35 | gcatccacagtaaatt | 2-12-2 | GCatccacagtaaaTT | 35_1 | 749 | -17 |
| 36 | gaaggttatttaattc | 2-11-3 | GAaggttatttaaTTC | 36_1 | 773 | -13 |
| 37 | ctaatcgaatgcagca | 4-9-3 | CTAAtcgaatgcaGCA | 37_1 | 805 | -22 |
| 38 | tacccaatctaatcga | 3-10-3 | TACccaatctaatCGA | 38_1 | 813 | -20 |
| 39 | tagttacccaatctaa | 3-10-3 | TAGttacccaatcTAA | 39_1 | 817 | -19 |
| 40 | catttagttacccaat | 3-10-3 | CATttagttacccAAT | 40_1 | 821 | -18 |
| 41 | tcatttagttacccaa | 3-10-3 | TCAtttagttaccCAA | 41_1 | 822 | -19 |
| 42 | ttcatttagttaccca | 2-10-4 | TTcatttagttaCCCA | 42_1 | 823 | -22 |
| 43 | gaattaatttcatttagt | 4-10-4 | GAATtaatttcattTAGT | 43_1 | 829 | -19 |
| 44 | cagtgaggaattaattt | 4-9-4 | CAGTgaggaattaATTT | 44_1 | 837 | -20 |
| 45 | ccaacagtgaggaatt | 4-8-4 | CCAAcagtgaggAATT | 45_1 | 842 | -21 |
| 46 | cccaacagtgaggaat | 3-10-3 | CCCaacagtgaggAAT | 46_1 | 843 | -22 |
| 47 | tatacccaacagtgagg | 2-12-3 | TAtacccaacagtgAGG | 47_1 | 846 | -21 |
| 48 | ttatacccaacagtgag | 2-11-4 | TTatacccaacagTGAG | 48_1 | 847 | -21 |
| 49 | tttatacccaacagtga | 3-11-3 | TTTatacccaacagTGA | 49_1 | 848 | -21 |
| 50 | cctttatacccaacag | 3-10-3 | CCTttatacccaaCAG | 50_1 | 851 | -23 |
| 51 | taacctttatacccaa | 4-8-4 | TAACctttatacCCAA | 51_1 | 854 | -22 |
| 52 | aataacctttataccca | 3-10-4 | AATaacctttataCCCA | 52_1 | 855 | -23 |
| 53 | gtaaataacctttata | 3-11-2 | GTAaataacctttaTA | 53_1 | 859 | -14 |
| 54 | actgtaaataacctttat | 4-10-4 | ACTGtaaataacctTTAT | 54_1 | 860 | -20 |
| 55 | atatatatgcaatgag | 3-11-2 | ATAtatatgcaatgAG | 55_1 | 903 | -14 |
| 56 | agatatatatgcaatg | 2-12-2 | AGatatatatgcaaTG | 56_1 | 905 | -12 |
| 57 | gagatatatatgcaat | 3-10-3 | GAGatatatatgcAAT | 57_1 | 906 | -15 |
| 58 | ccagagatatatatgc | 2-11-3 | CCagagatatataTGC | 58_1 | 909 | -19 |
| 59 | caatattccagagatat | 4-9-4 | CAATattccagagATAT | 59_1 | 915 | -20 |
| 60 | gcaatattccagagata | 4-10-3 | GCAAtattccagagATA | 60_1 | 916 | -22 |
| 61 | agcaatattccagagat | 3-11-3 | AGCaatattccagaGAT | 61_1 | 917 | -22 |
| 62 | cagcaatattccagag | 3-9-4 | CAGcaatattccAGAG | 62_1 | 919 | -22 |
| 63 | aatcagcaatattccag | 4-9-4 | AATCagcaatattCCAG | 63_1 | 921 | -23 |
| 64 | acaatcagcaatattcc | 4-9-4 | ACAAtcagcaataTTCC | 64_1 | 923 | -21 |
| 65 | actaagtagttacacttct | 2-14-3 | ACtaagtagttacactTCT | 65_1 | 957 | -20 |
| 66 | ctaagtagttacacttc | 4-11-2 | CTAAgtagttacactTC | 66_1 | 958 | -18 |
| 67 | gactaagtagttacactt | 3-12-3 | GACtaagtagttacaCTT | 67_1 | 959 | -20 |
| 68 | tgactaagtagttaca | 3-9-4 | TGActaagtagtTACA | 68_1 | 962 | -19 |
| 69 | ctttgactaagtagtta | 4-10-3 | CTTTgactaagtagTTA | 69_1 | 964 | -19 |
| 70 | ctctttgactaagtag | 3-10-3 | CTCtttgactaagTAG | 70_1 | 967 | -19 |
| 71 | gctctttgactaagta | 4-10-2 | GCTCtttgactaagTA | 71_1 | 968 | -21 |
| 72 | ccttaaatactgttgac | 2-11-4 | CCttaaatactgtTGAC | 72_1 | 1060 | -20 |
| 73 | cttaaatactgttgac | 2-12-2 | CTtaaatactgttgAC | 73_1 | 1060 | -13 |
| 74 | tccttaaatactgttg | 3-10-3 | TCCttaaatactgTTG | 74_1 | 1062 | -18 |
| 75 | tctccttaaatactgtt | 4-11-2 | TCTCcttaaatactgTT | 75_1 | 1063 | -19 |
| 76 | tatcatagttctcctt | 2-10-4 | TAtcatagttctCCTT | 76_1 | 1073 | -21 |
| 77 | agtatcatagttctcc | 3-10-3 | AGTatcatagttcTCC | 77_1 | 1075 | -22 |
| 78 | gagtatcatagttctc | 2-11-3 | GAgtatcatagttCTC | 78_1 | 1076 | -18 |
| 79 | agagtatcatagttct | 2-10-4 | AGagtatcatagTTCT | 79_1 | 1077 | -18 |
| 79 | agagtatcatagttct | 3-10-3 | AGAgtatcatagtTCT | 79_2 | 1077 | -19 |
| 80 | cagagtatcatagttc | 3-10-3 | CAGagtatcatagTTC | 80_1 | 1078 | -18 |
| 81 | ttcagagtatcatagt | 4-10-2 | TTCAgagtatcataGT | 81_1 | 1080 | -18 |
| 82 | cttcagagtatcatag | 3-9-4 | CTTcagagtatcATAG | 82_1 | 1081 | -19 |
| 83 | ttcttcagagtatcata | 4-11-2 | TTCTtcagagtatcaTA | 83_1 | 1082 | -19 |
| 84 | tttcttcagagtatcat | 3-10-4 | TTTcttcagagtaTCAT | 84_1 | 1083 | -20 |
| 85 | gagaaaggctaagttt | 4-9-3 | GAGAaaggctaagTTT | 85_1 | 1099 | -19 |
| 86 | gacactcttgtacatt | 2-10-4 | GAcactcttgtaCATT | 86_1 | 1213 | -19 |
| 87 | tgagacactcttgtaca | 2-13-2 | TGagacactcttgtaCA | 87_1 | 1215 | -18 |
| 88 | tgagacactcttgtac | 2-11-3 | TGagacactcttgTAC | 88_1 | 1216 | -18 |
| 89 | ctttattaaactccat | 2-10-4 | CTttattaaactCCAT | 89_1 | 1266 | -18 |
| 90 | accaaactttattaaa | 4-10-2 | ACCAaactttattaAA | 90_1 | 1272 | -14 |
| 91 | aaacctctactaagtg | 4-10-2 | AAACctctactaagTG | 91_1 | 1288 | -16 |
| 92 | agattaagacagttga | 2-11-3 | AGattaagacagtTGA | 92_1 | 1310 | -16 |
| 93 | aagtaggagcaagaggc | 2-12-3 | AAgtaggagcaagaGGC | 93_1 | 1475 | -22 |
| 94 | aaagtaggagcaagagg | 4-10-3 | AAAGtaggagcaagAGG | 94_1 | 1476 | -20 |
| 95 | gttaagcagccaggag | 2-12-2 | GTtaagcagccaggAG | 95_1 | 1806 | -20 |
| 96 | agggtaggatgggtag | 2-12-2 | AGggtaggatgggtAG | 96_1 | 1842 | -20 |
| 97 | aagggtaggatgggta | 3-11-2 | AAGggtaggatgggTA | 97_1 | 1843 | -20 |
| 98 | caagggtaggatgggt | 2-12-2 | CAagggtaggatggGT | 98_2 | 1844 | -20 |
| 98 | caagggtaggatgggt | 3-11-2 | CAAgggtaggatggGT | 98_1 | 1844 | -21 |
| 99 | ccaagggtaggatggg | 2-12-2 | CCaagggtaggatgGG | 99_1 | 1845 | -22 |
| 100 | tccaagggtaggatgg | 2-12-2 | TCcaagggtaggatGG | 100_1 | 1846 | -20 |
| 101 | cttccaagggtaggat | 4-10-2 | CTTCcaagggtaggAT | 101_1 | 1848 | -21 |
| 102 | atcttccaagggtagga | 3-12-2 | A TCttccaagggtagGA | 102_1 | 1849 | -22 |
| 103 | agaagtgatggctcatt | 2-11-4 | AGaagtgatggctCATT | 103_1 | 1936 | -21 |
| 104 | aagaagtgatggctcat | 3-10-4 | AAGaagtgatggcTCAT | 104_1 | 1937 | -21 |
| 105 | gaagaagtgatggctca | 3-11-3 | GAAgaagtgatggcTCA | 105_1 | 1938 | -21 |
| 106 | atgaaatgtaaactggg | 4-9-4 | ATGAaatgtaaacTGGG | 106_1 | 1955 | -21 |
| 107 | caatgaaatgtaaactgg | 4-10-4 | CAATgaaatgtaaaCTGG | 107_1 | 1956 | -20 |
| 108 | gcaatgaaatgtaaactg | 4-10-4 | GCAAtgaaatgtaaACTG | 108_1 | 1957 | -20 |
| 109 | agcaatgaaatgtaaact | 4-10-4 | AGCAatgaaatgtaAACT | 109_1 | 1958 | -20 |
| 110 | gagcaatgaaatgtaaac | 4-10-4 | GAGCaatgaaatgtAAAC | 110_1 | 1959 | -19 |
| 111 | tgaattcccatatccga | 2-12-3 | TGaattcccatatcCGA | 111_1 | 1992 | -22 |
| 112 | agaattatgaccatat | 2-11-3 | AGaattatgaccaTAT | 112_1 | 2010 | -15 |
| 113 | aggtaagaattatgacc | 3-10-4 | AGGtaagaattatGACC | 113_1 | 2014 | -21 |
| 114 | tcaggtaagaattatgac | 4-10-4 | TCAGgtaagaattaTGAC | 114_1 | 2015 | -22 |
| 115 | cttcaggtaagaattatg | 4-10-4 | CTTCaggtaagaatTATG | 115_1 | 2017 | -21 |
| 116 | tcttcaggtaagaatta | 4-9-4 | TCTTcaggtaagaATTA | 116_1 | 2019 | -20 |
| 117 | cttcttcaggtaagaat | 4-9-4 | CTTCttcaggtaaGAAT | 117_1 | 2021 | -21 |
| 118 | tcttcttcaggtaagaa | 4-10-3 | TCTTcttcaggtaaGAA | 118_1 | 2022 | -20 |
| 119 | tcttcttcaggtaaga | 3-10-3 | TCTtcttcaggtaAGA | 119_1 | 2023 | -20 |
| 120 | tggtctaagagaagaag | 3-10-4 | TGGtctaagagaaGAAG | 120_1 | 2046 | -20 |
| 121 | gttggtctaagagaag | 4-9-3 | GTTGgtctaagagAAG | 121_1 | 2049 | -19 |
| 123 | cagttggtctaagagaa | 2-11-4 | CAgttggtctaagAGAA | 123_1 | 2050 | -20 |
| 124 | gcagttggtctaagagaa | 3-13-2 | GCAgttggtctaagagAA | 124_1 | 2050 | -22 |
| 122 | agttggtctaagagaa | 3-9-4 | AGTtggtctaagAGAA | 122_1 | 2050 | -20 |
| 126 | gcagttggtctaagaga | 2-13-2 | GCagttggtctaagaGA | 126_1 | 2051 | -21 |
| 125 | cagttggtctaagaga | 4-10-2 | CAGTtggtctaagaGA | 125_1 | 2051 | -21 |
| 127 | gcagttggtctaagag | 2-11-3 | GCagttggtctaaGAG | 127_1 | 2052 | -21 |
| 128 | ctcatatcagggcagt | 2-10-4 | CTcatatcagggCAGT | 128_1 | 2063 | -24 |
| 129 | cacacatgttctttaac | 4-11-2 | CACAcatgttctttaAC | 129_1 | 2087 | -18 |
| 130 | taaatacacacatgttct | 3-11-4 | TAAatacacacatgTTCT | 130_1 | 2092 | -19 |
| 131 | gtaaatacacacatgttc | 4-11-3 | GTAAatacacacatgTTC | 131_1 | 2093 | -19 |
| 132 | tgtaaatacacacatgtt | 4-10-4 | TGTAaatacacacaTGTT | 132_1 | 2094 | -22 |
| 133 | gatcatgtaaatacacac | 4-10-4 | GATCatgtaaatacACAC | 133_1 | 2099 | -20 |
| 134 | agatcatgtaaatacaca | 4-10-4 | AGATcatgtaaataCACA | 134_1 | 2100 | -21 |
| 135 | caaagatcatgtaaatacac | 4-12-4 | CAAAgatcatgtaaatACAC | 135_1 | 2101 | -19 |
| 136 | acaaagatcatgtaaataca | 4-12-4 | ACAAagatcatgtaaaTACA | 136_1 | 2102 | -20 |
| 137 | gaatacaaagatcatgta | 4-10-4 | GAATacaaagatcaTGTA | 137_1 | 2108 | -20 |
| 138 | agaatacaaagatcatgt | 4-10-4 | AGAAtacaaagatcATGT | 138_1 | 2109 | -20 |
| 139 | cagaatacaaagatcatg | 4-10-4 | CAGAatacaaagatCATG | 139_1 | 2110 | -21 |
| 140 | gcagaatacaaagatca | 4-9-4 | GCAGaatacaaagATCA | 140_1 | 2112 | -22 |
| 141 | aggcagaatacaaagat | 4-11-2 | AGGCagaatacaaagAT | 141_1 | 2114 | -19 |
| 142 | aaggcagaatacaaaga | 4-10-3 | AAGGcagaatacaaAGA | 142_1 | 2115 | -19 |
| 143 | attagtgagggacgaa | 3-10-3 | ATTagtgagggacGAA | 143_1 | 2132 | -18 |
| 144 | cattagtgagggacga | 2-11-3 | CAttagtgagggaCGA | 144_1 | 2133 | -20 |
| 145 | gagggtgatggattag | 2-11-3 | GAgggtgatggatTAG | 145_1 | 2218 | -19 |
| 146 | ttaggagtaataaagg | 2-10-4 | TT aggagtaataAAG G | 146_1 | 2241 | -14 |
| 147 | ttaatgaatttggttg | 3-11-2 | TTAatgaatttggtTG | 147_1 | 2263 | -13 |
| 148 | ctttaatgaatttggt | 2-12-2 | CTttaatgaatttgGT | 148_1 | 2265 | -14 |
| 149 | catggattacaactaa | 4-10-2 | CATGgattacaactAA | 149_1 | 2322 | -16 |
| 150 | tcatggattacaacta | 2-11-3 | TCatggattacaaCTA | 150_1 | 2323 | -16 |
| 151 | gtcatggattacaact | 3-11-2 | GTCatggattacaaCT | 151_1 | 2324 | -18 |
| 152 | cattaaatctagtcat | 2-10-4 | CAttaaatctagTCAT | 152_1 | 2335 | -16 |
| 153 | gacattaaatctagtca | 4-10-3 | GACAttaaatctagTCA | 153_1 | 2336 | -19 |
| 154 | agggacattaaatcta | 4-10-2 | AGGGacattaaatcTA | 154_1 | 2340 | -18 |
| 155 | caaagcattataacca | 4-9-3 | CAAAgcattataaCCA | 155_1 | 2372 | -18 |
| 156 | acttactaggcagaag | 2-10-4 | ACttactaggcaGAAG | 156_1 | 2415 | -19 |
| 157 | cagagttaactgtaca | 4-10-2 | CAGAgttaactgtaCA | 157_1 | 2545 | -20 |
| 158 | ccagagttaactgtac | 4-10-2 | CCAGagttaactgtAC | 158_1 | 2546 | -20 |
| 159 | gccagagttaactgta | 2-12-2 | GCcagagttaactgTA | 159_1 | 2547 | -20 |
| 160 | tgggccagagttaact | 2-12-2 | TGggccagagttaaCT | 160_1 | 2550 | -21 |
| 161 | cagcatctatcagact | 2-12-2 | CAgcatctatcagaCT | 161_1 | 2576 | -19 |
| 162 | tgaaataacatgagtcat | 3-11-4 | TGAaataacatgagTCAT | 162_1 | 2711 | -19 |
| 163 | gtgaaataacatgagtc | 3-10-4 | GTGaaataacatgAGTC | 163_1 | 2713 | -19 |
| 164 | tctgtttatgtcactg | 4-10-2 | TCTGtttatgtcacTG | 164_1 | 2781 | -20 |
| 165 | gtctgtttatgtcact | 4-10-2 | GTCTgtttatgtcaCT | 165_1 | 2782 | -22 |
| 166 | tggtctgtttatgtca | 2-10-4 | TGgtctgtttatGTCA | 166_1 | 2784 | -21 |
| 167 | ttggtctgtttatgtc | 4-10-2 | TTGGtctgtttatgTC | 167_1 | 2785 | -20 |
| 168 | tcacccattgtttaaa | 2-12-2 | TCacccattgtttaAA | 168_1 | 2842 | -15 |
| 169 | ttcagcaaatattcgt | 2-10-4 | TTcagcaaatatTCGT | 169_1 | 2995 | -17 |
| 170 | gtgtgttcagcaaatat | 3-10-4 | GTGtgttcagcaaATAT | 170_1 | 2999 | -21 |
| 171 | tctattgttaggtatc | 3-10-3 | TCTattgttaggtATC | 171_1 | 3053 | -18 |
| 172 | attgcccatcttactg | 2-12-2 | A TtgcccatcttacTG | 172_1 | 3118 | -19 |
| 173 | tattgcccatcttact | 3-11-2 | TATtgcccatcttaCT | 173_1 | 3119 | -21 |
| 174 | aaatattgcccatctt | 2-11-3 | AAatattgcccatCTT | 174_1 | 3122 | -17 |
| 175 | ataaccttatcataca | 3-11-2 | ATAaccttatcataCA | 175_1 | 3174 | -16 |
| 176 | tataaccttatcatac | 2-11-3 | TAtaaccttatcaTAC | 176_1 | 3175 | -14 |
| 177 | ttataaccttatcata | 3-11-2 | TTAtaaccttatcaTA | 177_1 | 3176 | -14 |
| 178 | tttataaccttatcat | 3-10-3 | TTTataaccttatCAT | 178_1 | 3177 | -16 |
| 179 | actgctattgctatct | 2-11-3 | ACtgctattgctaTCT | 179_1 | 3375 | -19 |
| 180 | aggactgctattgcta | 2-11-3 | AGgactgctattgCTA | 180_1 | 3378 | -21 |
| 181 | gaggactgctattgct | 3-11-2 | GAGgactgctattgCT | 181_1 | 3379 | -22 |
| 182 | acgtagaataataaca | 2-12-2 | ACgtagaataataaCA | 182_1 | 3561 | -11 |
| 183 | ccaagtgatataatgg | 2-10-4 | CCaagtgatataATGG | 183_1 | 3613 | -19 |
| 184 | ttagcagaccaagtga | 2-10-4 | TTagcagaccaaGTGA | 184_1 | 3621 | -21 |
| 185 | gtttagcagaccaagt | 2-12-2 | GTttagcagaccaaGT | 185_1 | 3623 | -19 |
| 186 | tgacagtgattatatt | 2-12-2 | TGacagtgattataTT | 186_1 | 3856 | -13 |
| 187 | tgtccaagatattgac | 4-10-2 | TGTCcaagatattgAC | 187_1 | 3868 | -18 |
| 188 | gaatatcctagattgt | 3-10-3 | GAAtatcctagatTGT | 188_1 | 4066 | -18 |
| 189 | caaactgagaatatcc | 2-11-3 | CAaactgagaataTCC | 189_1 | 4074 | -16 |
| 190 | gcaaactgagaatatc | 3-11-2 | GCAaactgagaataTC | 190_1 | 4075 | -16 |
| 191 | tcctattacaatcgta | 3-11-2 | TCCtattacaatcgTA | 191_1 | 4214 | -19 |
| 192 | ttcctattacaatcgt | 4-10-2 | TTCCtattacaatcGT | 192_1 | 4215 | -19 |
| 193 | actaatgggaggattt | 2-12-2 | ACtaatgggaggatTT | 193_1 | 4256 | -15 |
| 194 | tagttcagagaataag | 2-12-2 | TAgttcagagaataAG | 194_1 | 4429 | -13 |
| 195 | taacatatagttcaga | 2-11-3 | TAacatatagttcAGA | 195_1 | 4436 | -15 |
| 196 | ataacatatagttcag | 3-11-2 | AT AacatatagttcAG | 196_1 | 4437 | -14 |
| 197 | cataacatatagttca | 2-12-2 | CAtaacatatagttCA | 197_1 | 4438 | -13 |
| 198 | tcataacatatagttc | 2-12-2 | TCataacatatagtTC | 198_1 | 4439 | -12 |
| 199 | tagctcctaacaatca | 4-10-2 | TAG CtcctaacaatCA | 199_1 | 4507 | -22 |
| 200 | ctccaatctttgtata | 4-10-2 | CTCCaatctttgtaTA | 200_1 | 4602 | -20 |
| 201 | tctccaatctttgtat | 4-10-2 | TCTCcaatctttgtAT | 201_1 | 4603 | -19 |
| 202 | tctatttcagccaatc | 2-12-2 | TCtatttcagccaaTC | 202_1 | 4708 | -17 |
| 203 | cggaagtcagagtgaa | 3-10-3 | CGGaagtcagagtGAA | 203_1 | 4782 | -19 |
| 204 | ttaagcatgaggaata | 4-10-2 | TTAAgcatgaggaaTA | 204_1 | 4798 | -16 |
| 205 | tgattgagcacctctt | 3-10-3 | TGAttgagcacctCTT | 205_1 | 4831 | -22 |
| 206 | gactaattatttcgtt | 3-11-2 | GACtaattatttcgTT | 206_1 | 4857 | -15 |
| 207 | tgactaattatttcgt | 3-10-3 | TGActaattatttCGT | 207_1 | 4858 | -17 |
| 208 | gtgactaattatttcg | 3-10-3 | GTGactaattattTCG | 208_1 | 4859 | -17 |
| 209 | ctgcttgaaatgtgac | 4-10-2 | CTGCttgaaatgtgAC | 209_1 | 4870 | -20 |
| 210 | cctgcttgaaatgtga | 2-11-3 | CCtgcttgaaatgTGA | 210_1 | 4871 | -21 |
| 211 | atcctgcttgaaatgt | 2-10-4 | ATcctgcttgaaATGT | 211_1 | 4873 | -20 |
| 212 | attataaatctattct | 3-10-3 | ATTataaatctatTCT | 212_1 | 5027 | -13 |
| 213 | gctaaatactttcatc | 2-11-3 | GCtaaatactttcATC | 213_1 | 5151 | -16 |
| 214 | cattgtaacataccta | 2-10-4 | CAttgtaacataCCTA | 214_1 | 5251 | -19 |
| 215 | gcattgtaacatacct | 2-12-2 | GCattgtaacatacCT | 215_1 | 5252 | -18 |
| 216 | taatattgcaccaaat | 2-12-2 | TAatattgcaccaaAT | 216_1 | 5295 | -13 |
| 217 | gataatattgcaccaa | 2-11-3 | GAtaatattgcacCAA | 217_1 | 5297 | -16 |
| 218 | agataatattgcacca | 2-12-2 | AGaatattgcacCA | 218_1 | 5298 | -16 |
| 219 | gccaagaagataatat | 2-10-4 | GCcaagaagataATAT | 219_1 | 5305 | -17 |
| 220 | cacagccacataaact | 4-10-2 | CACAgccacataaaCT | 220_1 | 5406 | -21 |
| 221 | ttgtaattgtggaaac | 2-12-2 | TTgtaattgtggaaAC | 221_1 | 5463 | -12 |
| 222 | tgacttgtaattgtgg | 2-11-3 | TGacttgtaattgTGG | 222_1 | 5467 | -18 |
| 223 | tctaactgaaatagtc | 2-12-2 | TCtaactgaaatagTC | 223_1 | 5503 | -13 |
| 224 | gtggttctaactgaaa | 3-11-2 | GTGgttctaactgaAA | 224_1 | 5508 | -16 |
| 225 | caatatgggacttggt | 2-12-2 | CAatatgggacttgGT | 225_1 | 5522 | -18 |
| 226 | atgacaatatgggact | 3-11-2 | ATGacaatatgggaCT | 226_1 | 5526 | -17 |
| 227 | tatgacaatatgggac | 4-10-2 | TATGacaatatgggAC | 227_1 | 5527 | -17 |
| 228 | atatgacaatatggga | 4-10-2 | ATATgacaatatggGA | 228_1 | 5528 | -17 |
| 229 | cttcacttaataatta | 2-11-3 | CTtcacttaataaTTA | 229_1 | 5552 | -13 |
| 230 | ctgcttcacttaataa | 4-10-2 | CTGCttcacttaatAA | 230_1 | 5555 | -18 |
| 231 | aagactgcttcactta | 2-11-3 | AAgactgcttcacTTA | 231_1 | 5559 | -17 |
| 232 | gaatgccctaattatg | 4-10-2 | GAATgccctaattaTG | 232_1 | 5589 | -19 |
| 233 | tggaatgccctaatta | 3-11-2 | TGGaatgccctaatTA | 233_1 | 5591 | -19 |
| 234 | gcaaatgccagtaggt | 3-11-2 | GCAaatgccagtagGT | 234_1 | 5642 | -23 |
| 235 | ctaatggaaggatttg | 3-11-2 | CTAatggaaggattTG | 235_1 | 5673 | -15 |
| 236 | aatatagaacctaatg | 2-12-2 | AAtatagaacctaaTG | 236_1 | 5683 | -10 |
| 237 | gaaagaatagaatgtt | 3-10-3 | GAAagaatagaatGTT | 237_1 | 5769 | -12 |
| 238 | atgggtaatagattat | 3-11-2 | ATGggtaatagattAT | 238_1 | 5893 | -15 |
| 239 | gaaagagcacagggtg | 2-12-2 | GAaagagcacagggTG | 239_1 | 6103 | -18 |
| 240 | ctacatagagggaatg | 4-10-2 | CTACatagagggaaTG | 240_1 | 6202 | -18 |
| 241 | gcttcctacatagagg | 2-10-4 | GCttcctacataGAGG | 241_1 | 6207 | -24 |
| 242 | tgcttcctacatagag | 4-10-2 | TGCTtcctacatagAG | 242_1 | 6208 | -22 |
| 243 | tgggcttgaaatatgt | 2-11-3 | TGggcttgaaataTGT | 243_1 | 6417 | -19 |
| 244 | cattatatttaagaac | 3-11-2 | CATtatatttaagaAC | 244_1 | 6457 | -11 |
| 245 | tcggttatgttatcat | 2-10-4 | TCggttatgttaTCAT | 245_1 | 6470 | -19 |
| 246 | cactttatctggtcgg | 2-10-4 | CActttatctggTCGG | 246_1 | 6482 | -22 |
| 247 | aaattggcacagcgtt | 3-10-3 | AAAttggcacagcGTT | 247_1 | 6505 | -18 |
| 248 | accgtgacagtaaatg | 4-9-3 | ACCGtgacagtaaATG | 248_1 | 6577 | -20 |
| 249 | tgggaaccgtgacagta | 2-13-2 | TGggaaccgtgacagTA | 249_1 | 6581 | -22 |
| 250 | ccacatataggtcctt | 2-11-3 | CCacatataggtcCTT | 250_1 | 6597 | -21 |
| 251 | catattgctaccatac | 2-11-3 | CAtattgctaccaTAC | 251_1 | 6617 | -18 |
| 252 | tcatattgctaccata | 3-10-3 | TCAtattgctaccATA | 252_1 | 6618 | -19 |
| 253 | caattgtcatattgct | 4-8-4 | CAATtgtcatatTGCT | 253_1 | 6624 | -21 |
| 254 | cattcaattgtcatattg | 3-12-3 | CATtcaattgtcataTTG | 254_1 | 6626 | -18 |
| 255 | tttctactgggaatttg | 4-9-4 | TTTCtactgggaaTTTG | 255_1 | 6644 | -20 |
| 256 | caattagtgcagccag | 3-10-3 | CAAttagtgcagcCAG | 256_1 | 6672 | -21 |
| 257 | gaataatgttcttatcc | 4-10-3 | GAATaatgttcttaTCC | 257_1 | 6704 | -20 |
| 258 | cacaaattgaataatgttct | 4-13-3 | CACAaattgaataatgtTCT | 258_1 | 6709 | -20 |
| 259 | catgcacaaattgaataat | 4-11-4 | CATGcacaaattgaaTAAT | 259_1 | 6714 | -20 |
| 260 | atcctgcaatttcacat | 3-11-3 | ATCctgcaatttcaCAT | 260_1 | 6832 | -22 |
| 261 | ccaccatagctgatca | 2-12-2 | CCaccatagctgatCA | 261_1 | 6868 | -22 |
| 262 | accaccatagctgatca | 2-12-3 | ACcaccatagctgaTCA | 262_1 | 6868 | -23 |
| 263 | caccaccatagctgatc | 2-13-2 | CAccaccatagctgaTC | 263_1 | 6869 | -21 |
| 264 | tagtcggcaccaccat | 2-12-2 | TAgtcggcaccaccAT | 264_1 | 6877 | -22 |
| 265 | cttgtagtcggcaccac | 1-14-2 | CttgtagtcggcaccAC | 265_1 | 6880 | -21 |
| 266 | cttgtagtcggcacca | 1-13-2 | CttgtagtcggcacCA | 266_1 | 6881 | -21 |
| 267 | cgcttgtagtcggcac | 2-12-2 | CGcttgtagtcggcAC | 267_1 | 6883 | -21 |
| 268 | tcaataaagatcaggc | 3-11-2 | TCAataaagatcagGC | 268_1 | 6942 | -17 |
| 269 | tggacttacaagaatg | 2-12-2 | TGgacttacaagaaTG | 269_1 | 6986 | -14 |
| 270 | atggacttacaagaat | 3-11-2 | ATGgacttacaagaAT | 270_1 | 6987 | -15 |
| 271 | gctcaagaaattggat | 4-10-2 | GCTCaagaaattggAT | 271_1 | 7073 | -19 |
| 272 | tactgtagaacatggc | 4-10-2 | TACTgtagaacatgGC | 272_1 | 7133 | -21 |
| 273 | gcaattcatttgatct | 4-9-3 | GCAAttcatttgaTCT | 273_1 | 7239 | -20 |
| 274 | tgaagggaggagggacac | 2-14-2 | TGaagggaggagggacAC | 274_1 | 7259 | -20 |
| 275 | agtggtgaagggaggag | 2-13-2 | AGtggtgaagggaggAG | 275_1 | 7265 | -21 |
| 276 | tagtggtgaagggaggag | 2-14-2 | TAgtggtgaagggaggAG | 276_1 | 7265 | -21 |
| 277 | atagtggtgaagggaggag | 1-16-2 | AtagtggtgaagggaggAG | 277_1 | 7265 | -20 |
| 278 | tagtggtgaagggagga | 2-13-2 | TAgtggtgaagggagGA | 278_1 | 7266 | -21 |
| 279 | atagtggtgaagggagga | 2-14-2 | ATagtggtgaagggagGA | 279_1 | 7266 | -21 |
| 280 | tagtggtgaagggagg | 3-11-2 | TAGtggtgaagggaGG | 280_1 | 7267 | -21 |
| 281 | atagtggtgaagggagg | 3-12-2 | ATAgtggtgaagggaGG | 281_1 | 7267 | -22 |
| 282 | gatagtggtgaagggagg | 2-14-2 | GAtagtggtgaagggaGG | 282_1 | 7267 | -21 |
| 283 | atagtggtgaagggag | 4-10-2 | ATAGtggtgaagggAG | 283_1 | 7268 | -20 |
| 284 | gatagtggtgaagggag | 2-12-3 | GAtagtggtgaaggGAG | 284_1 | 7268 | -21 |
| 285 | gagatagtggtgaagg | 2-10-4 | GAgatagtggtgAAGG | 285_1 | 7271 | -20 |
| 286 | catgggagatagtggt | 4-10-2 | CATGggagatagtgGT | 286_1 | 7276 | -22 |
| 287 | acaaataatggttactct | 4-10-4 | ACAAataatggttaCTCT | 287_1 | 7302 | -20 |
| 288 | acacacaaataatggtta | 4-10-4 | ACACacaaataatgGTTA | 288_1 | 7306 | -20 |
| 289 | gagggacacacaaataat | 3-11-4 | GAGggacacacaaaTAAT | 289_1 | 7311 | -21 |
| 290 | atatagagaggctcaa | 4-8-4 | ATATagagaggcTCAA | 290_1 | 7390 | -21 |
| 291 | ttgatatagagaggct | 2-10-4 | TTgatatagagaGGCT | 291_1 | 7393 | -20 |
| 292 | gcatttgatatagaga | 4-9-3 | GCATttgatatagAGA | 292_1 | 7397 | -20 |
| 293 | tttgcatttgatatag | 2-11-3 | TTtgcatttgataTAG | 293_1 | 7400 | -15 |
| 294 | ctggaagaataggttc | 3-11-2 | CTGgaagaataggtTC | 294_1 | 7512 | -17 |
| 295 | actggaagaataggtt | 4-10-2 | ACTGgaagaataggTT | 295_1 | 7513 | -18 |
| 296 | tactggaagaataggt | 4-10-2 | TACTggaagaatagGT | 296_1 | 7514 | -18 |
| 297 | tggcttatcctgtact | 4-10-2 | TGGCttatcctgtaCT | 297_1 | 7526 | -25 |
| 298 | atggcttatcctgtac | 2-10-4 | ATggcttatcctGTAC | 298_1 | 7527 | -22 |
| 299 | tatggcttatcctgta | 4-10-2 | TATGgcttatcctgTA | 299_1 | 7528 | -22 |
| 300 | gtatggcttatcctgt | 3-10-3 | GTAtggcttatccTGT | 300_1 | 7529 | -23 |
| 301 | atgaatatatgcccagt | 2-11-4 | ATgaatatatgccCAGT | 301_1 | 7547 | -22 |
| 302 | gatgaatatatgccca | 2-10-4 | GAtgaatatatgCCCA | 302_1 | 7549 | -22 |
| 303 | caagatgaatatatgcc | 3-10-4 | CAAgatgaatataTGCC | 303_1 | 7551 | -21 |
| 304 | gacaacatcagtataga | 4-9-4 | GACAacatcagtaTAGA | 304_1 | 7572 | -22 |
| 305 | caagacaacatcagta | 4-8-4 | CAAGacaacatcAGTA | 305_1 | 7576 | -20 |
| 306 | cactcctagttccttt | 3-10-3 | CACtcctagttccTTT | 306_1 | 7601 | -22 |
| 307 | aacactcctagttcct | 3-10-3 | AACactcctagttCCT | 307_1 | 7603 | -22 |
| 308 | taacactcctagttcc | 2-11-3 | TAacactcctagtTCC | 308_1 | 7604 | -20 |
| 309 | ctaacactcctagttc | 2-12-2 | CTaacactcctagtTC | 309_1 | 7605 | -18 |
| 310 | tgataacataactgtg | 2-12-2 | TGataacataactgTG | 310_1 | 7637 | -13 |
| 311 | ctgataacataactgt | 2-10-4 | CTgataacataaCTGT | 311_1 | 7638 | -18 |
| 312 | tttgaactcaagtgac | 4-10-2 | TTTGaactcaagtgAC | 312_1 | 7654 | -16 |
| 313 | tcctttacttagctag | 4-9-3 | TCCTttacttagcTAG | 313_1 | 7684 | -23 |
| 314 | gagtttggattagctg | 2-11-3 | GAgtttggattagCTG | 314_1 | 7764 | -20 |
| 315 | tgggatatgacaggga | 2-11-3 | TGggatatgacagGGA | 315_1 | 7838 | -21 |
| 316 | tgtgggatatgacagg | 4-10-2 | TGTGggatatgacaGG | 316_1 | 7840 | -22 |
| 317 | atatggaagggatatc | 4-10-2 | ATATggaagggataTC | 317_1 | 7875 | -17 |
| 318 | acaggatatggaaggg | 3-10-3 | ACAggatatggaaGGG | 318_1 | 7880 | -21 |
| 319 | atttcaacaggatatgg | 4-9-4 | ATTTcaacaggatATGG | 319_1 | 7885 | -20 |
| 320 | gagtaatttcaacagg | 2-11-3 | GAgtaatttcaacAGG | 320_1 | 7891 | -17 |
| 321 | agggagtaatttcaaca | 4-9-4 | AGGGagtaatttcAACA | 321_1 | 7893 | -22 |
| 322 | attagggagtaatttca | 4-9-4 | ATTAgggagtaatTTCA | 322_1 | 7896 | -21 |
| 323 | cttactattagggagt | 2-10-4 | CTtactattaggGAGT | 323_1 | 7903 | -20 |
| 324 | cagcttactattaggg | 2-11-3 | CAgcttactattaGGG | 324_1 | 7906 | -20 |
| 326 | atttcagcttactattag | 3-11-4 | ATTtcagcttactaTTAG | 326_1 | 7908 | -20 |
| 325 | tcagcttactattagg | 3-10-3 | TCAgcttactattAGG | 325_1 | 7907 | -20 |
| 327 | ttcagcttactattag | 2-10-4 | TTcagcttactaTTAG | 327_1 | 7908 | -17 |
| 328 | cagatttcagcttact | 4-10-2 | CAGAtttcagcttaCT | 328_1 | 7913 | -21 |
| 329 | gactacaactagaggg | 3-11-2 | GACtacaactagagGG | 329_1 | 7930 | -19 |
| 330 | agactacaactagagg | 4-10-2 | AGACtacaactagaGG | 330_1 | 7931 | -19 |
| 331 | aagactacaactagag | 2-12-2 | AAgactacaactagAG | 331_1 | 7932 | -13 |
| 332 | atgatttaattctagtcaaa | 4-12-4 | ATGAtttaattctagtCAAA | 332_1 | 7982 | -20 |
| 333 | tttaattctagtcaaa | 3-10-3 | TTTaattctagtcAAA | 333_1 | 7982 | -12 |
| 334 | gatttaattctagtca | 4-8-4 | GATTtaattctaGTCA | 334_1 | 7984 | -20 |
| 771 | tgatttaattctagtca | 3-10-4 | TGAtttaattctaGTCA | 771_1 | 7984 | -20 |
| 335 | atgatttaattctagtca | 4-11-3 | ATGAtttaattctagTCA | 335_1 | 7984 | -20 |
| 336 | gatgatttaattctagtca | 4-13-2 | GATGatttaattctagtCA | 336_1 | 7984 | -20 |
| 337 | gatttaattctagtca | 2-10-4 | GAtttaattctaGTCA | 337_1 | 7984 | -18 |
| 338 | gatgatttaattctagtc | 4-11-3 | GATGatttaattctaGTC | 338_1 | 7985 | -20 |
| 339 | tgatttaattctagtc | 2-12-2 | TGatttaattctagTC | 339_1 | 7985 | -13 |
| 340 | gagatgatttaattcta | 4-9-4 | GAGAtgatttaatTCTA | 340_1 | 7988 | -20 |
| 341 | gagatgatttaattct | 3-10-3 | GAGatgatttaatTCT | 341_1 | 7989 | -16 |
| 342 | cagattgatggtagtt | 4-10-2 | CAGAttgatggtagTT | 342_1 | 8030 | -19 |
| 343 | ctcagattgatggtag | 2-10-4 | CTcagattgatgGTAG | 343_1 | 8032 | -20 |
| 344 | gttagccctcagattg | 3-10-3 | GTTagccctcagaTTG | 344_1 | 8039 | -23 |
| 345 | tgtattgttagccctc | 2-10-4 | TGtattgttagcCCTC | 345_1 | 8045 | -24 |
| 346 | acttgtattgttagcc | 2-10-4 | ACttgtattgttAGCC | 346_1 | 8048 | -22 |
| 347 | agccagtatcagggac | 3-11-2 | AGCcagtatcagggAC | 347_1 | 8191 | -23 |
| 348 | ttgacaatagtggcat | 2-10-4 | TTgacaatagtgGCAT | 348_1 | 8213 | -20 |
| 349 | acaagtggtatcttct | 3-10-3 | ACAagtggtatctTCT | 349_1 | 8228 | -19 |
| 350 | aatctactttacaagt | 4-10-2 | AATCtactttacaaGT | 350_1 | 8238 | -16 |
| 351 | cacagtagatgcctgata | 2-12-4 | CAcagtagatgcctGATA | 351_1 | 8351 | -24 |
| 352 | gaacacagtagatgcc | 2-11-3 | GAacacagtagatGCC | 352_1 | 8356 | -21 |
| 353 | cttggaacacagtagat | 4-11-2 | CTTGgaacacagtagAT | 353_1 | 8359 | -20 |
| 354 | atatcttggaacacag | 3-10-3 | ATAtcttggaacaCAG | 354_1 | 8364 | -18 |
| 355 | tctttaatatcttggaac | 3-11-4 | TCTttaatatcttgGAAC | 355_1 | 8368 | -19 |
| 356 | tgatttctttaatatcttg | 2-13-4 | TGatttctttaatatCTTG | 356_1 | 8372 | -19 |
| 357 | tgatgatttctttaatatc | 2-13-4 | TGatgatttctttaaTATC | 357_1 | 8375 | -18 |
| 358 | aggctaagtcatgatg | 3-11-2 | AGGctaagtcatgaTG | 358_1 | 8389 | -19 |
| 359 | ttgatgaggctaagtc | 4-10-2 | TTGAtgaggctaagTC | 359_1 | 8395 | -19 |
| 360 | ccaggattatactctt | 3-11-2 | CCAggattatactcTT | 360_1 | 8439 | -20 |
| 361 | gccaggattatactct | 2-10-4 | GCcaggattataCTCT | 361_1 | 8440 | -23 |
| 362 | ctgccaggattatact | 3-11-2 | CTGccaggattataCT | 362_1 | 8442 | -21 |
| 363 | cagaaacttatactttatg | 4-13-2 | CAGAaacttatactttaTG | 363_1 | 8473 | -19 |
| 364 | aagcagaaacttatact | 4-9-4 | AAGCagaaacttaTACT | 364_1 | 8478 | -20 |
| 365 | gaagcagaaacttatact | 3-11-4 | GAAgcagaaacttaTACT | 365_1 | 8478 | -20 |
| 366 | tggaagcagaaacttatact | 3-15-2 | TGGaagcagaaacttataCT | 366_1 | 8478 | -21 |
| 367 | tggaagcagaaacttatac | 3-13-3 | TGGaagcagaaacttaTAC | 367_1 | 8479 | -20 |
| 368 | aagcagaaacttatac | 2-11-3 | AAgcagaaacttaTAC | 368_1 | 8479 | -13 |
| 369 | tggaagcagaaacttata | 3-11-4 | TGGaagcagaaactTATA | 369_1 | 8480 | -21 |
| 370 | aagggatattatggag | 4-10-2 | AAGGgatattatggAG | 370_1 | 8587 | -18 |
| 371 | tgccggaagatttcct | 2-12-2 | TGccggaagatttcCT | 371_1 | 8641 | -21 |
| 372 | atggattgggagtaga | 4-10-2 | ATGGattgggagtaGA | 372_1 | 8772 | -21 |
| 373 | agatggattgggagta | 2-12-2 | AGatggattgggagTA | 373_1 | 8774 | -18 |
| 374 | aagatggattgggagt | 3-11-2 | AAGatggattgggaGT | 374_1 | 8775 | -18 |
| 375 | acaagatggattggga | 2-10-4 | ACaagatggattGGGA | 375_1 | 8777 | -20 |
| 375 | acaagatggattggga | 2-12-2 | ACaagatggattggGA | 375_2 | 8777 | -17 |
| 376 | agaaggttcagacttt | 3-9-4 | AGAaggttcagaCTTT | 376_1 | 8835 | -20 |
| 377 | gcagaaggttcagact | 2-11-3 | GCagaaggttcagACT | 377 1 | 8837 | -21 |
| 377 | gcagaaggttcagact | 3-11-2 | GCAgaaggttcagaCT | 377_2 | 8837 | -22 |
| 378 | tgcagaaggttcagac | 4-10-2 | TGCAgaaggttcagAC | 378_1 | 8838 | -22 |
| 379 | agtgcagaaggttcag | 2-11-3 | AGtgcagaaggttCAG | 379_1 | 8840 | -20 |
| 379 | agtgcagaaggttcag | 4-10-2 | AGTGcagaaggttcAG | 379_2 | 8840 | -21 |
| 380 | aagtgcagaaggttca | 4-10-2 | AAGTgcagaaggttCA | 380_1 | 8841 | -20 |
| 381 | taagtgcagaaggttc | 2-10-4 | TAagtgcagaagGTTC | 381_1 | 8842 | -19 |
| 382 | tctaagtgcagaaggt | 2-10-4 | TCtaagtgcagaAGGT | 382_1 | 8844 | -21 |
| 383 | ctcaggagttctacttc | 3-12-2 | CTCaggagttctactTC | 383_1 | 8948 | -20 |
| 384 | ctcaggagttctactt | 3-10-3 | CTCaggagttctaCTT | 384_1 | 8949 | -21 |
| 385 | atggaggtgactcaggag | 1-15-2 | AtggaggtgactcaggAG | 385_1 | 8957 | -20 |
| 386 | atggaggtgactcagga | 2-13-2 | ATggaggtgactcagGA | 386_1 | 8958 | -21 |
| 387 | atggaggtgactcagg | 2-11-3 | ATggaggtgactcAG G | 387_1 | 8959 | -21 |
| 388 | tatggaggtgactcagg | 2-12-3 | TAtggaggtgactcAGG | 388_1 | 8959 | -21 |
| 389 | atatggaggtgactcagg | 2-14-2 | ATatggaggtgactcaGG | 389_1 | 8959 | -21 |
| 390 | tatggaggtgactcag | 4-10-2 | TATGgaggtgactcAG | 390_1 | 8960 | -21 |
| 391 | atatggaggtgactcag | 2-11-4 | ATatggaggtgacTCAG | 391_1 | 8960 | -22 |
| 392 | catatggaggtgactcag | 2-14-2 | CAtatggaggtgactcAG | 392_1 | 8960 | -20 |
| 393 | atatggaggtgactca | 3-10-3 | ATAtggaggtgacTCA | 393_1 | 8961 | -20 |
| 394 | catatggaggtgactca | 2-12-3 | CAtatggaggtgacTCA | 394_1 | 8961 | -21 |
| 395 | catatggaggtgactc | 2-10-4 | CAtatggaggtgACTC | 395_1 | 8962 | -20 |
| 396 | gcatatggaggtgactc | 2-13-2 | GCatatggaggtgacTC | 396_1 | 8962 | -21 |
| 397 | tgcatatggaggtgactc | 2-14-2 | TGcatatggaggtgacTC | 397_1 | 8962 | -21 |
| 398 | ttgcatatggaggtgactc | 1-16-2 | TtgcatatggaggtgacTC | 398_1 | 8962 | -20 |
| 399 | tttgcatatggaggtgactc | 1-17-2 | TttgcatatggaggtgacTC | 399_1 | 8962 | -21 |
| 400 | gcatatggaggtgact | 2-12-2 | GCatatggaggtgaCT | 400_1 | 8963 | -20 |
| 401 | tgcatatggaggtgact | 2-13-2 | TGcatatggaggtgaCT | 401_1 | 8963 | -20 |
| 402 | ttgcatatggaggtgact | 3-13-2 | TTGcatatggaggtgaCT | 402_1 | 8963 | -22 |
| 403 | tttgcatatggaggtgact | 1-16-2 | TttgcatatggaggtgaCT | 403_1 | 8963 | -20 |
| 404 | tgcatatggaggtgac | 3-11-2 | TGCatatggaggtgAC | 404_1 | 8964 | -20 |
| 405 | ttgcatatggaggtgac | 3-11-3 | TTGcatatggaggtGAC | 405_1 | 8964 | -21 |
| 406 | tttgcatatggaggtgac | 4-12-2 | TTTGcatatggaggtgAC | 406_1 | 8964 | -21 |
| 407 | tttgcatatggaggtga | 4-11-2 | TTTGcatatggaggtGA | 407_1 | 8965 | -21 |
| 408 | tttgcatatggaggtg | 2-10-4 | TTtgcatatggaGGTG | 408_1 | 8966 | -21 |
| 409 | aagtgaagttcaacagc | 2-11-4 | AAgtgaagttcaaCAGC | 409_1 | 8997 | -20 |
| 410 | tgggaagtgaagttca | 2-10-4 | TGggaagtgaagTTCA | 410_1 | 9002 | -20 |
| 411 | atgggaagtgaagttc | 2-11-3 | ATgggaagtgaagTTC | 411_1 | 9003 | -17 |
| 412 | gatgggaagtgaagtt | 4-9-3 | GATGggaagtgaaGTT | 412_1 | 9004 | -21 |
| 413 | ctgtgatgggaagtgaa | 3-11-3 | CTGtgatgggaagtGAA | 413_1 | 9007 | -20 |
| 414 | attgagtgaatccaaa | 3-10-3 | ATTgagtgaatccAAA | 414_1 | 9119 | -14 |
| 415 | aattgagtgaatccaa | 2-10-4 | AAttgagtgaatCCAA | 415_1 | 9120 | -16 |
| 416 | gataattgagtgaatcc | 4-10-3 | GATAattgagtgaaTCC | 416_1 | 9122 | -20 |
| 417 | gtgataattgagtgaa | 3-10-3 | GTGataattgagtGAA | 417_1 | 9125 | -16 |
| 418 | aagaaaggtgcaataa | 3-10-3 | AAGaaaggtgcaaTAA | 418_1 | 9155 | -14 |
| 419 | caagaaaggtgcaata | 2-10-4 | CAagaaaggtgcAATA | 419_1 | 9156 | -15 |
| 420 | acaagaaaggtgcaat | 4-10-2 | ACAAgaaaggtgcaAT | 420_1 | 9157 | -16 |
| 421 | atttaaactcacaaac | 2-12-2 | ATttaaactcacaaAC | 421_1 | 9171 | -10 |
| 422 | ctgttaggttcagcga | 2-10-4 | CTgttaggttcaGCGA | 422_1 | 9235 | -24 |
| 423 | tctgaatgaacatttcg | 4-9-4 | TCTGaatgaacatTTCG | 423_1 | 9260 | -20 |
| 424 | ctcattgaaggttctg | 2-10-4 | CTcattgaaggtTCTG | 424_1 | 9281 | -20 |
| 425 | ctaatctcattgaagg | 3-11-2 | CTAatctcattgaaGG | 425_1 | 9286 | -17 |
| 426 | cctaatctcattgaag | 2-12-2 | CCtaatctcattgaAG | 426_1 | 9287 | -16 |
| 427 | actttgatctttcagc | 3-10-3 | ACTttgatctttcAGC | 427_1 | 9305 | -20 |
| 428 | actatgcaacactttg | 2-12-2 | ACtatgcaacacttTG | 428_1 | 9315 | -15 |
| 429 | caaatagctttatcgg | 3-10-3 | CAAatagctttatCGG | 429_1 | 9335 | -17 |
| 430 | ccaaatagctttatcg | 2-10-4 | CCaaatagctttATCG | 430_1 | 9336 | -19 |
| 431 | tccaaatagctttatc | 4-10-2 | TCCAaatagctttaTC | 431_1 | 9337 | -18 |
| 432 | gatccaaatagcttta | 4-10-2 | GATCcaaatagcttTA | 432_1 | 9339 | -18 |
| 433 | atgatccaaatagctt | 2-10-4 | ATgatccaaataGCTT | 433_1 | 9341 | -19 |
| 434 | tatgatccaaatagct | 4-10-2 | TATGatccaaatagCT | 434_1 | 9342 | -18 |
| 435 | taaacagggctgggaat | 4-9-4 | TAAAcagggctggGAAT | 435_1 | 9408 | -22 |
| 436 | acttaaacagggctgg | 2-10-4 | ACttaaacagggCTGG | 436_1 | 9412 | -21 |
| 437 | acacttaaacagggct | 2-10-4 | ACacttaaacagGGCT | 437_1 | 9414 | -22 |
| 438 | gaacacttaaacaggg | 4-8-4 | GAACacttaaacAGGG | 438_1 | 9416 | -20 |
| 439 | agagaacacttaaacag | 4-9-4 | AGAGaacacttaaACAG | 439_1 | 9418 | -20 |
| 440 | ctacagagaacactta | 4-8-4 | CTACagagaacaCTTA | 440_1 | 9423 | -20 |
| 441 | atgctacagagaacact | 3-10-4 | ATGctacagagaaCACT | 441_1 | 9425 | -22 |
| 442 | ataaatgctacagagaaca | 4-11-4 | ATAAatgctacagagAACA | 442_1 | 9427 | -20 |
| 443 | agataaatgctacagaga | 2-12-4 | AGataaatgctacaGAGA | 443_1 | 9430 | -20 |
| 444 | tagagataaatgctaca | 4-9-4 | TAGAgataaatgcTACA | 444_1 | 9434 | -21 |
| 445 | tagatagagataaatgct | 4-11-3 | TAGAtagagataaatGCT | 445_1 | 9437 | -20 |
| 446 | caatatactagatagaga | 4-10-4 | CAATatactagataGAGA | 446_1 | 9445 | -21 |
| 447 | tacacaatatactagatag | 4-11-4 | TACAcaatatactagATAG | 447_1 | 9448 | -21 |
| 448 | ctacacaatatactag | 3-10-3 | CTAcacaatatacTAG | 448_1 | 9452 | -16 |
| 449 | gctacacaatatacta | 4-8-4 | GCTAcacaatatACTA | 449_1 | 9453 | -21 |
| 450 | atatgctacacaatatac | 4-10-4 | ATATgctacacaatATAC | 450_1 | 9455 | -20 |
| 451 | tgatatgctacacaat | 4-8-4 | TGATatgctacaCAAT | 451_1 | 9459 | -20 |
| 452 | atgatatgatatgctac | 4-9-4 | ATGAtatgatatgCTAC | 452_1 | 9464 | -21 |
| 453 | gaggagagagacaataaa | 4-10-4 | GAGGagagagacaaTAAA | 453_1 | 9495 | -20 |
| 454 | ctaggaggagagagaca | 3-11-3 | CTAggaggagagagACA | 454_1 | 9500 | -22 |
| 455 | tattctaggaggagaga | 4-10-3 | TATTctaggaggagAGA | 455_1 | 9504 | -21 |
| 456 | ttatattctaggaggag | 4-10-3 | TTATattctaggagGAG | 456_1 | 9507 | -21 |
| 457 | gtttatattctaggag | 3-9-4 | GTTtatattctaGGAG | 457_1 | 9510 | -20 |
| 458 | tggagtttatattctagg | 2-12-4 | TGgagtttatattcTAGG | 458_1 | 9512 | -22 |
| 459 | cgtaccaccactctgc | 2-11-3 | CGtaccaccactcTGC | 459_1 | 9590 | -25 |
| 460 | tgaggaaatcattcattc | 4-10-4 | TGAGgaaatcattcATTC | 460_1 | 9641 | -22 |
| 461 | tttgaggaaatcattcat | 4-10-4 | TTTGaggaaatcatTCAT | 461_1 | 9643 | -20 |
| 462 | aggctaatcctatttg | 4-10-2 | AGGCtaatcctattTG | 462_1 | 9657 | -22 |
| 463 | tttaggctaatcctat | 4-8-4 | TTTAggctaatcCTAT | 463_1 | 9660 | -22 |
| 464 | tgctccagtgtaccct | 3-11-2 | TGCtccagtgtaccCT | 464_1 | 9755 | -27 |
| 465 | tagtagtactcgatag | 2-10-4 | TAgtagtactcgATAG | 465_1 | 9813 | -18 |
| 466 | ctaattgtagtagtactc | 3-12-3 | CTAattgtagtagtaCTC | 466_1 | 9818 | -20 |
| 467 | tgctaattgtagtagt | 2-10-4 | TGctaattgtagTAGT | 467_1 | 9822 | -19 |
| 468 | agtgctaattgtagta | 4-10-2 | AGTGctaattgtagTA | 468_1 | 9824 | -19 |
| 469 | gcaagtgctaattgta | 4-10-2 | GCAAgtgctaattgTA | 469_1 | 9827 | -20 |
| 470 | gaggaaatgaactaattta | 4-13-2 | GAGGaaatgaactaattTA | 470_1 | 9881 | -18 |
| 471 | caggaggaaatgaacta | 4-11-2 | CAGGaggaaatgaacTA | 471_1 | 9886 | -19 |
| 472 | ccctagagtcatttcc | 2-11-3 | CCctagagtcattTCC | 472_1 | 9902 | -24 |
| 473 | atcttacatgatgaagc | 3-11-3 | ATCttacatgatgaAGC | 473_1 | 9925 | -20 |
| 475 | agacacactcagatttcag | 2-15-2 | AG acacactcagatttcAG | 475_1 | 9967 | -20 |
| 474 | gacacactcagatttcag | 3-13-2 | GACacactcagatttcAG | 474_1 | 9967 | -20 |
| 476 | aagacacactcagatttcag | 3-15-2 | AAGacacactcagatttcAG | 476_1 | 9967 | -21 |
| 477 | agacacactcagatttca | 2-13-3 | AG acacactcagattTCA | 477_1 | 9968 | -20 |
| 478 | aagacacactcagatttca | 3-13-3 | AAGacacactcagattTCA | 478_1 | 9968 | -21 |
| 479 | aaagacacactcagatttca | 2-14-4 | AAagacacactcagatTTCA | 479_1 | 9968 | -20 |
| 480 | gaaagacacactcagatttc | 3-14-3 | GAAagacacactcagatTTC | 480_1 | 9969 | -20 |
| 481 | aagacacactcagatttc | 4-11-3 | AAGAcacactcagatTTC | 481_1 | 9969 | -21 |
| 482 | aaagacacactcagatttc | 4-11-4 | AAAGacacactcagaTTTC | 482_1 | 9969 | -20 |
| 483 | tgaaagacacactcagattt | 4-14-2 | TGAAagacacactcagatTT | 483_1 | 9970 | -20 |
| 484 | tgaaagacacactcagatt | 2-13-4 | TGaaagacacactcaGATT | 484_1 | 9971 | -21 |
| 485 | tgaaagacacactcagat | 3-12-3 | TGAaagacacactcaGAT | 485_1 | 9972 | -20 |
| 486 | attgaaagacacactca | 4-10-3 | ATTGaaagacacacTCA | 486_1 | 9975 | -19 |
| 487 | tcattgaaagacacact | 2-11-4 | TCattgaaagacaCACT | 487_1 | 9977 | -18 |
| 488 | ttccatcattgaaaga | 3-9-4 | TTCcatcattgaAAGA | 488_1 | 9983 | -18 |
| 489 | ataataccacttatcat | 4-9-4 | ATAAtaccacttaTCAT | 489_1 | 10010 | -20 |
| 490 | ttacttaatttctttgga | 2-12-4 | TTacttaatttcttTGGA | 490_1 | 10055 | -20 |
| 491 | ttagaactagctttatca | 3-12-3 | TTAgaactagctttaTCA | 491_1 | 10101 | -20 |
| 492 | gaggtacaaatatagg | 3-10-3 | GAGgtacaaatatAGG | 492_1 | 10171 | -18 |
| 493 | cttatgatacaactta | 3-10-3 | CTTatgatacaacTTA | 493_1 | 10384 | -15 |
| 494 | tcttatgatacaactt | 2-11-3 | TCttatgatacaaCTT | 494_1 | 10385 | -15 |
| 495 | ttcttatgatacaact | 3-11-2 | TTCttatgatacaaCT | 495_1 | 10386 | -15 |
| 496 | cagtttcttatgatac | 2-11-3 | CAgtttcttatgaTAC | 496_1 | 10390 | -16 |
| 497 | gcagtttcttatgata | 3-11-2 | GCAgtttcttatgaTA | 497_1 | 10391 | -19 |
| 498 | tacaaatgtctattaggtt | 4-12-3 | TACAaatgtctattagGTT | 498_1 | 10457 | -21 |
| 499 | tgtacaaatgtctattag | 4-11-3 | TGTAcaaatgtctatTAG | 499_1 | 10460 | -20 |
| 500 | agcatcacaattagta | 3-11-2 | AGCatcacaattagTA | 500_1 | 10535 | -18 |
| 501 | ctaatgatagtgaagc | 3-11-2 | CTAatgatagtgaaGC | 501_1 | 10548 | -17 |
| 502 | agctaatgatagtgaa | 3-11-2 | AGCtaatgatagtgAA | 502_1 | 10550 | -16 |
| 503 | atgccttgacatatta | 4-10-2 | ATGCcttgacatatTA | 503_1 | 10565 | -20 |
| 504 | ctcaagattattgacac | 4-9-4 | CTCAagattattgACAC | 504_1 | 10623 | -20 |
| 505 | acctcaagattattga | 2-10-4 | ACctcaagattaTTGA | 505_2 | 10626 | -18 |
| 505 | acctcaagattattga | 3-9-4 | ACCtcaagattaTTGA | 505_1 | 10626 | -20 |
| 506 | aacctcaagattattg | 4-10-2 | AACCtcaagattatTG | 506_1 | 10627 | -17 |
| 507 | cacaaacctcaagattatt | 4-13-2 | CACAaacctcaagattaTT | 507_1 | 10628 | -20 |
| 508 | gtacttaattagacct | 3-9-4 | GTActtaattagACCT | 508_1 | 10667 | -21 |
| 509 | agtacttaattagacc | 4-9-3 | AGTActtaattagACC | 509_1 | 10668 | -20 |
| 510 | gtatgaggtggtaaac | 4-10-2 | GTATgaggtggtaaAC | 510_1 | 10688 | -18 |
| 511 | aggaaacagcagaagtg | 2-11-4 | AGgaaacagcagaAGTG | 511_1 | 10723 | -21 |
| 512 | gcacaacccagaggaa | 2-12-2 | GCacaacccagaggAA | 512_1 | 10735 | -20 |
| 513 | caagcacaacccagag | 3-11-2 | CAAgcacaacccagAG | 513_1 | 10738 | -20 |
| 514 | ttcaagcacaacccag | 3-10-3 | TTCaagcacaaccCAG | 514_1 | 10740 | -21 |
| 515 | aattcaagcacaaccc | 2-10-4 | AAttcaagcacaACCC | 515_1 | 10742 | -20 |
| 516 | taataattcaagcacaacc | 4-13-2 | TAATaattcaagcacaaCC | 516_1 | 10743 | -20 |
| 517 | actaataattcaagcac | 4-9-4 | ACTAataattcaaGCAC | 517_1 | 10747 | -20 |
| 518 | ataatactaataattcaagc | 4-12-4 | ATAAtactaataattcAAGC | 518_1 | 10749 | -19 |
| 519 | tagatttgtgaggtaa | 2-10-4 | TAgatttgtgagGTAA | 519_1 | 11055 | -18 |
| 520 | agccttaattctccat | 4-10-2 | AGCCttaattctccAT | 520_1 | 11091 | -24 |
| 521 | aatgatctagagcctta | 4-9-4 | AATGatctagagcCTTA | 521_1 | 11100 | -22 |
| 522 | ctaatgatctagagcc | 3-10-3 | CTAatgatctagaGCC | 522_1 | 11103 | -22 |
| 523 | actaatgatctagagc | 3-9-4 | ACTaatgatctaGAGC | 523_1 | 11104 | -21 |
| 524 | cattaacatgttcttatt | 3-11-4 | CATtaacatgttctTATT | 524_1 | 11165 | -19 |
| 525 | acaagtacattaacatgttc | 4-12-4 | ACAAgtacattaacatGTTC | 525_1 | 11170 | -22 |
| 526 | ttacaagtacattaacatg | 4-11-4 | TTACaagtacattaaCATG | 526_1 | 11173 | -20 |
| 527 | gctttattcatgtttat | 4-9-4 | GCTTtattcatgtTTAT | 527_1 | 11195 | -22 |
| 528 | gctttattcatgttta | 3-11-2 | GCTttattcatgttTA | 528_1 | 11196 | -18 |
| 529 | agagctttattcatgttt | 3-13-2 | AGAgctttattcatgtTT | 529_1 | 11197 | -20 |
| 530 | ataagagctttattcatg | 4-10-4 | ATAAgagctttattCATG | 530_1 | 11200 | -21 |
| 531 | cataagagctttattca | 4-9-4 | CATAagagctttaTTCA | 531_1 | 11202 | -21 |
| 532 | agcataagagctttat | 4-8-4 | AGCAtaagagctTTAT | 532_1 | 11205 | -22 |
| 533 | tagattgtttagtgca | 3-10-3 | TAGattgtttagtGCA | 533_1 | 11228 | -20 |
| 534 | gtagattgtttagtgc | 2-10-4 | GTagattgtttaGTGC | 534_1 | 11229 | -21 |
| 535 | gacaattctagtagatt | 4-9-4 | GACAattctagtaGATT | 535_1 | 11238 | -21 |
| 536 | ctgacaattctagtag | 3-9-4 | CTGacaattctaGTAG | 536_1 | 11241 | -20 |
| 537 | gctgacaattctagta | 4-10-2 | GCTGacaattctagTA | 537_1 | 11242 | -21 |
| 538 | aggattaagatacgta | 2-12-2 | AGgattaagatacgTA | 538_1 | 11262 | -15 |
| 539 | caggattaagatacgt | 2-11-3 | CAggattaagataCGT | 539_1 | 11263 | -17 |
| 540 | tcaggattaagatacg | 3-11-2 | TCAggattaagataCG | 540_1 | 11264 | -16 |
| 541 | ttcaggattaagatac | 2-10-4 | TTcaggattaagATAC | 541_1 | 11265 | -15 |
| 542 | aggaagaaagtttgattc | 4-10-4 | AGGAagaaagtttgATTC | 542_1 | 11308 | -21 |
| 543 | tcaaggaagaaagtttga | 4-10-4 | TCAAggaagaaagtTTGA | 543_1 | 11311 | -20 |
| 544 | ctcaaggaagaaagtttg | 4-10-4 | CTCAaggaagaaagTTTG | 544_1 | 11312 | -20 |
| 545 | tgctcaaggaagaaagt | 3-10-4 | TGCtcaaggaagaAAGT | 545_1 | 11315 | -21 |
| 546 | aattatgctcaaggaaga | 4-11-3 | AATTatgctcaaggaAGA | 546_1 | 11319 | -20 |
| 547 | taggataccacattatga | 4-12-2 | TAGGataccacattatGA | 547_1 | 11389 | -22 |
| 548 | cataatttattccattcctc | 2-15-3 | CAtaatttattccattcCTC | 548_1 | 11449 | -22 |
| 549 | tgcataatttattccat | 4-10-3 | TGCAtaatttattcCAT | 549_1 | 11454 | -22 |
| 550 | actgcataatttattcc | 4-10-3 | ACTGcataatttatTCC | 550_1 | 11456 | -21 |
| 551 | ctaaactgcataatttatt | 4-11-4 | CTAAactgcataattTATT | 551_1 | 11458 | -20 |
| 552 | ataactaaactgcata | 2-10-4 | ATaactaaactgCATA | 552_1 | 11465 | -16 |
| 553 | ttattaataactaaactgc | 3-12-4 | TTAttaataactaaaCTGC | 553_1 | 11468 | -19 |
| 554 | tagtacattattaataact | 4-13-2 | TAGTacattattaataaCT | 554_1 | 11475 | -18 |
| 555 | cataactaaggacgtt | 4-10-2 | CATAactaaggacgTT | 555_1 | 11493 | -17 |
| 556 | tcataactaaggacgt | 2-11-3 | TCataactaaggaCGT | 556_1 | 11494 | -16 |
| 557 | cgtcataactaaggac | 4-10-2 | CGTCataactaaggAC | 557_1 | 11496 | -17 |
| 558 | tcgtcataactaagga | 2-12-2 | TCgtcataactaagGA | 558_1 | 11497 | -16 |
| 559 | atcgtcataactaagg | 2-10-4 | ATcgtcataactAAGG | 559_1 | 11498 | -17 |
| 560 | gttagtatcttacatt | 2-11-3 | GTtagtatcttacATT | 560_1 | 11525 | -15 |
| 561 | ctctattgttagtatc | 3-10-3 | CTCtattgttagtATC | 561_1 | 11532 | -17 |
| 562 | agtatagagttactgt | 3-10-3 | AGTatagagttacTGT | 562_1 | 11567 | -19 |
| 563 | ttcctggtgatacttt | 4-10-2 | TTCCtggtgatactTT | 563_1 | 11644 | -21 |
| 564 | gttcctggtgatactt | 4-10-2 | GTTCctggtgatacTT | 564_1 | 11645 | -21 |
| 565 | tgttcctggtgatact | 2-12-2 | TGttcctggtgataCT | 565_1 | 11646 | -20 |
| 566 | ataaacatgaatctctcc | 2-12-4 | ATaaacatgaatctCTCC | 566_1 | 11801 | -20 |
| 567 | ctttataaacatgaatctc | 3-12-4 | CTTtataaacatgaaTCTC | 567_1 | 11804 | -19 |
| 568 | ctgtctttataaacatg | 3-10-4 | CTGtctttataaaCATG | 568_1 | 11810 | -19 |
| 569 | ttgttataaatctgtctt | 2-12-4 | TTgttataaatctgTCTT | 569_1 | 11820 | -18 |
| 570 | ttaaatttattcttggata | 3-12-4 | TTAaatttattcttgGATA | 570_1 | 11849 | -19 |
| 571 | cttaaatttattcttgga | 2-12-4 | CTtaaatttattctTGGA | 571_1 | 11851 | -19 |
| 572 | cttcttaaatttattcttg | 4-13-2 | CTTCttaaatttattctTG | 572_1 | 11853 | -18 |
| 573 | tatgtttctcagtaaag | 4-9-4 | TATGtttctcagtAAAG | 573_1 | 11877 | -19 |
| 574 | gaattatctttaaacca | 3-10-4 | GAAttatctttaaACCA | 574_1 | 11947 | -18 |
| 575 | cccttaaatttctaca | 3-11-2 | CCCttaaatttctaCA | 575_1 | 11980 | -20 |
| 576 | acactgctcttgtacc | 4-10-2 | ACACtgctcttgtaCC | 576_1 | 11995 | -23 |
| 577 | tgacaacactgctctt | 3-10-3 | TGAcaacactgctCTT | 577_1 | 12000 | -21 |
| 578 | tacatttattgggctc | 4-10-2 | TACAtttattgggcTC | 578_1 | 12081 | -19 |
| 579 | gtacatttattgggct | 2-10-4 | GTacatttattgGGCT | 579_1 | 12082 | -23 |
| 580 | ttggtacatttattgg | 3-10-3 | TTGgtacatttatTGG | 580_1 | 12085 | -18 |
| 581 | catgttggtacatttat | 4-10-3 | CATGttggtacattTAT | 581_1 | 12088 | -21 |
| 582 | aatcatgttggtacat | 4-10-2 | AATCatgttggtacAT | 582_1 | 12092 | -16 |
| 583 | aaatcatgttggtaca | 2-12-2 | AAatcatgttggtaCA | 583_1 | 12093 | -14 |
| 584 | gacaagtttggattaa | 3-11-2 | GACaagtttggattAA | 584_1 | 12132 | -14 |
| 585 | aatgttcagatgcctc | 2-10-4 | AAtgttcagatgCCTC | 585_1 | 12197 | -21 |
| 586 | gcttaatgttcagatg | 2-12-2 | GCttaatgttcagaTG | 586_1 | 12201 | -17 |
| 587 | cgtacatagcttgatg | 4-10-2 | CGTAcatagcttgaTG | 587_1 | 12267 | -20 |
| 588 | gtgaggaattaggata | 3-11-2 | GTGaggaattaggaTA | 588_1 | 12753 | -17 |
| 589 | gtaacaatatggtttg | 3-11-2 | GTAacaatatggttTG | 589_1 | 12780 | -15 |
| 590 | gaaatattgtagacta | 2-11-3 | GAaatattgtagaCTA | 590_1 | 13151 | -14 |
| 591 | ttgaaatattgtagac | 3-11-2 | TTGaaatattgtagAC | 591_1 | 13153 | -12 |
| 592 | aagtctagtaatttgc | 2-10-4 | AAgtctagtaatTTGC | 592_1 | 13217 | -17 |
| 593 | gctcagtagattataa | 4-10-2 | GCTCagtagattatAA | 593_1 | 13259 | -17 |
| 594 | catacactgttgctaa | 3-10-3 | CATacactgttgcTAA | 594_1 | 13296 | -19 |
| 595 | atggtctcaaatcatt | 3-10-3 | ATGgtctcaaatcATT | 595_1 | 13314 | -17 |
| 596 | caatggtctcaaatca | 4-10-2 | CAATggtctcaaatCA | 596_1 | 13316 | -18 |
| 597 | ttcctattgattgact | 4-10-2 | TTCCtattgattgaCT | 597_1 | 13568 | -20 |
| 598 | tttctgttcacaacac | 4-10-2 | TTTCtgttcacaacAC | 598_1 | 13600 | -17 |
| 599 | aggaacccactaatct | 2-11-3 | AGgaacccactaaTCT | 599_1 | 13702 | -20 |
| 600 | taaatggcaggaaccc | 3-11-2 | TAAatggcaggaacCC | 600_1 | 13710 | -19 |
| 601 | gtaaatggcaggaacc | 4-10-2 | GTAAatggcaggaaCC | 601_1 | 13711 | -20 |
| 602 | ttgtaaatggcaggaa | 2-11-3 | TTgtaaatggcagGAA | 602_1 | 13713 | -16 |
| 603 | ttatgagttaggcatg | 2-10-4 | TTatgagttaggCATG | 603_1 | 13835 | -19 |
| 604 | ccaggtgaaactttaa | 3-11-2 | CCAggtgaaactttAA | 604_1 | 13935 | -17 |
| 605 | cccttagtcagctcct | 3-10-3 | CCCttagtcagctCCT | 605_1 | 13997 | -30 |
| 606 | acccttagtcagctcc | 2-10-4 | ACccttagtcagCTCC | 606_1 | 13998 | -27 |
| 607 | cacccttagtcagctc | 2-11-3 | CAcccttagtcagCTC | 607_1 | 13999 | -24 |
| 608 | tctcttactaggctcc | 3-10-3 | TCTcttactaggcTCC | 608_1 | 14091 | -24 |
| 609 | cctatctgtcatcatg | 2-11-3 | CCtatctgtcatcATG | 609_1 | 14178 | -20 |
| 610 | tcctatctgtcatcat | 3-11-2 | TCCtatctgtcatcAT | 610_1 | 14179 | -20 |
| 611 | gagaagtgtgagaagc | 3-11-2 | GAGaagtgtgagaaGC | 611_1 | 14808 | -19 |
| 612 | catccttgaagtttag | 4-10-2 | CATCcttgaagtttAG | 612_1 | 14908 | -19 |
| 613 | taataagatggctccc | 3-10-3 | TAAtaagatggctCCC | 613_1 | 15046 | -21 |
| 614 | caaggcataataagat | 3-11-2 | CAAggcataataagAT | 614_1 | 15053 | -14 |
| 615 | ccaaggcataataaga | 2-10-4 | CCaaggcataatAAGA | 615_1 | 15054 | -18 |
| 616 | tgatccaattctcacc | 2-12-2 | TGatccaattctcaCC | 616_1 | 15151 | -19 |
| 617 | atgatccaattctcac | 3-10-3 | ATGatccaattctCAC | 617_1 | 15152 | -19 |
| 618 | cgcttcatcttcaccc | 3-11-2 | CGCttcatcttcacCC | 618_1 | 15260 | -26 |
| 619 | tatgacactgcatctt | 2-10-4 | TAtgacactgcaTCTT | 619_1 | 15317 | -19 |
| 620 | gtatgacactgcatct | 3-10-3 | GTAtgacactgcaTCT | 620_1 | 15318 | -21 |
| 621 | tgtatgacactgcatc | 2-10-4 | TGtatgacactgCATC | 621_1 | 15319 | -20 |
| 622 | ttctcttctgtaagtc | 4-10-2 | TTCTcttctgtaagTC | 622_1 | 15363 | -19 |
| 623 | ttctacagaggaacta | 2-10-4 | TTctacagaggaACTA | 623_1 | 15467 | -17 |
| 624 | actacagttctacaga | 3-10-3 | ACTacagttctacAGA | 624_1 | 15474 | -19 |
| 625 | ttcccacaggtaaatg | 4-10-2 | TTCCcacaggtaaaTG | 625_1 | 15561 | -21 |
| 626 | attatttgaatatactcatt | 4-12-4 | ATTAtttgaatatactCATT | 626_1 | 15594 | -20 |
| 627 | tgggaggaaattatttg | 4-10-3 | TGGGaggaaattatTTG | 627_1 | 15606 | -20 |
| 628 | tgactcatcttaaatg | 4-10-2 | TGACtcatcttaaaTG | 628_1 | 15621 | -17 |
| 629 | ctgactcatcttaaat | 3-11-2 | CTGactcatcttaaAT | 629_1 | 15622 | -16 |
| 630 | tttactctgactcatc | 3-10-3 | TTTactctgactcATC | 630_1 | 15628 | -17 |
| 631 | tattggaggaattatt | 3-11-2 | TATtggaggaattaTT | 631_1 | 15642 | -14 |
| 632 | gtattggaggaattat | 3-11-2 | GTAttggaggaattAT | 632_1 | 15643 | -16 |
| 633 | tggtatacttctctaagtat | 2-15-3 | TGgtatacttctctaagTAT | 633_1 | 15655 | -22 |
| 634 | gatctcttggtatact | 4-10-2 | GATCtcttggtataCT | 634_1 | 15666 | -20 |
| 635 | cagacaactctatacc | 2-12-2 | CAgacaactctataCC | 635_1 | 15689 | -18 |
| 636 | aacatcagacaactcta | 4-9-4 | AACAtcagacaacTCTA | 636_1 | 15693 | -21 |
| 637 | taacatcagacaactc | 4-10-2 | TAACatcagacaacTC | 637_1 | 15695 | -16 |
| 638 | tttaacatcagacaactc | 4-10-4 | TTTAacatcagacaACTC | 638_1 | 15695 | -20 |
| 639 | atttaacatcagacaa | 2-12-2 | ATttaacatcagacAA | 639_1 | 15698 | -11 |
| 640 | cctatttaacatcagac | 2-11-4 | CCtatttaacatcAGAC | 640_1 | 15700 | -20 |
| 641 | tccctatttaacatca | 3-10-3 | TCCctatttaacaTCA | 641_1 | 15703 | -21 |
| 642 | tcaacgactattggaat | 4-9-4 | TCAAcgactattgGAAT | 642_1 | 15737 | -20 |
| 643 | cttatattctggctat | 4-9-3 | CTTAtattctggcTAT | 643_1 | 15850 | -20 |
| 644 | atccttatattctggc | 4-10-2 | ATCCttatattctgGC | 644_1 | 15853 | -23 |
| 645 | gatccttatattctgg | 2-10-4 | GAtccttatattCTGG | 645_1 | 15854 | -21 |
| 646 | tgatccttatattctg | 3-10-3 | TGAtccttatattCTG | 646_1 | 15855 | -19 |
| 647 | attgaaacttgatcct | 4-8-4 | ATTGaaacttgaTCCT | 647_1 | 15864 | -21 |
| 648 | actgtcattgaaactt | 2-10-4 | ACtgtcattgaaACTT | 648_1 | 15870 | -16 |
| 649 | tcttactgtcattgaa | 3-11-2 | TCTtactgtcattgAA | 649_1 | 15874 | -16 |
| 650 | aggatcttactgtcatt | 2-11-4 | AGgatcttactgtCATT | 650_1 | 15877 | -21 |
| 651 | gcaaatcaactccatc | 3-10-3 | GCAaatcaactccATC | 651_1 | 15896 | -20 |
| 652 | gtgcaaatcaactcca | 3-10-3 | GTGcaaatcaactCCA | 652_1 | 15898 | -22 |
| 653 | caattatttctttgtgc | 4-10-3 | CAATtatttctttgTGC | 653_1 | 15910 | -21 |
| 654 | tggcaacaattatttctt | 3-11-4 | TGGcaacaattattTCTT | 654_1 | 15915 | -21 |
| 655 | gctggcaacaattatt | 3-9-4 | GCTggcaacaatTATT | 655_1 | 15919 | -21 |
| 656 | atccatttctactgcc | 4-10-2 | ATCCatttctactgCC | 656_1 | 15973 | -24 |
| 657 | taatatctattgatttcta | 4-11-4 | TAATatctattgattTCTA | 657_1 | 15988 | -20 |
| 658 | tcaatagtgtagggca | 2-12-2 | TCaatagtgtagggCA | 658_1 | 16093 | -18 |
| 659 | ttcaatagtgtagggc | 3-11-2 | TTCaatagtgtaggGC | 659_1 | 16094 | -19 |
| 660 | aggttaattaattcaatag | 4-11-4 | AGGTtaattaattcaATAG | 660_1 | 16102 | -21 |
| 661 | catttgtaatccctag | 3-10-3 | CATttgtaatcccTAG | 661_2 | 16163 | -20 |
| 661 | catttgtaatccctag | 3-9-4 | CATttgtaatccCTAG | 661_1 | 16163 | -22 |
| 662 | acatttgtaatcccta | 3-10-3 | ACAtttgtaatccCTA | 662_1 | 16164 | -20 |
| 663 | aacatttgtaatccct | 2-10-4 | AAcatttgtaatCCCT | 663_2 | 16165 | -21 |
| 663 | aacatttgtaatccct | 3-9-4 | AACatttgtaatCCCT | 663_1 | 16165 | -22 |
| 664 | taaatttcaagttctg | 2-11-3 | TAaatttcaagttCTG | 664_1 | 16184 | -14 |
| 665 | gtttaaatttcaagttct | 3-11-4 | GTTtaaatttcaagTTCT | 665_1 | 16185 | -19 |
| 666 | ccaagtttaaatttcaag | 4-10-4 | CCAAgtttaaatttCAAG | 666_1 | 16189 | -21 |
| 667 | acccaagtttaaatttc | 4-9-4 | ACCCaagtttaaaTTTC | 667_1 | 16192 | -22 |
| 668 | catacagtgacccaagttt | 2-14-3 | CAtacagtgacccaagTTT | 668_1 | 16199 | -23 |
| 669 | acatcccatacagtga | 2-11-3 | ACatcccatacagTGA | 669_1 | 16208 | -21 |
| 670 | agcacagctctacatc | 2-10-4 | AGcacagctctaCATC | 670_1 | 16219 | -22 |
| 671 | atatagcacagctcta | 3-9-4 | ATAtagcacagcTCTA | 671_1 | 16223 | -21 |
| 672 | tccatatagcacagct | 3-11-2 | TCCatatagcacagCT | 672_1 | 16226 | -22 |
| 673 | atttccatatagcaca | 3-9-4 | ATTtccatatagCACA | 673_1 | 16229 | -20 |
| 674 | tttatttccatatagca | 4-9-4 | TTTAtttccatatAGCA | 674_1 | 16231 | -22 |
| 675 | tttatttccatatagc | 3-10-3 | TTTatttccatatAGC | 675_1 | 16232 | -18 |
| 676 | aaggagaggagattatg | 4-9-4 | AAGGagaggagatTATG | 676_1 | 16409 | -21 |
| 677 | agttcttgtgttagct | 3-11-2 | AGTtcttgtgttagCT | 677_1 | 16456 | -21 |
| 678 | gagttcttgtgttagc | 2-12-2 | GAgttcttgtgttaGC | 678_1 | 16457 | -20 |
| 679 | attaattatccatccac | 3-10-4 | ATTaattatccatCCAC | 679_1 | 16590 | -21 |
| 680 | atcaattaattatccatc | 3-11-4 | ATCaattaattatcCATC | 680_1 | 16593 | -19 |
| 681 | agaatcaattaattatcc | 3-12-3 | AGAatcaattaattaTCC | 681_1 | 16596 | -18 |
| 682 | tgagataccgtgcatg | 2-12-2 | TGagataccgtgcaTG | 682_1 | 16656 | -18 |
| 683 | aatgagataccgtgca | 2-10-4 | AAtgagataccgTGCA | 683_1 | 16658 | -21 |
| 684 | ctgtggttaggctaat | 3-11-2 | CTGtggttaggctaAT | 684_1 | 16834 | -19 |
| 685 | aagagtaagggtctgtggtt | 1-17-2 | AagagtaagggtctgtggTT | 685_1 | 16842 | -21 |
| 686 | gatgggttaagagtaa | 4-9-3 | GATGggttaagagTAA | 686_1 | 16854 | -19 |
| 687 | agcagatgggttaaga | 3-11-2 | AGCagatgggttaaGA | 687_1 | 16858 | -20 |
| 688 | tgtaaacatttgtagc | 2-10-4 | TGtaaacatttgTAGC | 688_1 | 16886 | -19 |
| 689 | cctgcttataaatgta | 3-11-2 | CCTgcttataaatgTA | 689_1 | 16898 | -19 |
| 690 | tgccctgcttataaat | 4-10-2 | TGCCctgcttataaAT | 690_1 | 16901 | -23 |
| 691 | tcttcttagttcaata | 2-12-2 | TCttcttagttcaaTA | 691_1 | 16935 | -15 |
| 692 | tggtttctaactacat | 2-10-4 | TGgtttctaactACAT | 692_1 | 16980 | -18 |
| 693 | agtttggtttctaacta | 2-12-3 | AGtttggtttctaaCTA | 693_1 | 16983 | -19 |
| 694 | gaatgaaacttgcctg | 3-10-3 | GAAtgaaacttgcCTG | 694_1 | 17047 | -18 |
| 695 | attatccttacatgat | 3-10-3 | ATTatccttacatGAT | 695_1 | 17173 | -17 |
| 696 | gtacccaattatcctt | 2-11-3 | GTacccaattatcCTT | 696_1 | 17180 | -21 |
| 697 | tgtacccaattatcct | 3-10-3 | TGTacccaattatCCT | 697_1 | 17181 | -24 |
| 698 | ttgtacccaattatcc | 2-11-3 | TTgtacccaattaTCC | 698_1 | 17182 | -20 |
| 699 | tttgtacccaattatc | 3-11-2 | TTTgtacccaattaTC | 699_1 | 17183 | -17 |
| 700 | agcagcaggttatatt | 4-10-2 | AGCAgcaggttataTT | 700_1 | 17197 | -22 |
| 701 | tgggaagtggtctggg | 3-10-3 | TGGgaagtggtctGGG | 701_1 | 17292 | -25 |
| 702 | ctggagagtgataata | 3-11-2 | CTGgagagtgataaTA | 702_1 | 17322 | -17 |
| 703 | aatgctggattacgtc | 4-10-2 | AATGctggattacgTC | 703_1 | 17354 | -19 |
| 704 | caatgctggattacgt | 2-11-3 | CAatgctggattaCGT | 704_1 | 17355 | -19 |
| 705 | ttgttcagaagtatcc | 2-10-4 | TTgttcagaagtATCC | 705_1 | 17625 | -19 |
| 706 | gatgatttgcttggag | 2-10-4 | GAtgatttgcttGGAG | 706_1 | 17646 | -21 |
| 707 | gaaatcattcacaacc | 3-10-3 | GAAatcattcacaACC | 707_1 | 17860 | -17 |
| 708 | ttgtaacatctactac | 3-10-3 | TTGtaacatctacTAC | 708_1 | 17891 | -16 |
| 709 | cattaagcagcaagtt | 3-11-2 | CATtaagcagcaagTT | 709_1 | 17923 | -17 |
| 710 | ttactagatgtgagca | 3-11-2 | TTActagatgtgagCA | 710_1 | 17942 | -18 |
| 711 | tttactagatgtgagc | 2-11-3 | TTtactagatgtgAGC | 711_1 | 17943 | -18 |
| 712 | gaccaagcaccttaca | 3-11-2 | GACcaagcaccttaCA | 712_1 | 17971 | -22 |
| 713 | agaccaagcaccttac | 3-10-3 | AGAccaagcacctTAC | 713_1 | 17972 | -22 |
| 714 | atgggttaaataaagg | 2-10-4 | ATgggttaaataAAGG | 714_1 | 18052 | -15 |
| 715 | tcaaccagagtattaa | 2-12-2 | TCaaccagagtattAA | 715_1 | 18067 | -13 |
| 716 | gtcaaccagagtatta | 3-11-2 | GTCaaccagagtatTA | 716_1 | 18068 | -18 |
| 717 | attgtaaagctgatat | 2-11-3 | ATtgtaaagctgaTAT | 717_1 | 18135 | -14 |
| 718 | cacataattgtaaagc | 2-10-4 | CAcataattgtaAAGC | 718_1 | 18141 | -16 |
| 719 | gaggtctgctatttac | 2-11-3 | GAggtctgctattTAC | 719_1 | 18274 | -19 |
| 720 | tgtagattcaatgcct | 2-11-3 | TGtagattcaatgCCT | 720_1 | 18404 | -20 |
| 721 | cctcattatactatga | 2-11-3 | CCtcattatactaTGA | 721_1 | 18456 | -19 |
| 722 | ccttatgctatgacac | 2-12-2 | CCttatgctatgacAC | 722_1 | 18509 | -18 |
| 723 | tccttatgctatgaca | 4-10-2 | TCCTtatgctatgaCA | 723_1 | 18510 | -22 |
| 724 | aagatgtttaagtata | 3-10-3 | AAGatgtttaagtATA | 724_1 | 18598 | -13 |
| 725 | ctgattattaagatgt | 2-10-4 | CTgattattaagATGT | 725_1 | 18607 | -17 |
| 726 | tggaaaggtatgaatt | 2-12-2 | TGgaaaggtatgaaTT | 726_1 | 18808 | -13 |
| 727 | acttgaatggcttgga | 2-12-2 | ACttgaatggcttgGA | 727_1 | 18880 | -18 |
| 728 | aacttgaatggcttgg | 3-10-3 | AACttgaatggctTGG | 728_1 | 18881 | -19 |
| 729 | caatgtgttactattt | 4-10-2 | CAATgtgttactatTT | 729_1 | 19004 | -16 |
| 730 | acaatgtgttactatt | 3-10-3 | ACAatgtgttactATT | 730_1 | 19005 | -15 |
| 731 | catctgctatataaga | 4-10-2 | CATCtgctatataaGA | 731_1 | 19063 | -18 |
| 732 | cctagagcaaatactt | 4-10-2 | CCTAgagcaaatacTT | 732_1 | 19223 | -20 |
| 733 | cagagttaataataag | 3-10-3 | CAGagttaataatAAG | 733_1 | 19327 | -13 |
| 734 | gttcaagcacaacgaa | 4-10-2 | GTTCaagcacaacgAA | 734_1 | 19493 | -18 |
| 735 | agggttcaagcacaac | 2-11-3 | AGggttcaagcacAAC | 735_1 | 19496 | -18 |
| 736 | tgttggagacactgtt | 2-12-2 | TGttggagacactgTT | 736_1 | 19677 | -17 |
| 737 | aaggaggagttaggac | 3-11-2 | AAGgaggagttaggAC | 737_1 | 19821 | -18 |
| 738 | ctatgccatttacgat | 4-10-2 | CTATgccatttacgAT | 738_1 | 19884 | -21 |
| 739 | tcaaatgcagaattag | 2-12-2 | TCaaatgcagaattAG | 739_1 | 19913 | -12 |
| 740 | agtgacaatcaaatgc | 2-10-4 | AGtgacaatcaaATGC | 740_1 | 19921 | -18 |
| 741 | aagtgacaatcaaatg | 2-11-3 | AAgtgacaatcaaATG | 741_1 | 19922 | -12 |
| 742 | gtgtaccaagtaacaa | 3-11-2 | GTGtaccaagtaacAA | 742_1 | 19978 | -16 |
| 743 | tgggatgttaaactga | 3-10-3 | TGGgatgttaaacTGA | 743_1 | 20037 | -20 |

| | | | | | | |
|---|---|---|---|---|---|---|
| Motif sequences represent the contiguous sequence of nucleobases present in the oligonucleotide. Designs refer to the gapmer design, F-G-F', where each number represents the number of consecutive modified nucleosides, e.g. 2' modified nucleosides (first number=5' flank), followed by the number of DNA nucleosides (second number= gap region), followed by the number of modified nucleosides, e.g. 2' modified nucleosides (third number=3' flank), optionally preceded by or followed by further repeated regions of DNA and LNA, which are not necessarily part of the contiguous sequence that is complementary to the target nucleic acid. Oligonucleotide compounds represent specific designs of a motif sequence. Capital letters represent beta-D-oxy LNA nucleosides, lowercase letters represent DNA nucleosides, all LNA C are 5-methyl cytosine, all internucleoside linkages are phosphorothioate internucleoside linkages. | | | | | | |

**Table 6: Oligonucleotides targeting mouse PD-L1 transcript (SEQ ID NO: 4) designs of these, as well as specific oligonucleotide compounds (indicated by CMP ID NO) designed based on the motif sequence.**

| **SEQ ID NO** | **Motif sequence** | **Design** | **Oligonucleotide Compound** | **CMP ID NO** | **Start on SEQ ID NO: 4** | **dG** |
|---|---|---|---|---|---|---|
| 744 | agtttacattttctgc | 3-10-3 | AGTttacattttcTGC | 744_1 | 4189 | -20 |
| 745 | tatgtgaagaggagag | 3-10-3 | TATgtgaagaggaGAG | 745_1 | 7797 | -19 |
| 746 | cacctttaaaacccca | 3-10-3 | CACctttaaaaccCCA | 746_1 | 9221 | -23 |
| 747 | tcctttataatcacac | 3-10-3 | TCCtttataatcaCAC | 747_1 | 10386 | -19 |
| 748 | acggtattttcacagg | 3-10-3 | ACGgtattttcacAGG | 748_1 | 12389 | -21 |
| 749 | gacactacaatgagga | 3-10-3 | GACactacaatgaGGA | 749_1 | 15088 | -20 |
| 750 | tggtttttaggactgt | 3-10-3 | TGGtttttaggacTGT | 750_1 | 16410 | -21 |
| 751 | cgacaaattctatcct | 3-10-3 | CGAcaaattctatCCT | 751_1 | 18688 | -20 |
| 752 | tgatatacaatgctac | 3-10-3 | TGAtatacaatgcTAC | 752_1 | 18735 | -16 |
| 753 | tcgttgggtaaattta | 3-10-3 | TCGttgggtaaatTTA | 753_1 | 19495 | -17 |
| 754 | tgctttataaatggtg | 3-10-3 | TGCtttataaatgGTG | 754_1 | 19880 | -19 |

| | | | | | | |
|---|---|---|---|---|---|---|
| Motif sequences represent the contiguous sequence of nucleobases present in the oligonucleotide. Designs refer to the gapmer design, F-G-F', where each number represents the number of consecutive modified nucleosides, e.g. 2' modified nucleosides (first number=5' flank), followed by the number of DNA nucleosides (second number= gap region), followed by the number of modified nucleosides, e.g. 2' modified nucleosides (third number=3' flank), optionally preceded by or followed by further repeated regions of DNA and LNA, which are not necessarily part of the contiguous sequence that is complementary to the target nucleic acid. Oligonucleotide compounds represent specific designs of a motif sequence. Capital letters represent beta-D-oxy LNA nucleosides, lowercase letters represent DNA nucleosides, all LNA C are 5-methyl cytosine, all internucleoside linkages are phosphorothioate internucleoside linkages. | | | | | | |

**Table 7: Oligonucleotide motif sequences and antisense compounds with 5' ca biocleavable linker.**

| SEQ ID NO | motif sequence | oligonucleotide compound with ca linker | CMP ID NO |
|---|---|---|---|
| 755 | caagtttacattttctgc | cₒaₒAGTttacattttcTGC | 755_1 |
| 756 | catatgtgaagaggagag | cₒaₒTATgtgaagaggaGAG | 756_1 |
| 757 | cacctttaaaacccca | cₒaₒCACctttaaaaccCCA | 757_1 |
| 758 | catcctttataatcacac | cₒaₒTCCtttataatcaCAC | 758_1 |
| 759 | caacggtattttcacagg | cₒaₒACGgtattttcacAGG | 759_1 |
| 760 | cagacactacaatgagga | cₒaₒGACactacaatgaGGA | 760_1 |
| 761 | catggtttttaggactgt | cₒaₒTGGtttttaggacTGT | 761_1 |
| 762 | cacgacaaattctatcct | cₒaₒCGAcaaattctatCCT | 762_1 |
| 763 | catgatatacaatgctac | cₒaₒTGAtatacaatgcTAC | 763_1 |
| 764 | catcgttgggtaaattta | cₒaₒTCGttgggtaaatTTA | 764_1 |
| 765 | catgctttataaatggtg | cₒaₒTGCtttataaatgGTG | 765_1 |
| 766 | caacaaataatggttactct | cₒaₒACAAataatggttaCTCT | 766_1 |
| 767 | cacagattgatggtagtt | cₒaₒCAGAttgatggtagTT | 767_1 |
| 768 | cacctatttaacatcagac | cₒaₒCCtatttaacatcAGAC | 768_1 |
| 769 | cactaattgtagtagtactc | cₒaₒCTAattgtagtagtaCTC | 769_1 |
| 770 | caataaacatgaatctctcc | cₒaₒATaaacatgaatctCTCC | 770_1 |

| | | | |
|---|---|---|---|
| Capital letters represent beta-D-oxy LNA nucleosides, lowercase letters represent DNA nucleosides, all LNA C are 5-methyl cytosine, subscript o represent a phosphodiester internucleoside linkage and unless otherwise indicated other internucleoside linkages are phosphorothioate internucleoside linkages. | | | |

**Table 8: GalNAc conjugated antisense oligonucleotide compounds.**

| **antisense oligonucleotide conjugate** | **CMP ID NO** |
|---|---|
| G N2-C6ₒcₒaₒAGTttacattttcTG C | 755_2 |
| GN2-C6ₒcₒaₒTATgtgaagaggaGAG | 756_2 |
| GN2-C6ₒcₒaₒCACctttaaaaccCCA | 757_2 |
| GN2-C6ₒcₒaₒTCCtttataatcaCAC | 758_2 |
| G N2-C6ₒcₒaₒACGgtattttcacAGG | 759_2 |
| G N2-C6ₒcₒaₒGACactacaatgaGGA | 760_2 |
| G N2-C6ₒcₒaₒTGGtttttaggacTGT | 761_2 |
| G N2-C6ₒcₒaₒCGAcaaattctatCCT | 762_2 |
| G N2-C6ₒcₒaₒTGAtatacaatgcTAC | 763_2 |
| G N2-C6ₒcₒaₒTCGttgggtaaatTTA | 764_2 |
| G N2-C6ₒcₒaₒTGCtttataaatgGTG | 765_2 |
| G N2-C6ₒcₒaₒACAAataatggttaCTCT | 766_2 |
| G N2-C6ₒcₒaₒCAGAttgatggtagTT | 767_2 |
| G N2-C6ₒcₒaₒCCtatttaacatcAGAC | 768_2 |
| GN2-C6ₒcₒaₒCTAattgtagtagtaCTC | 769_2 |
| GN2-C6ₒcₒaₒATaaacatgaatctCTCC | 770_2 |

| | |
|---|---|
| GN2 represents the trivalent GalNAc cluster shown in Figure 3, C6 represents an amino alkyl group with 6 carbons, capital letters represent beta-D-oxy LNA nucleosides, lowercase letters represent DNA nucleosides, all LNA C are 5-methyl cytosine, subscript o represent a phosphodiester nucleoside linkage and unless otherwise indicated internucleoside linkages are phosphorothioate internucleoside linkages. Chemical drawings representing some of the molecules are shown in figures 4 to 8. | |

### AAV/HBV mouse models

### Pasteur model:

HLA-A2.1-/HLA-DR1-transgenic H-2 class I-/class II-knockout (here referred to as HLA-A2/DR1) mice were created and bred at the Institut Pasteur. These mice represent an *in vivo* experimental model for human immune function studies without any interference with mouse MHC response (Pajot et al 2004 Eur J Immunol. 34(11):3060-9.

Adeno-associated virus (AAV) vector, AAV serotype 2/8 carrying a replication competent HBV DNA genome was used in these studies. The AAV-HBV vector (batch GVPN #6163) was diluted in sterile Phosphate buffered Saline (PBS) to reach a titer of 5 x 10¹¹ vg/mL. Mice were injected intravenously (i.v.) with 100µL of this diluted solution (dose/mouse: 5 x 10¹⁰ vg) in a tail vein. Complete viral particles containing HBV DNA were detected in the blood of HBV-carrier mice. HBcAg was detected for up to one year in the liver together with HBV circulating proteins HBeAg and HBsAg in the blood. In all AAV2/8-HBV-transduced mice, HBsAg, HBeAg, and HBV DNA persisted in serum for at least one year (Dion et al 2013 J Virol 87:5554-5563).

### Shanghai model:

In this model, mice infected with a recombinant adeno-associated virus (AAV) carrying the HBV genome (AAV/HBV) maintains stable viremia and antigenimia for more than 30 weeks (Dan Yang, et al. 2014 Cellular & Molecular Immunology 11, 71-78).

Male C57BL/6 mice (4-6 weeks old), specific pathogen free, were purchased from SLAC (Shanghai Laboratory Animal Center of Chinese Academy of Sciences) and housed in an animal care facility in individually ventilated cages. Guidelines were followed for the care and use of animals as indicated by WuXi IACUC (Institutional Animal Care and Use Committee, WUXI IACUC protocol number R20131126-Mouse). Mice were allowed to acclimate to the new environment for 3 days and are grouped according to the experimental design.

Recombinant AAV-HBV was diluted in PBS, 200 µL per injection. This recombinant virus carries 1.3 copies of the HBV genome (genotype D, serotype ayw).

On day 0, all mice were injected through tail vein with 200 µL AAV-HBV. On days 6, 13 and 20 after AAV injection, all mice in were submandibularly bled (0.1 ml blood/mouse) for serum collection. On day 22 post injection, mice with stable viremia were ready for oligonucleotide treatment. The oligonucleotides can be unconjugated or GalNAc conjugated.

### DNA vaccine

Plasmid DNA were endotoxin-free and manufactured by Plasmid-Factory (Germany). pCMV-S2.S ayw encodes the preS2 and S domains of the HBsAg (genotype D), and its expression is controlled by the cytomegalovirus immediate early gene promoter (Michel et al 1995 Proc Natl Acad Sci U S A 92:5307-5311). pCMV-HBc encodes the HBV capsid carrying the hepatitis core (HBc) Ag (Dion et al 2013 J Virol 87:5554-5563).

Treatment with DNA vaccine was conducted as described here. Five days prior to vacciantion cardiotoxine (CaTx, Latoxan refL81-02, 50 µl/ muscle) was injected into the muscle of the mice. CaTx depolarizees the muscular fibers to induce cell degeneration, 5 days post injection, new muscular fibers will appear and will receive the DNA vaccine for a better efficacy for transfection. The pCMV-S2.S ayw and pCMVCore at 1 mg/ml each were mixed in equal amount and each mouse received a total of 100 µg by bilateral intramuscular injection into cardiotoxin-treated tibialis anterior muscles as previously described in Michel et al 1995 Proc Natl Acad Sci U S A 92:5307-5311, under anesthesia (100 µL of 12.5 mg/mL ketamine, 1.25 mg/mL xylazine).

### Anti-PD-L1 antibody

This is a mouse anti mouse PD-L1 IgG1 antibody clone 6E11 internally produced at Genetech. It is a surrogate antibody that cross blocks Atezolizumab and has similar in vitro blocking activity Atezolizumabproduced internally at Roche. The antibody was adminstredadministered by intraperitoneal (i.p.) injection at a dose of 12.5 µg/g.

### Oligonucleotide synthesis

Oligonucleotide synthesis is generally known in the art. Below is a protocol which may be applied. The oligonucleotides of the present invention may have been produced by slightly varying methods in terms of apparatus, support and concentrations used.

Oligonucleotides are synthesized on uridine universal supports using the phosphoramidite approach on an Oligomaker 48 at 1 µmol scale. At the end of the synthesis, the oligonucleotides are cleaved from the solid support using aqueous ammonia for 5-16hours at 60°C. The oligonucleotides are purified by reverse phase HPLC (RP-HPLC) or by solid phase extractions and characterized by UPLC, and the molecular mass is further confirmed by ESI-MS.

### Elongation of the oligonucleotide:

The coupling of β-cyanoethyl- phosphoramidites (DNA-A(Bz), DNA- G(ibu), DNA- C(Bz), DNA-T, LNA-5-methyl-C(Bz), LNA-A(Bz), LNA- G(dmf), or LNA-T) is performed by using a solution of 0.1 M of the 5'-O-DMT-protected amidite in acetonitrile and DCI (4,5-dicyanoimidazole) in acetonitrile (0.25 M) as activator. For the final cycle a phosphoramidite with desired modifications can be used, e.g. a C6 linker for attaching a conjugate group or a conjugate group as such. Thiolation for introduction of phosphorthioate linkages is carried out by using xanthane hydride (0.01 M in acetonitrile/pyridine 9:1). Phosphordiester linkages can be introduced using 0.02 M iodine in THF/Pyridine/water 7:2:1. The rest of the reagents are the ones typically used for oligonucleotide synthesis.

For post solid phase synthesis conjugation a commercially available C6 amino linker phosphoramidite can be used in the last cycle of the solid phase synthesis and after deprotection and cleavage from the solid support the aminolinked deprotected oligonucleotide is isolated. The conjugates are introduced via activation of the functional group using standard synthesis methods.

Alternatively, the conjugate moiety can be added to the oligonucleotide while still on the solid support by using a GalNAc- or GalNAc-cluster phosphoramidite as described in PCT/EP2015/073331 or in EP appl. NO. 15194811.4.

### Purification by RP-HPLC:

The crude compounds are purified by preparative RP-HPLC on a Phenomenex Jupiter C18 10µ 150x10 mm column. 0.1 M ammonium acetate pH 8 and acetonitrile is used as buffers at a flow rate of 5 mL/min. The collected fractions are lyophilized to give the purified compound typically as a white solid.

### Abbreviations:

- DCI:: 4,5-Dicyanoimidazole
- DCM:: Dichloromethane
- DMF:: Dimethylformamide
- DMT:: 4,4'-Dimethoxytrityl
- THF:: Tetrahydrofurane
- Bz:: Benzoyl
- Ibu:: Isobutyryl
- RP-HPLC:: Reverse phase high performance liquid chromatography

### Tₘ Assay

Oligonucleotide and RNA target (phosphate linked, PO) duplexes are diluted to 3 mM in 500 ml RNase-free water and mixed with 500 ml 2x Tₘ-buffer (200mM NaCl, 0.2mM EDTA, 20mM Naphosphate, pH 7.0). The solution is heated to 95ºC for 3 min and then allowed to anneal in room temperature for 30 min. The duplex melting temperatures (Tₘ) is measured on a Lambda 40 UV/VIS Spectrophotometer equipped with a Peltier temperature programmer PTP6 using PE Templab software (Perkin Elmer). The temperature is ramped up from 20ºC to 95ºC and then down to 25ºC, recording absorption at 260 nm. First derivative and the local maximums of both the melting and annealing are used to assess the duplex Tₘ.

### Tissue specific In vitro linker cleavage assay

FAM-labeled oligonucleotides with the biocleavable linker to be tested (e.g. a DNA phosphodiester linker (PO linker)) are subjected to *in vitro* cleavage using homogenates of the relevant tissues (e.g. liver or kidney) and Serum.

The tissue and serum samples are collected from a suitable animal (e.g. mice, monkey, pig or rat) and homogenized in a homogenisation buffer (0,5% Igepal CA-630, 25 mM Tris pH 8.0, 100 mM NaCl, pH 8.0 (adjusted with 1 N NaOH).The tissue homogenates and Serum are spiked with oligonucleotide to concentrations of 200 µg/g tissue. The samples are incubated for 24 hours at 37°C and thereafter the samples are extracted with phenol - chloroform. The solutions are subjected to AIE HPLC analyses on a Dionex Ultimate 3000 using an Dionex DNApac p-100 column and a gradient ranging from 10mM - 1 M sodium perchlorate at pH 7.5. The content of cleaved and non-cleaved oligonucleotide are determined against a standard using both a fluorescence detector at 615 nm and a uv detector at 260 nm.

### S1 nuclease cleavage assay

FAM-labelled oligonucleotides with S1 nuclease susceptible linkers (e.g. a DNA phosphodiester linker (PO linker)) are subjected to *in vitro* cleavage in S1 nuclease extract or Serum.

100 µM of the oligonucleotides are subjected to *in vitro* cleavage by S1 nuclease in nuclease buffer (60 U pr. 100 µL) for 20 and 120 minutes. The enzymatic activity is stopped by adding EDTA to the buffer solution. The solutions are subjected to AIE HPLC analyses on a Dionex Ultimate 3000 using an Dionex DNApac p-100 column and a gradient ranging from 10mM - 1 M sodium perchlorate at pH 7.5. The content of cleaved and non-cleaved oligonucleotide is determined against a standard using both a fluorescence detector at 615 nm and a uv detector at 260 nm.

### Preparation of liver mononuclear cells

Liver cells from AAV/HBV mice were prepared as described below and according to a method described by Tupin et al 2006 Methods Enzymol 417:185-201 with minor modifications. After mouse euthanasia, the liver was perfused with 10 ml of sterile PBS via hepatic portal vein using syringe with G25 needle. When organ is pale, the organ was harvested in Hank's Balanced Salt Solution (HBSS) (GIBCO® HBSS, 24020) + 5 % decomplemented fetal calf serum (FCS). The harvested liver was gently pressed through 100 µm cell-strainer (BD Falcon, 352360) and cells were suspended in 30 ml of HBSS + 5 % FCS. Cell suspension was centrifuged at 50 g for 5 min. Supernatants were then centrifuged at 289 g for 10 min at 4 °C. After centrifugation, supernatants were discarded and pellets were re-suspended in 15 mL at room temperature in a 35 % isotonic Percoll solution (GE Healthcare Percoll #17-0891-01 diluted into RPMI 1640 (GIBCO, 31870)) and transferred to a 15 ml tube. Cells were further centrifuged at 1360g for 25 min at room temperature. The supernatant was discarded by aspiration and the pellet containing mononuclear cells was washed twice with HBSS + 5 % FCS.

Cells were cultured in complete medium (a-minimal essential medium (Gibco, 22571) supplemented with 10 % FCS (Hyclone, # SH30066, lot APG21570), 100 U/mL penicillin + 100 µg/mL streptomycin + 0.3 mg/mL L-glutamine (Gibco, 10378), 1X non-essential amino acids (Gibco, 11140), 10 mM Hepes (Gibco, 15630), 1 mM sodium pyruvate (Gibco, 11360) and 50 µM β-mercaptoethanol (LKB, 1830)).

### Surface labeling of cells

Cells were seeded in U-bottom 96-well plates and washed with PBS FACS (PBS containing 1 % bovine serum albumin and 0.01% sodium azide). Cells were incubated with 5 µL of PBS FACS containing a rat anti-mouse CD16/CD32 antibody and a viability marker LD fixable yellow, Thermofisher, L34959 for 10 min in the dark at 4°C. Then, cells were stained for 20 min in the dark at 4°C with 25 µL of PBS FACS containing monoclonal antibodies (Mab) against NK P46 BV421 (Rat Mab anti mouse NK P46, Biolegend, 137612) and F4/80 (rat Mab anti-mouse F4/80 FITC, BD Biolegend, 123108) and two supplemental surface markers: PD1 (rat Mab anti-mouse PD1 PE, BD Biosciences, 551892) and PDL1 (rat Mab anti-mouse PDL1 BV711, Biolegend, 124319) were also added.

### Intracelluar cytokine staining (ICS) assay

ICS assays were performed on both splenocytes and liver mononuclear cells. Cells were seeded in Ubottom 96-well plates. Plates with cells were incubated overnight at 37 °C either in complete medium alone as negative control or with the peptides described in Table 9 at a concentration of 2 µg/ml. Brefeldin A at 2µg/mL (Sigma, B6542) was added after one hour of incubation.

After the overnight culture, cells were washed with PBS FACS and incubated with 5 µL of PBS FACS containing rat anti-mouse CD16/CD32 antibody and a viability marker LD fixable yellow, Thermofisher, L34959 for 10 min in the dark at 4°C. Then, cells were stained for 20 min in the dark at 4°C with 25 µL of PBS FACS containing Mab. The mix was composed of monoclonal antibodies against CD3 (hamster Mab anti-mouse CD3-PerCP, BD Biosciences, 553067), CD8 (rat Mab anti-mouse CD8-APC-H7, BD Biosciences, 560182), CD4 (rat Mab anti-mouse CD4-PE-Cy7, BD Biosciences, 552775), and NK cells (Rat Mab anti mouse NK P46 BV421, Biolegend, 137612). Cells were fixed after several washes and permeabilized for 20 min in the dark at room temperature with Cytofix/Cytoperm, washed with Perm/Wash solution (BD Biosciences, 554714) at 4 °C.

Intracellular cytokine staining with antibodies against IFNγ (rat Mab anti-mouse IFNγ-APC, clone XMG1.2, BD Biosciences, 554413) and tumor necrosis factor alpha (TNFα) (rat Mab anti-mouse TNFα-FITC, clone MP6-XT22; 1/250 (BD Biosciences 554418) was performed for 30 min in the dark at 4 °C. Before analysis by flow cytometry using the MACSQuant Analyzer, cells were washed with Perm/Wash and re-suspended in PBS FACS containing 1% Formaldehyde.

Live CD3+CD8+CD4- and cells CD3+CD8-CD4+ were gated and presented on dot-plot. Two regions were defined to gate for positive cells for each cytokine. Numbers of events found in these gates were divided by total number of events in parental population to yield percentages of responding T cells. For each mouse, the percentage obtained in medium alone was considered as background and subtracted from the percentage obtained with peptide stimulations.

Threshold of positivity was defined according to experiment background i.e. the mean percentage of stained cells obtained for each group in medium alone condition plus two standard deviations. Only percentage of cytokine represented at least 5 events were considered as positive.

**Table 9: HLA-A2/DR1 restricted epitopes contained in the HBV Core protein and the Envelope domains of the HBsAg (S2+S).**

| **Protein** | **Start Position** | **End Position** | **Sequence** | **HLA restriction** | **References** |
|---|---|---|---|---|---|
| Core | 18 | 27 | FLPSDFFPSV (SEQ ID NO: 773) | A2 | Bertoletti et al Gastroenterology 1997;112:193-199 |
| | 111 | 125 | GRETVLEYLVSFGVW (SEQ ID NO: 774) | DR1 | (Bertoletti et al Gastroenterology 1997;112:193-199 |
| Envelope (S2+S) | 114 | 128 | TTFHQTLQDPRVRGL (SEQ ID NO: 775) | DR1 | Pajot et al Microbes Infect 2006;8:2783-2790. |
| | 179 | 194 | QAGFFLLTRILTIPQS (SEQ ID NO: 776) | A2 + DR1 | Pajot et al Microbes Infect 2006;8:2783-2790. |
| | 183 | 191 | FLLTRILTI (SEQ ID NO: 777) | A2 | Sette et al J Immunol 1994;153:5586-5592. |
| | 200 | 214 | TSLNFLGGTTVCLGQ (SEQ ID NO: 778) | A2 + DR1 | Pajot et al Microbes Infect 2006;8:2783-2790. |
| | 204 | 212 | FLGGTTVCL (SEQ ID NO: 779) | A2 | Rehermann et al J Exp Med 1995;181: 1047-1058. |
| | 335 | 343 | WLSLLVPFV (SEQ ID NO: 780) | A2 | Nayersina et alJ Immunol 1993;150: 4659-4671. |
| | 337 | 357 | SLLVPFVQWFVGLSPTVWLSV (SEQ ID NO: 781) | A2 + DR1 | Loirat et al J Immunol 2000;165: 4748-4755 |
| | 348 | 357 | GLSPTVWLSV (SEQ ID NO: 782) | A2 | Loirat et al J Immunol 2000;165: 4748-4755 |
| | 370 | 379 | SILSPFLPLL (SEQ ID NO: 783) | A2 | Mizukoshi et al J Immunol 2004;173: 5863-5871. |

### Example 1 Testing in vitro efficacy

A gene walk was performed across the human PD-L1 transcript primarily using 16 to 20mer gapmers. Efficacy testing was performed in an *in vitro* experiment in the human leukemia monocytic cell line THP1 and in the human non-Hodgkin's K lymphoma cell line (KARPAS-299).

### Cell lines

THP1 and Karpas-299 cell line were originally purchased from European Collection of Authenticated Cell Cultures (ECACC) and maintained as recommended by the supplier in a humidified incubator at 37°C with 5% CO₂.

### Oligonucleotide efficacy

THP-1 cells (3.104 in RPMI-GLutamax, 10% FBS, 1% Pen-Strep (Thermo Fisher Scientific) were added to the oligonucleotides (4-5 ul) into 96-well round bottom plates and cultured for 6 days in a final volume of 100 µl/well. Oligonucleotides were screened at one single concentration (20 µM) and in dose-range concentrations from 25 µM to 0.004 µM (1:3 dilution in water). Total mRNA was extracted using the MagNA Pure 96 Cellular RNA Large Volume Kit on the MagNA Pure 96 System (Roche Diagnostics) according to the manufacturer's instructions. For gene expression analysis, RT-qPCR was performed using the TaqMan RNA-to-ct 1-Step kit (Thermo Fisher Scientific) on the QuantStudio machine (Applied Biosystems) with pre-designed Taqman primers targeting human PDL1 and ACTB used as endogenous control (Thermo Fisher Scientific). The relative PD-L1 mRNA expression level was calculated using 2(-Delta Delta C(T)) method and the percentage of inhibition as the % compared to the control sample (non-treated cells).

Karpas-299 cells were cultured in RPMI 1640, 2 mM Glutamine and 20% FBS (Sigma). The cells were plated at 10000 cell/well in 96 wells plates incubated for 24 hours before addition of oligonucleotides dissolved in PBS. Final concentration of oligonucleotides was in a single dose of 5 µM, in a final culture volume was 100 µl/well or added in a dose response ranging from 50 µM, 15.8 µM, 5.0 µM, 1.58 µM, 0.5 µM, 0.158 µM, 0.05 µM, to 0.0158 µM in 100 µL culture volume. The cells were harvested 3 days after addition of oligonucleotide compounds and RNA was extracted using the PureLink Pro 96 RNA Purification kit (Ambion), according to the manufacturer's instructions. cDNA was synthesized using M-MLT Reverse Transcriptase, random decamers RETROscript, RNase inhibitor (Ambion) and 100 mM dNTP set (Invitrogen, PCR Grade) according to the manufacturer's instruction. For gene expressions analysis, qPCR was performed using TaqMan Fast Advanced Master Mix (2X) (Ambion) in a duplex set up with TaqMan primer assays for the PD-L1 (Applied Biosystems; Hs01125299_m1) and TBP (Applied Biosystems; 4325803). The relative PD-L1 mRNA expression level is shown in table 10 as % of control sample (PBS-treated cells).

**Table 10: in vitro efficacy of anti-PD-L1 compounds in THP1 and KARPAS-299 cell lines (Average from n=3 experiments). PD-L1 mRNA levels are normalized to TBP in KARPAS-299 cells or ACTB in THP1 cells and shown as % of control (PBS treated cells).**

| **CMP ID NO** | **KARPAS-299 cells 5 µM CMP** | | **THP1 cells 20 µM CMP** | | **Compound (CMP)** | **Start on SEQ ID NO 1** |
|---|---|---|---|---|---|---|
| | **% mRNA of control** | **sd** | **% mRNA of control** | **sd** | | |
| 4_1 | 50 | 1 | 32 | 11 | TAattggctctacTGC | 236 |
| 5_1 | 25 | 5 | 9 | 6 | TCGCataagaatgaCT | 371 |
| 6_1 | 29 | 2 | 15 | 5 | TGaacacacagtcgCA | 382 |
| 7_1 | 27 | 7 | 3 | 1 | CTGaacacacagtCGC | 383 |
| 8_1 | 23 | 4 | 11 | 3 | TCTgaacacacagtCG | 384 |
| 9_1 | 32 | 3 | 19 | 6 | TTCtgaacacacagTC | 385 |
| 10_1 | 57 | 5 | 39 | 16 | ACaagtcatgttaCTA | 463 |
| 11_1 | 75 | 5 | 37 | 12 | ACacaagtcatgttAC | 465 |
| 12_1 | 22 | 2 | 10 | 3 | CTtacttagatgcTGC | 495 |
| 13_1 | 33 | 4 | 23 | 11 | ACttacttagatgCTG | 496 |
| 14_1 | 33 | 7 | 21 | 6 | GACttacttagatgCT | 497 |
| 15_1 | 41 | 6 | 18 | 10 | AGacttacttagaTGC | 498 |
| 16_1 | 96 | 14 | 40 | 7 | GCAggaagagactTAC | 506 |
| 17_1 | 22 | 2 | 9 | 3 | AATAaattccgttCAGG | 541 |
| 18_1 | 34 | 6 | 21 | 9 | GCAAataaattcCGTT | 545 |
| 18_2 | 51 | 4 | 27 | 11 | GCAaataaattccGTT | 545 |
| 19_1 | 38 | 5 | 23 | 7 | AG CAaataaattcCGT | 546 |
| 20_1 | 73 | 8 | 56 | 15 | CAGAgcaaataaatTCC | 548 |
| 21_1 | 83 | 8 | 65 | 10 | TGGAcagagcaaataAAT | 551 |
| 22_1 | 86 | 6 | 80 | 8 | ATGGacagagcaAATA | 554 |
| 23_1 | 44 | 4 | 30 | 2 | CAgaatggacagaGCA | 558 |
| 24_1 | 63 | 10 | 40 | 11 | TTCtcagaatggacAG | 562 |
| 25_1 | 31 | 1 | 39 | 5 | CTGAactttgacATAG | 663 |
| 26_1 | 60 | 4 | 56 | 19 | AAgacaaacccagacTGA | 675 |
| 27_1 | 36 | 4 | 34 | 10 | TATAagacaaacccAGAC | 678 |
| 28_1 | 40 | 4 | 28 | 13 | TTATaagacaaaccCAGA | 679 |
| 29_1 | 30 | 2 | 18 | 6 | TGTTataagacaaaCCC | 682 |
| 30_1 | 77 | 3 | 67 | 10 | TAGAacaatggtaCTTT | 708 |
| 31_1 | 81 | 17 | 20 | 14 | GTAGaacaatggtaCT | 710 |
| 32_1 | 29 | 5 | 14 | 8 | AGGtagaacaatgGTA | 712 |
| 33_1 | 32 | 1 | 43 | 20 | AAGAggtagaacaATGG | 714 |
| 34_1 | 70 | 4 | 35 | 13 | GCatccacagtaaaTT | 749 |
| 35_1 | 83 | 2 | 66 | 21 | GAaggttatttaaTTC | 773 |
| 36_1 | 18 | 2 | 15 | 5 | CTAAtcgaatgcaGCA | 805 |
| 37_1 | 64 | 7 | 35 | 10 | TACccaatctaatCGA | 813 |
| 38_1 | 69 | 1 | 49 | 13 | TAGttacccaatcTAA | 817 |
| 39_1 | 49 | 5 | 26 | 9 | CATttagttacccAAT | 821 |
| 40_1 | 23 | 7 | 8 | 2 | TCAtttagttaccCAA | 822 |
| 41_1 | 24 | 6 | 12 | 3 | TTcatttagttaCCCA | 823 |
| 42_1 | 51 | 7 | 40 | 5 | GAATtaatttcattTAGT | 829 |
| 43_1 | 71 | 9 | 45 | 3 | CAGTgaggaattaATTT | 837 |
| 44_1 | 60 | 5 | 45 | 17 | CCAAcagtgaggAATT | 842 |
| 45_1 | 63 | 1 | 37 | 15 | CCCaacagtgaggAAT | 843 |
| 46_1 | 31 | 3 | 29 | 12 | TAtacccaacagtgAGG | 846 |
| 47_1 | 44 | 3 | 27 | 0 | TTatacccaacagTGAG | 847 |
| 48_1 | 38 | 3 | 26 | 6 | TTTatacccaacagTGA | 848 |
| 49_1 | 20 | 4 | 7 | 1 | CCTttatacccaaCAG | 851 |
| 50_1 | 22 | 3 | 6 | 2 | TAACctttatacCCAA | 854 |
| 51_1 | 28 | 1 | 29 | 16 | AATaacctttataCCCA | 855 |
| 52_1 | 80 | 11 | 48 | 10 | GTAaataacctttaTA | 859 |
| 53_1 | 54 | 4 | 37 | 14 | ACTGtaaataacctTTAT | 860 |
| 54_1 | 81 | 4 | 53 | 15 | AT AtatatgcaatgAG | 903 |
| 55_1 | 86 | 12 | 70 | 15 | AGatatatatgcaaTG | 905 |
| 56_1 | 56 | 8 | 27 | 7 | GAGatatatatgcAAT | 906 |
| 57_1 | 28 | 7 | 13 | 5 | CCagagatatataTGC | 909 |
| 58_1 | 88 | 13 | 69 | 23 | CAATattccagagATAT | 915 |
| 59_1 | 29 | 3 | 14 | 6 | GCAAtattccagagATA | 916 |
| 60_1 | 25 | 3 | 14 | 3 | AGCaatattccagaGAT | 917 |
| 61_1 | 29 | 4 | 17 | 2 | CAGcaatattccAGAG | 919 |
| 62_1 | 27 | 3 | 14 | 3 | AATCagcaatattCCAG | 921 |
| 63_1 | 23 | 6 | 12 | 6 | ACAAtcagcaataTTCC | 923 |
| 64_1 | 53 | 9 | 43 | 15 | ACtaagtagttacactTCT | 957 |
| 65_1 | 32 | 5 | 14 | 6 | CTAAgtagttacactTC | 958 |
| 66_1 | 35 | 4 | 31 | 6 | GACtaagtagttacaCTT | 959 |
| 67_1 | 64 | 10 | 55 | 14 | TGActaagtagtTACA | 962 |
| 68_1 | 62 | 11 | 57 | 16 | CTTTgactaagtagTTA | 964 |
| 69_1 | 42 | 9 | 59 | 13 | CTCtttgactaagTAG | 967 |
| 70_1 | 81 | 6 | 56 | 12 | GCTCtttgactaagTA | 968 |
| 71_1 | 27 | 3 | 39 | 9 | CCttaaatactgtTGAC | 1060 |
| 72_1 | 75 | 5 | 36 | 7 | CTtaaatactgttgAC | 1060 |
| 73_1 | 35 | 6 | 43 | 13 | TCCttaaatactgTTG | 1062 |
| 74_1 | 57 | 4 | 79 | 25 | TCTCcttaaatactgTT | 1063 |
| 75_1 | 53 | 6 | 28 | 6 | TAtcatagttctCCTT | 1073 |
| 76_1 | 26 | 4 | 9 | 2 | AGTatcatagttcTCC | 1075 |
| 77_1 | 74 | 5 | 39 | 12 | GAgtatcatagttCTC | 1076 |
| 78_1 | 49 | 5 | 35 | 6 | AGagtatcatagTTCT | 1077 |
| 78_2 | 74 | 6 | 36 | 8 | AGAgtatcatagtTCT | 1077 |
| 79_1 | 19 | 2 | 19 | 13 | CAGagtatcatagTTC | 1078 |
| 80_1 | 23 | 2 | 26 | 2 | TTCAgagtatcataGT | 1080 |
| 81_1 | 35 | 3 | 36 | 11 | CTTcagagtatcATAG | 1081 |
| 82_1 | 24 | 6 | 20 | 7 | TTCTtcagagtatcaTA | 1082 |
| 83_1 | 20 | 2 | 16 | 2 | TTTcttcagagtaTCAT | 1083 |
| 84_1 | 33 | 4 | 37 | 10 | GAGAaaggctaagTTT | 1099 |
| 85_1 | 42 | 2 | 35 | 18 | GAcactcttgtaCATT | 1213 |
| 86_1 | 50 | 4 | 54 | 8 | TGagacactcttgtaCA | 1215 |
| 87_1 | 50 | 8 | 28 | 8 | TGagacactcttgTAC | 1216 |
| 88_1 | 61 | 4 | 33 | 6 | CTttattaaactCCAT | 1266 |
| 89_1 | 71 | 8 | 43 | 12 | ACCAaactttattaAA | 1272 |
| 90_1 | 62 | 5 | 42 | 9 | AAACctctactaagTG | 1288 |
| 91_1 | 22 | 3 | 12 | 5 | AGattaagacagtTGA | 1310 |
| 92_1 | 46 | 3 | ND | ND | AAgtaggagcaagaGGC | 1475 |
| 93_1 | 42 | 4 | 60 | 24 | AAAGtaggagcaagAGG | 1476 |
| 94_1 | 86 | 15 | 46 | 10 | GTtaagcagccaggAG | 1806 |
| 95_1 | 66 | 6 | 82 | 27 | AGggtaggatgggtAG | 1842 |
| 96_1 | 83 | 19 | 62 | 36 | AAGggtaggatgggTA | 1843 |
| 97_1 | 60 | 9 | 69 | 5 | CAAgggtaggatggGT | 1844 |
| 97_2 | 76 | 13 | 34 | 7 | CAagggtaggatggGT | 1844 |
| 98_1 | 65 | 8 | 76 | 28 | CCaagggtaggatgGG | 1845 |
| 99_1 | 61 | 2 | 75 | 17 | TCcaagggtaggatGG | 1846 |
| 100_1 | 83 | 4 | 82 | 13 | CTTCcaagggtaggAT | 1848 |
| 101_1 | 45 | 3 | 52 | 14 | A TCttccaagggtagGA | 1849 |
| 102_1 | 29 | 2 | 17 | 7 | AGaagtgatggctCATT | 1936 |
| 103_1 | 26 | 3 | 22 | 1 | AAGaagtgatggcTCAT | 1937 |
| 104_1 | 34 | 6 | 22 | 2 | GAAgaagtgatggcTCA | 1938 |
| 105_1 | 41 | 5 | 21 | 5 | ATGAaatgtaaacTGGG | 1955 |
| 106_1 | 40 | 8 | 29 | 6 | CAATgaaatgtaaaCTGG | 1956 |
| 107_1 | 24 | 3 | 16 | 4 | GCAAtgaaatgtaaACTG | 1957 |
| 108_1 | 30 | 4 | 20 | 6 | AGCAatgaaatgtaAACT | 1958 |
| 109_1 | 44 | 4 | 34 | 14 | GAGCaatgaaatgtAAAC | 1959 |
| 110_1 | 18 | 1 | 13 | 3 | TGaattcccatatcCGA | 1992 |
| 111_1 | 69 | 8 | 35 | 8 | AGaattatgaccaTAT | 2010 |
| 112_1 | 77 | 7 | 38 | 10 | AGGtaagaattatGACC | 2014 |
| 113_1 | 97 | 10 | 56 | 13 | TCAGgtaagaattaTGAC | 2015 |
| 114_1 | 69 | 8 | 54 | 21 | CTTCaggtaagaatTATG | 2017 |
| 115_1 | 91 | 7 | 115 | 42 | TCTTcaggtaagaATTA | 2019 |
| 116_1 | 88 | 6 | 104 | 36 | CTTCttcaggtaaGAAT | 2021 |
| 117_1 | 85 | 6 | 118 | 17 | TCTTcttcaggtaaGAA | 2022 |
| 118_1 | 105 | 14 | 102 | 9 | TCTtcttcaggtaAGA | 2023 |
| 119_1 | 37 | 2 | 76 | 18 | TGGtctaagagaaGAAG | 2046 |
| 120_1 | 46 | 6 | 81 | 11 | GTTGgtctaagagAAG | 2049 |
| 121_1 | 74 | 11 | 64 | 4 | AGTtggtctaagAGAA | 2050 |
| 122_1 | 74 | 9 | 55 | 21 | CAgttggtctaagAGAA | 2050 |
| 123_1 | 65 | 9 | 95 | 21 | GCAgttggtctaagagAA | 2050 |
| 124_1 | 63 | 7 | ND | ND | CAGTtggtctaagaGA | 2051 |
| 125_1 | 65 | 6 | ND | ND | GCagttggtctaagaGA | 2051 |
| 126_1 | 67 | 14 | 104 | 34 | GCagttggtctaaGAG | 2052 |
| 127_1 | 22 | 6 | 10 | 3 | CTcatatcagggCAGT | 2063 |
| 128_1 | 50 | 4 | 46 | 9 | CACAcatgttctttaAC | 2087 |
| 129_1 | 22 | 4 | 12 | 12 | TAAatacacacatgTTCT | 2092 |
| 130_1 | 24 | 2 | 43 | 28 | GTAAatacacacatgTTC | 2093 |
| 131_1 | 33 | 3 | 20 | 12 | TGTAaatacacacaTGTT | 2094 |
| 132_1 | 73 | 17 | 57 | 21 | GATCatgtaaatacACAC | 2099 |
| 133_1 | 47 | 5 | 28 | 14 | AGATcatgtaaataCACA | 2100 |
| 134_1 | 35 | 6 | 26 | 11 | CAAAgatcatgtaaatACAC | 2101 |
| 135_1 | 30 | 2 | 14 | 3 | ACAAagatcatgtaaaTACA | 2102 |
| 136_1 | 52 | 6 | 24 | 18 | GAATacaaagatcaTGTA | 2108 |
| 137_1 | 33 | 5 | 20 | 6 | AGAAtacaaagatcATGT | 2109 |
| 138_1 | 37 | 1 | 22 | 15 | CAGAatacaaagatCATG | 2110 |
| 139_1 | 85 | 6 | 53 | 8 | GCAGaatacaaagATCA | 2112 |
| 140_1 | 79 | 4 | 40 | 6 | AGGCagaatacaaagAT | 2114 |
| 141_1 | 56 | 2 | 53 | 20 | AAGGcagaatacaaAGA | 2115 |
| 142_1 | 28 | 5 | 20 | 5 | ATTagtgagggacGAA | 2132 |
| 143_1 | 26 | 2 | 22 | 10 | CAttagtgagggaCGA | 2133 |
| 144_1 | 29 | 6 | 16 | 4 | GAgggtgatggatTAG | 2218 |
| 145_1 | 45 | 6 | 22 | 5 | TTaggagtaataAAGG | 2241 |
| 146_1 | 65 | 7 | 44 | 9 | TTAatgaatttggtTG | 2263 |
| 147_1 | 84 | 8 | 43 | 10 | CTttaatgaatttgGT | 2265 |
| 148_1 | 32 | 0 | 15 | 3 | CATGgattacaactAA | 2322 |
| 149_1 | 33 | 2 | 20 | 4 | TCatggattacaaCTA | 2323 |
| 150_1 | 29 | 1 | 11 | 3 | GTCatggattacaaCT | 2324 |
| 151_1 | 64 | 2 | 40 | 9 | CAttaaatctagTCAT | 2335 |
| 152_1 | 97 | 8 | 63 | 22 | GACAttaaatctagTCA | 2336 |
| 153_1 | 92 | 7 | ND | ND | AGGGacattaaatcTA | 2340 |
| 154_1 | 35 | 4 | 25 | 15 | CAAAgcattataaCCA | 2372 |
| 155_1 | 34 | 3 | 24 | 6 | ACttactaggcaGAAG | 2415 |
| 156_1 | 102 | 6 | 113 | 18 | CAGAgttaactgtaCA | 2545 |
| 157_1 | 102 | 10 | 103 | 15 | CCAGagttaactgtAC | 2546 |
| 158_1 | 88 | 7 | 95 | 18 | GCcagagttaactgTA | 2547 |
| 159_1 | 78 | 10 | ND | ND | TGggccagagttaaCT | 2550 |
| 160_1 | 59 | 5 | 26 | 5 | CAgcatctatcagaCT | 2576 |
| 161_1 | 78 | 8 | 42 | 10 | TGAaataacatgagTCAT | 2711 |
| 162_1 | 31 | 6 | ND | ND | GTGaaataacatgAGTC | 2713 |
| 163_1 | 18 | 2 | 11 | 3 | TCTGtttatgtcacTG | 2781 |
| 164_1 | 56 | 5 | 29 | 9 | GTCTgtttatgtcaCT | 2782 |
| 165_1 | 37 | 8 | 12 | 5 | TGgtctgtttatGTCA | 2784 |
| 166_1 | 39 | 1 | 19 | 3 | TTGGtctgtttatgTC | 2785 |
| 167_1 | 41 | 3 | 35 | 14 | TCacccattgtttaAA | 2842 |
| 168_1 | 18 | 3 | 14 | 4 | TTcagcaaatatTCGT | 2995 |
| 169_1 | 36 | 8 | 13 | 2 | GTGtgttcagcaaATAT | 2999 |
| 170_1 | 18 | 2 | 11 | 4 | TCTattgttaggtATC | 3053 |
| 171_1 | 67 | 4 | 26 | 12 | ATtgcccatcttacTG | 3118 |
| 172_1 | 71 | 2 | 33 | 9 | TATtgcccatcttaCT | 3119 |
| 173_1 | 47 | 4 | 20 | 5 | AAatattgcccatCTT | 3122 |
| 174_1 | 74 | 4 | 34 | 7 | ATAaccttatcataCA | 3174 |
| 175_1 | 98 | 19 | 44 | 12 | TAtaaccttatcaTAC | 3175 |
| 176_1 | 100 | 10 | 64 | 11 | TTAtaaccttatcaTA | 3176 |
| 177_1 | 72 | 38 | 28 | 5 | TTTataaccttatCAT | 3177 |
| 178_1 | 47 | 6 | 34 | 6 | ACtgctattgctaTCT | 3375 |
| 179_1 | 41 | 3 | 23 | 6 | AGgactgctattgCTA | 3378 |
| 180_1 | 32 | 6 | 27 | 7 | GAGgactgctattgCT | 3379 |
| 181_1 | 83 | 1 | 46 | 20 | ACgtagaataataaCA | 3561 |
| 182_1 | 94 | 4 | 52 | 9 | CCaagtgatataATGG | 3613 |
| 183_1 | 49 | 2 | 16 | 3 | TTagcagaccaaGTGA | 3621 |
| 184_1 | 96 | 3 | 26 | 5 | GTttagcagaccaaGT | 3623 |
| 185_1 | 78 | 3 | 46 | 10 | TGacagtgattataTT | 3856 |
| 186_1 | 88 | 5 | 45 | 21 | TGTCcaagatattgAC | 3868 |
| 187_1 | 46 | 6 | 23 | 6 | GAAtatcctagatTGT | 4066 |
| 188_1 | 79 | 3 | 45 | 14 | CAaactgagaataTCC | 4074 |
| 189_1 | 63 | 5 | 27 | 8 | GCAaactgagaataTC | 4075 |
| 190_1 | 77 | 9 | 37 | 11 | TCCtattacaatcgTA | 4214 |
| 191_1 | 74 | 10 | 36 | 9 | TTCCtattacaatcGT | 4215 |
| 192_1 | 91 | 8 | 51 | 28 | ACtaatgggaggatTT | 4256 |
| 193_1 | 95 | 14 | 67 | 24 | TAgttcagagaataAG | 4429 |
| 194_1 | 86 | 5 | 47 | 16 | TAacatatagttcAGA | 4436 |
| 195_1 | 87 | 4 | 81 | 20 | ATAacatatagttcAG | 4437 |
| 196_1 | 101 | 6 | 67 | 20 | CAtaacatatagttCA | 4438 |
| 197_1 | 91 | 6 | 60 | 13 | TCataacatatagtTC | 4439 |
| 198_1 | 61 | 3 | 31 | 10 | TAGCtcctaacaatCA | 4507 |
| 199_1 | 79 | 12 | 49 | 11 | CTCCaatctttgtaTA | 4602 |
| 200_1 | 74 | 2 | 58 | 13 | TCTCcaatctttgtAT | 4603 |
| 201_1 | 53 | 3 | 33 | 10 | TCtatttcagccaaTC | 4708 |
| 202_1 | 25 | 4 | 30 | 9 | CGGaagtcagagtGAA | 4782 |
| 203_1 | 32 | 5 | 21 | 7 | TTAAgcatgaggaaTA | 4798 |
| 204_1 | 34 | 10 | 26 | 11 | TGAttgagcacctCTT | 4831 |
| 205_1 | 81 | 12 | 62 | 12 | GACtaattatttcgTT | 4857 |
| 206_1 | 57 | 7 | 37 | 7 | TGActaattatttCGT | 4858 |
| 207_1 | 26 | 5 | 21 | 6 | GTGactaattattTCG | 4859 |
| 208_1 | 48 | 3 | 33 | 13 | CTGCttgaaatgtgAC | 4870 |
| 209_1 | 32 | 1 | 34 | 13 | CCtgcttgaaatgTGA | 4871 |
| 210_1 | 60 | 5 | 50 | 19 | ATcctgcttgaaATGT | 4873 |
| 211_1 | 111 | 8 | 110 | 26 | ATTataaatctatTCT | 5027 |
| 212_1 | 107 | 1 | 67 | 12 | GCtaaatactttcATC | 5151 |
| 213_1 | 26 | 3 | 19 | 6 | CAttgtaacataCCTA | 5251 |
| 214_1 | 33 | 2 | 20 | 4 | GCattgtaacatacCT | 5252 |
| 215_1 | 89 | 8 | 53 | 16 | TAatattgcaccaaAT | 5295 |
| 216_1 | 25 | 2 | 29 | 9 | GAtaatattgcacCAA | 5297 |
| 217_1 | 27 | 1 | 27 | 6 | AGataatattgcacCA | 5298 |
| 218_1 | 79 | 6 | 45 | 11 | GCcaagaagataATAT | 5305 |
| 219_1 | 159 | 16 | 68 | 14 | CACAgccacataaaCT | 5406 |
| 220_1 | 90 | 2 | 72 | 12 | TTgtaattgtggaaAC | 5463 |
| 221_1 | 10 | 2 | 11 | 5 | TGacttgtaattgTGG | 5467 |
| 222_1 | 82 | 1 | 67 | 18 | TCtaactgaaatagTC | 5503 |
| 223_1 | 30 | 1 | 32 | 9 | GTGgttctaactgaAA | 5508 |
| 224_1 | 53 | 7 | 53 | 15 | CAatatgggacttgGT | 5522 |
| 225_1 | 44 | 1 | 33 | 10 | ATGacaatatgggaCT | 5526 |
| 226_1 | 49 | 1 | 41 | 14 | TATGacaatatgggAC | 5527 |
| 227_1 | 77 | 1 | 54 | 15 | ATATgacaatatggGA | 5528 |
| 228_1 | 100 | 3 | 98 | 29 | CTtcacttaataaTTA | 5552 |
| 229_1 | 90 | 12 | 80 | 19 | CTGCttcacttaatAA | 5555 |
| 230_1 | 91 | 0 | 79 | 23 | AAgactgcttcacTTA | 5559 |
| 231_1 | 49 | 8 | 77 | 34 | GAATgccctaattaTG | 5589 |
| 232_1 | 17 | 7 | 88 | 33 | TGGaatgccctaatTA | 5591 |
| 233_1 | 40 | 5 | 35 | 10 | GCAaatgccagtagGT | 5642 |
| 234_1 | 81 | 6 | 72 | 25 | CTAatggaaggattTG | 5673 |
| 235_1 | 97 | 17 | 87 | 25 | AAtatagaacctaaTG | 5683 |
| 236_1 | 98 | 4 | 83 | 21 | GAAagaatagaatGTT | 5769 |
| 237_1 | 93 | 2 | 102 | 26 | ATGggtaatagattAT | 5893 |
| 238_1 | 110 | 24 | 44 | 14 | GAaagagcacagggTG | 6103 |
| 239_1 | 66 | 5 | 36 | 10 | CTACatagagggaaTG | 6202 |
| 240_1 | 70 | 4 | 34 | 8 | GCttcctacataGAGG | 6207 |
| 241_1 | 64 | NA | 33 | 6 | TGCTtcctacatagAG | 6208 |
| 242_1 | 30 | NA | 19 | 7 | TGggcttgaaataTGT | 6417 |
| 243_1 | 88 | 6 | 69 | 15 | CATtatatttaagaAC | 6457 |
| 244_1 | 8 | 2 | 5 | 2 | TCggttatgttaTCAT | 6470 |
| 245_1 | 18 | 9 | 12 | 4 | CActttatctggTCGG | 6482 |
| 246_1 | 37 | 2 | 19 | 5 | AAAttggcacagcGTT | 6505 |
| 247_1 | 46 | 12 | 29 | 8 | ACCGtgacagtaaATG | 6577 |
| 248_1 | 31 | 2 | 25 | 2 | TGggaaccgtgacagTA | 6581 |
| 249_1 | 17 | 2 | 23 | 9 | CCacatataggtcCTT | 6597 |
| 250_1 | 15 | 6 | 23 | 7 | CAtattgctaccaTAC | 6617 |
| 251_1 | 4 | 2 | 9 | 2 | TCAtattgctaccATA | 6618 |
| 252_1 | 65 | 12 | 85 | 14 | CAATtgtcatatTGCT | 6624 |
| 253_1 | 20 | 2 | 51 | 7 | CATtcaattgtcataTTG | 6626 |
| 254_1 | 48 | 8 | 91 | 41 | TTTCtactgggaaTTTG | 6644 |
| 255_1 | 11 | 5 | 23 | 8 | CAAttagtgcagcCAG | 6672 |
| 256_1 | 43 | 7 | 62 | 13 | GAATaatgttcttaTCC | 6704 |
| 257_1 | 28 | 2 | 36 | 19 | CACAaattgaataatgtTCT | 6709 |
| 258_1 | 64 | 4 | 78 | 22 | CATGcacaaattgaaTAAT | 6714 |
| 259_1 | 53 | 8 | 104 | 73 | ATCctgcaatttcaCAT | 6832 |
| 260_1 | 54 | 5 | 59 | 14 | CCaccatagctgatCA | 6868 |
| 261_1 | 42 | 8 | 52 | 22 | ACcaccatagctgaTCA | 6868 |
| 262_1 | 68 | 5 | 118 | 66 | CAccaccatagctgaTC | 6869 |
| 263_1 | 40 | 2 | 73 | 20 | TAgtcggcaccaccAT | 6877 |
| 264_1 | 64 | 6 | 72 | 35 | CttgtagtcggcaccAC | 6880 |
| 265_1 | 56 | 4 | 82 | 35 | CttgtagtcggcacCA | 6881 |
| 266_1 | 41 | 5 | 46 | 21 | CGcttgtagtcggcAC | 6883 |
| 267_1 | 51 | 4 | 33 | 14 | TCAataaagatcagGC | 6942 |
| 268_1 | 61 | 2 | 49 | 10 | TGgacttacaagaaTG | 6986 |
| 269_1 | 45 | 7 | 40 | 9 | ATGgacttacaagaAT | 6987 |
| 270_1 | 51 | 12 | 36 | 12 | GCTCaagaaattggAT | 7073 |
| 271_1 | 17 | 0 | 14 | 5 | TACTgtagaacatgGC | 7133 |
| 272_1 | 15 | 3 | 11 | 3 | GCAAttcatttgaTCT | 7239 |
| 273_1 | 64 | 11 | ND | ND | TGaagggaggagggacAC | 7259 |
| 274_1 | 52 | 6 | 50 | 28 | AGtggtgaagggaggAG | 7265 |
| 275_1 | 79 | 7 | ND | ND | TAgtggtgaagggaggAG | 7265 |
| 276_1 | 81 | 6 | ND | ND | AtagtggtgaagggaggAG | 7265 |
| 277_1 | 70 | 9 | ND | ND | TAgtggtgaagggagGA | 7266 |
| 278_1 | 84 | 9 | ND | ND | ATagtggtgaagggagGA | 7266 |
| 279_1 | 40 | 6 | 64 | 53 | TAGtggtgaagggaGG | 7267 |
| 280_1 | 42 | 10 | ND | ND | ATAgtggtgaagggaGG | 7267 |
| 281_1 | 63 | 7 | ND | ND | GAtagtggtgaagggaGG | 7267 |
| 282_1 | 27 | 7 | 38 | 11 | ATAGtggtgaagggAG | 7268 |
| 283_1 | 60 | 22 | ND | ND | GAtagtggtgaaggGAG | 7268 |
| 284_1 | 23 | 3 | 97 | 54 | GAgatagtggtgAAGG | 7271 |
| 285_1 | 51 | 6 | 72 | 19 | CATGggagatagtgGT | 7276 |
| 286_1 | 7 | 1 | 21 | 9 | ACAAataatggttaCTCT | 7302 |
| 287_1 | 66 | 8 | 48 | 20 | ACACacaaataatgGTTA | 7306 |
| 288_1 | 67 | 6 | 58 | 20 | GAGggacacacaaaTAAT | 7311 |
| 289_1 | 46 | 2 | 50 | 21 | ATATagagaggcTCAA | 7390 |
| 290_1 | 22 | 6 | ND | ND | TTgatatagagaGGCT | 7393 |
| 291_1 | 11 | 2 | 17 | 3 | GCATttgatatagAGA | 7397 |
| 292_1 | 70 | 18 | 44 | 8 | TTtgcatttgataTAG | 7400 |
| 293_1 | 30 | 1 | 30 | 9 | CTGgaagaataggtTC | 7512 |
| 294_1 | 53 | 5 | 42 | 10 | ACTGgaagaataggTT | 7513 |
| 295_1 | 56 | 2 | 41 | 15 | TACTggaagaatagGT | 7514 |
| 296_1 | 80 | 8 | 53 | 13 | TGGCttatcctgtaCT | 7526 |
| 297_1 | 73 | 6 | 52 | 14 | ATggcttatcctGTAC | 7527 |
| 298_1 | 75 | 7 | 89 | 25 | TATGgcttatcctgTA | 7528 |
| 299_1 | 52 | 5 | 50 | 11 | GTAtggcttatccTGT | 7529 |
| 300_1 | 27 | 3 | 31 | 6 | ATgaatatatgccCAGT | 7547 |
| 301_1 | 41 | 8 | 33 | 9 | GAtgaatatatgCCCA | 7549 |
| 302_1 | 8 | 2 | ND | ND | CAAgatgaatataTGCC | 7551 |
| 303_1 | 32 | 5 | 37 | 14 | GACAacatcagtaTAGA | 7572 |
| 304_1 | 28 | 5 | 30 | 23 | CAAGacaacatcAGTA | 7576 |
| 305_1 | 47 | 5 | 41 | 9 | CACtcctagttccTTT | 7601 |
| 306_1 | 39 | 6 | 33 | 7 | AACactcctagttCCT | 7603 |
| 307_1 | 68 | 3 | 42 | 14 | TAacactcctagtTCC | 7604 |
| 308_1 | 115 | 5 | 69 | 22 | CTaacactcctagtTC | 7605 |
| 309_1 | 97 | 16 | 57 | 14 | TGataacataactgTG | 7637 |
| 310_1 | 36 | 1 | 23 | 10 | CTgataacataaCTGT | 7638 |
| 311_1 | 38 | 5 | 24 | 5 | TTTGaactcaagtgAC | 7654 |
| 312_1 | 42 | 3 | 39 | 5 | TCCTttacttagcTAG | 7684 |
| 313_1 | 15 | 2 | 14 | 3 | GAgtttggattagCTG | 7764 |
| 314_1 | 49 | 28 | ND | ND | TGggatatgacagGGA | 7838 |
| 315_1 | 34 | 6 | ND | ND | TGTGggatatgacaGG | 7840 |
| 316_1 | 47 | 3 | 37 | 8 | ATATggaagggataTC | 7875 |
| 317_1 | 11 | 3 | ND | ND | ACAggatatggaaGGG | 7880 |
| 318_1 | 48 | 4 | ND | ND | ATTTcaacaggatATGG | 7885 |
| 319_1 | 18 | 2 | 16 | 4 | GAgtaatttcaacAG G | 7891 |
| 320_1 | 74 | 6 | 44 | 5 | AGGGagtaatttcAACA | 7893 |
| 321_1 | 38 | 5 | 56 | 28 | ATTAgggagtaatTTCA | 7896 |
| 322_1 | 66 | 9 | 32 | 11 | CTtactattaggGAGT | 7903 |
| 323_1 | 13 | 1 | 15 | 5 | CAgcttactattaGGG | 7906 |
| 324_1 | 26 | 4 | 20 | 9 | TCAgcttactattAGG | 7907 |
| 325_1 | 43 | 4 | 17 | 2 | ATTtcagcttactaTTAG | 7908 |
| 326_1 | 54 | 5 | 57 | 16 | TTcagcttactaTTAG | 7908 |
| 327_1 | 28 | 3 | 8 | 2 | CAGAtttcagcttaCT | 7913 |
| 328_1 | 43 | 4 | 37 | 16 | GACtacaactagagGG | 7930 |
| 329_1 | 45 | 12 | 36 | 10 | AGACtacaactagaGG | 7931 |
| 330_1 | 99 | 8 | 94 | 32 | AAgactacaactagAG | 7932 |
| 331_1 | 59 | 4 | 52 | 19 | ATGAtttaattctagtCAAA | 7982 |
| 332_1 | 100 | 2 | 84 | 23 | TTTaattctagtcAAA | 7982 |
| 333_1 | 91 | 9 | 60 | 19 | GATTtaattctaGTCA | 7984 |
| 771_1 | 74 | 6 | 50 | 5 | TGAtttaattctaGTCA | 7984 |
| 334_1 | 73 | 5 | 54 | 12 | ATGAtttaattctagTCA | 7984 |
| 335_1 | 15 | 1 | 26 | 3 | GATGatttaattctagtCA | 7984 |
| 336_1 | 71 | 22 | 49 | 16 | GAtttaattctaGTCA | 7984 |
| 337_1 | 43 | 5 | 30 | 11 | GATGatttaattctaGTC | 7985 |
| 338_1 | 98 | 5 | 90 | 27 | TGatttaattctagTC | 7985 |
| 339_1 | 87 | 21 | 86 | 2 | GAGAtgatttaatTCTA | 7988 |
| 340_1 | 92 | 5 | 85 | 27 | GAGatgatttaatTCT | 7989 |
| 341_1 | 7 | 1 | 7 | 1 | CAGAttgatggtagTT | 8030 |
| 342_1 | 7 | 2 | 24 | 11 | CTcagattgatgGTAG | 8032 |
| 343_1 | 3 | 1 | 14 | 9 | GTTagccctcagaTTG | 8039 |
| 344_1 | 14 | 5 | 20 | 7 | TGtattgttagcCCTC | 8045 |
| 345_1 | 10 | 2 | 11 | 5 | ACttgtattgttAGCC | 8048 |
| 346_1 | 52 | 4 | 52 | 17 | AGCcagtatcagggAC | 8191 |
| 347_1 | 33 | 3 | 18 | 8 | TTgacaatagtgGCAT | 8213 |
| 348_1 | 7 | 2 | 13 | 5 | ACAagtggtatctTCT | 8228 |
| 349_1 | 63 | 8 | 44 | 15 | AATCtactttacaaGT | 8238 |
| 350_1 | 36 | 2 | ND | ND | CAcagtagatgcctGATA | 8351 |
| 351_1 | 24 | 2 | 30 | 9 | GAacacagtagatGCC | 8356 |
| 352_1 | 23 | 4 | 103 | 14 | CTTGgaacacagtagAT | 8359 |
| 353_1 | 20 | 2 | 45 | 2 | ATAtcttggaacaCAG | 8364 |
| 354_1 | 25 | 3 | 24 | 6 | TCTttaatatcttgGAAC | 8368 |
| 355_1 | 39 | 2 | 41 | 10 | TGatttctttaatatCTTG | 8372 |
| 356_1 | 54 | 5 | 88 | 43 | TGatgatttctttaaTATC | 8375 |
| 357_1 | 31 | 4 | 45 | 27 | AGGctaagtcatgaTG | 8389 |
| 358_1 | 18 | 3 | 43 | 20 | TTGAtgaggctaagTC | 8395 |
| 359_1 | 6 | 2 | 11 | 2 | CCAggattatactcTT | 8439 |
| 360_1 | 43 | 5 | 40 | 14 | GCcaggattataCTCT | 8440 |
| 361_1 | 56 | 8 | 73 | 13 | CTGccaggattataCT | 8442 |
| 362_1 | 23 | 1 | 33 | 7 | CAGAaacttatactttaTG | 8473 |
| 363_1 | 49 | 8 | 45 | 14 | AAGCagaaacttaTACT | 8478 |
| 364_1 | 39 | 6 | 37 | 4 | GAAgcagaaacttaTACT | 8478 |
| 365_1 | 26 | 4 | 45 | 13 | TGGaagcagaaacttataCT | 8478 |
| 366_1 | 21 | 4 | 44 | 5 | TGGaagcagaaacttaTAC | 8479 |
| 367_1 | 97 | 4 | 70 | 22 | AAgcagaaacttaTAC | 8479 |
| 368_1 | 34 | 3 | 32 | 11 | TGGaagcagaaactTATA | 8480 |
| 369_1 | 71 | 7 | 46 | 19 | AAGGgatattatggAG | 8587 |
| 370_1 | 51 | 9 | 79 | 38 | TGccggaagatttcCT | 8641 |
| 371_1 | 45 | 6 | 52 | 25 | ATGGattgggagtaGA | 8772 |
| 372_1 | 27 | 7 | 30 | 8 | AGatggattgggagTA | 8774 |
| 373_1 | 13 | 3 | 28 | 6 | AAGatggattgggaGT | 8775 |
| 374_1 | 42 | 10 | 44 | 11 | ACaagatggattGGGA | 8777 |
| 374_2 | 41 | 3 | 45 | 14 | ACaagatggattggGA | 8777 |
| 375_1 | 83 | 9 | 88 | 32 | AGAaggttcagaCTTT | 8835 |
| 376_1 | 40 | 5 | 33 | 3 | GCAgaaggttcagaCT | 8837 |
| 376_2 | 28 | 5 | 20 | 4 | GCagaaggttcagACT | 8837 |
| 377_1 | 70 | 2 | 43 | 8 | TGCAgaaggttcagAC | 8838 |
| 378_1 | 23 | 3 | 55 | 17 | AGtgcagaaggttCAG | 8840 |
| 378_2 | 51 | 6 | 41 | 8 | AGTGcagaaggttcAG | 8840 |
| 379_1 | 34 | 6 | 35 | 7 | AAGTgcagaaggttCA | 8841 |
| 380_1 | 44 | 11 | 24 | 6 | TAagtgcagaagGTTC | 8842 |
| 381_1 | 37 | 5 | 45 | 9 | TCtaagtgcagaAGGT | 8844 |
| 382_1 | 75 | 5 | 147 | 26 | CTCaggagttctactTC | 8948 |
| 383_1 | 90 | 10 | 141 | 55 | CTCaggagttctaCTT | 8949 |
| 384_1 | 73 | 8 | 234 | 116 | AtggaggtgactcaggAG | 8957 |
| 385_1 | 33 | 4 | 42 | 7 | ATggaggtgactcagGA | 8958 |
| 386_1 | 24 | 3 | 29 | 14 | ATggaggtgactcAGG | 8959 |
| 387_1 | 37 | 2 | 65 | 15 | TAtggaggtgactcAGG | 8959 |
| 388_1 | 50 | 10 | 81 | 19 | ATatggaggtgactcaGG | 8959 |
| 389_1 | 42 | 5 | 61 | 10 | TATGgaggtgactcAG | 8960 |
| 390_1 | 36 | 2 | 76 | 50 | ATatggaggtgacTCAG | 8960 |
| 391_1 | 52 | 6 | 64 | 6 | CAtatggaggtgactcAG | 8960 |
| 392_1 | 63 | 5 | 57 | 6 | ATAtggaggtgacTCA | 8961 |
| 393_1 | 53 | 7 | 64 | 12 | CAtatggaggtgacTCA | 8961 |
| 394_1 | 51 | 5 | 56 | 24 | CAtatggaggtgACTC | 8962 |
| 395_1 | 23 | 3 | 41 | 34 | GCatatggaggtgacTC | 8962 |
| 396_1 | 34 | 3 | 54 | 10 | TGcatatggaggtgacTC | 8962 |
| 397_1 | 54 | 5 | 71 | 24 | TtgcatatggaggtgacTC | 8962 |
| 398_1 | 61 | 11 | 59 | 13 | TttgcatatggaggtgacTC | 8962 |
| 399_1 | 25 | 2 | 30 | 6 | GCatatggaggtgaCT | 8963 |
| 400_1 | 34 | 4 | 25 | 9 | TGcatatggaggtgaCT | 8963 |
| 401_1 | 25 | 4 | 31 | 20 | TTGcatatggaggtgaCT | 8963 |
| 402_1 | 51 | 6 | 37 | 11 | TttgcatatggaggtgaCT | 8963 |
| 403_1 | 26 | 1 | 33 | 5 | TGCatatggaggtgAC | 8964 |
| 404_1 | 25 | 2 | 69 | 19 | TTGcatatggaggtGAC | 8964 |
| 405_1 | 26 | 4 | 24 | 4 | TTTGcatatggaggtgAC | 8964 |
| 406_1 | 19 | 3 | 20 | 7 | TTTGcatatggaggtGA | 8965 |
| 407_1 | 16 | 5 | 46 | 16 | TTtgcatatggaGGTG | 8966 |
| 408_1 | 9 | 2 | 9 | 6 | AAgtgaagttcaaCAGC | 8997 |
| 409_1 | 26 | 8 | 109 | 52 | TGggaagtgaagTTCA | 9002 |
| 410_1 | 31 | 5 | 24 | 5 | ATgggaagtgaagTTC | 9003 |
| 411_1 | 49 | 9 | 19 | 10 | GATGggaagtgaaGTT | 9004 |
| 412_1 | 28 | 10 | 17 | 9 | CTGtgatgggaagtGAA | 9007 |
| 413_1 | 54 | 4 | 34 | 8 | ATTgagtgaatccAAA | 9119 |
| 414_1 | 11 | 1 | 14 | 2 | AAttgagtgaatCCAA | 9120 |
| 415_1 | 58 | 6 | 14 | 2 | GATAattgagtgaaTCC | 9122 |
| 416_1 | 5 | 1 | 16 | 3 | GTGataattgagtGAA | 9125 |
| 417_1 | 73 | 5 | 61 | 14 | AAGaaaggtgcaaTAA | 9155 |
| 418_1 | 86 | 6 | 64 | 13 | CAagaaaggtgcAATA | 9156 |
| 419_1 | 75 | 19 | 64 | 14 | ACAAgaaaggtgcaAT | 9157 |
| 420_1 | 75 | 8 | 50 | 13 | ATttaaactcacaaAC | 9171 |
| 421_1 | 21 | 8 | 23 | 6 | CTgttaggttcaGCGA | 9235 |
| 422_1 | 54 | 10 | 30 | 5 | TCTGaatgaacatTTCG | 9260 |
| 423_1 | 11 | 4 | 15 | 5 | CTcattgaaggtTCTG | 9281 |
| 424_1 | 87 | 3 | 52 | 8 | CTAatctcattgaaGG | 9286 |
| 425_1 | 95 | 1 | 85 | 13 | CCtaatctcattgaAG | 9287 |
| 426_1 | 31 | 7 | 22 | 7 | ACTttgatctttcAGC | 9305 |
| 427_1 | 64 | 7 | 49 | 16 | ACtatgcaacacttTG | 9315 |
| 428_1 | 18 | 6 | 21 | 3 | CAAatagctttatCGG | 9335 |
| 429_1 | 19 | 6 | 17 | 4 | CCaaatagctttATCG | 9336 |
| 430_1 | 35 | 4 | 27 | 8 | TCCAaatagctttaTC | 9337 |
| 431_1 | 75 | 8 | 43 | 7 | GATCcaaatagcttTA | 9339 |
| 432_1 | 67 | 11 | 32 | 8 | ATgatccaaataGCTT | 9341 |
| 433_1 | 53 | 5 | 43 | 6 | TATGatccaaatagCT | 9342 |
| 434_1 | 97 | 9 | 66 | 29 | TAAAcagggctggGAAT | 9408 |
| 435_1 | 58 | 12 | 44 | 17 | ACttaaacagggCTGG | 9412 |
| 436_1 | 58 | 10 | 30 | 12 | ACacttaaacagGGCT | 9414 |
| 437_1 | 87 | 38 | 41 | 3 | GAACacttaaacAGGG | 9416 |
| 438_1 | 70 | 4 | 59 | 33 | AGAGaacacttaaACAG | 9418 |
| 439_1 | 83 | 17 | 28 | 9 | CTACagagaacaCTTA | 9423 |
| 440_1 | 49 | 12 | 27 | 4 | ATGctacagagaaCACT | 9425 |
| 441_1 | 53 | 10 | 24 | 13 | ATAAatgctacagagAACA | 9427 |
| 442_1 | 23 | 6 | 20 | 10 | AGataaatgctacaGAGA | 9430 |
| 443_1 | 48 | 6 | 27 | 7 | TAGAgataaatgcTACA | 9434 |
| 444_1 | 51 | 3 | 32 | 8 | TAGAtagagataaatGCT | 9437 |
| 445_1 | 38 | 5 | ND | ND | CAATatactagataGAGA | 9445 |
| 446_1 | 52 | 3 | 31 | 1 | TACAcaatatactagATAG | 9448 |
| 447_1 | 65 | 6 | 48 | 11 | CTAcacaatatacTAG | 9452 |
| 448_1 | 67 | 9 | 29 | 2 | GCTAcacaatatACTA | 9453 |
| 449_1 | 103 | 17 | 65 | 15 | ATATgctacacaatATAC | 9455 |
| 450_1 | 71 | 13 | 129 | 22 | TGATatgctacaCAAT | 9459 |
| 451_1 | 19 | 4 | 9 | 1 | ATGAtatgatatgCTAC | 9464 |
| 452_1 | 75 | 10 | 45 | 21 | GAGGagagagacaaTAAA | 9495 |
| 453_1 | 68 | 6 | 43 | 10 | CTAggaggagagagACA | 9500 |
| 454_1 | 72 | 7 | 79 | 25 | TATTctaggaggagAGA | 9504 |
| 455_1 | 31 | 3 | 29 | 9 | TTATattctaggagGAG | 9507 |
| 456_1 | 38 | 5 | 62 | 17 | GTTtatattctaGGAG | 9510 |
| 457_1 | 15 | 6 | 15 | 8 | TGgagtttatattcTAGG | 9512 |
| 458_1 | 34 | 3 | 21 | 3 | CGtaccaccactcTGC | 9590 |
| 459_1 | 41 | 5 | 55 | 22 | TGAGgaaatcattcATTC | 9641 |
| 460_1 | 81 | 8 | 47 | 22 | TTTGaggaaatcatTCAT | 9643 |
| 461_1 | 76 | 8 | 39 | 5 | AGGCtaatcctattTG | 9657 |
| 462_1 | 93 | 12 | 216 | 12 | TTTAggctaatcCTAT | 9660 |
| 463_1 | 15 | 6 | 30 | 9 | TGCtccagtgtaccCT | 9755 |
| 464_1 | 27 | 3 | 25 | 6 | TAgtagtactcgATAG | 9813 |
| 465_1 | 9 | 2 | 7 | 3 | CTAattgtagtagtaCTC | 9818 |
| 466_1 | 52 | 3 | 32 | 6 | TGctaattgtagTAGT | 9822 |
| 467_1 | 68 | 11 | 36 | 16 | AGTGctaattgtagTA | 9824 |
| 468_1 | 35 | 6 | 32 | 3 | GCAAgtgctaattgTA | 9827 |
| 469_1 | 91 | 9 | ND | ND | GAGGaaatgaactaattTA | 9881 |
| 470_1 | 92 | 5 | ND | ND | CAGGaggaaatgaacTA | 9886 |
| 471_1 | 67 | 5 | 42 | 6 | CCctagagtcattTCC | 9902 |
| 472_1 | 35 | 5 | 20 | 8 | ATCttacatgatgaAGC | 9925 |
| 473_1 | 13 | 1 | 20 | 5 | GACacactcagatttcAG | 9967 |
| 474_1 | 24 | 4 | 20 | 2 | AGacacactcagatttcAG | 9967 |
| 475_1 | 25 | 4 | 24 | 7 | AAGacacactcagatttcAG | 9967 |
| 476_1 | 26 | 6 | 19 | 4 | AGacacactcagattTCA | 9968 |
| 477_1 | 28 | 4 | 32 | 13 | AAGacacactcagattTCA | 9968 |
| 478_1 | 31 | 8 | 37 | 6 | AAagacacactcagatTTCA | 9968 |
| 479_1 | 63 | 7 | 51 | 26 | GAAagacacactcagatTTC | 9969 |
| 480_1 | 37 | 10 | ND | ND | AAGAcacactcagatTTC | 9969 |
| 481_1 | 41 | 4 | ND | ND | AAAGacacactcagaTTTC | 9969 |
| 482_1 | 19 | 5 | 48 | 14 | TGAAagacacactcagatTT | 9970 |
| 483_1 | 60 | 8 | 68 | 10 | TGaaagacacactcaGATT | 9971 |
| 484_1 | 42 | 8 | 63 | 22 | TGAaagacacactcaGAT | 9972 |
| 485_1 | 48 | 9 | 41 | 20 | ATTGaaagacacacTCA | 9975 |
| 486_1 | 27 | 6 | 27 | 12 | TCattgaaagacaCACT | 9977 |
| 487_1 | 88 | 13 | 121 | 33 | TTCcatcattgaAAGA | 9983 |
| 488_1 | 80 | 12 | ND | ND | ATAAtaccacttaTCAT | 10010 |
| 489_1 | 13 | 4 | 27 | 15 | TTacttaatttcttTGGA | 10055 |
| 490_1 | 32 | 5 | 60 | 24 | TTAgaactagctttaTCA | 10101 |
| 491_1 | 58 | 10 | 55 | 17 | GAGgtacaaatatAGG | 10171 |
| 492_1 | 4 | 1 | 12 | 3 | CTTatgatacaacTTA | 10384 |
| 493_1 | 37 | 6 | 35 | 5 | TCttatgatacaaCTT | 10385 |
| 494_1 | 30 | 0 | 27 | 6 | TTCttatgatacaaCT | 10386 |
| 495_1 | 27 | 8 | 18 | 3 | CAgtttcttatgaTAC | 10390 |
| 496_1 | 25 | 10 | 25 | 6 | GCAgtttcttatgaTA | 10391 |
| 497_1 | 77 | 6 | 72 | 29 | TACAaatgtctattagGTT | 10457 |
| 498_1 | 66 | 5 | 69 | 17 | TGTAcaaatgtctatTAG | 10460 |
| 499_1 | 27 | 10 | 20 | 4 | AGCatcacaattagTA | 10535 |
| 500_1 | 31 | 10 | 25 | 5 | CTAatgatagtgaaGC | 10548 |
| 501_1 | 21 | 7 | 30 | 8 | AGCtaatgatagtgAA | 10550 |
| 502_1 | 35 | 5 | 39 | 8 | ATGCcttgacatatTA | 10565 |
| 503_1 | 64 | 11 | 79 | 26 | CTCAagattattgACAC | 10623 |
| 504_2 | 25 | 4 | 83 | 32 | ACctcaagattaTTGA | 10626 |
| 504_1 | 94 | 7 | 22 | 6 | ACCtcaagattaTTGA | 10626 |
| 505_1 | 31 | 6 | 34 | 10 | AACCtcaagattatTG | 10627 |
| 506_1 | 55 | 6 | 62 | 17 | CACAaacctcaagattaTT | 10628 |
| 507_1 | 66 | 12 | 40 | 4 | GTActtaattagACCT | 10667 |
| 508_1 | 78 | 5 | 80 | 10 | AGTActtaattagACC | 10668 |
| 509_1 | 36 | 5 | 42 | 15 | GTATgaggtggtaaAC | 10688 |
| 510_1 | 40 | 4 | 48 | 22 | AGgaaacagcagaAGTG | 10723 |
| 511_1 | 27 | 7 | 13 | 6 | GCacaacccagaggAA | 10735 |
| 512_1 | 54 | 5 | ND | ND | CAAgcacaacccagAG | 10738 |
| 513_1 | 35 | 7 | ND | ND | TTCaagcacaaccCAG | 10740 |
| 514_1 | 49 | 6 | 52 | 15 | AAttcaagcacaACCC | 10742 |
| 515_1 | 72 | 4 | 106 | 49 | TAATaattcaagcacaaCC | 10743 |
| 516_1 | 43 | 4 | 57 | 21 | ACTAataattcaaGCAC | 10747 |
| 517_1 | 37 | 3 | 60 | 12 | ATAAtactaataattcAAGC | 10749 |
| 518_1 | 9 | 3 | 6 | 1 | TAgatttgtgagGTAA | 11055 |
| 519_1 | 59 | 10 | 31 | 5 | AGCCttaattctccAT | 11091 |
| 520_1 | 41 | 4 | 34 | 9 | AATGatctagagcCTTA | 11100 |
| 521_1 | 34 | 6 | 34 | 7 | CTAatgatctagaGCC | 11103 |
| 522_1 | 52 | 6 | 52 | 17 | ACTaatgatctaGAGC | 11104 |
| 523_1 | 60 | 4 | 54 | 10 | CATtaacatgttctTATT | 11165 |
| 524_1 | 57 | 4 | 55 | 8 | ACAAgtacattaacatGTTC | 11170 |
| 525_1 | 53 | 6 | 44 | 5 | TTACaagtacattaaCATG | 11173 |
| 526_1 | 54 | 11 | 49 | 17 | GCTTtattcatgtTTAT | 11195 |
| 527_1 | 34 | 7 | 17 | 5 | GCTttattcatgttTA | 11196 |
| 528_1 | 11 | 2 | 21 | 4 | AGAgctttattcatgtTT | 11197 |
| 529_1 | 22 | 4 | 33 | 7 | ATAAgagctttattCATG | 11200 |
| 530_1 | 30 | 5 | 32 | 15 | CATAagagctttaTTCA | 11202 |
| 531_1 | 77 | 8 | 24 | 4 | AGCAtaagagctTTAT | 11205 |
| 532_1 | 8 | 3 | 15 | 6 | TAGattgtttagtGCA | 11228 |
| 533_1 | 4 | 2 | 10 | 2 | GTagattgtttaGTGC | 11229 |
| 534_1 | 41 | 6 | 33 | 11 | GACAattctagtaGATT | 11238 |
| 535_1 | 50 | 1 | 37 | 7 | CTGacaattctaGTAG | 11241 |
| 536_1 | 49 | 7 | 36 | 6 | GCTGacaattctagTA | 11242 |
| 537_1 | 59 | 2 | 42 | 11 | AGgattaagatacgTA | 11262 |
| 538_1 | 28 | 11 | 28 | 4 | CAggattaagataCGT | 11263 |
| 539_1 | 96 | 5 | 20 | 6 | TCAggattaagataCG | 11264 |
| 540_1 | 70 | 11 | 59 | 11 | TTcaggattaagATAC | 11265 |
| 541_1 | 53 | 5 | 28 | 4 | AGGAagaaagtttgATTC | 11308 |
| 542_1 | 92 | 13 | 59 | 12 | TCAAggaagaaagtTTGA | 11311 |
| 543_1 | 44 | 3 | 67 | 7 | CTCAaggaagaaagTTTG | 11312 |
| 544_1 | 43 | 4 | 32 | 4 | TGCtcaaggaagaAAGT | 11315 |
| 545_1 | 41 | 7 | 44 | 20 | AATTatgctcaaggaAGA | 11319 |
| 546_1 | 11 | 4 | 26 | 8 | TAGGataccacattatGA | 11389 |
| 547_1 | 25 | 4 | 26 | 12 | CAtaatttattccattcCTC | 11449 |
| 548_1 | 64 | 6 | ND | ND | TGCAtaatttattcCAT | 11454 |
| 549_1 | 48 | 17 | 49 | 7 | ACTGcataatttatTCC | 11456 |
| 550_1 | 91 | 10 | 92 | 15 | CTAAactgcataattTATT | 11458 |
| 551_1 | 85 | 8 | 38 | 9 | ATaactaaactgCATA | 11465 |
| 552_1 | 86 | 4 | ND | ND | TTAttaataactaaaCTGC | 11468 |
| 553_1 | 91 | 13 | 92 | 21 | TAGTacattattaataaCT | 11475 |
| 554_1 | 50 | 4 | 37 | 7 | CATAactaaggacgTT | 11493 |
| 555_1 | 41 | 5 | 30 | 7 | TCataactaaggaCGT | 11494 |
| 556_1 | 80 | 7 | 55 | 13 | CGTCataactaaggAC | 11496 |
| 557_1 | 86 | 3 | 59 | 11 | TCgtcataactaagGA | 11497 |
| 558_1 | 51 | 9 | 33 | 12 | ATcgtcataactAAGG | 11498 |
| 559_1 | 91 | 6 | 65 | 26 | GTtagtatcttacATT | 11525 |
| 560_1 | 30 | 3 | 41 | 8 | CTCtattgttagtATC | 11532 |
| 561_1 | 59 | 8 | 18 | 6 | AGTatagagttacTGT | 11567 |
| 562_1 | 65 | 11 | 41 | 11 | TTCCtggtgatactTT | 11644 |
| 563_1 | 57 | 13 | 45 | 13 | GTTCctggtgatacTT | 11645 |
| 564_1 | 57 | 15 | 30 | 7 | TGttcctggtgataCT | 11646 |
| 565_1 | 17 | 4 | 35 | 4 | ATaaacatgaatctCTCC | 11801 |
| 566_1 | 16 | 3 | 30 | 4 | CTTtataaacatgaaTCTC | 11804 |
| 567_1 | 60 | 5 | 45 | 11 | CTGtctttataaaCATG | 11810 |
| 568_1 | 20 | 2 | 19 | 5 | TTgttataaatctgTCTT | 11820 |
| 569_1 | 68 | 9 | 44 | 4 | TTAaatttattcttgGATA | 11849 |
| 570_1 | 76 | 8 | 48 | 12 | CTtaaatttattctTGGA | 11851 |
| 571_1 | 62 | 5 | 66 | 5 | CTTCttaaatttattctTG | 11853 |
| 572_1 | 28 | 4 | 44 | 10 | TATGtttctcagtAAAG | 11877 |
| 573_1 | 29 | 6 | 36 | 11 | GAAttatctttaaACCA | 11947 |
| 574_1 | 74 | 6 | 34 | 7 | CCCttaaatttctaCA | 11980 |
| 575_1 | 37 | 8 | 30 | 9 | ACACtgctcttgtaCC | 11995 |
| 576_1 | 45 | 14 | 27 | 6 | TGAcaacactgctCTT | 12000 |
| 577_1 | 2 | 1 | 12 | 5 | TACAtttattgggcTC | 12081 |
| 578_1 | 65 | 14 | 39 | 9 | GTacatttattgGGCT | 12082 |
| 579_1 | 34 | 4 | 53 | 12 | TTGgtacatttatTGG | 12085 |
| 580_1 | 41 | 7 | 35 | 6 | CATGttggtacattTAT | 12088 |
| 581_1 | 11 | 4 | 12 | 5 | AATCatgttggtacAT | 12092 |
| 582_1 | 96 | 16 | 48 | 9 | AAatcatgttggtaCA | 12093 |
| 583_1 | 71 | 15 | 42 | 13 | GACaagtttggattAA | 12132 |
| 584_1 | 46 | 34 | 39 | 6 | AAtgttcagatgCCTC | 12197 |
| 585_1 | 37 | 26 | 28 | 12 | GCttaatgttcagaTG | 12201 |
| 586_1 | 75 | 8 | 43 | 12 | CGTAcatagcttgaTG | 12267 |
| 587_1 | 41 | 10 | 28 | 5 | GTGaggaattaggaTA | 12753 |
| 588_1 | 41 | 5 | 27 | 9 | GTAacaatatggttTG | 12780 |
| 589_1 | 67 | 10 | 37 | 7 | GAaatattgtagaCTA | 13151 |
| 590_1 | 97 | 10 | 80 | 12 | TTGaaatattgtagAC | 13153 |
| 591_1 | 64 | 10 | 47 | 9 | AAgtctagtaatTTGC | 13217 |
| 592_1 | 84 | 7 | 60 | 9 | GCTCagtagattatAA | 13259 |
| 593_1 | 42 | 8 | 32 | 9 | CATacactgttgcTAA | 13296 |
| 594_1 | 101 | 6 | 79 | 17 | ATGgtctcaaatcATT | 13314 |
| 595_1 | 53 | 14 | 46 | 7 | CAATggtctcaaatCA | 13316 |
| 596_1 | 47 | 6 | 36 | 6 | TTCCtattgattgaCT | 13568 |
| 597_1 | 97 | 12 | 41 | 6 | TTTCtgttcacaacAC | 13600 |
| 598_1 | 85 | 1 | 49 | 11 | AGgaacccactaaTCT | 13702 |
| 599_1 | 56 | 3 | 34 | 7 | TAAatggcaggaacCC | 13710 |
| 600_1 | 15 | 4 | 24 | 8 | GTAAatggcaggaaCC | 13711 |
| 601_1 | 40 | 6 | 26 | 8 | TTgtaaatggcagGAA | 13713 |
| 602_1 | 59 | 12 | 26 | 6 | TTatgagttaggCATG | 13835 |
| 603_1 | 62 | 2 | 42 | 10 | CCAggtgaaactttAA | 13935 |
| 604_1 | 77 | 9 | 55 | 18 | CCCttagtcagctCCT | 13997 |
| 605_1 | 82 | 13 | 42 | 11 | ACccttagtcagCTCC | 13998 |
| 606_1 | 74 | 1 | 39 | 10 | CAcccttagtcagCTC | 13999 |
| 607_1 | 76 | 9 | 30 | 8 | TCTcttactaggcTCC | 14091 |
| 608_1 | 82 | 5 | 50 | 13 | CCtatctgtcatcATG | 14178 |
| 609_1 | 82 | 1 | 48 | 12 | TCCtatctgtcatcAT | 14179 |
| 610_1 | 41 | 6 | 50 | 13 | GAGaagtgtgagaaGC | 14808 |
| 611_1 | 70 | 5 | 84 | 19 | CATCcttgaagtttAG | 14908 |
| 612_1 | 64 | 14 | 61 | 16 | TAAtaagatggctCCC | 15046 |
| 613_1 | 85 | 2 | 51 | 14 | CAAggcataataagAT | 15053 |
| 614_1 | 47 | 1 | 35 | 10 | CCaaggcataatAAGA | 15054 |
| 615_1 | 74 | 8 | 53 | 11 | TGatccaattctcaCC | 15151 |
| 616_1 | 63 | 4 | 41 | 11 | ATGatccaattctCAC | 15152 |
| 617_1 | 46 | 7 | 42 | 9 | CGCttcatcttcacCC | 15260 |
| 618_1 | 104 | 4 | 15 | 4 | TAtgacactgcaTCTT | 15317 |
| 619_1 | 8 | 3 | 8 | 5 | GTAtgacactgcaTCT | 15318 |
| 620_1 | 21 | 3 | 27 | 10 | TGtatgacactgCATC | 15319 |
| 621_1 | 37 | 7 | 38 | 11 | TTCTcttctgtaagTC | 15363 |
| 622_1 | 49 | 7 | 36 | 11 | TTctacagaggaACTA | 15467 |
| 623_1 | 47 | 1 | 32 | 10 | ACTacagttctacAGA | 15474 |
| 624_1 | 78 | 8 | 69 | 6 | TTCCcacaggtaaaTG | 15561 |
| 625_1 | 70 | 7 | ND | ND | ATTAtttgaatatactCATT | 15594 |
| 626_1 | 73 | 7 | 49 | 25 | TGGGaggaaattatTTG | 15606 |
| 627_1 | 80 | 5 | 64 | 11 | TGACtcatcttaaaTG | 15621 |
| 628_1 | 71 | 6 | 66 | 19 | CTGactcatcttaaAT | 15622 |
| 629_1 | 31 | 6 | 41 | 6 | TTTactctgactcATC | 15628 |
| 630_1 | 88 | 2 | 68 | 18 | TATtggaggaattaTT | 15642 |
| 631_1 | 53 | 2 | 27 | 6 | GTAttggaggaattAT | 15643 |
| 632_1 | 23 | 3 | 39 | 7 | TGgtatacttctctaagTAT | 15655 |
| 633_1 | 42 | 9 | 33 | 3 | GATCtcttggtataCT | 15666 |
| 634_1 | 38 | 1 | 30 | 16 | CAgacaactctataCC | 15689 |
| 635_1 | 10 | 2 | 19 | 3 | AACAtcagacaacTCTA | 15693 |
| 636_1 | 13 | 1 | 11 | 3 | TAACatcagacaacTC | 15695 |
| 637_1 | 14 | 2 | 27 | 2 | TTTAacatcagacaACTC | 15695 |
| 638_1 | 101 | 14 | 81 | 16 | ATttaacatcagacAA | 15698 |
| 639_1 | 14 | 1 | 17 | 1 | CCtatttaacatcAGAC | 15700 |
| 640_1 | 65 | 2 | ND | ND | TCCctatttaacaTCA | 15703 |
| 641_1 | 41 | 6 | 42 | 12 | TCAAcgactattgGAAT | 15737 |
| 642_1 | 37 | 2 | 29 | 5 | CTTAtattctggcTAT | 15850 |
| 643_1 | 31 | 7 | 35 | 4 | ATCCttatattctgGC | 15853 |
| 644_1 | 13 | 3 | 8 | 1 | GAtccttatattCTGG | 15854 |
| 645_1 | 25 | 5 | 20 | 4 | TGAtccttatattCTG | 15855 |
| 646_1 | 33 | 6 | 54 | 10 | ATTGaaacttgaTCCT | 15864 |
| 647_1 | 43 | 3 | 27 | 6 | ACtgtcattgaaACTT | 15870 |
| 648_1 | 54 | 7 | 32 | 12 | TCTtactgtcattgAA | 15874 |
| 649_1 | 12 | 1 | 25 | 2 | AGgatcttactgtCATT | 15877 |
| 650_1 | 13 | 4 | 11 | 3 | GCAaatcaactccATC | 15896 |
| 651_1 | 10 | 5 | 16 | 3 | GTGcaaatcaactCCA | 15898 |
| 652_1 | 7 | 0 | 36 | 18 | CAATtatttctttgTGC | 15910 |
| 653_1 | 21 | 3 | 31 | 7 | TGGcaacaattattTCTT | 15915 |
| 654_1 | 75 | 9 | 73 | 24 | GCTggcaacaatTATT | 15919 |
| 655_1 | 21 | 6 | 39 | 6 | ATCCatttctactgCC | 15973 |
| 656_1 | 25 | 3 | 38 | 8 | TAATatctattgattTCTA | 15988 |
| 657_1 | 14 | 2 | 11 | 5 | TCaatagtgtagggCA | 16093 |
| 658_1 | 11 | 4 | 10 | 3 | TTCaatagtgtaggGC | 16094 |
| 659_1 | 18 | 1 | 32 | 12 | AGGTtaattaattcaATAG | 16102 |
| 660_1 | 33 | 7 | 25 | 10 | CATttgtaatccCTAG | 16163 |
| 660_2 | 64 | 14 | 31 | 8 | CATttgtaatcccTAG | 16163 |
| 661_1 | 48 | 6 | 34 | 6 | ACAtttgtaatccCTA | 16164 |
| 662_2 | 29 | 6 | 23 | 5 | AAcatttgtaatCCCT | 16165 |
| 662_1 | 30 | 6 | 18 | 6 | AACatttgtaatCCCT | 16165 |
| 663_1 | 49 | 1 | 26 | 6 | TAaatttcaagttCTG | 16184 |
| 664_1 | 17 | 3 | 30 | 10 | GTTtaaatttcaagTTCT | 16185 |
| 665_1 | 22 | 7 | 40 | 9 | CCAAgtttaaatttCAAG | 16189 |
| 666_1 | 89 | 11 | ND | ND | ACCCaagtttaaaTTTC | 16192 |
| 667_1 | 60 | 16 | 87 | 8 | CAtacagtgacccaagTTT | 16199 |
| 668_1 | 65 | 9 | 50 | 12 | ACatcccatacagTGA | 16208 |
| 669_1 | 83 | 8 | 103 | 4 | AGcacagctctaCATC | 16219 |
| 670_1 | 80 | 9 | 150 | 36 | ATAtagcacagcTCTA | 16223 |
| 671_1 | 57 | 14 | ND | ND | TCCatatagcacagCT | 16226 |
| 672_1 | 53 | 10 | 106 | 8 | ATTtccatatagCACA | 16229 |
| 673_1 | 78 | 3 | 96 | 14 | TTTAtttccatatAGCA | 16231 |
| 674_1 | 77 | 9 | 31 | 7 | TTTatttccatatAGC | 16232 |
| 675_1 | 32 | 6 | ND | ND | AAGGagaggagatTATG | 16409 |
| 676_1 | 32 | 5 | 24 | 6 | AGTtcttgtgttagCT | 16456 |
| 677_1 | 19 | 4 | 17 | 4 | GAgttcttgtgttaGC | 16457 |
| 678_1 | 14 | 3 | 25 | 3 | ATTaattatccatCCAC | 16590 |
| 679_1 | 11 | 2 | 20 | 6 | ATCaattaattatcCATC | 16593 |
| 680_1 | 31 | 5 | 40 | 11 | AGAatcaattaattaTCC | 16596 |
| 681_1 | 8 | 3 | 30 | 10 | TGagataccgtgcaTG | 16656 |
| 682_1 | 11 | 3 | ND | ND | AAtgagataccgTGCA | 16658 |
| 683_1 | 15 | 3 | 33 | 10 | CTGtggttaggctaAT | 16834 |
| 684_1 | 45 | 7 | 38 | 7 | AagagtaagggtctgtggTT | 16842 |
| 685_1 | 24 | 5 | ND | ND | GATGggttaagagTAA | 16854 |
| 686_1 | 11 | 2 | ND | ND | AGCagatgggttaaGA | 16858 |
| 687_1 | ND | ND | 51 | 7 | TGtaaacatttgTAGC | 16886 |
| 688_1 | 83 | 1 | 54 | 11 | CCTgcttataaatgTA | 16898 |
| 689_1 | 103 | 4 | 73 | 14 | TGCCctgcttataaAT | 16901 |
| 690_1 | 104 | 2 | 64 | 22 | TCttcttagttcaaTA | 16935 |
| 691_1 | ND | ND | 60 | 9 | TGgtttctaactACAT | 16980 |
| 692_1 | ND | ND | 94 | 22 | AGtttggtttctaaCTA | 16983 |
| 693_1 | 8 | 2 | 17 | 5 | GAAtgaaacttgcCTG | 17047 |
| 694_1 | 98 | 6 | 51 | 9 | ATTatccttacatGAT | 17173 |
| 695_1 | 48 | 4 | 18 | 4 | GTacccaattatcCTT | 17180 |
| 696_1 | 94 | 2 | 48 | 9 | TGTacccaattatCCT | 17181 |
| 697_1 | 31 | 5 | 42 | 13 | TTgtacccaattaTCC | 17182 |
| 698_1 | 41 | 4 | 39 | 6 | TTTgtacccaattaTC | 17183 |
| 699_1 | 63 | 0 | 28 | 12 | AGCAgcaggttataTT | 17197 |
| 700_1 | 99 | 6 | 43 | 12 | TGGgaagtggtctGGG | 17292 |
| 701_1 | 103 | 2 | 28 | 5 | CTGgagagtgataaTA | 17322 |
| 702_1 | 52 | 6 | 27 | 9 | AATGctggattacgTC | 17354 |
| 703_1 | 67 | 3 | 37 | 7 | CAatgctggattaCGT | 17355 |
| 704_1 | 36 | 10 | 80 | 12 | TTgttcagaagtATCC | 17625 |
| 705_1 | 19 | 9 | 47 | 9 | GAtgatttgcttGGAG | 17646 |
| 706_1 | 44 | NA | 60 | 9 | GAAatcattcacaACC | 17860 |
| 707_1 | 46 | 9 | 32 | 9 | TTGtaacatctacTAC | 17891 |
| 708_1 | 56 | 0 | 79 | 17 | CATtaagcagcaagTT | 17923 |
| 709_1 | 30 | 9 | 46 | 7 | TTActagatgtgagCA | 17942 |
| 710_1 | 29 | 4 | 36 | 6 | TTtactagatgtgAG C | 17943 |
| 711_1 | 41 | 13 | 41 | 6 | GACcaagcaccttaCA | 17971 |
| 712_1 | 36 | 19 | 49 | 11 | AGAccaagcacctTAC | 17972 |
| 713_1 | 30 | 6 | 34 | 7 | ATgggttaaataAAGG | 18052 |
| 714_1 | 70 | 2 | 24 | 8 | TCaaccagagtattAA | 18067 |
| 715_1 | 11 | 4 | 26 | 8 | GTCaaccagagtatTA | 18068 |
| 716_1 | 126 | 56 | 26 | 6 | ATtgtaaagctgaTAT | 18135 |
| 717_1 | 73 | 1 | 42 | 10 | CAcataattgtaAAGC | 18141 |
| 718_1 | 23 | 9 | 55 | 18 | GAggtctgctattTAC | 18274 |
| 719_1 | 50 | 1 | 42 | 11 | TGtagattcaatgCCT | 18404 |
| 720_1 | 79 | 3 | 39 | 10 | CCtcattatactaTGA | 18456 |
| 721_1 | 27 | 6 | 30 | 8 | CCttatgctatgacAC | 18509 |
| 722_1 | 26 | 7 | 50 | 13 | TCCTtatgctatgaCA | 18510 |
| 723_1 | 59 | 1 | 48 | 12 | AAGatgtttaagtATA | 18598 |
| 724_1 | 54 | 2 | 50 | 13 | CTgattattaagATGT | 18607 |
| 725_1 | 92 | 10 | 84 | 19 | TGgaaaggtatgaaTT | 18808 |
| 726_1 | 24 | 8 | 61 | 16 | ACttgaatggcttgGA | 18880 |
| 727_1 | 8 | 4 | 51 | 14 | AACttgaatggctTGG | 18881 |
| 728_1 | 35 | 4 | 35 | 10 | CAATgtgttactatTT | 19004 |
| 729_1 | 36 | 9 | 53 | 11 | ACAatgtgttactATT | 19005 |
| 730_1 | 70 | 2 | 41 | 11 | CATCtgctatataaGA | 19063 |
| 731_1 | 38 | NA | 42 | 9 | CCTAgagcaaatacTT | 19223 |
| 732_1 | 102 | 15 | 15 | 4 | CAG agttaataatAAG | 19327 |
| 733_1 | 37 | 10 | 8 | 5 | GTTCaagcacaacgAA | 19493 |
| 734_1 | 13 | 1 | 38 | 11 | AGggttcaagcacAAC | 19496 |
| 735_1 | 49 | NA | 36 | 11 | TGttggagacactgTT | 19677 |
| 736_1 | 48 | NA | 32 | 10 | AAGgaggagttaggAC | 19821 |
| 737_1 | 36 | NA | 64 | 11 | CTATgccatttacgAT | 19884 |
| 738_1 | 105 | 19 | 66 | 19 | TCaaatgcagaattAG | 19913 |
| 739_1 | 44 | NA | 41 | 6 | AGtgacaatcaaATGC | 19921 |
| 740_1 | 107 | NA | 68 | 18 | AAgtgacaatcaaATG | 19922 |
| 741_1 | 102 | 4 | 27 | 6 | GTGtaccaagtaacAA | 19978 |
| 742_1 | 110 | 10 | 30 | 16 | TGGgatgttaaacTGA | 20037 |

### Example 2 - Testing in vitro efficacy in a dose response curve

A selection of oligonucleotides from Table 10 were tested in KARPAS-299 cells using half-log serial dilutions in in PBS (50 µM, 15.8 µM, 5.0 µM, 1.58 µM, 0.5 µM, 0.158 µM, 0.05 µM, to 0.0158 µM oligonucleotide) in the in vitro efficacy assay described in Example 1. IC 50 and max inhibition (% residual PD-L1 expression) was assessed for the oligonucleotides.

EC50 calculations were performed in GraphPad Prism6. The IC50 and maximum PD-L1 knock down level is shown in table 11 as % of control (PBS) treated cells.

**Table 11: Max inhibition as % of saline and EC50 in KARPAS-299 cell line.**

| **CMP ID NO** | **Max Inhibition (% residual PD-L1 expression; % of saline-treated)** | | **EC50 (µM)** | | **Compound CMP** | **Start on SEQ ID NO: 1** |
|---|---|---|---|---|---|---|
| | **Avg** | **SD** | **Avg** | **SD** | | |
| 6_1 | 11 | 3.3 | 0.69 | 0.11 | TCGCataagaatgaCT | 371 |
| 8_1 | 29 | 1.7 | 0.06 | 0.01 | CTGaacacacagtCGC | 383 |
| 9_1 | 19 | 1.7 | 0.23 | 0.02 | TCTgaacacacagtCG | 384 |
| 13_1 | 14 | 4.7 | 0.45 | 0.12 | CTtacttagatgcTGC | 495 |
| 41_1 | 10 | 1.8 | 0.19 | 0.02 | TCAtttagttaccCAA | 822 |
| 42_1 | 17 | 1.3 | 0.19 | 0.02 | TTcatttagttaCCCA | 823 |
| 58_1 | 23 | 1.5 | 0.17 | 0.01 | CCagagatatataTGC | 909 |
| 77_1 | 24 | 2.4 | 0.16 | 0.02 | AGTatcatagttcTCC | 1075 |
| 92_1 | 12 | 2.4 | 0.25 | 0.03 | AGattaagacagtTGA | 1310 |
| 111_1 | 3 | 2.0 | 0.27 | 0.03 | TGaattcccatatcCGA | 1992 |
| 128_1 | 11 | 1.8 | 0.25 | 0.03 | CTcatatcagggCAGT | 2063 |
| 151_1 | 16 | 2.7 | 0.28 | 0.05 | GTCatggattacaaCT | 2324 |
| 164_1 | 19 | 1.6 | 0.15 | 0.01 | TCTGtttatgtcacTG | 2781 |
| 166_1 | 36 | 1.7 | 0.11 | 0.02 | TGgtctgtttatGTCA | 2784 |
| 169_1 | 10 | 1.6 | 0.22 | 0.02 | TTcagcaaatatTCGT | 2995 |
| 171_1 | 12 | 2.0 | 0.21 | 0.02 | TCTattgttaggtATC | 3053 |
| 222_1 | 1 | 2.0 | 0.21 | 0.02 | TGacttgtaattgTGG | 5467 |
| 233_1 | 1 | 4.3 | 0.89 | 0.17 | TGGaatgccctaatTA | 5591 |
| 245_1 | 4 | 2.0 | 0.17 | 0.02 | TCggttatgttaTCAT | 6470 |
| 246_1 | 7 | 2.1 | 0.25 | 0.03 | CActttatctggTCGG | 6482 |
| 250_1 | 0 | 2.5 | 0.23 | 0.03 | CCacatataggtcCTT | 6597 |
| 251_1 | 0 | 2.8 | 0.75 | 0.10 | CAtattgctaccaTAC | 6617 |
| 252_1 | 3 | 2.2 | 0.19 | 0.02 | TCAtattgctaccATA | 6618 |
| 256_1 | 5 | 2.2 | 0.32 | 0.03 | CAAttagtgcagcCAG | 6672 |
| 272_1 | 1 | 3.2 | 0.69 | 0.10 | TACTgtagaacatgGC | 7133 |
| 273_1 | 3 | 2.8 | 0.28 | 0.04 | GCAAttcatttgaTCT | 7239 |
| 287_1 | 1 | 1.4 | 0.13 | 0.01 | ACAAataatggttaCTCT | 7302 |
| 292_1 | 2 | 2.1 | 0.21 | 0.02 | GCATttgatatagAGA | 7397 |
| 303_1 | 0 | 1.2 | 0.21 | 0.01 | CAAgatgaatataTGCC | 7551 |
| 314_1 | 3 | 2.1 | 0.39 | 0.04 | GAgtttggattagCTG | 7764 |
| 318_1 | 3 | 1.4 | 0.14 | 0.01 | ACAggatatggaaGGG | 7880 |
| 320_1 | 2 | 2.4 | 0.22 | 0.03 | GAgtaatttcaacAGG | 7891 |
| 324_1 | 0 | 2.4 | 0.44 | 0.05 | CAgcttactattaGGG | 7906 |
| 336_1 | 0 | 2.5 | 0.21 | 0.03 | GATGatttaattctagtCA | 7984 |
| 342_1 | 1 | 2.2 | 0.12 | 0.01 | CAGAttgatggtagTT | 8030 |
| 343_1 | 4 | 1.8 | 0.11 | 0.01 | CTcagattgatgGTAG | 8032 |
| 344_1 | 0 | 0.9 | 0.12 | 0.01 | GTTagccctcagaTTG | 8039 |
| 345_1 | 0 | 2.3 | 0.36 | 0.04 | TGtattgttagcCCTC | 8045 |
| 346_1 | 1 | 2.1 | 0.22 | 0.02 | ACttgtattgttAGCC | 8048 |
| 349_1 | 4 | 2.9 | 0.21 | 0.03 | ACAagtggtatctTCT | 8228 |
| 359_1 | 6 | 2.9 | 0.39 | 0.05 | TTGAtgaggctaagTC | 8395 |
| 360_1 | 0 | 1.7 | 0.18 | 0.02 | CCAggattatactcTT | 8439 |
| 374_1 | 5 | 1.7 | 0.33 | 0.03 | AAGatggattgggaGT | 8775 |
| 408_1 | 3 | 1.8 | 0.21 | 0.02 | TTtgcatatggaGGTG | 8966 |
| 409_1 | 0 | 1.8 | 0.21 | 0.02 | AAgtgaagttcaaCAGC | 8997 |
| 415_1 | 0 | 1.4 | 0.23 | 0.02 | AAttgagtgaatCCAA | 9120 |
| 417_1 | 7 | 0.9 | 0.15 | 0.01 | GTGataattgagtGAA | 9125 |
| 424_1 | 6 | 3.2 | 0.19 | 0.03 | CTcattgaaggtTCTG | 9281 |
| 429_1 | 5 | 2.5 | 0.48 | 0.05 | CAAatagctttatCGG | 9335 |
| 430_1 | 1 | 2.7 | 0.68 | 0.09 | CCaaatagctttATCG | 9336 |
| 458_1 | 0 | 4.1 | 0.35 | 0.07 | TGgagtttatattcTAGG | 9512 |
| 464_1 | 0 | 4.1 | 0.56 | 0.10 | TGCtccagtgtaccCT | 9755 |
| 466_1 | 1 | 2.1 | 0.21 | 0.02 | CTAattgtagtagtaCTC | 9818 |
| 474_1 | 0 | 2.4 | 0.27 | 0.03 | GACacactcagatttcAG | 9967 |
| 490_1 | 0 | 1.9 | 0.29 | 0.03 | TTacttaatttcttTGGA | 10055 |
| 493_1 | 3 | 1.8 | 0.20 | 0.02 | CTTatgatacaacTTA | 10384 |
| 512_1 | 0 | 3.3 | 0.63 | 0.10 | GCacaacccagaggAA | 10735 |
| 519_1 | 5 | 1.5 | 0.15 | 0.01 | TAgatttgtgagGTAA | 11055 |
| 529_1 | 0 | 2.7 | 0.24 | 0.03 | AGAgctttattcatgtTT | 11197 |
| 533_1 | 6 | 1.5 | 0.14 | 0.01 | TAGattgtttagtGCA | 11228 |
| 534_1 | 5 | 0.9 | 0.06 | 0.00 | GTagattgtttaGTGC | 11229 |
| 547_1 | 1 | 1.6 | 0.26 | 0.02 | TAGGataccacattatGA | 11389 |
| 566_1 | 0 | 3.0 | 0.40 | 0.06 | ATaaacatgaatctCTCC | 11801 |
| 567_1 | 2 | 2.5 | 0.34 | 0.04 | CTTtataaacatgaaTCTC | 11804 |
| 578_1 | 2 | 1.3 | 0.09 | 0.01 | TACAtttattgggcTC | 12081 |
| 582_1 | 1 | 1.6 | 0.20 | 0.02 | AATCatgttggtacAT | 12092 |
| 601_1 | 1 | 2.1 | 0.47 | 0.05 | GTAAatggcaggaaCC | 13711 |
| 619_1 | 4 | 3.4 | 0.44 | 0.08 | TAtgacactgcaTCTT | 15317 |
| 620_1 | 1 | 1.2 | 0.12 | 0.01 | GTAtgacactgcaTCT | 15318 |
| 636_1 | 0 | 1.3 | 0.19 | 0.01 | AACAtcagacaacTCTA | 15693 |
| 638_1 | 0 | 2.2 | 0.36 | 0.04 | TAACatcagacaacTC | 15695 |
| 637_1 | 0 | 2.1 | 0.21 | 0.02 | TTTAacatcagacaACTC | 15695 |
| 640_1 | 2 | 3.3 | 0.42 | 0.06 | CCtatttaacatcAGAC | 15700 |
| 645_1 | 1 | 2.9 | 0.34 | 0.04 | GAtccttatattCTGG | 15854 |
| 650_1 | 0 | 2.4 | 0.24 | 0.03 | AGgatcttactgtCATT | 15877 |
| 651_1 | 4 | 3.4 | 0.33 | 0.05 | GCAaatcaactccATC | 15896 |
| 652_1 | 0 | 1.3 | 0.16 | 0.01 | GTGcaaatcaactCCA | 15898 |
| 653_1 | 4 | 2.0 | 0.09 | 0.01 | CAATtatttctttgTGC | 15910 |
| 658_1 | 3 | 1.6 | 0.32 | 0.02 | TCaatagtgtagggCA | 16093 |
| 659_1 | 5 | 1.4 | 0.20 | 0.01 | TTCaatagtgtaggGC | 16094 |
| 660_1 | 4 | 2.1 | 0.22 | 0.02 | AGGTtaattaattcaATAG | 16102 |
| 665_1 | 3 | 1.8 | 0.18 | 0.02 | GTTtaaatttcaagTTCT | 16185 |
| 678_1 | 3 | 2.1 | 0.43 | 0.04 | GAgttcttgtgttaGC | 16457 |
| 679_1 | 0 | 3.5 | 0.31 | 0.05 | ATTaattatccatCCAC | 16590 |
| 680_1 | 4 | 1.6 | 0.12 | 0.01 | ATCaattaattatcCATC | 16593 |
| 682_1 | 3 | 2.4 | 0.27 | 0.03 | TGagataccgtgcaTG | 16656 |
| 683_1 | 0 | 3.2 | 0.16 | 0.03 | AAtgagataccgTGCA | 16658 |
| 684_1 | 2 | 2.3 | 0.25 | 0.03 | CTGtggttaggctaAT | 16834 |
| 687_1 | 5 | 1.3 | 0.13 | 0.01 | AGCagatgggttaaGA | 16858 |
| 694_1 | 0 | 1.7 | 0.16 | 0.02 | GAAtgaaacttgcCTG | 17047 |
| 706_1 | 15 | 3.6 | 0.27 | 0.06 | GAtgatttgcttGGAG | 17646 |
| 716_1 | 10 | 2.1 | 0.15 | 0.02 | GTCaaccagagtatTA | 18068 |
| 728_1 | 5 | 1.2 | 0.09 | 0.01 | AACttgaatggctTGG | 18881 |
| 733_1 | 0 | 12.7 | 8.01 | 3.62 | CAGagttaataatAAG | 19327 |
| 734_1 | 0 | 14.6 | 3.49 | 2.39 | GTTCaagcacaacgAA | 19493 |
| 735_1 | 0 | 2.5 | 0.30 | 0.04 | AGggttcaagcacAAC | 19496 |

A selection of oligonucleotides from Table 6 were tested in THP-1 cells using 1:3 serial in water from 25 µM to 0.004 µM in the in vitro efficacy assay described in Example 1. IC 50 and max inhibition (Percent residual PD-L1 expresson) was assessed for the oligonucleotides.

EC50 calculations were performed in GraphPad Prism6. The IC50 and maximum PD-L1 knock down level is shown in table 12 as % of control (PBS) treated cells.

**Table 12: Max inhibition as % of saline and EC50 in THP1 cell line.**

| **CMP ID NO** | **Max Inhibition (% residual PD-L1 expression; % of saline)** | | **EC50 (µM)** | | **Compound CMP** | **Start on SEQ ID NO: 1** |
|---|---|---|---|---|---|---|
| | **Avq** | **SD** | **Avg** | **SD** | | |
| 6_1 | 12 | 11.5 | 0.73 | 0.38 | TCGCataagaatgaCT | 371 |
| 8_1 | 6 | 5.6 | 0.11 | 0.04 | CTGaacacacagtCGC | 383 |
| 9_1 | 1 | 14.3 | 0.36 | 0.27 | TCTgaacacacagtCG | 384 |
| 13_1 | 2 | 12.4 | 0.49 | 0.31 | CTtacttagatgcTGC | 495 |
| 41_1 | 14 | 14.6 | 0.38 | 0.27 | TCAtttagttaccCAA | 822 |
| 42_1 | 21 | 10.4 | 0.22 | 0.10 | TTcatttagttaCCCA | 823 |
| 58_1 | 6 | 19.8 | 0.97 | 0.81 | CCagagatatataTGC | 909 |
| 77_1 | 5 | 4.8 | 0.14 | 0.04 | AGTatcatagttcTCC | 1075 |
| 92_1 | 0 | 12.9 | 0.57 | 0.39 | AGattaagacagtTGA | 1310 |
| 128_1 | 15 | 10.1 | 0.23 | 0.13 | CTcatatcagggCAGT | 2063 |
| 151_1 | 9 | 14.4 | 0.18 | 0.15 | GTCatggattacaaCT | 2324 |
| 164_1 | 16 | 22.0 | 0.57 | 0.60 | TCTGtttatgtcacTG | 2781 |
| 166_1 | 13 | 11.9 | 0.17 | 0.11 | TGgtctgtttatGTCA | 2784 |
| 169_1 | 0 | 9.3 | 0.22 | 0.11 | TTcagcaaatatTCGT | 2995 |
| 171_1 | 11 | 12.9 | 0.28 | 0.20 | TCTattgttaggtATC | 3053 |
| 222_1 | 16 | 19.7 | 0.68 | 0.64 | TGacttgtaattgTGG | 5467 |
| 245_1 | 14 | 6.1 | 0.26 | 0.08 | TCggttatgttaTCAT | 6470 |
| 246_1 | 28 | 7.3 | 0.10 | 0.20 | CActttatctggTCGG | 6482 |
| 252_1 | 19 | 8.0 | 0.29 | 0.12 | TCAtattgctaccATA | 6618 |
| 272_1 | 3 | 9.7 | 0.25 | 0.14 | TACTgtagaacatgGC | 7133 |
| 314_1 | 13 | 9.6 | 0.31 | 0.15 | GAgtttggattagCTG | 7764 |
| 344_1 | 11 | 8.0 | 0.14 | 0.06 | GTTagccctcagaTTG | 8039 |
| 349_1 | 12 | 12.5 | 0.18 | 0.14 | ACAagtggtatctTCT | 8228 |
| 415_1 | 11 | 9.6 | 0.26 | 0.12 | AAttgagtgaatCCAA | 9120 |
| 493_1 | 15 | 16.5 | 0.48 | 0.34 | CTTatgatacaacTTA | 10384 |
| 512_1 | 43 | 14.1 | 0.31 | 0.68 | GCacaacccagaggAA | 10735 |
| 519_1 | 9 | 12.2 | 0.45 | 0.26 | TAgatttgtgagGTAA | 11055 |
| 533_1 | 11 | 13.6 | 0.29 | 0.21 | TAGattgtttagtGCA | 11228 |
| 534_1 | 9 | 6.5 | 0.09 | 0.03 | GTagattgtttaGTGC | 11229 |
| 582_1 | 0 | 12.3 | 0.33 | 0.23 | AATCatgttggtacAT | 12092 |
| 619_1 | 8 | 10.4 | 0.32 | 0.18 | TAtgacactgcaTCTT | 15317 |
| 620_1 | 12 | 24.6 | 1.10 | 1.08 | GTAtgacactgcaTCT | 15318 |
| 638_1 | 2 | 5.4 | 0.00 | 0.00 | TAACatcagacaacTC | 15695 |
| 645_1 | 20 | 29.6 | 1.10 | 1.50 | GAtccttatattCTGG | 15854 |
| 651_1 | 0 | 11.2 | 0.14 | 0.09 | GCAaatcaactccATC | 15896 |
| 658_1 | 11 | 13.8 | 0.48 | 0.32 | TCaatagtgtagggCA | 16093 |
| 659_1 | 0 | 8.2 | 0.11 | 0.06 | TTCaatagtgtaggGC | 16094 |
| 733_1 | 0 | 69.6 | 11.03 | 26.95 | CAGagttaataatAAG | 19327 |
| 734_1 | 36 | 16.8 | 2.84 | 2.12 | GTTCaagcacaacgAA | 19493 |

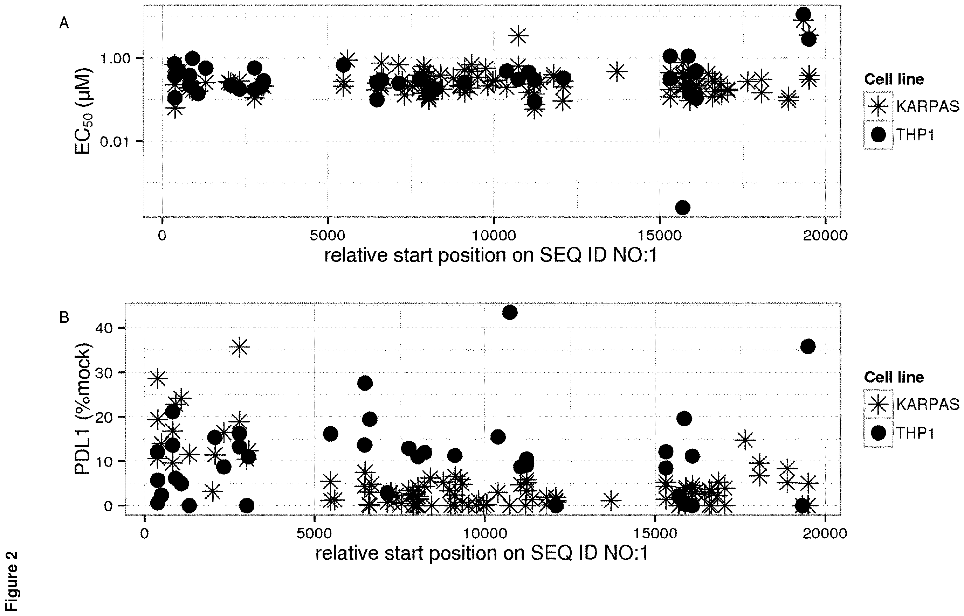

The results in table 7 and 8 are also shown in figure 2 in relation to their position where they target the PD-L1 pre mRNA of SEQ ID NO: 1.

From this it can be seen that almost all of the compounds have EC50 values below 1 µM and a target knock down below 25% of the PD-L1 expression level in the control cells (treated with saline).

### Example 3 - In vitro potency and efficacy and in vivo PD-L1 reduction in poly(I:C) induced mice using naked and GalNAc conjugated PD-L1 antisense oligonucleotides

Efficacy and potency testing was performed in an *in vitro* experiment in in dose-response studies in MCP-11 cells using the oligonucleotides in table 6. The same oligonucleotides as well as GalNAc conjugated versions (Table 8 CMP ID NO 755_2 - 765_2) were tested *in vivo* in poly(I:C) induced C57BL/6J female mice for their ability to reduce PD-L1 mRNA and protein expression

### In vitro assay

MCP-11 cells (originally purchased from ATCC) suspended in DMEM (Sigma cat. no. D0819) supplemented with 10% horse serum, 2 mM L-glutamine, 0.025 mg/ml gentamicin and 1 mM sodium pyruvate were added at a density of 8000 cells/well to the oligonucleotides (10 µl) in 96-well round bottom plates and cultured for 3 days in a final volume of 200 µl/well in a humidified incubator at 37°C with 5% CO₂. Oligonucleotides were screened in dose-range concentrations (50 µM, 15.8 µM, 5.0 µM, 1.58 µM, 0.5 µM, 0.158 µM, 0.05 µM and 0.0158 µM).

Total mRNA was extracted using the PureLink Pro 96 RNA Purification kit (Ambion), according to the manufacturer's instructions. cDNA was synthesized using M-MLT Reverse Transcriptase, random decamers RETROscript, RNase inhibitor (Ambion) and 100 mM dNTP set (Invitrogen, PCR Grade) according to the manufacturer's instruction. For gene expressions analysis, qPCR was performed using TaqMan Fast Advanced Master Mix (2X) (Ambion) in a duplex set up with TaqMan primer assays for the PD-L1 (Thermo Fisher Scientific; FAM-MGB Mm00452054-m1) and Gusb (Thermo Fisher Scientific; VIC-MGB-PL Mm01197698-m1). The relative PD-L1 mRNA expression level is shown in table 9 as % of residual PD-L1 expression in % of PBS control samples (PBS-treated cells). EC50 calculations were performed in GraphPad Prism6. The EC50 and maximum PD-L1 knockdown level is shown in table 13 as % of control (PBS) cells.

### In vivo assay

C57BL/6J female mice (20-23 g; 5 mice per group) were injected s.c. with 5 mg/kg unconjugated oligonucleotides to mouse PD-L1 or 2.8 mg/kg GalNAc-conjugated oligonucleotides to mouse PD-L1. Three days later, the mice were injected i.v. with 10 mg/kg poly(I:C) (LWM, Invivogen). The mice were sacrificed 5 h after poly(I:C) injection and liver samples were placed in RNAlater (Thermo Fisher Scientific) for RNA extraction or frozen at dry ice for protein extraction.

Total mRNA was extracted from homogenized liver samples using the PureLink Pro 96 RNA Purification kit (Ambion), according to the manufacturer's instructions. cDNA was synthesized using M-MLT Reverse Transcriptase, random decamers RETROscript, RNase inhibitor (Ambion) and 100 mM dNTP set (Invitrogen, PCR Grade) according to the manufacturer's instruction. For gene expressions analysis, qPCR was performed using TaqMan® Fast Advanced Master Mix TaqMan Fast Advanced Master Mix (2X) (Ambion) in a duplex set up with TaqMan primer assays for the PD-L1 mRNA (Thermo Fisher Scientific; FAM-MGB Mm00452054-m1) and TBP (Thermo Fisher Scientific; VIC-MGB-PL Mm00446971_m1). The relative PD-L1 mRNA expression level is shown in table 13 as % of control samples from mice injected with saline and poly(I:C).

Liver homogenates were prepared by homogenizing liver samples in 2 ml per 100 mg tissue T-PER® Tissue Protein Extraction Reagent (Thermo Fisher Scientific) mixed with 1x Halt Protease Inhibitor Cocktail, EDTA-Free (Thermo Fisher Scientific). Protein concentrations in liver homogenates were measured using Coomassie Plus (Bradford) Assay Reagent (Thermo Scientific) according to the manufacturer's instructions. Liver homogenates (40 µg protein) were separated on 4-12% Bis-Tris Plus polyacrylamide gels (Thermo Fisher Scientific) in 1xMOPS running buffer and transferred to nitrocellulose membranes using iBLOT Dry blotting system (Thermo Fisher Scientific) according to the manufacturer's instructions. Each blot was cut in to two parts horizontally at the 64 kDa band. Following blocking in TBS containing 5% skim milk and 0.05% Tween20, the membranes were incubated overnight at 4°C with rabbit monoclonal anti-vinculin (Abcam cat. no. ab129002) diluted 1:10000 (upper membranes) or goat polyclonal anti-mPD-L1 (R&D Systems cat. no. AF1019) diluted 1:1000 (lower membranes) in TBS containing 5% skim milk and 0.05% Tween20. The membranes were washed in TBS containing 0.05% Tween20 and exposed for 1 h at room temperature to HRP-conjugated swine anti-rabbit IgG (DAKO) diluted 1:3000 (upper membranes) or HRP-conjugated rabbit anti-goat IgG (DAKO) diluted 1:2000 in TBS containing 5% skim milk and 0.05% Tween20. Following washing of the membranes, the reactivity was detected using ECL select (Amersham GE Healthcare). For each group of mice treated with oligonucleotides, the intensity of the PD-L1 bands in relation to vinculin bands were evaluated by comparison with the PD-L1/vinculin band intensities of mice injected with saline and poly(I:C) (control). Results are shown in table 13, and westernblots with pairs of naked and conjugated oligonucleotides are shown in figure 9A-E.

**Table 13: In vitro and in vivo efficacy of oligonucleotides to mouse PD-L1**

| **CMP ID NO** | **Compound CMP** | **Max Inhibition (%of PBS)** | **EC50 (µM)** | **PD-L1 mRNA (% of control)** | **PD-L1 protein (relative to control)** |
|---|---|---|---|---|---|
| 744_1 | AGTttacattttcTGC | 9.1 | 0.56 | 86 | ++ |
| 746_1 | CACctttaaaaccCCA | 5.0 | 0.46 | 181 | nd |
| 747_1 | TCCtttataatcaCAC | 4.4 | 0.52 | 104 | ++ |
| 748_1 | ACGgtattttcacAGG | 1.8 | 0.26 | 102 | +++ |
| 749_1 | GACactacaatgaGGA | 7.6 | 1.21 | 104 | nd |
| 750_1 | TGGtttttaggacTGT | 12.4 | 0.74 | 84 | nd |
| 751_1 | CGAcaaattctatCCT | 9.9 | 0.69 | 112 | nd |
| 752_1 | TGAtatacaatgcTAC | 10.5 | 1.11 | 142 | +++ |
| 753_1 | TCGttgggtaaatTTA | 5.7 | 0.53 | 116 | +++ |
| 754_1 | TGCtttataaatgGTG | 5.2 | 0.35 | 98 | nd |
| 755_2 | 5'-G N2-C6-caAGTttacattttcTG C | nd | nd | 58 | + |
| 757_2 | 5'-GN2-C6-caCACctttaaaaccCCA | nd | nd | 62 | nd |
| 758_2 | 5'-GN2-C6-caTCCtttataatcaCAC | nd | nd | 53 | + |
| 759_2 | 5'-G N2-C6-caACGgtattttcacAG G | nd | nd | 66 | + |
| 760_2 | 5'-GN2-C6-caGACactacaatgaGGA | nd | nd | 101 | nd |
| 761_2 | 5'-GN2-C6-caTGGtttttaggacTGT | nd | nd | 99 | nd |
| 762_2 | 5'-GN2-C6-caCGAcaaattctatCCT | nd | nd | 84 | nd |
| 763_2 | 5'-GN2-C6-caTGAtatacaatgcTAC | nd | nd | 93 | +++ |
| 764_2 | 5'-GN2-C6-caTCGttgggtaaatTTA | nd | nd | 53 | + |
| 765_2 | 5'-GN2-C6-caTGCtttataaatgGTG | nd | nd | 106 | nd |

| | | | | | |
|---|---|---|---|---|---|
| +++: similar to PD-L1/vinculin band intensity of control; ++: weaker than PD-L1/vinculin band intensity of control; +: much weaker than PD-L1/vinculin band intensity of control; nd= not determined. | | | | | |

From the data in table 13 it can be seen that GalNAc conjugation of the oligonucleotides clearly improves the *in vivo* PD-L1 reduction. The reduction of mRNA generally correlates with a reduction in PD-L1 protein. Except for CMP ID NO: 754_1, a low *in vitro* EC50 value generally reflects a good *in vivo* PD-L1 mRNA reduction once the oligonucleotide is conjugated to GalNAc.

### Example 4 - In vivo PK/PD in sorted hepatocytes and non-parenchymal cells from poly(I:C) induced mice

The distribution of naked and GalNAc conjugated oligonucleotides as well as PD-L1 mRNA reduction was investigated in hepatocytes and non-parenchymal cells isolated from poly(I:C) induced mice.

C57BU6J female mice (n=3 per group) were injected s.c. with 5 mg/kg unconjugated oligonucleotide (748_1) or 7 mg/kg GalNAc-conjugated oligonucleotides (759_2) targeting mouse PD-L1 mRNA. Two days later, the mice were injected i.p. with 15 mg/kg poly(I:C) (LWM, Invivogen). The mice were anesthesized 18-20 h after poly(I:C) injection and the liver was perfused at a flow rate of 7 ml per min through the vena cava using Hank's balanced salt solution containing 15 mM Hepes and 0.38 mM EGTA for 5 min followed by collagenase solution (Hank's balanced salt solution containing 0.17 mg/ml Collagenase type 2 (Worthington 4176), 0.03% BSA, 3.2 mM CaCl₂ and 1.6 g/l NaHCO₃) for 12 min. Following perfusion , the liver was removed and the liver capsule was opened, the liver suspension was filtered through 70 µm cell strainer using William E medium and an aliquot of the cell suspension (= mixed liver cells) was removed for later analysis. The rest of the cell suspension was centrifuged for 3 min at 50xg. The supernatant was collected for later purification of non-parenchymal cells. The pellet was resuspended in 25 ml William E medium (Sigma cat. no. W1878 complemented with 1x Pen/Strep, 2 mM L-glutamine and 10% FBS (ATCC #30-2030)), mixed with 25 ml William E medium containing 90% percoll and the hepatocytes were precipitated by centrifugation at 50xg for 10 min. Following 2x washing in William E medium, the precipitated hepatocytes were resuspended in Williams E medium. The supernant containing non-parenchymal cells was centrifuged at 500xg 7 min and the cells were resuspended in 4 ml RPMI medium and centrifugated through two layers of percoll (25% and 50% percoll) at 1800xg for 30 min. Following collection of the non-parenchymal cells between the two percoll layers, the cells were washed and resuspended in RPMI medium.

Total mRNA was extracted from purified hepatocytes, non-parenchymal cells and total liver suspension (non-fractionated liver cells) using the PureLink Pro 96 RNA Purification kit (Ambion), according to the manufacturer's instructions. cDNA was synthesized using M-MLT Reverse Transcriptase, random decamers RETROscript, RNase inhibitor (Ambion) and 100 mM dNTP set (Invitrogen, PCR Grade) according to the manufacturer's instruction. For gene expressions analysis, qPCR was performed using TaqMan Fast Advanced Master Mix (2X) (Ambion) in a duplex set up with TaqMan primer assays for the PD-L1 (Thermo Fisher Scientific; FAM-MGB Mm00452054-m1) and TBP (Thermo Fisher Scientific; VIC-MGB-PL Mm00446971_m1). The relative PD-L1 mRNA expression level is shown in table 10 as % of control samples from mice injected with saline and poly(I:C).

Oligonucleotide content analysis was performed using ELISA employing a biotinylated capture probe with the sequence 5'-TACCGT-s-Bio-3' and a digoxigenin conjugated detection probe with the sequence 5'- DIG-C12-S1-CCTGTG - 3'. The probes consisted of only LNA with a phosphodiester backbone. Liver samples (approximately 50 mg) were homogenized in 1.4 mL MagNa pure lysis buffer (Roche Cat. No 03604721001) in a 2 mL Eppendorf tube containing one 5mm stainless steel bead. Samples were homogenized on Retsch MM400 homogenizer (Merck Eurolab) until a uniform lysate was obtained. The samples were incubated for 30 min at room temperature. Standards were generated by spiking the unconjugated antisense oligonucleotide compound (CMP ID NO 748_1) in defined concentrations into an untreated liver sample and processing them as the samples. Spike-in concentrations are chosen to match the expected sample oligo content (within ∼10-fold).

The homogenized samples were diluted a minimum of 10 times in 5 x SSCT buffer (750 mM NaCl, and 75 mM sodium citrate, containing 0.05 % (v/v) Tween-20, pH 7.0) and a dilution series of 6 times 2 fold dilutions using capture-detection solution (35 nM capture probe and 35 nM detection probe in 5xSSCT buffer) were made and incubated for 30 min at room temperature. The samples were transferred to a 96 well streptavidin coated plate (Nunc Cat. No. 436014) with 100 µL in each well. The plates were incubated for 1 hour at room temperature with gentle agitation. Wash three times with 2 x SSCT buffer and add 100 µL anti-DIG-AP Fab fragment (Roche Applied Science, Cat. No. 11 093 274 910) diluted 1:4000 in PBST (Phosphate buffered saline, containing 0.05 % (v/v) Tween-20, pH 7.2, freshly made) was added to each well and incubated for 1 hour at room temperature under gentle agitation. Wash three times with 2 x SSCT buffer and add 100 µL of alkaline phosphatase (AP) substrate solution (Blue Phos Substrate, KPL product code 50-88-00, freshly prepared). The intensity of the color was measured spectrophotometrically at 615 nm after 30 minutes incubation with gentle agitation. Raw data were exported from the readers (Gen5 2.0 software) to excel format and further analyzed in excel. Standard curves were generated using GraphPad Prism 6 software and a logistic 4PL regression model.

**Table 14: PD-L1 expression and oligo content in total liver suspension, hepatocytes and non-parenchymal cells from poly(I:C) mice treated with unconjugated and GalNAc-conjugated oligonucleotides, n=3.**

| **Cell type** | **CMP ID no** | **PD-L1 expression (% of saline-poly(I:C))** | | **oligo content (ng/10⁵ cells)** | |
|---|---|---|---|---|---|
| | | **Avg** | **SD** | **Avg** | **SD** |
| Total liver | 748_1 | 31 | 12.4 | 2.3 | 0.3 |
| | 759_2 | 28 | 5.3 | 8.3 | 1.1 |
| Hepatocytes | 748_1 | 33 | 8.0 | 5.1 | 3.7 |
| | 759_2 | 7 | 1.0 | 43.8 | 18.9 |
| Non-parenchymal cells | 748_1 | 31 | 10.1 | 2.2 | 0.7 |
| | 759_2 | 66 | 1.6 | 1.7 | 0.9 |

The results show that naked (CMP ID NO: 748_1) and conjugated (CMP ID NO: 759_2) oligonucleotide reduce PD-L1 mRNA equally well in total liver cells. In isolated hepatocytes, the effect of the conjugated oligonucleotide is almost 5 fold stronger than the effect of the naked oligonucleotide, while naked oligonucleotides showed two fold stronger effect than GalNAc-conjugated oligonucleotides in non-parenchymal cells. In hepatocytes and non- parenchymal cells the reduction of PD-L1 mRNA expression correlates to some extent with the oligonucleotide content in these cell types.

### Example 5 - In vivo PD-L1 knock down in AAV/HBV mice using naked and GalNAc conjugated PD-L1 antisense oligonucleotides

In the present study AAV/HBV mice were treated with naked or conjugated to GalNAc PD-L1 antisense oligonucleotides, and the PD-L1 mRNA expression and HBV gene expression was evaluated in the liver.

Female HLA-A2/DR1 mice 5-8 weeks old (5 animals pr. group) were pretreated at week -1 vehicle (saline), naked PD-L1 antisense oligonucleotides (CMP ID NO 752_1 at 5 mg/kg s.c.) and GalNAc PD-L1 antisense oligonucleotides (CMP ID NO 763_2 at 7 mg/kg s.c.), these doses correspond to equimolar concentrations of the oligonucleotides. The mice were transduced by 5x 10¹⁰ vg AAV-HBV at week 0 (for further details see description AAV/HBV mouse model in the Materials and Methods section). From W1 post AAV-HBV transduction to W4, mice received 4 additional s.c. injections of PD-L1 oligonucleotides or vehicle (saline solution), given one week apart.

Blood samples were taken one week before transduction and one week after each injection.

Mice were sacrificed two weeks after the last injections and their liver were removed following PBS perfusion. The liver was cut in smaller pieces and directly frozen.

To measure HBV gene expression, DNA was extracted from serum with Qiagen Biorobot using the QIAamp One for all nucleic acid kit, Cat.# 965672, serum was diluted 1:20 dilution in PBS a total of 100 µl was lysed in 200ul Buffer AL. DNA was eluted from the kit in 100 µl.

For the Real-Time qPCR the TaqMan Gene Expression Master Mix (cat.#4369016, Applied Biosystems) was used together with a primer mix prepared by adding 1:1:0.5 of the following primers F3_core, R3_core, P3_core (Integrated DNA Technologies, all reconstituted at 100uM each)

Forward (F3_core): CTG TGC CTT GGG TGG CTT T (SEQ ID NO: 784)
Reverse (R3_core): AAG GAA AGA AGT CAG AAG GCA AAA (SEQ ID NO: 785)
Probe (P3_core):56-FAM-AGC TCC AAA/ZEN/TTC TTT ATA AGG GTC GAT GTC CAT G-3IABkFQ (SEQ ID NO: 786)

A standard curve using HBV plasmid (Genotype D, GTD) was prepared using 10-fold dilutions starting with 1x10⁹ copies/µl down to 1 copy/µl and used in 5µl per reaction.

For each reaction 10µl Gene Expression Master Mix, 4.5µl water, 0.5µl Primer mix and 5µl sample or standard was added and the qPCR was run.

For the analysis the copy number / ml / well was calculated using the standard curve. The results are shown in table 15.

PD-L1 mRNA expression was measured using qPCR.

mRNA was extracted from frozen liver pieces that were added to 2ml tubes containing ceramic beads (Lysing Matrix D tubes, 116913500, mpbio) and 1ml of Trizol.

The liver piece was homogenized using the Precellys Tissue Disruptor.200µl Chloroform was added to the homogenate, vortexed and centrifuged at 4°C for 20min at 10000rpm. The RNA containing clear phase (around 500ul) was transferred into a fresh tube and the same volume of 70% EtOH was added. After mixing well the solution was transferred onto a RNeasy spin column and RNA was further extracted following the RNeasy Kit's manual RNeasy Mini Kit, cat.# 74104, Qiagen (including the RNA digestion RNase-free DNase Set, cat.# 79254). Elution in 50µl H₂O. The final RNA concentration was measured and adjusted to 100ng/ul for all samples.

The qPCR was conducted on 7.5µl RNA using the Taqman RNA-to-ct 1-step Kit, cat.# 4392938, Thermo Fisher according to the manufactures instructions. The fprimer mixed used contained PD-L1_1-3 (Primer number Mm00452054_m1, Mm03048247_m1 and Mm03048248_m1) and endogounous controls (ATCB Mm00607939_s1, CANX Mm00500330_m1, YWHAZ Mm03950126_s1 and GUSB Mm01197698_m1)

Data were analysed using the 2^-ddct method. The mean of all four endogenous controls was used to calculate dct values. The PD-L1 expression relative to mean of the endogenous controls and in % of saline

**Table 15: PD-L1 mRNA expression and HBV DNA in AAV/HBV mice treated with unconjugated and GalNAc-conjugated oligonucleotides, n=5.**

| | **CMP ID no** | **PD-L1 mRNA expression (% of saline)** | | **HBV DNA expression (% of saline)** | |
|---|---|---|---|---|---|
| | | **Avq** | **SD** | **Avg** | **SD** |
| Naked | 752_1 | 55 | 35 | 72 | 16 |
| GalNAc conjugated | 763_2 | 34 | 3 | 79 | 9 |

From these results it can be seen that both naked and GalNAc conjugated oligonucleotides are capable of reducing PD-L1 mRNA expression in the liver of an AAV/HBV mouse, with the GalNAc conjugated oligonucleotide being somewhat better. Both oligonucleotides also resulted in some reduction in HBV DNA in the serum.

### Example 6 - In vivo effect on T cell response in AAV/HBV mice

In the present study AAV/HBV mice from Pasteur were treated with an antibody or antisense oligonucleotides targeting PD-L1. The antisense oligonucleotides were either naked or conjugated to GalNAc. During the treatment the animals were immunized with a DNA vaccine against HBs and HBc antigens (see Materials and Methods section) to ensure efficient T cell priming by the antigen presenting cells. It was evaluated how the treatment affected the cell population in liver and spleen, as well as the PD-L1 expression on these populations and whether a HBV specific T cell response could be identified.

### Treatment protocol:

Female HLA-A2/DR1 mice were treated according to the protocols below. The study was conducted in two separate sub-studies, with slight differences in the administration regimens as indicated in Table 16 and 17 below.

DNA vaccine and anti-PD-L1 antibody was administered as described in the materials and method section. The antisense oligonucleotides used were CMP ID NO 748_1 (naked) at 5 mg/kg and CMP ID NO: 759_2 (GalNAc conjugated) at 7mg/kg, both where administered as subcutaneous injections (s.c.).

**Table 16: AAV/HBV mouse treatment protocol with DNA vaccine and DNA vaccine + anti-PD-L1 antibody, 6 mice in each group**

| Day | Vehicle (Group 10) | DNA vaccine (Group 11) | DNA vaccine + anti-PDL-1 Ab (Group 13) |
|---|---|---|---|
| 0 | AAV/HBV | | |
| 29* | Animal randomization | | |
| 34 | Saline+Isotype | - | Ab |
| 41 | Saline+Isotype | - | Ab |
| 48 | Saline+Isotype | - | Ab |
| 50 | - | CaTx | CaTx |
| 55* | PBS+Isotype | DNA | DNA+Ab |
| 62 | Saline+Isotype | - | Ab |
| 69 | PBS+Isotype | DNA | DNA+Ab |
| 76* | Saline+Isotype | - | Ab |
| 83 | Saline+Isotype | - | Ab |
| 97* | Sacrifice | | |

| | | | |
|---|---|---|---|
| Isotype= mouse IgG control Ab, CaTx = cardiotoxine, DNA = DNA vaccine, Ab=anti-PD-L1 Ab and *= serum collection | | | |

**Table 17: AAV/HBV mouse treatment protocol with DNA vaccine and DNA vaccine + naked or conjugated PD-L1 oligonucleotide (ASO), 7 mice in each group**

| Day | Vehicle (Group 1) | DNA vaccine (Group 2) | DNA vaccine + PDL-1 ASO (Group 7) | DNA vaccine + GN-PDL-1 ASO (Group 8) |
|---|---|---|---|---|
| 0 | AAV/HBV | | | |
| 29* | Animal randomization | | | |
| 39 | Saline | Saline | | |
| 41 | | Saline | ASO | GN-ASO |
| 46 | Saline | Saline | | |
| 49 | | Saline | ASO | GN-ASO |
| 53 | Saline | Saline | | |
| 55 | CaTx | CaTx | CaTx | CaTx |
| 56 | | Saline | ASO | GN-ASO |
| 59 | PBS+ Saline | DNA+PBS | DNA | DNA |
| 62* | | Saline | ASO | GN-ASO |
| 67 | Saline | Saline | | |
| 70 | | Saline | ASO | GN-ASO |
| 74 | PBS+ Saline | DNA+PBS | DNA | DNA |
| 77 | | Saline | ASO | GN-ASO |
| 81 | Saline | Saline | | |
| 84* | | Saline | ASO | GN-ASO |
| 88 | Saline | Saline | | |
| 91 | | Saline | ASO | GN-ASO |
| 102 | Sacrifice | | | |

| | | | | |
|---|---|---|---|---|
| DNA = DNA vaccine, CaTx = cardiotoxine, Ab=anti-PD-L1 Ab, ASO= naked PDL-1 oligonucleotide, GN-ASO= GalNAc-PDL-1 oligonucleotide and *= serum collection | | | | |

At the time of sacrifice blood, spleen and liver mononuclear cells of each mouse from each group were collected and depleted of red blood cells (Lysing Buffer, BD biosciences, 555899). The liver mononuclear cells required a specific preparation as described in the materials and method section.

### Cell populations:

In the liver the cell population was analyzed by surface labeling on liver mononuclear cells (see materials and methods) using cytometry.

No significant changes were noticed in the frequencies of NK cells in the spleen and liver of treated mice compared to control groups (i.e. vehicle and DNA-immunized groups). Table 18 show that in the liver, groups treated with naked PD-L1 oligonucleotide (CMP ID NO 748_1) and GalNAc conjugated PD-L1 oligonucleotide (CMP ID NO: 759_2) had a significant increase in T cell numbers compared to either control groups (i.e. vehicle and DNA-immunized groups) also presented in figure 10A. This increase was due to an increase in both CD4+ and CD8+ T cell populations (Table 18 and figure 10B and 10C, respectively).

**Table 18: T-cells in the liver following treatment in millions of cells**

| | T-cells (millinons) | | CD4+ T-cells (millions) | | CD8+ T-cells (millions) | |
|---|---|---|---|---|---|---|
| | Avg | Std | Avg | Std | Avg | Std |
| Vehicle (Group 1) | 0.77 | 0.44 | 0.51 | 0.35 | 0.11 | 0.05 |
| DNA vaccine (Group 2) | 0.90 | 0.24 | 0.58 | 0.16 | 0.16 | 0.08 |
| DNA vaccine + anti-PD-L1 Ab (Group 13) | 1.98 | 0.90 | 1.40 | 0.81 | 0.41 | 0.23 |
| Vehicle (Group 10) | 1.73 | 0.87 | 1.13 | 0.55 | 0.40 | 0.25 |
| DNA vaccine (Group 11) | 1.27 | 0.97 | 0.79 | 0.58 | 0.32 | 0.32 |
| DNA vaccine + PD-L1 ASO (Group 7) | 3.78 | 1.31 | 2.46 | 0.72 | 0.79 | 0.39 |
| DNA vaccine + GN-PD-L1 ASO (Group 8) | 3.33 | 0.66 | 2.18 | 0.40 | 0.67 | 0.17 |

### PD-L1 expression:

The expression of PD-L1 protein was evaluated on macrophages, B and T cells from spleen and liver at time of sacrifice. The presence of PD-L1 antibody in the surface labeling antibody mix (see materials and methods) allowed quantification of PD-L1 expressing cells by cytometry.

In spleen, no significant difference between the treatments was observed in the % of macrophages, B cells and CD4+ T cells expressing PD-L1. The % of the CD8+ T cells expressing PD-L1 was lower in mice treated with naked PD-L1 oligonucleotide (CMP ID NO 748_1) and GalNAc conjugated PD-L1 oligonucleotide (CMP ID NO: 759_2) when compared to the other treatments (data not shown).

In liver, PD-L1 was expressed mainly on CD8+ T cells with a mean frequency of 32% and 41% in the control groups (the two vehicle and DNA vaccination groups combined, respectively, figure 11A). Treatment with naked PD-L1 oligonucleotide or GalNAc PD-L1 oligonucleotide resulted in a decrease of the frequency of CD8+ T cells expressing PD-L1 (see table 19 figure 11A). Significant differences in the % of cells expressing PD-L1 were also noticed for B cells and CD4+ T-cells after ASO treatment, although these cell types express significantly less PD-L1 than the CD8+ T cells (see table 19 and figure 11B and C). Treatment with anti-PD-L1 Ab, also resulted in an apparent decrease in the PD-L1 expression in all cell types. It is, however, possible that this decrease is due to partly blockage of the PD-L1 epitope by the anti-PD-L1 antibody used for treatment, so that the PD-L1 detection antibody in the surface labeling antibody mix is prevented from binding to PD-L1. Therefore what appears to be a PD-L1 down regulation by the anti-PD-L1 antibody used for treatment may be the result of epitope competition between the treatment antibody and the detection antibody.

**Table 19: % of liver cell population with PD-L1 expression**

| | % of CD8+ T-cells | | % of CD4+ T-cells | | % of B-cells | |
|---|---|---|---|---|---|---|
| | Avg | Std | Avg | Std | Avg | Std |
| Vehicle (Group 10) | 35.5 | 4.7 | 0.75 | 0.52 | 5.9 | 1.5 |
| DNA vaccine (Group 11) | 36.8 | 7.7 | 0.61 | 0.08 | 5.5 | 1.1 |
| DNA vaccine + anti-PD-L1 Ab (Group 13) | 18.6 | 12.3 | 0.33 | 0.10 | 2.9 | 1.7 |
| Vehicle (Group 1) | 28.5 | 11.5 | 0.64 | 0.21 | 5.9 | 1.7 |
| DNA vaccine (Group 2) | 44.9 | 14.4 | 1.43 | 0.69 | 8.7 | 3.1 |
| DNA vaccine + PD-L1 ASO (Group 7) | 9.6 | 2.4 | 0.37 | 0.21 | 2.9 | 0.8 |
| DNA vaccine + GN-PD-L1 ASO Group 8) | 14.6 | 3.3 | 0.31 | 0.11 | 2.8 | 0.8 |

### HBV specific T cell response:

NK cells and CD4+ and CD8+ T cells producing pro-inflammatory cytokines were detected using the intracellular cytokine staining assays (see Materials and Methods section) detecting IFNγ and TNFα production.

In the spleen no NK cells and few CD4+ T cells secreting IFNγ- and TNFα were detectable (frequency < 0.1%) at sacrifice. IFNγ-producing CD8+ T cells targeting the two HBV antigens were detected in mice treated with naked PD-L1 oligonucleotide or GalNAc PD-L1 oligonucleotide as well as in mice from this study which received only DNA vaccine (data not shown).

In the livers of DNA-immunized HBV-carrier mice, no IFNγ-producing NK cells were detected at sacrifice, whereas IFNγ-secreting CD4+ T cells specific for Core or for S2+S were detected in the liver of a few DNA-immunized mice at a low frequency (< 0.4%, data not shown). HBV S2+S -specific CD8+ T cells producing IFNγ were detected in the majority of DNA-immunized mice. The frequency of IFNγ-secreting CD8+ T cells increased in mice treated with combination of DNA vaccine and naked PD-L1 oligonucleotide or GalNAc PD-L1 oligonucleotide, whereas treatment with anti-PD-L1 antibody did not add any apparent additional effect to the DNA vaccination (figure 12). IFNγ-producing CD8+ T cells targeting the envelope and core antigens were detected in most DNA-immunized groups (except anti-PD-L1 antibody) (figure 12B). Most of the S2-S specific T cells produced both IFNγ and TNFα (figure 12C). The results are also shown in Table 20.

**Table 20: % of HBV antigen (S2-S or core) specific CD8+ T cells from total IFNγ or IFNγ + TNFα cell population**

| | PreS2-S specific T cells (% of IFNγ cells) | | Core specific T cells (% of IFNγ cells) | | S2-S specific T cells (% of IFNγ + TNFα) | |
|---|---|---|---|---|---|---|
| | Avg | Std | Avg | Std | Avg | Std |
| Vehicle (Group 10) | 0.15 | 0.37 | 0.18 | 0.43 | 0.00 | 0.00 |
| DNA vaccine (Group 11) | 1.48 | 1.10 | 0.47 | 0.53 | 0.42 | 1.02 |
| DNA vaccine + anti-PDL-1 Ab | 1.18 | 0.95 | 0 | 0 | 0.38 | 0.49 |
| Vehicle (Group 1) | 0.17 | 0.45 | 0.11 | 0.28 | 0.00 | 0.00 |
| DNA vaccine (Group 2) | 1.70 | 1.02 | 0.27 | 0.51 | 0.98 | 0.90 |
| DNA vaccine + PDL-1 ASO | 2.56 | 1.60 | 0.78 | 0.80 | 1.44 | 1.55 |
| DNA vaccine + GN-PDL-1 ASO | 3.83 | 2.18 | 0.68 | 1.16 | 2.62 | 1.62 |

### Example 7 - In vivo effect on HBV antigen and HBV DNA in the serum of AAV/HBV mice

In the present study AAV/HBV mice from Shanghai (see Materials and Methods section) were treated with the GalNAc conjugated PD-L1 antisense oligonucleotide CMP ID NO 759_2.

It was evaluated how the treatment affected the HBe and HBs antigens and HBV DNA levels in the serum compared to vehicle treated animals.

### Treatment protocol:

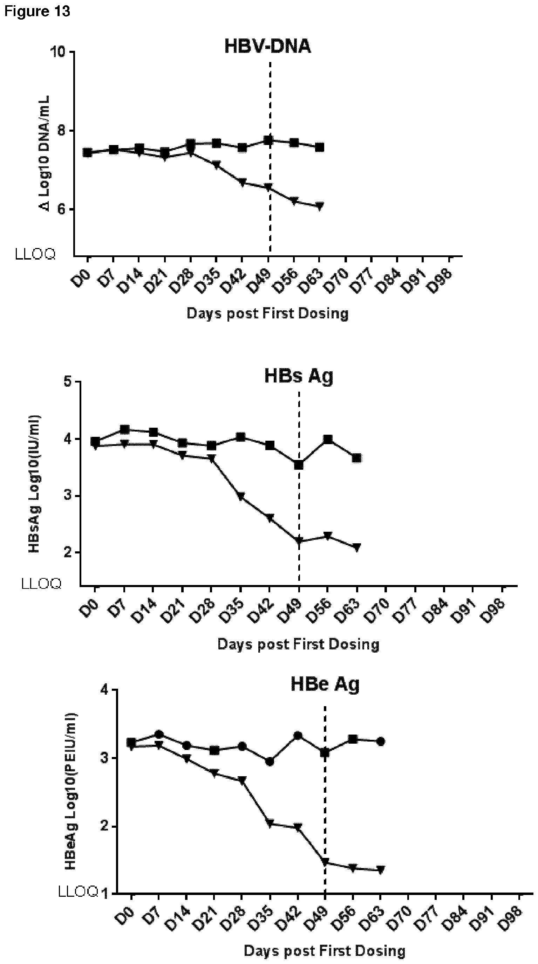

Male C57BL/6 mice infected with recombinant adeno-associated virus (AAV) carrying the HBV genome (AAV/HBV) as described under the Shanghai model in the materials and method section were used in this study. The mice (6 mice pr. group) were injected once a week for 8 weeks with the antisense oligonucleotide CMP ID NO: 759_2 at 5 mg/kg or vehicle (saline) both where administered as subcutaneous injections (s.c.). Blood samples were collected each week during treatment as well as 6 weeks post treatment. HBV DNA, HBsAg and HBeAg levels were measured in the serum samples as described below. The results for the first 10 weeks are shown in table 21 and in figure 13. The study was still ongoing at the time of filing the application therefore data for the remaining 4 weeks have not been obtained.

### HBsAg and HBeAg detection:

Serum HBsAg and HBeAg levels were determined in the serum of infected AAV-HBV mouse using the HBsAg chemoluminescence immunoassay (CLIA) and the HBeAg CLIA kit (Autobio diagnostics Co. Ltd., Zhengzhou,China, Cat. no.CL0310-2 and CL0312-2 respectively), according to the manufacturer's protocol. Briefly, 50µl of serum was transferred to the respective antibody coated microtiter plate and 50µl of enzyme conjugate reagent was added. The plate was incubated for 60 min on a shaker at room temperature before all wells were washed six times with washing buffer using an automatic washer. 25µl of substrate A and then 25µl of substrate B was added to each well. The plate was incubated for 10 min at RT before luminescence was measured using an Envision luminescence reader. HBsAg is given in the unit IU/ml; where 1 ng HBsAg =1.14 IU. HBeAg is given in the unit NCU/ml serum.

### HBV DNA extraction and qPCR:

Initially mice serum was diluted by a factor of 10 (1:10) with Phosphate buffered saline (PBS). DNA was extracted using the MagNA Pure 96 (Roche) robot. 50µl of the diluted serum was mixed in a processing cartridge with 200ul MagNA Pure 96 external lysis buffer (Roche, Cat. no. 06374913001) and incubated for 10 minutes. DNA was then extracted using the "MagNA Pure 96 DNA and Viral Nucleic Acid Small Volume Kit" (Roche, Cat. no. 06543588001) and the "Viral NA Plasma SV external lysis 2.0" protocol. DNA elution volume was 50µl.

Quantification of extracted HBV DNA was performed using a Taqman qPCR machine (ViiA7, life technologies). Each DNA sample was tested in duplicate in the PCR. 5µl of DNA sample was added to 15µl of PCR mastermix containing 10µl TaqMan Gene Expression Master Mix (Applied Biosystems, Cat. no. 4369016), 0.5 µl PrimeTime XL qPCR Primer/Probe (IDT) and 4.5µl distilled water in a 384 well plate and the PCR was performed using the following settings: UDG Incubation (2min, 50°C), Enzyme Activation (10min, 95°C) and PCR (40 cycles with 15sec, 95° for Denaturing and 1min, 60°C for annealing and extension). DNA copy numbers were calculated from Cₜ values based on a HBV plasmid DNA standard curve by the ViiA7 software.

Sequences for TaqMan primers and probes (IDT):
Forward core primer (F3_core): CTG TGC CTT GGG TGG CTT T (SEQ ID NO: 784)
Reverse primer (R3_core): AAG GAA AGA AGT CAG AAG GCA AAA (SEQ ID NO: 785)
Taqman probe (P3_core): 56-FAM/AGC TCC AAA /ZEN/TTC TTT ATA AGG GTC GAT GTC CAT G/3IABkFQ (SEQ ID NO: 786).

**Table 21: HBV-DNA, HBsAg and HBeAg levels in serum from AAV/HBV mice following treatment with GalNAc conjugated PD-L1 antisense oligonucleotide.**

| | **Saline** | | | | | | **CMP ID NO: 759_2 at 5 mg/kg** | | | | | |
|---|---|---|---|---|---|---|---|---|---|---|---|---|
| | **HBV-DNA** | | **HBsAg** | | **HBeAg** | | **HBV-DNA** | | **HBsAg** | | **HBeAg** | |
| Day | Avg | Std | Avg | Std | Avg | Std | Avg | Std | Avg | Std | Avg | Std |
| 0 | 7.46 | 0.35 | 3.96 | 0.48 | 3.23 | 0.14 | 7.44 | 0.29 | 3.87 | 0.40 | 3.17 | 0.13 |
| 7 | 7.53 | 0.23 | 4.17 | 0.45 | 3.35 | 0.10 | 7.53 | 0.20 | 3.91 | 0.42 | 3.19 | 0.18 |
| 14 | 7.57 | 0.24 | 4.12 | 0.49 | 3.19 | 0.11 | 7.45 | 0.22 | 3.90 | 0.50 | 2.99 | 0.27 |
| 21 | 7.47 | 0.27 | 3.93 | 0.51 | 3.12 | 0.05 | 7.33 | 0.47 | 3.71 | 0.76 | 2.78 | 0.26 |
| 28 | 7.68 | 0.26 | 3.88 | 0.67 | 3.18 | 0.13 | 7.45 | 0.46 | 3.65 | 0.93 | 2.67 | 0.38 |
| 35 | 7.69 | 0.21 | 4.03 | 0.54 | 2.95 | 0.08 | 7.13 | 0.75 | 2.98 | 1.05 | 2.04 | 0.38 |
| 42 | 7.58 | 0.23 | 3.89 | 0.65 | 3.34 | 0.10 | 6.69 | 0.89 | 2.60 | 1.05 | 1.98 | 0.45 |
| 49 | 7.77 | 0.17 | 3.54 | 1.06 | 3.08 | 0.26 | 6.56 | 1.26 | 2.19 | 0.70 | 1.47 | 0.37 |
| 56 | 7.71 | 0.24 | 3.99 | 0.86 | 3.28 | 0.05 | 6.21 | 1.48 | 2.28 | 0.84 | 1.38 | 0.30 |
| 63 | 7.59 | 0.28 | 3.67 | 1.07 | 3.25 | 0.13 | 6.08 | 1.39 | 2.08 | 0.71 | 1.35 | 0.30 |

From this study it can be seen that GalNAc conjugated PD-L1 antisense oligonucleotide CMP NO 759_2 has a significant effect on the reduction of HBV-DNA, HBsAg and HBeAg levels in serum after 6 weeks of treatment, and effect that is sustained for at least 2 weeks after the treatment has ended.

### Example 8 - In vitro PD-L1 knock down in human primary hepatocytes using GalNAc conjugated PD-L1 oligonucleotides

The ability of GalNAc conjugated PD-L1 antisense oligonucleotide compounds to reduce the PD-L1 transcript in primary human hepatocytes was investigated using genomics.

### Cell culture

Cryopreserved human hepatocytes were suspended in WME supplemented with 10% fetal calf serum, penicillin (100 U/ml), streptomycin (0.1 mg/ml) and L-glutamine (0.292 mg/ml) at a density of approx. 5 x 10⁶ cells/ml and seeded into collagen-coated 24-well plates (Becton Dickinson AG, Allschwil, Switzerland) at a density of 2 x 10⁵ cells/well. Cells were pre-cultured for 4h allowing for attachment to cell culture plates before start of treatment with oligonucleotides at a final concentration of 100 µM. The oligonucleotides used are shown in table 21 and table 8, vehicle was PBS. Seeding medium was replaced by 315 µl of serum free WME (supplemented with penicillin (100 U/ml), streptomycin (0.1 mg/ml), L-glutamine (0.292 mg/ml)) and 35 µl of 1 mM oligonucleotide stock solutions in PBS were added to the cell culture and left on the cells for 24 hours or 66 hours.

### Library preparation

Transcript expression profiling was performed using Illumina Stranded mRNA chemistry on the Illumina sequencing platform with a sequencing strategy of 2 x 51 bp paired end reads and a minimum read depth of 30M per specimen (Q squared EA). Cells were lysed in the wells by adding 350 µl of Qiagen RLT buffer and were accessioned in a randomization scheme.

mRNA was purified using the Qiagen RNeasy Mini Kit. mRNA was quantitated and integrity was assessed using an Agilent Bioanalyzer. Upon initial quality assessment of the isolated RNA, it was observed that all samples met the input quality metric of 100ng with RIN scores >7.0.

Sequencing libraries were generated for all samples using the Illumina TruSeq Stranded mRNA Library Preparation, starting with 100 ng of total RNA. Final cDNA libraries were analyzed for size distribution and using an Agilent Bioanalyzer (DNA 1000 kit), quantitated by qPCR (KAPA Library Quant Kit) and normalized to 2 nM in preparation for sequencing. The Standard Cluster Generation Kit v5 was used to bind the cDNA libraries to the flow cell surface and the cBot isothermally to amplify the attached cDNA constructs up to clonal clusters of ∼1000 copies each. The DNA sequence was determined by sequencing-by-synthesis technology using the TruSeq SBS Kit.

### Data processing

Illumina paired-end sequencing reads of length 2x51bp were mapped on the human reference genome hg19 using the GSNAP short read alignment program. SAM-format alignments were converted into sorted alignment BAM-format files using the SAMTOOLS program. Gene read counts were estimated for PD-L1 based on the exon annotation from NCBI RefSeq, specified by the corresponding GTF file for hg19. A normalization step accounting for the different library size of each sample was applied using the DESeq2 R package.

The reduction in PD-L1 transcript after incubation with GalNAc conjugated PD-L1 antisense oligonucleotide compounds are shown in table 22.

**Table 22: PD-L1 transcript reduction in human primary hepatocytes following treatment with GalNAc conjugated oligonucleotides, n=4**

| **Compound** | **PD-L1 expression level 24 h (library size adjusted counts)** | **PD-L1 expression level 66 h (library size adjusted counts)** |
|---|---|---|
| Vehicle | 259 | 156 |
| | 159 | 168 |
| | 192 | 136 |
| | 202 | 211 |
| 767_2 | 7 | 7 |
| | 11 | 14 |
| | 22 | 9 |
| | 28 | 15 |
| 766_2 | 16 | 13 |
| | 15 | 10 |
| | 17 | 11 |
| | 29 | 13 |
| 769_2 | 15 | 21 |
| | 18 | 18 |
| | 25 | 18 |
| | 26 | 25 |
| 768_2 | 41 | 25 |
| | 27 | 48 |
| | 31 | 25 |
| | 34 | 22 |
| 770_2 | 21 | 16 |
| | 44 | 62 |
| | 67 | 51 |
| | 38 | 63 |

All five GalNAc conjugated antisense compounds showed significant PD-L1 transcript reduction after 24 and 66 hour incubation when compared to samples treated with vehicle.

### Example 9 - EC50 of conjugated and naked PD-L1 antisense oligonucleotides in HBV infected ASGPR-HepaRG cells

The potency of two naked and the equivalent GalNAc conjugated PD-L1 antisense oligonucleotides were compared in HBV infected ASGPR-HepaRG cells.

### Cell line

HepaRG cells (Biopredic International, Saint-Gregoire, France) were cultured in Williams E medium (supplemented with 10% HepaRG growth supplement (Biopredic). From this cell line a HepaRG cell line stably overexpressing human ASGPR1 and ASGPR2 was generated using a lentiviral method. Proliferating HepaRG cells were transduced at MOI 300 with a lentivirus produced on demand by Sirion biotech (CLV-CMV-ASGPR1-T2a_ASGPR2-IRES-Puro) coding for Human ASGPR1 and 2 under the control of a CMV promoter and a puromycin resistance gene. Transduced cells were selected for 11 days with 1µg/ml puromycin and then maintained in the same concentration of antibiotic to ensure stable expression of the transgenes. ASGPR1/2 overexpression was confirmed both at mRNA level by RT-qPCR (ASGPR1: 8560 fold vs non-transduced, ASGPR2: 2389 fold vs non transduced), and at protein level by flow cytometry analysis.

The cells were differentiated using 1.8% DMSO for at least 2 weeks before infection. HBV genotype D was derived from HepG2.2.15 cell culture supernatant and was concentrated using PEG precipitation. To evaluate activity of test compounds against HBV, differentiated ASGPR-HepaRG cells in 96 well plates were infected with HBV at an MOI of 20 to 30 for 20 h, before the cells were washed 4 times with PBS to remove the HBV inoculum.

### Oligonucleotide potency

### The following oligonucleotides

| **Naked PD-L1 ASO** | **Equivalent GalNAc conjugated PD-L1 ASO** |
|---|---|
| CPM ID NO: 640_1 | CPM ID NO: 768_2 |
| CPM ID NO: 466_1 | CPM ID NO: 769_2 |

were added to the HBV infected ASGPR-HepaRG cells on day 7 and day 10 post infection using serial dilutions from 25 µM to 0.4 nM (1:4 dilutions in PBS). Cells were harvested on day 13 post infection.

Total mRNA was extracted using the MagNA Pure 96 Cellular RNA Large Volume Kit on the MagNA Pure 96 System (Roche Diagnostics) according to the manufacturer's instructions. For gene expression analysis, RT-qPCR was performed as described in Example 5.

Data were analysed using the 2^-ddct method. ActinB was used as the endogenous control to calculate dct values. The PD-L1 expression is relative to the endogenous controls and to the saline vehicle.

EC50 calculations were performed in GraphPad Prism6 and is shown in table 23.

**Table 23: EC50 in ASGPR-HepaRG HBV infected cells, n=4.**

| **CMP ID NO** | **EC50 (µM)** |
|---|---|
| **640_1** | 2.25 |
| **768_2** | 0.10 |
| **466_1** | 5.82 |
| **769_2** | 0.13 |

These data clearly shows that GalNAc conjugation of the PD-L1 antisense oligonucleotides improves the EC50 values significantly.

### Example 10 - Stimulation T cell function in PBMCs derived from chronic HBV patients

It was investigated whether naked PD-L1 antisense compounds could increase the T cells function of chronically infected HBV (CHB) patients after ex-vivo HBV antigen stimulation of the peripheral blood mononuclear cells (PBMCs).

Frozen PBMCs from three chronic HBV infected patients were thawed and seeded at a density of 200'000 cells/well in 100µl medium (RPM11640 + GlutaMax+ 8% Human Serum + 25mM Hepes + 1% PenStrep). The next day, cells were stimulated with 1µM PepMix HBV Large Envelope Protein or 1µM PepMix HBV Core Protein (see table 9) with or without 5µM of CMP ID NO: 466_1 or CMP ID NO: 640_1 in 100µl medium containing 100pg/ml IL-12 and 5ng/ml IL-7 (Concanavalin stimulation was only applied at day 8). Four days later PD-L1 antisense oligonucleotide treatment was renewed with medium containing 50IU IL-2. At day 8 after the first stimulation the cells were re-stimulated with PepMix or 5µg/ml Concanavalin A plus PD-L1 antisense oligonucleotide for 24h. For the last 5h of the stimulation, 0.1µl Brefeldin A, 0.1µl Monensin and 3µl anti-human CD-107 (APC) were added.

After 24h the cells were washed with Stain Buffer (PBS + 1% BSA + 0.09% Sodium Azide + EDTA) and surface staining was applied for 30min at 4°C [anti-human CD3 (BV 605), anti-human CD4 (FITC), anti-human CD8 (BV711), anti-human PDL1 (BV421), anti-human PD1 (PerCP-Cy5.5) and Live and Dead stain (BV510) (BD Biosciences)]. Cells were fixed in BD Fixation Buffer for 15min at 4°C. The next morning, cells were permeabilized with BD Perm/Wash Buffer for 15min at 4°C and intracellular staining was done for 30min at 4°C [anti-human INFγ (PE)]. After washing in Perm/Wash Buffer cells were dissolved in 250µl stain buffer.

FACS measurement was performed on a BD Fortessa (BD Biosciences). For the analysis, the whole cell population was first gated on live cells (Live and Death stain, BV510), and then on CD3+ (BV605) cells. The CD3+ cells were then graphed as CD107a+ (APC) vs IFNγ+ (PE).

The results are shown in table 24.

**Table 24: Effect of PD-L1 ASO treatment on CD3+ T cell from PBMCs isolated from three chronically HBV infected patients.**

| | **No antigen stimulation** | | | **Envelope antigen** | | | **Core antigen** | | |
|---|---|---|---|---|---|---|---|---|---|
| | **Saline** | **CMP 466_1** | **CMP 640_1** | **Saline** | **CMP 466_1** | **CMP 640_1** | **Saline** | **CMP 466_1** | **CMP 640_1** |
| **INFγ-/CD107+** | 1.16 | 4.95 | 4.81 | 4.7 | 9.12 | 8.62 | 3.84 | 9.66 | 7.31 |
| | 2.7 | 3.59 | 2.74 | 2.57 | 3.69 | 3.2 | 3.25 | 3.34 | 2.92 |
| | 3 | 3.87 | 3.98 | 4.59 | 12.5 | 10.9 | 9.23 | 6.11 | 6.88 |
| **INFγ+/CD107+** | 0.12 | 1.03 | 1.15 | 3.19 | 17.3 | 18.9 | 2.38 | 15.1 | 5.75 |
| | 0.49 | 3.12 | 1.75 | 2.73 | 7 | 5.34 | 1.63 | 2.35 | 1.9 |
| | 0.24 | 1.13 | 1.5 | 1.6 | 8.16 | 3.06 | 1.68 | 1.9 | 1.91 |
| **INFγ+/ CD107-** | 0.33 | 1.43 | 1.08 | 5.11 | 7.74 | 9.47 | 3.14 | 7.76 | 2.83 |
| | 0.61 | 2.9 | 2.26 | 7.84 | 5.79 | 5.78 | 2.33 | 2.82 | 2.95 |
| | 0.17 | 1.57 | 1.72 | 1.22 | 2.58 | 0.99 | 0.1 | 0.61 | 1.04 |

From these data it can be seen that the antigen stimulation by itself is capable of inducing T cell activation (increase % of CD3+ cells expressing INFγ and/or CD107a) in the PBMCs of CHB patients (n=3). The addition of PD-L1 antisense oligonucleotide CMP 466_1 or 640_1 resulted in an additional increase of CD3+ T cell response. This increase was mainly observed in the HBV envelop stimulated group.

## Claims

1. An antisense oligonucleotide of formula CCtatttaacatcAGAC, wherein capital letters represent beta-D-oxy LNA nucleosides, lowercase letters represent DNA nucleosides, all LNA C are 5-methyl cytosine and all internucleoside linkages are phosphorothioate internucleoside linkages.

2. An antisense oligonucleotide conjugate comprising the oligonucleotide of claim 1 and a conjugate moiety covalently attached to said oligonucleotide.

3. The antisense oligonucleotide conjugate of claim 2, wherein a linker is present between the oligonucleotide and the conjugate moiety.

4. The antisense oligonucleotide conjugate of claim 2 or claim 3, wherein the conjugate moiety is a asialoglycoprotein receptor targeting moiety.

5. The antisense oligonucleotide conjugate of claim 4, wherein the asialoglycoprotein receptor targeting moiety is a tri-valent N-acetylgalactosamine (GaINAc) moiety.

6. The antisense oligonucleotide conjugate of any one of claims 3 to 5, wherein the linker is a physiologically labile linker.

7. The antisense oligonucleotide conjugate of claim 6, wherein the physiologically labile linker is a nuclease susceptible linker.

8. The antisense oligonucleotide conjugate of claim 6 or claim 7, wherein the physiologically labile linker comprises a cytidine-adenosine dinucleotide.

9. The antisense oligonucleotide conjugate of claim 2 wherein a linker is present between the oligonucleotide and the conjugate moiety; further wherein the conjugate moiety comprises an asialoglycoprotein receptor targeting moiety that is a tri-valent N-acetylgalactosamine (GaINAc) moiety; wherein the linker is a physiologically labile linker; further wherein the physiologically labile linker comprises a cytidine-adenosine dinucleotide.

10. A pharmaceutical composition comprising the antisense oligonucleotide of claim 1 or the antisense oligonucleotide conjugate of any one of claims 2 to 9 and a pharmaceutically acceptable diluent, solvent, carrier, salt and/or adjuvant.

11. The pharmaceutical composition according to claim 10 wherein the pharmaceutically acceptable diluent is sterile phosphate buffered saline.

12. The pharmaceutical composition according to claim 10 or claim 11 wherein the pharmaceutically acceptable salt is sodium.

13. The pharmaceutical composition according to claim 10 or claim 11 wherein the pharmaceutically acceptable salt is potassium.

14. An *in vitro* method for modulating PD-L1 expression in a target cell which is expressing PD-L1, said method comprising administering the antisense oligonucleotide of claim 1, the antisense oligonucleotide conjugate of any one of claims 2 to 9 or the pharmaceutical composition of any one of claims 10 to 13 in an effective amount to said cell.

15. The antisense oligonucleotide of claim 1, the antisense oligonucleotide conjugate of any one of claims 2 to 9 or the pharmaceutical composition of any one of claims 10 to 13 for use in restoration of immune response against a virus.

16. The antisense oligonucleotide, antisense oligonucleotide conjugate or pharmaceutical composition for use according to claim 15, wherein the virus is HBV.

17. The antisense oligonucleotide of claim 1, the antisense oligonucleotide conjugate of any one of claims 2 to 9 or the pharmaceutical composition of any one of claims 10 to 13 for use in restoration of immune response against a parasite.

18. The antisense oligonucleotide, antisense oligonucleotide conjugate or pharmaceutical composition for use of any one of claims 15 to 16, wherein the restoration of the immune response is an increase in the liver of CD8+ T cells specific to one or more HBV antigens when compared to a control.

19. The antisense oligonucleotide of claim 1, antisense oligonucleotide conjugate of any one of claims 2 to 9 or the pharmaceutical composition of any one of claims 10 to 13 for use as a medicament.

20. The antisense oligonucleotide of claim 1, the antisense oligonucleotide conjugate of any one of claims 2 to 9 or the pharmaceutical composition of claims 10 to 13 for use in the treatment of HBV infection.

## Patentansprüche

1. Antisense-Oligonukleotid der Formel CCtatttaacatcAGAC, wobei Großbuchstaben beta-D-Oxy-LNA-Nukleoside darstellen, Kleinbuchstaben DNA-Nukleoside darstellen, alle LNA C 5-Methylcytosin sind und alle Internukleosid-Bindungen Phosphorothioat-Internukleosid-Bindungen sind.

2. Antisense-Oligonukleotid-Konjugat, umfassend das Oligonukleotid nach Anspruch 1 und eine Konjugatkomponente, die kovalent an das Oligonukleotid gebunden ist.

3. Antisense-Oligonukleotid-Konjugat nach Anspruch 2, wobei ein Linker zwischen dem Oligonukleotid und der Konjugatkomponente vorhanden ist.

4. Antisense-Oligonukleotid-Konjugat nach Anspruch 2 oder 3, wobei die Konjugatkomponente eine auf den Asialoglykoprotein-Rezeptor zielende Komponente ist.

5. Antisense-Oligonukleotid-Konjugat nach Anspruch 4, wobei die auf den Asialoglykoprotein-Rezeptor zielende Komponente eine dreiwertige N-Acetylgalactosamin (GalNAc) 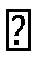 Komponente ist.

6. Antisense-Oligonukleotid-Konjugat nach einem der Ansprüche 3 bis 5, wobei der Linker ein physiologisch labiler Linker ist.

7. Antisense-Oligonukleotid-Konjugat nach Anspruch 6, wobei der physiologisch labile Linker ein Nukleaseanfälliger Linker ist.

8. Antisense-Oligonukleotid-Konjugat nach Anspruch 6 oder 7, wobei der physiologisch labile Linker ein Cytidin-Adenosin-Dinukleotid umfasst.

9. Antisense-Oligonukleotid-Konjugat nach Anspruch 2, wobei ein Linker zwischen dem Oligonukleotid und der Konjugatkomponente vorhanden ist; wobei ferner die Konjugatkomponente eine auf den Asialoglykoprotein-Rezeptor zielende Komponente umfasst, die eine dreiwertige N-Acetylgalactosamin (GalNAc) 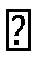 Komponente ist; wobei der Linker ein physiologisch labiler Linker ist; wobei ferner der physiologisch labile Linker ein Cytidin-Adenosin-Dinukleotid umfasst.

10. Pharmazeutische Zusammensetzung, umfassend das Antisense-Oligonukleotid nach Anspruch 1 oder das Antisense-Oligonukleotid-Konjugat nach einem der Ansprüche 2 bis 9 und ein(en) pharmazeutisch akzeptables/akzeptablen Verdünnungsmittel, Lösungsmittel, Träger, Salz und/oder Adjuvans.

11. Pharmazeutische Zusammensetzung gemäß Anspruch 10, wobei das pharmazeutisch akzeptable Verdünnungsmittel sterile phosphatgepufferte Kochsalzlösung ist.

12. Pharmazeutische Zusammensetzung gemäß Anspruch 10 oder Anspruch 11, wobei das pharmazeutisch akzeptable Salz Natrium ist.

13. Pharmazeutische Zusammensetzung gemäß Anspruch 10 oder Anspruch 11, wobei das pharmazeutisch akzeptable Salz Kalium ist.

14. *In*-*vitro*-Verfahren zum Modulieren der PD-Ll-Expression in einer Zielzelle, die PD-L1 exprimiert, wobei das Verfahren das Verabreichen des Antisense-Oligonukleotids nach Anspruch 1, des Antisense-Oligonukleotid-Konjugats nach einem der Ansprüche 2 bis 9 oder der pharmazeutischen Zusammensetzung nach einem der Ansprüche 10 bis 13 in einer wirksamen Menge an die Zelle umfasst.

15. Antisense-Oligonukleotid nach Anspruch 1, Antisense-Oligonukleotid-Konjugat nach einem der Ansprüche 2 bis 9 oder pharmazeutische Zusammensetzung nach einem der Ansprüche 10 bis 13 zur Verwendung bei der Wiederherstellung der Immunantwort gegen ein Virus.

16. Antisense-Oligonukleotid, Antisense-Oligonukleotid-Konjugat oder pharmazeutische Zusammensetzung zur Verwendung gemäß Anspruch 15, wobei das Virus HBV ist.

17. Antisense-Oligonukleotid nach Anspruch 1, Antisense-Oligonukleotid-Konjugat nach einem der Ansprüche 2 bis 9 oder pharmazeutische Zusammensetzung nach einem der Ansprüche 10 bis 13 zur Verwendung bei der Wiederherstellung der Immunantwort gegen einen Parasiten.

18. Antisense-Oligonukleotid, Antisense-Oligonukleotid-Konjugat oder pharmazeutische Zusammensetzung zur Verwendung nach einem der Ansprüche 15 bis 17, wobei die Wiederherstellung der Immunantwort eine Zunahme von CD8+ T-Zellen, die spezifisch für ein oder mehrere HBV-Antigene sind, in der Leber im Vergleich zu einer Kontrolle ist.

19. Antisense-Oligonukleotid nach Anspruch 1, Antisense-Oligonukleotid-Konjugat nach einem der Ansprüche 2 bis 9 oder pharmazeutische Zusammensetzung nach einem der Ansprüche 10 bis 13 zur Verwendung als Arzneimittel.

20. Antisense-Oligonukleotid nach Anspruch 1, Antisense-Oligonukleotid-Konjugat nach einem der Ansprüche 2 bis 9 oder pharmazeutische Zusammensetzung nach einem der Ansprüche 10 bis 13 zur Verwendung bei der Behandlung einer HBV-Infektion.

## Revendications

1. Oligonucléotide antisens de formule CCtatttaacatcAGAC, dans lequel les lettres en majuscules représentent des bêta-D-oxy-nucléosides LNA, les lettres minuscules représentent des nucléosides d'ADN, tous les LNA C sont la 5-méthyl-cytosine et toutes les liaisons internucléosidiques sont des liaisons internucléosidiques phosphorothioate.

2. Conjugué d'oligonucléotide antisens comprenant l'oligonucléotide selon la revendication 1 et un fragment conjugué lié de façon covalente audit oligonucléotide.

3. Conjugué d'oligonucléotide antisens selon la revendication 2, dans lequel un lieur est présent entre l'oligonucléotide et le fragment de conjugué.

4. Conjugué d'oligonucléotide antisens selon la revendication 2 ou la revendication 3, dans lequel le fragment conjugué est un fragment de ciblage de récepteur d'asialoglycoprotéine.

5. Conjugué d'oligonucléotide antisens selon la revendication 4, dans lequel le fragment de ciblage de récepteur d'asialoglycoprotéine est un fragment N-acétylgalactosamine (GalNAc) trivalent.

6. Conjugué d'oligonucléotide antisens selon l'une quelconque des revendications 3 à 5, dans lequel le lieur est un lieur physiologiquement labile.

7. Conjugué d'oligonucléotide antisens selon la revendication 6, dans lequel le lieur physiologiquement labile est un lieur sensible aux nucléases.

8. Conjugué d'oligonucléotide antisens selon la revendication 6 ou la revendication 7, dans lequel le lieur physiologiquement labile comprend un dinucléotide cytidine-adénosine.

9. Conjugué d'oligonucléotide antisens selon la revendication 2 dans lequel un lieur est présent entre l'oligonucléotide et le fragment conjugué ; en outre dans lequel le fragment conjugué comprend un fragment de ciblage de récepteur d'asialoglycoprotéine qui est un fragment N-acétylgalactosamine (GalNAc) trivalent ; dans lequel le lieur est un lieur physiologiquement labile ; en outre dans lequel le lieur comprend un dinucléotide cytidine-adénosine.

10. Composition pharmaceutique comprenant l'oligonucléotide antisens selon la revendication 1 ou le conjugué d'oligonucléotide antisens selon l'une quelconque des revendications 2 à 9 et un diluant, solvant, véhicule, sel et/ou adjuvant pharmaceutiquement acceptable.

11. Composition pharmaceutique selon la revendication 10 dans laquelle le diluant pharmaceutiquement acceptable est un tampon phosphate salin stérile.

12. Composition pharmaceutique selon la revendication 10 ou la revendication 11 dans laquelle le sel pharmaceutiquement acceptable est le sel de sodium.

13. Composition pharmaceutique selon la revendication 10 ou la revendication 11 dans laquelle le sel pharmaceutiquement acceptable est le sel de potassium.

14. Procédé *in vitro* de modulation de l'expression de PD-L1 dans une cellule cible qui exprime PD-L1, ledit procédé comprenant l'administration de l'oligonucléotide antisens selon la revendication 1, du conjugué d'oligonucléotide antisens selon l'une quelconque des revendications 2 à 9 ou de la composition pharmaceutique selon l'une quelconque des revendications 10 à 13 en une quantité efficace à ladite cellule.

15. Oligonucléotide antisens selon la revendication 1, conjugué d'oligonucléotide antisens selon l'une quelconque des revendications 2 à 9 ou composition pharmaceutique selon l'une quelconque des revendications 10 à 13 pour utilisation dans la restauration de la réponse immunitaire contre un virus.

16. Oligonucléotide antisens, conjugué d'oligonucléotide antisens ou composition pharmaceutique pour utilisation selon la revendication 15, le virus étant VHB.

17. Oligonucléotide antisens selon la revendication 1, conjugué d'oligonucléotide antisens selon l'une quelconque des revendications 2 à 9 ou composition pharmaceutique selon l'une quelconque des revendications 10 à 13 pour utilisation dans la restauration de la réponse immunitaire contre un parasite.

18. Oligonucléotide antisens, conjugué d'oligonucléotide antisens ou composition pharmaceutique pour utilisation selon l'une quelconque des revendications 15 à 17, la restauration de la réponse immunitaire étant une augmentation dans le foie de lymphocytes T CD8+ spécifiques d'un ou plusieurs antigènes de VHB par rapport à un témoin.

19. Oligonucléotide antisens selon la revendication 1, conjugué d'oligonucléotide antisens selon l'une quelconque des revendications 2 à 9 ou composition pharmaceutique selon l'une quelconque des revendications 10 à 13 pour utilisation en tant que médicament.

20. Oligonucléotide antisens selon la revendication 1, conjugué d'oligonucléotide antisens selon l'une quelconque des revendications 2 à 9 ou composition pharmaceutique des revendications 10 à 13 pour utilisation dans le traitement d'une infection par VHB.


### SEQUENCE LISTING

<110> F. Hoffmann-La Roche AG
   Roche Innovation Center Copenhagen A/S
<120> OLIGONUCLEOTIDES FOR REDUCTION OF PD-L1 EXPRESSION
<130> P33358-WO
<160> 786
<170> PatentIn version 3.5
<210> 1
   <211> 20064
   <212> DNA
   <213> homo sapiens
<400> 1 
ggcgcaacgc tgagcagctg gcgcgtcccg cgcggcccca gttctgcgca gcttcccgag 60

gctccgcacc agccgcgctt ctgtccgcct gcaggtaggg agcgttgttc ctccgcgggt 120

gcccacggcc cagtatctct ggctagctcg ctgggcactt taggacggag ggtctctaca 180

ccctttcttt gggatggaga gaggagaagg gaaagggaac gcgatggtct agggggcagt 240

agagccaatt acctgttggg gttaataaga acaggcaatg catctggcct tcctccaggc 300

gcgattcagt tttgctctaa aaataattta tacctctaaa aataaataag ataggtagta 360

taggataggt agtcattctt atgcgactgt gtgttcagaa tatagctctg atgctaggct 420

ggaggtctgg acacgggtcc aagtccaccg ccagctgctt gctagtaaca tgacttgtgt 480

aagttatccc agctgcagca tctaagtaag tctcttcctg cgctaagcag gtccaggatc 540

cctgaacgga atttatttgc tctgtccatt ctgagaaccc aaaggagtcc taaaagagga 600

atggaggagc ctaagaataa aaatagtata ataaaacatt tcttagacac attgaccttg 660

gcctatgtca aagttcagtc tgggtttgtc ttataacaca aggagtaaaa gtaccattgt 720

tctacctctt tttttaatac ttgaaaaaaa tttactgtgg atgcttttct atgaattaaa 780

taaccttcta aaaaatgttt tcattgctgc attcgattag attgggtaac taaatgaaat 840

taattcctca ctgttgggta taaaggttat ttacagtggt tctgtcttag ccattcactg 900

aactcattgc atatatatct ctggaatatt gctgattgtt tccttcaagt aaacttagaa 960

gtgtaactac ttagtcaaag agcctgaata ttttaaaggc cttttgaaga aaactgaaaa 1020

tgctttccag aaaggatgta tcagttgaca atgacagtcg tcaacagtat ttaaggagaa 1080

ctatgatact ctgaagaaaa acttagcctt tctcagtaaa agtaggtagg cagaggccac 1140

atgacagcag ttagagtgtg gtcttcaagg aagtcacaga aatactgtgg ggaattgaaa 1200

ccccatgtgg aaaatgtaca agagtgtctc agtgtgactg agaaggaggt tgggcatggg 1260

gtttcatgga gtttaataaa gtttggtcac ttagtagagg tttaataaat caactgtctt 1320
 
aatctttgat cctacttaag aatttttttt ttgtttttgt agagatgggg ctcttgttat 1380

gttgcccagg ctgttctcga actcctagcc tcaggcgatc ctccctcctc aggctccaga 1440

agtcctggga ttactggcgg gagccaccat gcaggcctct tgctcctact tttgagaaag 1500

gaagtttaac cggttttttt tgtctttttt tttttttttt tgagacagag tctcactctg 1560

ttgcccatgc tggagtgcag tggtgcaatc tcagctcact gcctcccggg ttcaagtgat 1620

tctcctgcct cagcctcccg agtagctggg actacaggca cctgccacca cgcccagcta 1680

atttttgtat ttttagtaga aatggggttt caccatattg gccaggctga tctcgaactc 1740

ctgacctcag gtgatccgcc tgcctcggcc tcccaaagtg ctgggattac aggcatgagc 1800

cactgctcct ggctgcttaa ctttttctct atctcatcct cctacccatc ctacccttgg 1860

aagatagaga agtagtatta gttccatagt gttatactgg gcttccccca gggacaaacc 1920

cacttcccca acctgaatga gccatcactt cttccccagt ttacatttca ttgctcttta 1980

aatgtctcca ttcggatatg ggaattcaca tatggtcata attcttacct gaagaagatg 2040

tcagtcttct tctcttagac caactgccct gatatgaggt ttagaggtta aagaacatgt 2100

gtgtatttac atgatctttg tattctgcct tttcgtccct cactaatgac agctgcaccc 2160

caaggaaatg gagctgtgga agagagggtt tgataagaaa ttaagtaaat attggatcta 2220

atccatcacc ctccaggaag cctttattac tcctaaaaat ttcaaccaaa ttcattaaag 2280

gacaagaact ccaccagagt aggccataaa cattggcaaa attagttgta atccatgact 2340

agatttaatg tccctttgtt ttattcccat atggttataa tgctttgctt ggcattaggg 2400

gtattttaag ttttcttctg cctagtaagt gaatttgtgt ttataataca ataatcataa 2460

aatatcacat taatatttta taactgtaca gttataaaat attttataag taatatttat 2520

attttataag taatatttta taactgtaca gttaactctg gcccaaggaa aagatagtct 2580

gatagatgct gcagccccat tttagcaaat gtgacctcac aggcctgaat gccatcgcta 2640

ttccacatct acaggataga cggaaaggaa agaaataaaa aaataggtac ctaacactgg 2700

caagaggatg atgactcatg ttatttcact taaccttttt atcttttaac atgaaggact 2760

catacaggtt gataagaaac cagtgacata aacagaccaa aaaatgatca gatctttcaa 2820

attagcaaaa aaataatatt ttttaaacaa tgggtgaaaa tacagtgtaa cagtaccaat 2880

tatcaacatg tgttgagaac cagaaaaatg ttctttttct ttgatcagca acactatttg 2940

ggaaaatcta tcctcagggc ctagcctggg gccctggcac acagtaggca ctcaacgaat 3000

atttgctgaa cacacaaata cttatgatat ttttaaaaat tggcaacaat ctgataccta 3060

acaatagagg gattaaatat tatggaactg ttaaataaga tgcttatgaa taccatgcag 3120

taagatgggc aatatttatg ccataagctt taatgaaaca aatgggtatt aaatgtatga 3180

taaggttata aattactttt taaaagatta cagggaaaaa aattgaaaga tatacactga 3240
 
aatgtttttt gctcacagtg gtgacaaggt ttctcagcac tggcactgtt gacgttttag 3300

gctgtatgtc tttgctgtgg gaggctggcc tgtgcactgc agggtgtttg gcagcactct 3360

tggcctctgc ccctagatag caatagcagt cctccctcaa ccagcccaat tttgacaacc 3420

aaaaatgttt ccaggcatca ccagatgctc cctgggtgag agtgatgaaa tagtagggga 3480

ttttcccctt cttttcttat tttctgtaat tccattatat tactttaata ataaagaaaa 3540

aaacataaaa aataaacgaa tgttattatt ctacgtcagt ttggatgttt ggactccatt 3600

ttggggttct ttccattata tcacttggtc tgctaaacat tctacggttt ggtaaggtga 3660

agtgattcat gaaattttgg ttttattttt ttcctgatac taaaaataaa acattctttc 3720

acttggaaat ttggacacag aacaccaaaa aaaatccata atctcatctc tctttttctg 3780

tcttttcctt ccttttttcc ctttaaaaac aataaagagt gaaacctacc tgttctccct 3840

ctaatttaat tcctaaatat aatcactgtc aatatcttgg acatttcctg tgtctaaaca 3900

cacacacaca cttttttttt tcagcaaaag tggatttctg ctacatgtag tgttctgcaa 3960

cttactttct atgtgtttac aaaatcagta catgtacata tgctgaattc agtccttaat 4020

ggtattatat tttgtgaata taccaaaatt tgtttaacca cttagacaat ctaggatatt 4080

ctcagtttgc tgttatgagc aatgctcttc ctttacatat acagacatat atatatatat 4140

gtgtgtgtgt gtgtttttgt tttagtagga tagatttcta ggagagggtg aaaggtctta 4200

tgacatccgc atttacgatt gtaataggaa gtatcaaagt gccccctaaa gaaaaaaatc 4260

ctcccattag tgggtaagaa agcctatttg ttcatatctt cacaaacact aaatattaga 4320

aatatttaca attgtggtca agctcataag tgaaaatggt atttcatatc ttatattttt 4380

tattgtgaga ttgaacatct ttcatatgtt tacatgtcac ctgtatttct tattctctga 4440

actatatgtt atgacctttc actttttttc ctcatgggtt atgtgtagtt tgtatagttg 4500

tcttattgat tgttaggagc tatttatata ttaggaacat taatctcctg tcttatatat 4560

acgtggcatc gattagttga tcatttgtga gttcatgtct gtatacaaag attggagagg 4620

cactaagagg gaaaacttac ctctttctta tcaaagtttg taaatatatg tataacagaa 4680

gagggagaaa atattaataa atgcacagat tggctgaaat agagtataaa tcttttactc 4740

ccctacttca acataaactg caaaaggaga gtgacttttc tttcactctg acttccgtat 4800

tcctcatgct taaaatagtg cctagcacag aagaggtgct caatcagtgt ttgctaaacg 4860

aaataattag tcacatttca agcaggatga ctaaatgaag aatagaatct aggcagatac 4920

tctggaagag tggctgtgag tcattcatat cttagtatga attagtcaaa tccaactctc 4980

tccccttccc actccccact gttagtagaa gaatctgttt attgagagaa tagatttata 5040

atttagaata agtgagaggg gcagaagagg agattttgaa ggatggcacc tgaaggagga 5100
 
ctagcatggc tgagacagtg aagtggaagc cttgaatagc taaagggtaa gatgaaagta 5160

tttagctgta gggggaaaaa gcattgacag gttggaaaag taaaagtcag attctccttg 5220

ctctgaaatt ttgtacaggg caggttctac taggtatgtt acaatgcaga aaaaacatga 5280

aataattgag aggaatttgg tgcaatatta tcttcttggc ttcttttgag tgggcagatt 5340

tttttcacgg cctgtaacta taataaattt gaaacttctc atcttttagt aacttttttc 5400

acttaagttt atgtggctgt gggcaatgga atgaagatat tgaacttcca attccctgtt 5460

gggtttccac aattacaagt caatcatgac tggttattag aagactattt cagttagaac 5520

caccaagtcc catattgtca tattgtatgt ttaattatta agtgaagcag tcttcttttc 5580

gtgttttcca taattagggc attccagaaa gatgaggata tttgctgtct ttatattcat 5640

gacctactgg catttgctga acggtaagac accaaatcct tccattaggt tctatatttt 5700

aaatatttta accatgagtt taaaactaaa atgatcattt aaaatgcatg caatttcttt 5760

atagagagaa cattctattc tttcttctac tttacacaat ggcaaagtct tctttctact 5820

ttacgcaatg ataaagttac ctgtgtcatt ttgtaaaaat atagagaata tagacaaatt 5880

gaaagacaca aaataatcta ttacccattt cccagggtta actactgaaa atatctgggg 5940

aaatggcctg tatgtataca tttatttgtt tgctttcaac aaggccaaga tcctttgatc 6000

tttcagtctt ggttgctctg tgacatgcct ttcctgatga ggatacttta aggaagaatt 6060

gtaagataca tggaaaatgt caggctaaca cagtactggc atcaccctgt gctctttcct 6120

gaactccata ccaatgtact tcttgccaga aaactgatca aaagtttagg gaagtaaaaa 6180

gagatgactg ttagaatcta ccattccctc tatgtaggaa gcaaataggt gtcctgtcaa 6240

aggacattct ggggatgtct acatgaaacc aagtctccct ggttgtaagg actccatctc 6300

catataatat ttatacagta atatatgttt ataaattgtg ggggcaactt gtttagctaa 6360

ttttattatt ctgctattgg gacactgtgt ctcagcatga gatatagtgt cccaaaacat 6420

atttcaagcc cattggataa aatatgtgtt tagcaagttc ttaaatataa tgataacata 6480

accgaccaga taaagtgatt tataaacgct gtgccaattt tgtaaatgtt tcgaggaatt 6540

ttcccttttc tgaagattgt ccttctttct ttttagcatt tactgtcacg gttcccaagg 6600

acctatatgt ggtagagtat ggtagcaata tgacaattga atgcaaattc ccagtagaaa 6660

aacaattaga cctggctgca ctaattgtct attgggaaat ggaggataag aacattattc 6720

aatttgtgca tggagaggaa gacctgaagg ttcagcatag tagctacaga cagagggccc 6780

ggctgttgaa ggaccagctc tccctgggaa atgctgcact tcagatcaca gatgtgaaat 6840

tgcaggatgc aggggtgtac cgctgcatga tcagctatgg tggtgccgac tacaagcgaa 6900

ttactgtgaa agtcaatggt aagaattatt atagatgaga ggcctgatct ttattgaaaa 6960

catattccaa gtgttgaaga cttttcattc ttgtaagtcc atacttattt tcaaacagaa 7020
 
cagcatagtc tgttcattca ttcattcaat tcatgaattc attcacataa ttatccaatt 7080

tcttgagcac ctatttgata gtcactggaa atccagagac aaacaacaca gagccatgtt 7140

ctacagtatg tacagttttc caaaaagaat ttctagtctt tactttttta ttacaaatgg 7200

aatacgtata cttgcaaata attcagatac tgtggaagag atcaaatgaa ttgcaaaagt 7260

gtccctcctc ccttcaccac tatctcccat ggcatgcaga gagagtaacc attatttgtg 7320

tgtccctcca gaaatttttt tattcaacta ctattttttt attttattag gtccgtcagt 7380

tttccttttt tgagcctctc tatatcaaat gcaaataaat atattcagaa caaaccccac 7440

tgtaaggttc acattaaaaa agacttgaag tcaccctatg aagacaaaaa ataatcacat 7500

taagtgtgaa agaacctatt cttccagtac aggataagcc atacttactg ggcatatatt 7560

catcttgaaa atctatactg atgttgtctt ggggaattga aaaggaacta ggagtgttag 7620

ttcctcggta ttgacccaca gttatgttat caggtcactt gagttcaaag ttttgtgttg 7680

gcactagcta agtaaaggaa aacacctctg ctttcattgt tgagtttcac agaattgaga 7740

gctgaaagga tcccaggcag gagcagctaa tccaaactcc cacaaagaac aaaaatcccc 7800

cagaggatct tctgttctta tatttcctgc aatggcgtcc ctgtcatatc ccacaatggc 7860

ctccctgcca tttggatatc ccttccatat cctgttgaaa ttactcccta atagtaagct 7920

gaaatctgcc cctctagttg tagtcttggg attatttcat ttacatgatg accttttaat 7980

atttgactag aattaaatca tctccccttg gtctttccat tcctgggcta actaccatca 8040

atctgagggc taacaataca agtagaaaaa gtatacattt gtcactgatc actgatcaat 8100

tattaatcaa tgatcactga taactataaa ctcaaaaaca aaatcatgtg gggattaaga 8160

gaaatgtatc agttttatgt tgtatttctg gtccctgata ctggctcagg taatgccact 8220

attgtcaaga agataccact tgtaaagtag atttaatttt cattatattt taccatatgc 8280

ttctccattc atgacatctc ttgagatgtt gtggtttata ctttcagttt ttctccagtc 8340

catccgcaaa tatcaggcat ctactgtgtt ccaagatatt aaagaaatca tcatgactta 8400

gcctcatcaa cagcattgct agatctggga tggaaaggaa gagtataatc ctggcagtca 8460

ggaagaaggc agcataaagt ataagtttct gcttccaaaa aaggtctctc atcagcctgt 8520

agggagtgtg tagggaaggg acagctgtcc ttgtagtagg gaagggtttt attcaggtcg 8580

tctgggctcc ataatatccc ttgtgtatct gcagtctcct ttgccatgga tcaacacaat 8640

aggaaatctt ccggcactga tggtttttcc aagggggagt tcttcctgga gcaaagcaaa 8700

tgaccaacca ggtttgagga cctgatttgt ttgacaattc catttgtatt tgtaaattac 8760

ttaattggca ttctactccc aatccatctt gtcatttgca tacagtggtt ttgggattga 8820

gttcagctat accaaaagtc tgaaccttct gcacttagaa caaggcaacc accaagcttc 8880
 
acttgcactg aggccgtgtc tccaatggaa atgaggcagc tggcttgcag gagcttccca 8940

actcagggaa gtagaactcc tgagtcacct ccatatgcaa atgatttcac agtaatgctg 9000

ttgaacttca cttcccatca cagcaaatgt gtggtaacat agcttcccca caggagttta 9060

ctcaccatgg tattttaaag gtgaaacatt tcaaaactga aatttgaaag aatttagttt 9120

tggattcact caattatcac tatcacttcg ggtgttattg cacctttctt gtttgtgagt 9180

ttaaatgcca gactctcagg ccactaactt tcaattaaaa gtgtttttct ttaatcgctg 9240

aacctaacag cagggaaaac gaaatgttca ttcagacttt cagaaccttc aatgagatta 9300

ggcagctgaa agatcaaagt gttgcatagt tgtcccgata aagctatttg gatcatatgg 9360

accaaatcga ctgctgtcat tccccaccaa ccccatctct ccccaaaatt cccagccctg 9420

tttaagtgtt ctctgtagca tttatctcta tctagtatat tgtgtagcat atcatatcat 9480

acttttctgt tttgtttatt gtctctctcc tcctagaata taaactccac aagcacaaag 9540

atttgggcct gttttataat attgttgcat ccccagggcc tgatatacag cagagtggtg 9600

gtacgaaaag agcacacaaa aaaatatttg ttgagtcaat gaatgaatga tttcctcaaa 9660

taggattagc ctaaaatttt ggaaacatga acagatttgg atatgtgaaa atttatttcc 9720

agactgttca tcaggaactg ttagcagctt ctaaagggta cactggagca gcagtagtaa 9780

aaggaggaag aggagcagct ctgctactgc tactatcgag tactactaca attagcactt 9840

gcttattctg tgtgttaggc cctgtactga acactctgtc taaattagtt catttcctcc 9900

tggaaatgac tctagggggt aagtgcttca tcatgtaaga tgagtatttt tcacattttg 9960

ttgtgtctga aatctgagtg tgtctttcaa tgatggaatc tttgattcca tgataagtgg 10020

tattattccc attttaagga tgaggaaact gaggtccaaa gaaattaagt aatttgccca 10080

aattcaccca gcctagaaaa tgataaagct agttctaaac ccaagcagat tagctctgaa 10140

gtctgggccc ttaataacca ctttttattg cctatatttg tacctctggt gtacgtatca 10200

agttatatgt tgacttcaaa actatcatga ccttttcttg gttttgattg tccaacatta 10260

gtatagtgtt ctgggtctgc aaaaattttg attactcatc tcatctgtaa aacattttga 10320

actcgtgtgt ttgtgcatgc acatttgtgt gtaattataa aaattttact ttctgttaat 10380

atataagttg tatcataaga aactgccgtt tttgaagagc aaaaaaaggt tgaatgttac 10440

cagttacatc tggttcaacc taatagacat ttgtacaaaa acagacattt taagaggttg 10500

aaataaaaat ttaataaaca atattttcag tttttactaa ttgtgatgct tcactatcat 10560

tagctaatat gtcaaggcat aatatacctt agggtgaact ttatcattaa caaaggtgga 10620

tggtgtcaat aatcttgagg tttgtgtttt tttatataac actgcgaggt ctaattaagt 10680

acttactgtt taccacctca tacagtggcc gataaaaagt gtcacttctg ctgtttcctc 10740

tgggttgtgc ttgaattatt agtattatct tcagtcctca gtttctttgt gggaaacttt 10800
 
ttaattagtt gtttaatttt gtaagatggt tagtttagtc aaaattagat aagagaattt 10860

gaaaatccgt agctacccca aagcaaccta cacataagaa ctattatttt tgtgttttga 10920

aatcataatt ttattgattt ccagtgtttc cactggtagt ggtttcattg atataggagt 10980

atcaaaacat cactcattat ttatttcagt ttcatttgat cctagccgtt ttgtattaac 11040

tctctgtgaa gaaattacct cacaaatcta ttgctgtcct tggtaaagga atggagaatt 11100

aaggctctag atcattagtg gttacactat agtattagaa gtaaaaaaaa gattatacca 11160

acaaaataag aacatgttaa tgtacttgta atgaataaac atgaataaag ctcttatgct 11220

atataggtgc actaaacaat ctactagaat tgtcagcaaa ctacgtatct taatcctgaa 11280

agggtcccaa accaatgatc taaaattgaa tcaaactttc ttccttgagc ataattactt 11340

aaatgattta ttaaaatagc cagcatttaa aagcttaaaa tgtaaatatc ataatgtggt 11400

atcctagata gcatcccaga acagaaaaag gatattaggg aaaaactgga ggaatggaat 11460

aaattatgca gtttagttat taataatgta ctaacgtcct tagttatgac gattgtacca 11520

tggtaatgta agatactaac aatagaggaa accgggtaag gagtatacag taactctata 11580

ctatctttgc aacttttttg taaatttaaa acttctaaaa taaagaacaa atttaaacat 11640

taaaaagtat caccaggaac atatatcact gtttacagat gaaatactat gtattttcat 11700

atctaatttc tgatcattga cttcaaatca gaaaagtgaa tgacacctca aaatcaggtt 11760

ttctgtttac tgaagtctaa gaaaagaaag cataccagct ggagagattc atgtttataa 11820

agacagattt ataacaacaa aaataaaata tccaagaata aatttaagaa gaagcacttt 11880

actgagaaac atatgaaaac ctgaacaaat ggagagggat attttgtatt tgaatagaaa 11940

gacttctggt ttaaagataa ttctctttaa attatttttt gtagaaattt aaggggtaca 12000

agagcagtgt tgtcacatgg atatattaca tagtggtgaa gtctggggtt ttagtgtaaa 12060

ttaatcttta cattttgttt gagcccaata aatgtaccaa catgattttt atagaaagat 12120

agtcattcct attaatccaa acttgtccca actttgaatt gaattgaggc agagctagca 12180

ggtgttcccc acggctgagg catctgaaca ttaagcatat ccctctgaga accagcctgc 12240

attgatactc tttctaatgt ggacagcatc aagctatgta cgtagttctg tgctcagcaa 12300

aagccctgac ttctttttgt ttatgtccta gccccataca acaaaatcaa ccaaagaatt 12360

ttggttgtgg atccagtcac ctctgaacat gaactgacat gtcaggctga gggctacccc 12420

aaggccgaag tcatctggac aagcagtgac catcaagtcc tgagtggtaa gaccaccacc 12480

accaattcca agagagagga gaagcttttc aatgtgacca gcacactgag aatcaacaca 12540

acaactaatg agattttcta ctgcactttt aggagattag atcctgagga aaaccataca 12600

gctgaattgg tcatcccagg taatattctg aatgtgtcca ttaaaatatg tctaacactg 12660
 
tcccctagca cctagcatga tgtctgccta tcatagtcat tcagtgattg ttgaataaat 12720

gaatgaatga ataacactat gtttacaaaa tatatcctaa ttcctcacct ccattcatcc 12780

aaaccatatt gttacttaat aaacattcag cagatattta tggaatatac cttttgttcc 12840

atgcattgta gtactcattg gatacacata gaataataag actcagttca cactcttcag 12900

gaaacagata aaaaactaag aaacaaacaa aaaacaggca atccaacacc atgtgggaaa 12960

tgctttcata gccgggaaac ctggggaata cctgagagga atactcaatt caggccttgt 13020

ttcaggaatc caaatcctgg cacatcagag ctgcttccct ctttccaggg tggcaggaaa 13080

taaatggaac atatttttct atcttatgcc aaacatgagg gaccctttct ccccggtgcc 13140

tctcccaagg tagtctacaa tatttcaact ctagcagtct gcttagtgca tagaacatga 13200

ggctgtgtgt ccctgggcaa attactagac ttctgtgtgc ttcactttcc ctgtaggatt 13260

ataatctact gagcaagctt attgtaaggg tcagattagc aacagtgtat gaaaatgatt 13320

tgagaccatt gcctgcacaa attcaactat ttttttttat ctcactactc tacagaagta 13380

ggtagggtgg gagacagagt ctgatgagag gctcagaatg tgaaagaaag tgaggcgagt 13440

gagcatgata tttaatataa acacaaagat attctgagaa gagctgctca ctgccccctc 13500

ccccaataca tgttgatagg aaaatgccac gtacttcagc aaaaacaact gaaaaattag 13560

atagaaaagt caatcaatag gaaaagataa tccaggacgg tgttgtgaac agaaagaggg 13620

ggaaaaaact ttagaaaatg atggggatgc tcttactggg gtacgagtcc tcaggtattg 13680

aactggcttt cagtaaaagc tagattagtg ggttcctgcc atttacaagc tgttttatga 13740

caacttactt gttgggtggc ctacagtaac tcacctaact gcactgagtc tgtttcctca 13800

tctgtaaatt ggggattttt ttttaaatac ctggcatgcc taactcataa agttgttctg 13860

aaactgaaat aaaacatacg tgaacaggca ttgtaaactg taagttacgg aaaaagctgg 13920

ctgttgttgt gtctttaaag tttcacctgg gtagtcaaag atggatcatg ggtctcagtg 13980

gagagctgag ccaggcagga gctgactaag ggtgagaggt gggagttagc agcctctgaa 14040

catctgtgta ccatgggacc ccctttcctc ctgcatggta ccccagacaa ggagcctagt 14100

aagagatact aatggcttgt tgtccagaga tgttcaaact gcagagaaag ataagacaac 14160

aagcattggc ctccaatcat gatgacagat aggaggaggt gggagctcct tagcagtgct 14220

ggttggcctt ccatgttcta ctgtgggcca tctctgccat gtactgtagg ctactagctt 14280

ctatattaaa gaatgcaaga ggggccagga gcggaggctc atgcctgtaa tctcagcact 14340

ttgggaggcc aaggtgggca gatcacttga ggtcaggagt ttgtgaccag cctggccaac 14400

atggtgaaac tctgccttta ctaaaaatat aaaaattagc tgggtgtggt ggtgtgcacc 14460

tgtaatccca gctactcggg agactgaggc acaagaattg cttgaacctg ggaggcggaa 14520

gttgcagtga gcccagattg cgccactgca ctccaccctg ggcaacagag aaagactctg 14580
 
cctcaaaaaa aaaaaaaaaa agcaagagga agtgaaataa tcaaggccgc catttaatag 14640

tgagcagcca ctccatgtgg tactgtgcaa gcacattata aatattagcc tcacaagaaa 14700

tgtattagca tttgtatttt gtacactggt taagtatctt gcccaagacc tcaaaactgg 14760

ttaagggcag cagaatttag ccccagcacc accttttcaa agcctgggct tctcacactt 14820

ctccatgctg ttcccatttt aacacaggta tctcgccatt ccagccactc aaactttggc 14880

atttaagaaa attatcctaa agctaaacta aacttcaagg atgaccattc tcctgacccc 14940

ttcccatcaa aattttatct ttagtcagtt tgttttcgtt ttgttttgtt tttcagaact 15000

acctctggca catcctccaa atgaaaggac tcacttggta attctgggag ccatcttatt 15060

atgccttggt gtagcactga cattcatctt ccgtttaaga aaaggtagta tttccttaat 15120

tgcagtggtc tccactgggg gtgaggaagg ggtgagaatt ggatcatggc tgcaaggaaa 15180

cccgacttaa cctctgcaag gtggtgcaaa ggcattccac tgttcaacag caattatatt 15240

gaagctgagt gggatcactg ggtgaagatg aagcgtaagg ggtgaggggc aggagaatgg 15300

gtatggatgg aggtagaaga tgcagtgtca tacagttttt ttctatcatg aaaataacca 15360

cagacttaca gaagagaaag agctaaaatg cccgtcattt tcagttgcat tttagtcttg 15420

cattagttgc aaccagctgg tttctgggta ccctaagtaa taaaaatagt tcctctgtag 15480

aactgtagta tgtttaccat agagtatttt gcaaaatttt tggtagagga tgttacataa 15540

tttgcatgtg ttcatttctc catttacctg tgggaacaat taaaatccag gaaaatgagt 15600

atattcaaat aatttcctcc catttaagat gagtcagagt aaataattcc tccaatactt 15660

agagaagtat accaagagat ccagtgatgg tatagagttg tctgatgtta aatagggaag 15720

tagaatatgg aaggggattc caatagtcgt tgaaaaattc cccataaccc cttacatggg 15780

ggaaagtagt gttaactgag agagtagaga taagctgttt ccaaaaatta tattcttaac 15840

aggactgaga tagccagaat ataaggatca agtttcaatg acagtaagat cctgagatgg 15900

agttgatttg cacaaagaaa taattgttgc cagcatgcat tttgaatatt tctctggaaa 15960

aaaagattag ttggcagtag aaatggatag aaatcaatag atattaaaat acctcagaat 16020

ttggttcatc tctgggaaaa gatgaaaaat aaaagtgtat actcctcaag aacatctagg 16080

atcaaaagca tgtgccctac actattgaat taattaacct cataagttgg gacctgtgga 16140

ataaggatgt ccaccagact tcctagggat tacaaatgtt tcacagaact tgaaatttaa 16200

acttgggtca ctgtatggga tgtagagctg tgctatatgg aaataaaaat gatttctttt 16260

tctcaaggga gaatgatgga tgtgaaaaaa tgtggcatcc aagatacaaa ctcaaagaag 16320

caaagtggta agaatatcag aaggaattgg gaagtaaaag tcaaaggaaa caaaaagcta 16380

aagcaataac aaagagaaat ccatcagtca taatctcctc tccttttaaa gaatgctggt 16440
 
tcccctttgc ctcacagcta acacaagaac tcctccaccg tctgaggagg tttaggagca 16500

gggaagggga aggagtcagc ttcatttgct aatcttctgt tgccctgcac cctagcagct 16560

ccttgcagca ggggacaagg atgacttagg tggatggata attaattgat tctaaaatat 16620

tgtgtgtcag tattgtaata ctatgttaat tgcaccatgc acggtatctc atttaatccc 16680

ccaccccttg ccattaccaa agagagagag agagagagag agagaaatac tagaatttat 16740

cctcatttta cagtagagaa aacagagggt caagaagata atgtaaagtg cccaagaaca 16800

cacagctgat cacaaaaatc aagcttgggg gccattagcc taaccacaga cccttactct 16860

taacccatct gcttcaatcc attttgctac aaatgtttac atttataagc agggcagaaa 16920

aacctcatcc aggttattga actaagaaga aagttatatt aaggtttcta atttttttaa 16980

tgtagttaga aaccaaactt aacaatgagc ccaagtttaa agcagtctaa ttaacctgga 17040

caagctcagg caagtttcat tctgtggccc atagcatcat ctgtgttgta aagctaagta 17100

gcaaatgttg tttgggtcat gctgggggac aagccatccc aatttgctca ggactgaggg 17160

gttttccagg atatcatgta aggataattg ggtacaaata taacctgctg ctttctctca 17220

tttcaaattt atcatttatc atatcagcaa ctatgagtta tgttttttat tagatttctt 17280

gttacttttt ccccagacca cttcccatga aattaatata ctattatcac tctccagata 17340

cacatttgga ggagacgtaa tccagcattg gaacttctga tcttcaagca gggattctca 17400

acctgtggtt taggggttca tcggggctga gcgtgacaag aggaaggaat gggcccgtgg 17460

gatgcaggca atgtgggact taaaaggccc aagcactgaa aatggaacct ggcgaaagca 17520

gaggaggaga atgaagaaag atggagtcaa acagggagcc tggagggaga ccttgatact 17580

ttcaaatgcc tgaggggctc atcgacgcct gtgacaggga gaaaggatac ttctgaacaa 17640

ggagcctcca agcaaatcat ccattgctca tcctaggaag acgggttgag aatccctaat 17700

ttgagggtca gttcctgcag aagtgccctt tgcctccact caatgcctca atttgttttc 17760

tgcatgactg agagtctcag tgttggaacg ggacagtatt tatgtatgag tttttcctat 17820

ttattttgag tctgtgaggt cttcttgtca tgtgagtgtg gttgtgaatg atttcttttg 17880

aagatatatt gtagtagatg ttacaatttt gtcgccaaac taaacttgct gcttaatgat 17940

ttgctcacat ctagtaaaac atggagtatt tgtaaggtgc ttggtctcct ctataactac 18000

aagtatacat tggaagcata aagatcaaac cgttggttgc ataggatgtc acctttattt 18060

aacccattaa tactctggtt gacctaatct tattctcaga cctcaagtgt ctgtgcagta 18120

tctgttccat ttaaatatca gctttacaat tatgtggtag cctacacaca taatctcatt 18180

tcatcgctgt aaccaccctg ttgtgataac cactattatt ttacccatcg tacagctgag 18240

gaagcaaaca gattaagtaa cttgcccaaa ccagtaaata gcagacctca gactgccacc 18300

cactgtcctt ttataataca atttacagct atattttact ttaagcaatt cttttattca 18360
 
aaaaccattt attaagtgcc cttgcaatat caatcgctgt gccaggcatt gaatctacag 18420

atgtgagcaa gacaaagtac ctgtcctcaa ggagctcata gtataatgag gagattaaca 18480

agaaaatgta ttattacaat ttagtccagt gtcatagcat aaggatgatg cgaggggaaa 18540

acccgagcag tgttgccaag aggaggaaat aggccaatgt ggtctgggac ggttggatat 18600

acttaaacat cttaataatc agagtaattt tcatttacaa agagaggtcg gtacttaaaa 18660

taaccctgaa aaataacact ggaattcctt ttctagcatt atatttattc ctgatttgcc 18720

tttgccatat aatctaatgc ttgtttatat agtgtctggt attgtttaac agttctgtct 18780

tttctattta aatgccacta aattttaaat tcataccttt ccatgattca aaattcaaaa 18840

gatcccatgg gagatggttg gaaaatctcc acttcatcct ccaagccatt caagtttcct 18900

ttccagaagc aactgctact gcctttcatt catatgttct tctaaagata gtctacattt 18960

ggaaatgtat gttaaaagca cgtattttta aaattttttt cctaaatagt aacacattgt 19020

atgtctgctg tgtactttgc tatttttatt tattttagtg tttcttatat agcagatgga 19080

atgaatttga agttcccagg gctgaggatc catgccttct ttgtttctaa gttatctttc 19140

ccatagcttt tcattatctt tcatatgatc cagtatatgt taaatatgtc ctacatatac 19200

atttagacaa ccaccatttg ttaagtattt gctctaggac agagtttgga tttgtttatg 19260

tttgctcaaa aggagaccca tgggctctcc agggtgcact gagtcaatct agtcctaaaa 19320

agcaatctta ttattaactc tgtatgacag aatcatgtct ggaacttttg ttttctgctt 19380

tctgtcaagt ataaacttca ctttgatgct gtacttgcaa aatcacattt tctttctgga 19440

aattccggca gtgtaccttg actgctagct accctgtgcc agaaaagcct cattcgttgt 19500

gcttgaaccc ttgaatgcca ccagctgtca tcactacaca gccctcctaa gaggcttcct 19560

ggaggtttcg agattcagat gccctgggag atcccagagt ttcctttccc tcttggccat 19620

attctggtgt caatgacaag gagtaccttg gctttgccac atgtcaaggc tgaagaaaca 19680

gtgtctccaa cagagctcct tgtgttatct gtttgtacat gtgcatttgt acagtaattg 19740

gtgtgacagt gttctttgtg tgaattacag gcaagaattg tggctgagca aggcacatag 19800

tctactcagt ctattcctaa gtcctaactc ctccttgtgg tgttggattt gtaaggcact 19860

ttatcccttt tgtctcatgt ttcatcgtaa atggcatagg cagagatgat acctaattct 19920

gcatttgatt gtcacttttt gtacctgcat taatttaata aaatattctt atttattttg 19980

ttacttggta caccagcatg tccatttctt tgtttatttt gtgtttaata aaatgttcag 20040

tttaacatcc cagtggagaa agtt 20064
<210> 2
   <211> 20261
   <212> DNA
   <213> macaca fascicularis
<400> 2 
   gtaaaatcaa ggtgcgttca gatgttggct tgttgtaaat ttctgtttat attaataaca 60
   taccaaatgt ggatttgttt taatcttcgg aactctttcc ggtgaaaacc tcatttacaa 120
   gaaaactgga ctgacaggtt tcactttctg tttcatttct atacatagct ttattcctag 180
   gacaccaaca ccactcgcta cccaaactga aagcttcccc gattccgccg aaggtcagga 240
   aagtccaatg ccgggcaaac tggatttgct gccttgcgca gaggtgggcg ggaccccgcc 300
   tccgggccgg gcgccaagtt gagcagctgg cacgcctcgc gaagccccag tcctgaagcc 360
   ccagtcctgc gctgcttccc gaggctccgc accagccgcg cttctctctg cctgcaggta 420
   gggagcgttg ttcctccgca ggtgcccacg gcccagcatc tctggctaac tcgctgggca 480
   ccttaggacg gaggatctct acaccctttc tttgggatgg agagaggagg agggaaaggg 540
   aaggcgatgg tctagggggc agtagagcca attacctgtt ggggttaata agaacaggca 600
   atgcatctgg gcttcctcca ggcgcaattc agttttgctc taaaaataat ttatacctct 660
   aaaaataaat aaggtgggta gtataggata ggtagtcatt cttatgcgac cgtgtgttca 720
   gaatatagct ctgatgctag gctggaagtc tggacacggg tcctagtcca ccgtcagctg 780
   cttgctagta atatgacttg tgtaagtcat cccagctgca gcagataagt aagtctcttc 840
   ctgcgctaag cacgtccagg acccctgaac ggaatttatt tgctctgtcc attctgaaaa 900
   cccaaaggag tcctaaaaga ggaatggagg agcctaagaa taaaaatagt ataataaaac 960
   atttcttaga catgttgacc ttggcctatg tcaaagttca gtctgggttt gtcttataac 1020
   ataaggagta aaagtaccat tgttctacct ctttttttaa tacttgaaaa aaaatttact 1080
   gtagatgctt ttctattaat taaataacct tctaaaaaat gtttttagtg ctgcattcga 1140
   ttaggttgga taactaaatg aaattaattc ctcactgttg ggtataaagg ttatttacag 1200
   tggttctgtc ttagctattc actgaacatc attacataga tatctctgga atattgctga 1260
   ttgtttccgt caataaactt agaagtgtaa ctacttagtc aaagagactg aatattttaa 1320
   aggcattttg aagaaaactg aaaatgcttt ccagaaagga tgtatcagtt gacaatgatg 1380
   gttgtcaaca gtatttaagg agaactatga tactctgaag aaaaacttag cctttctcag 1440
   tagaagcagg taggcagagg ccacatgaca gcagttagag tgtggtcttc aaggaagtca 1500
   cagaaatact gtggggaatt gaaaccccaa gtggaaaatg tacaagagtg tctcagtgtg 1560
   actgagaagg aggttgggct tggggtttaa cttaagaatt tttttctttt tcttttgtgg 1620
   agatagggct tttgttatgt tacccaggct ggtcttgaac tcctagcctc aggcgatcct 1680
   cccgcctcag gctgcagaag tcctgggatt actggccgga gccaccatgc aggcctcttg 1740
   ctcctacttt tgagaaagga agcttaacct tttttttttg tttgtttgtt tttgtttttt 1800
   gttttttttg agacgagtct cactctgttg cccaagctgg agtgcagtgg tgccatctca 1860
   gctcacggca acctctgcct cctgggttca agtgattctc ctgcctcagc ctcccgagta 1920
   gctgggacta caggcaccca ccaccacgcc cggctaattt ttgtattttt agtagagatg 1980
   gggtttcacc atattggtca ggctgatctc gaactcctga ctcaggtgat ccacctgcct 2040
   cagcctccca aagtgctggg attacaggcg tgagccaccg cgcctggcca cttgactttt 2100
   tctctatctc ttccttctac ccatcctacc cttggaagat agagaagtaa tatcagttcc 2160
   atcgtgttat actgggcttc ccccagggac aaacccactt ccccaacctg aatgagccat 2220
   cacttcttcc ccagtttaca tttcattgct ctttgaatgt ctccgttcgg atatgggaat 2280
   tcacatgtgg tcataattct tacctgaaga agactgacgt cttcttctct tagaccaact 2340
   gccctgatgt gaggtttaga ggttaaagaa catgtgtgta tttacatgat ctttgtattc 2400
   tgccttttcg tccctcacta atgacagctg aaccccaagg aaatagagct gtggaggaga 2460
   gggtttgatg agaaagtagg taaatattgg atctaatcca tcatcttcca ggaaacctcc 2520
   attacttcta aaaatttcaa ccaaattcgt taaaggacaa gaactccacc agagtagggc 2580
   cataaacatt ggcaaaatta gttgtaatct atgactagat ttaatgtccc tttgttttat 2640
   tcacatatgg ttataatgct ttgcttggaa ttaggggtat tttaagtttt cttctgccta 2700
   gtaagtgtat ttgtgcttat aatacaataa ttataaaata tcacattaat attttataac 2760
   tgtacagtta actctggccc aaggaaaaga tagtccggta gatgctgcag cctgattttg 2820
   tatctaaccc tggcaagagg ataatgactc atgttatttc acttaccctt tttatctttt 2880
   aacatgaagg gctcatatag gtcaataaga aaccagtgat ataaacagac caaaaaatga 2940
   tcagatcttt cacattagca aaaaaaaata ttttttaaac aatacccaac tgggtgaaaa 3000
   tacagtgtaa cagtaccaaa tatcaacatg tgtcaagaac cagaaaaatg tttgttttct 3060
   ttgatcagca acactatttg aggaaatcta tcctcagggc ctagcctggg gcctggcaca 3120
   cagtaggcac tgaacaaata tttgctgaac acacacatac ttatgatatt tttaaaattg 3180
   gcaacaatcc aatacccaat aatagaggaa ttaaatatta tagaactgtt caataagatg 3240
   cttacgaata tcatgcagta agatgggcaa tatttatatc ataagcttaa atgaaacaaa 3300
   tgggtattaa aggtatggta aggttataaa ttacttttta agagattaaa gggaaaagac 3360
   tgaaagatat atactgaaat gctcacagtg gtgacaaggt tccccagcct tggcactatt 3420
   gacattttgg gctatgtctt tgctgtggga ggctggcctg tgctctgcag gaggtttggc 3480
   agcactcttg gtttctaccc ctagatagca gtagcaaccc tccctcaacc agcccaattt 3540
   tgacaaccaa aaatgtttcc aggcatcacc agattctccc tgggtgagag tgatgaaaca 3600
   gtaggtgatt ttccccttct tttctcattt tctgtaattt tgtcacatta cgttaataat 3660
   aaggaaaaaa cataaaaaat agatgaattt attattctac ctcagtttgg atgtttggac 3720
   tccctttggg ggttctttcc attatatcac ttggtctgct aaacagtcta tggtttggca 3780
   aggtgaaatg attcatgaaa ttttgttttt attttttacc tgatactaaa agtaaaacat 3840
   tcattcgctt gaaaatttgg acacagaaca ccaaaaaaaa tccataatct catctctctt 3900
   tttctgtctt ttccttcctt ttttcccttt aaaaacaaga gtgaaaacct accggttctc 3960
   cctccaattt aattcctaaa tataatcact gttaacatct tggacatttc ctgtgtctaa 4020
   acacacatac tcactttttc ttttttttag caaaaagtgg atttctgcta catgtagtgt 4080
   tccgcaactt cctacatgtt tacaaaatca gtacatttac atatgctgaa ttcagtcctt 4140
   aatggtatta tattttgtga atataacaaa atttatttaa ccacttagac aatctaagat 4200
   attctcagtt tgctgttatg agcaatgctc ttcctttaca tatacatata tatgtgtgtg 4260
   tgtgtgtgtg tgtgtgtgtg tgtgtgtgtg ttttagtagg atagatttct aggagagggc 4320
   gaaatgtcat atgacatcca catttacaat tgtaatagga agtatcaaag tgccccctaa 4380
   aaaaaaaaat tcctcccatt agtgtgtgag aaagcctatt tgttcatatc ttcacaaaca 4440
   ctaaatatta gaaatattta caattgtgtt caagctaaga agtgaaaaat ggtatttcat 4500
   atcttataat ttttgttgtg agattgaaca tatttcctat gtttacatgt cacctgtatt 4560
   tcttattctc tgaactatac gttatgacct ttcacttatt ttcctcatgg gttatgtgta 4620
   gtttgtgtag ttgtcttatt gattgttagg agctatttat atattaggaa cattaatctc 4680
   ctgtcttata tgtatgtggc attgattagt tgatcatttg tgagttcatg tctgtataca 4740
   aagattagag aggcagtaag agggaaaact tacctctttc ttatcaaagt ttgtaaatat 4800
   atgtataaca gaagagagag aaaacattaa taaatgctga aataaagtat aaatttttta 4860
   ctccactact tcaacataaa ctacaaaagg agagtgactt ttctttcatg ctgacttcca 4920
   tattccccat gcctaaaata gtgcctagca cagaagaggt gctcaatcag tgtttgctaa 4980
   atgaaagaat tagtaacatt tcaagcagga tgactaaatg aagaatagaa tctaggcaga 5040
   tactctggaa gagtgactgt gagtcattca tggtcttggt atgaattagt caaatccagc 5100
   tttctcccct tcccactccc cactgttagt agaataatct gtttattgag agaatagatt 5160
   tataatttag aataagaggg gcagaagagg agattttgaa ggatggcacc tgaaggagga 5220
   ctagcatggc tgagatagtg aagtggaagt cttggaaagc taaagggtaa gatgaaagta 5280
   tttagccgta gggggaaaaa gcattgacag gttggaaatg taaaagtcag attctccttg 5340
   ctttgaaatt ttgtacaggg caggttctac taggtatgtt acaatgcaga aaaaacatga 5400
   aatagttgag aggaatttgg tgaaatatta tcttcctggc ttcttttgag tgagcagatt 5460
   tttttcaggg cctgtaacta taataaattt gaaacttctc atcttttagt aacttttttc 5520
   acttaagttt atgtggctgt gggcaatgga atgaaggtat tgaacttcct attccctgct 5580
   gggtttccac aattacaagt caatcatgac tggttattag aagactattt caattagaac 5640
   caccaagtcc cataatgtca tattgtgtgt ttaattatta agtaaagcag tcttcttttt 5700
   gtgttttcca taattagcac attccagaaa gatgaggata tttgctgtct ttatattcac 5760
   gatctactgg catttgctga atggtaagac accaaatcct tccattaggt tctatatttt 5820
   aaatatttta accatgagtt taaaactaaa atgataattt aaaatgcatg caattttctt 5880
   atagagagaa tattctcttc tttcttctac tttacacaat gacaaagtct ttcttctgct 5940
   ttacacaatg ataaagttcc ctgtgtcatt gtgtaaaaat atagagaata tagacaaatt 6000
   gaaagacaca aaataatcta ttacccattt cccagggtta actactgaaa atatctgggg 6060
   aaatggcctg tatgtatata tttatttgtt tgttttcaac aaggccagga tctttcaatc 6120
   tttcaatctt ggttgctctg tgacatgcct ttcctgatga gaatactcta aggaagaatt 6180
   gtaggataca tggaaaatgt cagggtaaca cagtactggc accaccctgc ggtcctttct 6240
   gaactccata ccaatgtact tcttgccaga aaactgatca aaagtttagg gaagtaaaaa 6300
   gagatgttag aatctaccat tccctctatg taggaagcaa ataggtgtcc agtcaaagga 6360
   cattctgggg atgtctacat gaaaccaagt ctcctggttg taagtactcc atctccatat 6420
   aatatttcta cagtaatata tgtttataaa ttgtgggggc aacttgttta gctaatttta 6480
   ttattctgtt attgggacac tatgtctctg catgagacat agtgtcccaa aacatatttc 6540
   aagcccattg gataaaatat gtatttagca agttcttaaa tataatgata acataactga 6600
   ccagataagg tgatttttaa atgctgtgcc aactttataa atgttttgag gaattttccc 6660
   ttttctgaag gttattcttc tttcttttta gcatttactg tcacggttcc caaggaccta 6720
   tatgtggtag agtatggcag caatatgaca attgaatgca aattcccagt agaaaaacaa 6780
   ttagacctga cttcactaat tgtctattgg gaaatggagg ataagaacat tattcaattt 6840
   gtgcatggag aggaagacct gaaggttcag catagtaact acagacagag ggcccagctg 6900
   ttgaaggacc agctctccct gggaaatgct gcacttcgga tcacagatgt gaaattgcag 6960
   gatgcagggg tttaccgctg catgatcagc tatggtggtg ccgactacaa gcggattacc 7020
   gtgaaagtca atggtaagaa ttattataga tgagaggcct gatctttata gaaaacatat 7080
   tctaagtgtt gaagactttt cattcttata agtccatact tattttcaaa cagaatagca 7140
   tagtctcttc attcattcat tcagttcatg aattcattca cgtaagtctc caattagcat 7200
   ttcttgagca cctatatgat agtcattgga aatccagaga caacacagag ccatgttcta 7260
   cggtatgtac agttttccaa aaataattcc tagtctttac ttttttatta taaatgtaat 7320
   acatatactt gcaaagaatt cagatactat ggaagagatc aaatgaattg caaaagtgtc 7380
   cctcctccct tcaccactat ctcccatgag ataaccaaga gacactccaa gagagtaacc 7440
   attatttgtg tgtccctcca gaaccttttt tattcaacta ccattttttt attttattag 7500
   gtctgtcagt tttccttttt tgagcctctc tatatcaaat gctaataaat atattcagat 7560
   caaaccccac tgtaaggttc atattaaaaa agacttgaag tctccctatg aagacaaaaa 7620
   ataatcatat taagtgtaaa agaacttatt cttccagtac agtataaact atactcactg 7680
   ggcatatatt catcttgaaa atctatactg atgttgtctt ggggaattga agaggaacta 7740
   ggagtgtcaa ttcctgggaa ctgacccaca gttatgtcat caggtcactt gagttcgaag 7800
   ttttgtgttg gcactagcta agtaaaggaa aacacctctg ctttcattgt tgagtttcat 7860
   agaattgaga gctgaaagga tcccaggcag gagcgactaa tccaaactcc cacaaagaac 7920
   aaaaatcccc cagaggatct tctgttcata tatttcctgc agtggcatcc ctgtcatatc 7980
   ccacaatggc atccctgcca tttggactcc ccttccatat cctgttgaaa ttactcccta 8040
   atagtaagct gaaatctgcc cctctagttg tagttttgga attatttcat ttccatgatg 8100
   accttttaat atttgactag aattaaatca tctccccttg gtatttccat tcctggacta 8160
   actaccatca atctgagggc taacaataca agtagaaaaa gtctacactt gtcattgatc 8220
   actgatcaat gattaatcaa tgatcactga taattataaa ctcaaaaaca aaatcatgta 8280
   gggattaaga gaaatgtatc agttttatgt tgtatttctg gtccctgatt ctggctcaaa 8340
   taatgctact attgtcaaga agatatcact tgtaaagtag atttaatttt cattatattt 8400
   taccatgtgc ttctccattc acggcatttc ttgagatgtt gtggtttata ctttcagttt 8460
   ttctccagtc catcagcaaa tatcaggcat ctactgtgtt ccaagatatt aaagaaatca 8520
   tcatgactta gcctcatcaa cagcattgct agatctggga tggaaaagaa gagtataatc 8580
   ctggcagtca ggaagaaggc tgcataaagt ataagtttct gcttccaaag aagatctctc 8640
   atcagcctgt agggagtgta tagggagggg acagctgtcc ttgtagtagc aaagggtttt 8700
   attcaggtca tctgggctcc ataatatccc ttgtgtatct gcagtctcct ttgccatgga 8760
   tcaacacaat aggaaatctt ccggcactga tggtttttcc aagggggagt tcttcaggga 8820
   gcaaagcaaa tgaccaacca ggtttgagga cctgatttga caattccatt ttgtatttta 8880
   aattagttaa tttgcattct agtcccaatc catcttgtca tttgcagaca gtggttttgg 8940
   ggttgagttg agctatacca aaagtctgaa ccttctgcac ttagaacaag gaaggcaacc 9000
   accaagcttc acttgcactg aggcagtgtc tccaatggaa acgaggtagc tggcttgcag 9060
   aagctttcca actcagggaa gtagaactcc tgagtcacct ccatatgcaa ataatttcac 9120
   agtactgctg ttgaacttca cttcccatca cagcaaatgt gtggtaacat agctttgcca 9180
   caggagttta ctcaccatgg gattttaaag gtgaaacatt tcaaaactga aatttgaaag 9240
   aatttagttt tggattcact caattatcat gatcactttg ggtgttattg cacctttcat 9300
   gtttgtgagt ttaaatacca gactctcagg cctctaactt tcaattaaaa gtgtttttct 9360
   ttaatcactg aacctaatag tagggaaaac gaaatgttca ttcagacttt caggaccttc 9420
   aatgagatga ggcagctgaa agatcaaagt gttgcatagt tatcccagta aagctatttg 9480
   gatcgtatgg accagatcaa ctgctgtcat tccccaccaa ccccatcttt ccccaaaatt 9540
   cccagccctg tttaagtgtt ctctgtagca tttatctcta tctagtatat tgtgtagcat 9600
   atcatatcat acttttctat tttgtttatt gtctctctcc tcctagaata taaactccac 9660
   aagcacagag atttgggtct gttttttaat attgttgtat ccccagggct tgacgtaaag 9720
   cagagtggta gtatgacaaa agcacacaaa aaaatatttg ttgagtcaat gaatgaatga 9780
   tttcctcaaa taggattagc ctaaaatttt ggaaacatga acagacttgg atatatgaaa 9840
   atttatttcc aaaactgttc atcaggaact gttagcacct tctaaagggt acactgaagc 9900
   agcagtagta aaaggaggag gaggagaagc agctctgcta ctactattat cgagtactac 9960
   tacaactatc gagtactact acaactatcg agtactacta caattagcac ttgcttattc 10020
   tgtgtgttag gtcctgtact gaacattctg cctaaattag ttcatttcct cctggaaatg 10080
   actctgtggg gtaggtgctt catcatgtaa gatgagtatt tttcacactt cactgtctct 10140
   gaaatctgag tgtgtctttc aatgatggaa tctttgattt catgataagt ggtattattc 10200
   ccattttaag gatgaggaaa ctggggtcca aagaaattaa gtaatttgcc caaattcacc 10260
   tagcctcgta aatgataaag ctagttctaa atccaagcag attggctctg aagtctgggc 10320
   ccttaataac cacttattgc ctgtatttgc acctctggtg tatgtatcaa gttatatatt 10380
   ggcttcaaaa ctatcatgac cttttcttga ttttgattgt tcaatattag tatagtgttc 10440
   tagatctagt agaccagggg tctgcaaaaa attttgatta ctcacctcat ctgtaaaaca 10500
   ttttaaactt gtgtgtctgt gcaggcacat ttgtgtgtaa ttataaaaaa tttactatct 10560
   attaatatat aggttgtacc gtaagaaaaa ttgccatttt tgaagagcaa aaaaggttga 10620
   atattaccag tttcatctgg ttcaacctaa tagacatttg tacaaaaaca gacattttaa 10680
   gaggatgaaa taaaaattta ataaacaata ttttcaattt ttactaattg tgacgcttca 10740
   ctattgttag ctaatatgtc aaggcatgat ataccttagg gtggaattta tcattaacaa 10800
   aggtggatag tgtcaataat cttgaggttt gtgttttttt atataacact gtgaggtcta 10860
   attaagtact taattgttta caacctcata cagtcgccaa taataagtgt cacttctgct 10920
   gtttcctctg ggttgtgctt gaattattag tattatcttc aatcctcagt ttctttgtgg 10980
   aaaacttttt aattagttgt ttaattttgt aagatggtta gtttagtcaa aattagataa 11040
   gagaatctga aaatccataa ttaccccaaa gcaacccact cataagaact attatttttg 11100
   tgttttgaaa tcataatttt attgatttcc agtgtttcca ctggtagtgg tttcattgat 11160
   gtaggagtat caaaacatca ctaattattt atttcagttt tgtttgatcc tagctgtttt 11220
   gtgttaactt tgaagaaatt acatcacaga tctattgttg tccttggtaa aggaatggag 11280
   agttaaggct ctagatcatt agtggttatg ctgtagtatt aggagtaaaa aaaaagatta 11340
   tatcaacaaa ataagaacat gttaatgtac ttgtaataga taaacatgaa taaagctctt 11400
   atgctatata gatgcactga acaatctact agaattgtca gcaaacggta tcttaatcct 11460
   aaaagggtcc caaaccaatg atctaaaatt gaatcaaact ttcttccttg agcataatta 11520
   tttaagtgat ttattaaaat agccagcatt taaaagctta aaatataagt atcataatgt 11580
   ggtatcctag atagatccca gaacagagaa aggatattag ggaaaaactg gaggaatgga 11640
   ataaattatg cagtttagtt attaataatg tactaatgtc cttagttatg accattgtac 11700
   catggtaaag taagatacta acaatagagg aaattgggta aggggtatat gtaactctat 11760
   actatctttg caattttttt gtaaatttaa aacttctaaa ataaagaaca aatttgaaca 11820
   ttaaaaagtg tcgccaggaa catgtatcac tgtttacaga tgaaacagta tgtattttta 11880
   tatctaattt ctgatcattg gcttcaaatc agaaaagtga atgacacatc aagatcaggt 11940
   tttctgttta ctaaataaag tctaagaaaa caaagcatac cagctggaga gattcatgtt 12000
   tataaagaca gatttataac aacaaaaata aaatatccaa gaataaattt aagaagaaat 12060
   agggcactat gtaaaaagta tagcacttta ctgagaaaca tatgaaaacc tgaatacatg 12120
   gagagaggta ttttatattt gaatagaaag attgctggtt taaagataat tctctttaaa 12180
   ttttttttgt agaaatttaa gaggtacaag agcagttttg tcacacggat atattacata 12240
   gtggtgaagt ctggggtttt agtgtaaatt aatctttaca ttttgtttga gcccaataaa 12300
   tgtaccaaca tgatttttat agcaagatag tcattcctat taacccaaac ttgtcccaac 12360
   tttgaactga actgaggcag agctagcagg tgttccccac tgctgaggca tctgaacatt 12420
   aagcgtatcc ctctgagaac cagcctgcat tgatcctctt tctaatgtag acagcatcaa 12480
   gctatatatc tagttctgtg ctcagcaaaa gccctgactt ctttttgctt atgtcctagc 12540
   tccatacaac aaaatcaacc aaagaatttt ggttgtcgat ccagtcacct ctgaacatga 12600
   actaacatgt caggctgagg gctaccccaa ggccgaagtc atttggacaa gcagtgacca 12660
   tcaagtcctg agtggtaaga ccaccaccac caattccaag agagaggaga agcttttaaa 12720
   tgtgaccagc acactgagaa tcaacacaac agctaatgag attttctact gcatttttag 12780
   gagattagat cctgaggaaa accatacagc tgaattggtc atcccaggta atattctgaa 12840
   tgtgtccatt aaaatatgtc taacactgtc ccctagcacc tagtatgatg tctgcctatc 12900
   atagtcattc agtgtttgtt gaataaatga attaatgaat aacattatat ttacaaaatg 12960
   tatcctaatt cctcacttcc attcatccaa atcatattgt tacttaataa acattcacca 13020
   aatatttatt gaatatgcct tttgttccat gcattgtagt actcatttga cacacataga 13080
   ataataagac tcacgttcac actcttcagg aaacagataa aaaacaaatg aacaaacaaa 13140
   aaacaggcaa tccaatacca tgtgggaaat gctttcatac catgtgggaa acctggggga 13200
   atacctgaga ggaatattca attcaggcca tgtttcagga atccaaatcc tggcacatca 13260
   gagccgcctc cttcttacta gggtttctgt ggcaggaaat aaatggaacg tatttttcta 13320
   tcttatgcca aacaggaggg accctttctc ccctgtgcct ctcccaaggt agtctacaat 13380
   atttcaacgc tagcagtctg tttagtgcac aggacatgag gctgtgtatc cctgggcaaa 13440
   ttgctacact tctgtgtgct tcactttctc tgtaggatta taacctactg agcaaggtta 13500
   ttgtgggggt caaattagca acagtgtatg aaaatgattt gagaccagtg cctgcacaaa 13560
   ttcaactatt tttttttatc tcactactct atagaagtag gtaggatggg agacagagtc 13620
   tgatgggagg ctcagaatgt gaaagtaagt gaggtgagtg agcatgatat ttcatataaa 13680
   cacaaagata ctctgagaag agcttctcac ttcccccgcc cccaatagat gttgacagga 13740
   aaatgccatg tacttcagca aaaacagctg aaaaattaga cataaaagtc aatcaatagg 13800
   aaaagataat ccaggatggt cttgtgaaca gaaagaggga aaaaaaaagt ttagaaaatg 13860
   atggggatgc tcttactggg gtatgagtcc tcaggtattc aactggcttt cagaaaaagc 13920
   tagactagtg ggttcctgcc atttaaaagc tgttttatga caacttactt gttgggtggc 13980
   ctacagtaac tcacttaact gtgctgagtc tgtttcctca tctgtaaatt ggggattttt 14040
   taaaataact ggcatgccta actcataaag ttgttctgaa actgaaataa aacatatatg 14100
   aacaggcatt gtaaactgta agttacggaa aaagctggct gttgttgtgt ctttaaagct 14160
   tcacctgggt agttagagat ggatcatggg tctcagtgga gagctgagcc aggcaggagc 14220
   tgactaaggg taagaggtgg gagttagcaa tctctgaaca tctgtgtgcc atgggacccc 14280
   ttttcctcct gcatggtacc ccagacaagg agcctagtaa gagatactaa tgacttgttg 14340
   tccagagatg ttcaaactgc agagaaagat aagacaacaa gcattggcct ccaatcatga 14400
   tgacagatag gaggaggtgg gagctcctta gcagtgctgg ttggttttcc atgttctact 14460
   gtgggccatc tctgccatgt actgtaggct actaacttct atattaaaaa atgcaagagg 14520
   ggccgggagt ggaggctcat gcctgtaatc tcagcacttt gggaggccaa ggtgggcaga 14580
   tcacttgagg tcaggagttt gtaaccagcc tggccaacat ggtgaatctc tgcctctact 14640
   aaaaatacaa aaattagcca gatgtggtgg cgtgcacccg taatcccagc tactcgggaa 14700
   gctgaggcac gagaattgct tgaacctggg aggcggaggt tgcagtgagc caagattgtg 14760
   ccactgccct ccagcctggg caacagagaa agactttgcc tcaaaaataa ataaataaat 14820
   aaataaataa ataaataaat aaatggaagt gaaataatca aggccaccat ttaatactga 14880
   gtagccactc catgtggtac tgtgctaagc acattataaa atattagcct cacaagaaat 14940
   gtattagcat ttgtattttg tacactggtt aagtatcttg cccaagacct caaaactggt 15000
   taaggggcag cagaatttaa ccccagcgcc accttttcaa agtctgggct tcttacactt 15060
   ctccatgctg ttcccatttt aacagatgta tctcgccatt ccagccactc aaactttggc 15120
   atttaagaaa attatcctaa agctaaacta aacttcaagg atgactattc tcctgatgac 15180
   cccttcccat caaaatttta tctttagtca gtttgttttt gttttgtttt gtttttcaga 15240
   actacctctg gcgcttcctc caaatgaaag gactcacttg gtaattctgg gagccatctt 15300
   tttactcctt ggtgtagcac tgacattcat cttctattta agaaaaggta gtatttcctt 15360
   aattgcagtg gtctccactg gggatgagga gggggtgaga attggatcga tcatggctgc 15420
   aaggaaacct gacttaacct ctgcagggtg gtgcaaaggc attccactat tcaacagtaa 15480
   ttatattgaa gctgcatggg atcactgggt gaagatgagg tgtaaggggt gagggacagg 15540
   agaatgggta tggatggagg tagaagatgc agtgtcatac aatttttttc tatcatgaaa 15600
   ataaccacag acttactgta aagaaggagc taaaatgcct gtcattttca gttgcatttt 15660
   agttttgcat tagttgcacc cagctggttt ctgggtactc taagtaataa aaatagttcc 15720
   tctgtagaac tgtagtattt tcaccataga gtattttgta aaattattgg tagaggatgt 15780
   tacataattt gcatgtgttc ctttctccat ttacctgtgg gaacaattaa aatccaggaa 15840
   aatgagtata ttcaaataat ttcctcccat ttatgatgat tcagagtaaa taattcctct 15900
   gatacttaga gaagtatacc aagagatcca gtgattgtat agagttgtct gatgttaaat 15960
   agggaagtag aatatggaag ggaattccaa tagtcgttga aaaattcccc acaacccctt 16020
   acatggggga aagtggtgtt aactgagata gtagagataa gctgttacca aaaattatgt 16080
   tcttaacagg attgagatag ccagaatata aggatcaagt ttcaatgaca gtaagatcct 16140
   gagatgcagt tgatttgcac aaagaaataa ttgttgccag cttgcatttt gaatatttct 16200
   ctggaaaaag agattagttg gcagtagaaa tgaatagaaa tcaatagata ttaaaatacc 16260
   tcagaatttg attcatctct gggaaaagat gaaaaataaa agtgtatagt cctcaagaaa 16320
   atctgggatc aaaagcatgg gccttaccct attgaattaa ttaacctcag aagttgggaa 16380
   ctgtggaata aggatgtcca ccagacttcc tagggattac aaatgtttca tagaacttga 16440
   aatttaaact tgggtcactg tatgggatgt agagctgtgc tatatggaaa taaaaatgat 16500
   ttctttttct caagggagaa tgatggatat gaaaaaatgt ggcattcgag ttacaaactc 16560
   aaagaagcaa cgtggtaaga atatcagaag gaattgggaa gtagaaggca aaggaaacaa 16620
   aaagctaaag caataacaaa gagaaatcca ttagtcataa tctcctctcc ttttaaagaa 16680
   tgctggttcc cctttgcctc acaactaata caagaacttc tccaccatct caggaagttt 16740
   agggatggcc ttcaagagta gagagtaggg agcagctctg tggagagagg agaggagcag 16800
   ggaaggggaa ggagtcagct tctctttgct aatctgttgc cctgcaccct agcagctccc 16860
   tgcagcaggg gacaaggttg acttaggtgg atggataatt aattgattct aaaatattgt 16920
   gtgtcagtat tgtatattgt aatactatgt taactgcgcc atgcacggta tctcatttaa 16980
   tcccccaccc cttgccatta ccaaaaagag agagagaaaa atactagaat tatcctcatt 17040
   ttacagtaga gaaaacagag ggtcaagaag ataaagtaaa gtgcccaaga acacacaact 17100
   gatcacaaat atcaagcttg gggtccatta gcctaaccac agacccttac tcttaaccca 17160
   tctgcttcaa tccattttgc tacaaatgtt tacatttata tgcagggcag aaaagtctca 17220
   tccagtttat tgaactaaga agaaagttat attaaggtgt ctaatttttt ttaatgtagt 17280
   tagaaaccaa acttaacaat gagcccaagt ttaaagcagt ctaattaact tgacaagctc 17340
   aggcaagttt cattctgtgg cctgtagcat catctgtgtt gtaaagctaa gtagcaaatg 17400
   ttatttgggt catcctgggg ggaaagtcat cccaatttgc tcaagactga ggggtttttc 17460
   aggatatcat gtaaggataa ttgggtacaa atataacctg cttctttctc tcatttcaaa 17520
   tttatcattt atcatctcag caactatgag ttatgttttt tattagattt cttgttactt 17580
   tttccccaga ccgctcccca tgaaattaat atactattat cactctccag atacacaatt 17640
   ggaggagacg taatccagca ttggaacttc tgatcttcaa gcagggattc tcagcctgtg 17700
   gtttgggggt tcgtcagggc tgagcatgac cagaggaatg aatgggcccg tgggatgcat 17760
   gcagtatggg acttaaaagg cccaagcact gaaaatggaa cctggcgaaa gcagaggagg 17820
   agaatgaaga aaaatggagt tgaacaggga gcgtggaggg agaccttgat actttcaaat 17880
   gcctgagggg ctcatcggtg catgtgacag ggagaaagga tacttctgaa caaggagcct 17940
   ccaagcaaat catccactgc tcatcttagg aaaacgggtt gagaatccct aatttgaggg 18000
   tcagttcctg cagaagtgcc ctttgcctcc actcaatgcc tcaatttgtt ttctgcgtga 18060
   ctgagggtcc cagtgttgga acagtattta tgtatgagat tttcctattt attttgagtc 18120
   tgtgaggtct tcttgtcatg ggagtgtggt tgtgaatgat ttcttttgaa gatatattgt 18180
   agtagatgtt acaattttgt cgccaaacta aacttgatgc ttaatgactt gctcacatct 18240
   agtaaaacat ggagtatttg taaggtgctt ggtctcctct ataactacaa gtacacattg 18300
   gaagcataaa gatcaaaccg ttgatttgta taggatgtca cctttattta acccattaat 18360
   actctgattg acttaatctt attctcagac ctcaagtgtc tgtgcagtat ctgttccatt 18420
   taaatatcag ctttataatt atgtggtacc atacacacat aatctccttt catcgctgta 18480
   accaccctgt tgtgatgacc actattattt tacccattgt acagctgagg aagcaaacag 18540
   attaagtaac ttgccaaaac cagtaaatag cagagctcag actgccaccc actgtccttt 18600
   tataatacaa tttacagcta tattttactt taagcaattc atttattcaa aacccattta 18660
   ttaagtgccc ttgcaatatc aatcactgta ccaggcattg aatctacaga tgtgagcaag 18720
   agaaagtacc tgtcctcaag gagcttggag tataataagg agattaataa gaaaatatat 18780
   tattacaatc tagtccagtg tcatagcata aggatgatgt gaggagaaaa gctgagcagt 18840
   gttgccaaga ggaggaaata ggccaatgtg gtctgggaca gttgaatgta tttaaacatc 18900
   ttaataatca aagtaatttt catttacaaa gagaagtcag tacttaaaat aaccctgaaa 18960
   aataacactg gaattccttt tctagcatta tatttatccc tgatttgcct ttgccataca 19020
   atctaatgct tgtttatata gtgtctgata ttgtttaaca gttctgtctt ttctattcaa 19080
   atgctattaa attttaaatt catacctttc catgattcaa aattcaaaag atcccatggg 19140
   agatggtttg aaaatctcca cttcatcctc caagccattc aagtttcctt tccagaagca 19200
   actgctactg ccttttattc atatgttctt ctaaagatag tctacatttg gaaatgtatg 19260
   ttaaaagcat atatttttaa atttttttcc ctaaatagta acacattata tgtctgctgt 19320
   gcactttgct atttttattt attgtagtgt ttcttatgta gcagatggaa tgaatttgaa 19380
   gctcccaagg gtcaggacac atgccttctt tgtttctaag ttatctttcc catagctttt 19440
   cataatcttt catatgattt agtacatgtt aaatatgtgc tacatataca tttagacaac 19500
   cagcatttgt taagtatttg ctctaggact gagtttggat ttatgtttgc tcaaaaggag 19560
   acccatgggc tctccagggt gcactgagtc aatctagtcc taaaaagcaa tcttattatt 19620
   aactctgtat gacagaatca tatctggaac ttttgttttc tgctttctgt caagtataaa 19680
   cttcactttg atgctgtact tgcaaaatca cattttcttt ctggaaattc cagtagtgta 19740
   ccttgactgc tagttaccct gtgccagaaa agcctcattc gttgtgcttg aaccctttaa 19800
   tgccaccagc tgtcatcact acacaggcct cctaagaggc ttcctggagg ttttgagatt 19860
   cagatgccct gagagatccc agagtttcct ttccctcttg gccacattct ggtgtcagtg 19920
   acaaggaata ccttcgcttt gccacccgtc aaggttgaag aaacagcgtc tccaacagag 19980
   ctccttgtgt tatctgtttg tacatgtgca tttgtacagt aatttgtgtg acagtgttct 20040
   ttgtgtgaat tacaggcaag aactgtggct gagcaaggca catagtctac tcagtctatt 20100
   cctaactcct ccttttggtg ttggatttgt aaggcacttt atcccttttg tctcatgttt 20160
   catcgtaaat ggcataggca gagatgatat ctaattctgc atttgattgt cactttttgt 20220
   acctgcatta atttaataaa atatccttat ttattttgtt a 20261
<210> 3
   <211> 20340
   <212> DNA
   <213> macaca fascicularis
<220>
   <221> misc_feature
   <222> (1658)..(1881)
   <223> n is a, c, g, or t
<220>
   <221> misc_feature
   <222> (2742)..(2745)
   <223> n is a, c, g, or t
<220>
   <221> misc_feature
   <222> (2747)..(2747)
   <223> n is a, c, g, or t
<220>
   <221> misc_feature
   <222> (2749)..(2749)
   <223> n is a, c, g, or t
<220>
   <221> misc_feature
   <222> (4764)..(4802)
   <223> n is a, c, g, or t
<220>
   <221> misc_feature
   <222> (8775)..(8778)
   <223> n is a, c, g, or t
<220>
   <221> misc_feature
   <222> (8980)..(8984)
   <223> n is a, c, g, or t
<220>
   <221> misc_feature
   <222> (9161)..(9293)
   <223> n is a, c, g, or t
<220>
   <221> misc_feature
   <222> (9344)..(9348)
   <223> n is a, c, g, or t
<220>
   <221> misc_feature
   <222> (9840)..(9874)
   <223> n is a, c, g, or t
<220>
   <221> misc_feature
   <222> (9958)..(9973)
   <223> n is a, c, g, or t
<220>
   <221> misc_feature
   <222> (10437)..(10452)
   <223> n is a, c, g, or t
<220>
   <221> misc_feature
   <222> (10603)..(10603)
   <223> n is a, c, g, or t
<220>
   <221> misc_feature
   <222> (10830)..(10830)
   <223> n is a, c, g, or t
<220>
   <221> misc_feature
   <222> (10989)..(11006)
   <223> n is a, c, g, or t
<220>
   <221> misc_feature
   <222> (11105)..(11122)
   <223> n is a, c, g, or t
<220>
   <221> misc_feature
   <222> (11457)..(11457)
   <223> n is a, c, g, or t
<220>
   <221> misc_feature
   <222> (13710)..(13710)
   <223> n is a, c, g, or t
<220>
   <221> misc_feature
   <222> (14015)..(14049)
   <223> n is a, c, g, or t
<220>
   <221> misc_feature
   <222> (14518)..(14585)
   <223> n is a, c, g, or t
<220>
   <221> misc_feature
   <222> (14689)..(14699)
   <223> n is a, c, g, or t
<220>
   <221> misc_feature
   <222> (14707)..(14767)
   <223> n is a, c, g, or t
<220>
   <221> misc_feature
   <222> (14837)..(14865)
   <223> n is a, c, g, or t
<220>
   <221> misc_feature
   <222> (15703)..(15741)
   <223> n is a, c, g, or t
<220>
   <221> misc_feature
   <222> (17403)..(17415)
   <223> n is a, c, g, or t
<220>
   <221> misc_feature
   <222> (19122)..(19135)
   <223> n is a, c, g, or t
<400> 3 
   ctgaaagctt ccccgattcc gccgaaggtc aggaaagtcc aatgccgggc aaactggatt 60
   tgctgccttg cgcagaggtg ggcgggaccc cgcctccggg ccgggcgcca agttgagcag 120
   ctggcacgcc tcgcgaagcc ccagtcctga agccccagtc ctgcgctgct tcccgaggct 180
   ccgcaccagc cgcgcttctc tctgcctgca ggtagggagc gttgttcctc cgcaggtgcc 240
   cacggcccag catctctggc taactcgctg ggcaccttag gacggaggat ctctacaccc 300
   tttctttggg atggagagag gaggagggaa agggaaggcg atggtctagg gggcagtaga 360
   gccaattacc tgttggggtt aataagaaca ggcaatgcat ctgggcttcc tccagcgcaa 420
   ttcagttttg ctctaaaaat aatttatacc tctaaaaata aataaggtgg gtagtatagg 480
   ataggtagtc attcttatgc gaccgtgtgt tcagaatata gctctgatgc taggctggaa 540
   gtctggacac gggtcctagt ccaccgtcag ctgcttgcta gtaatatgac ttgtgtaagt 600
   catcccagct gcagcagata agtaagtctc ttcctgcgct aagcacgtcc aggacccctg 660
   aacggaattt atttgctctg tccattctga aaacccaaag gagtcctaaa agaggaatgg 720
   aggagcctaa gaataaaaat agtataataa aacatttctt agacaggttg accttggcct 780
   atgtcaaagt tcagtctggg tttgtcttat aacataagga gtaaaagtac cattgttcta 840
   cctctttttt taatacttga aaaaaaattt actgtagatg cttttctatt aattaaataa 900
   ccttctaaaa aatgttttta gtgctgcatt cgattaggtt ggataactaa atgaaattaa 960
   ttcctcactg ttgggtataa aggttattta cagtggttct gtcttagcta ttcactgaac 1020
   atcattacat agatatctct ggaatattgc tgattgtttc cgtcaataaa cttagaagtg 1080
   taactactta gtcaaagaga ctgaatattt taaaggcatt ttgaagaaaa ctgaaaatgc 1140
   tttccagaaa ggatgtatca gttgacaatg atggttgtca acagtattta aggagaacta 1200
   tgatactctg aagaaaaact tagcctttct cagtagaagc aggtaggcag aggccacatg 1260
   acagcagtta gagtgtggtc ttcaaggaag tcacagaaat actgtgggga attgaaaccc 1320
   caagtggaaa atgtacaaga gtgtctcagt gtgactgaga aggaggttgg gcttggggtt 1380
   taacttaaga atttttcttt ttcttttgtg gagatagggc ttttgttatg ttacccaggc 1440
   tggtcttgaa ctcctagcct caggcgatcc tcccgcctca ggctgcagaa gtcctgggat 1500
   tactggccgg agccaccatg caggcctctt gctcctactt ttgagaaagg aagcttaacc 1560
   tttttttttt gtttgttttt gtttttgttt ttttttgtag acgagtctca ctctgttgcc 1620
   caagctggag tgcagtggtg ccatctcagc tcacagcnnn nnnnnnnnnn nnnnnnnnnn 1680
   nnnnnnnnnn nnnnnnnnnn nnnnnnnnnn nnnnnnnnnn nnnnnnnnnn nnnnnnnnnn 1740
   nnnnnnnnnn nnnnnnnnnn nnnnnnnnnn nnnnnnnnnn nnnnnnnnnn nnnnnnnnnn 1800
   nnnnnnnnnn nnnnnnnnnn nnnnnnnnnn nnnnnnnnnn nnnnnnnnnn nnnnnnnnnn 1860
   nnnnnnnnnn nnnnnnnnnn ngactttttc tctatctctt ccttctaccc atcctaccct 1920
   tggaagatag agaagtaata tcagttccat cgtgttatac tgggcttccc ccagggacaa 1980
   acccacttcc ccaacctgaa tgagccatca cttcttcccc agtttacatt tcattgctct 2040
   ttgaatgtct ccgttcggat atgggaattc acatgtggtc ataattctta cctgaagaag 2100
   actgacgtct tcttctctta gaccaactgc cctgatgtga ggtttagagg ttaaagaaca 2160
   tgtgtgtatt tacatgatct ttgtattctg ccttttcgtc cctcactaat gacagctgaa 2220
   ccccaaggaa atagagctgt ggaggagagg gtttgatgag aaagtaggta aatattggat 2280
   ctaatccatc atcttccagg aaacctccat tacttctaaa aatttcaacc aaattcgtta 2340
   aaggacaaga actccaccag agtagggcca taaacattgg caaaattagt tgtaatctat 2400
   gactagattt aatgtccctt tgttttattc acatatggtt ataatgcttt gcttggaatt 2460
   aggggtattt taagttttct tctgcctagt aagtgtattt gtgcttataa tacaataatt 2520
   ataaaatatc acattaatat tttataactg tacagttaac tctggcccaa ggaaaagata 2580
   gtccggtaga tgctgcagcc tgattttgta tctaaccctg gcaagaggat aatgactcat 2640
   gttatttcac ttaccctttt tatcttttaa catgaagggc tcatataggt caataagaaa 2700
   ccagtgatat aaacagacca aaaaatgatc agatctttca cnnnnanana aaaaaaatat 2760
   ttttaaacaa tacccaactg ggtgaaaata caatgtaaca gtaccaaata tcaacatgtg 2820
   tcaagaacca gaaaaatgtt tgttttcttt gatcagcaac actatttgag gaaatctatc 2880
   ctcagggcct agcctggggc ctggcacaca gtaggcactg aacaaatatt tgctgaacac 2940
   acacatactt atgatatttt taaaattggc aacaatccaa tacccaataa tagaggaatt 3000
   aaatattata gaactgttca ataagatgct tacgaatatc atgcagtaag atgggcaata 3060
   tttatatcat aagcttaaat gaaacaaatg ggtattaaag gtatggtaag gttataaatt 3120
   actttttaag agattaaagg gaaaagactg aaagatatat actgaaatgc tcacagtggt 3180
   gacaaggttc cccagccttg gcactattga cattttgggc tatgtctttg ctgtgggagg 3240
   ctggcctgtg ctctgcagga ggtttggcag cactcttggt ttctacccct agatagcagt 3300
   agcaaccctc cctcaaccag cccaattttg acaaccaaaa atgtttccag gcatcaccag 3360
   attctccctg ggtgagagtg atgaaacagt aggtgatttt ccccttcttt tctcattttc 3420
   tgtaattttg tcacattacg ttaataataa ggaaaaaaca taaaaaatag atgaatttat 3480
   tattctacct cagtttggat gtttggactc cctttggggg ttctttccat tatatcactt 3540
   ggtctgctaa acagtctatg gtttggcaag gtgaaatgat tcatgaaatt ttgtttttat 3600
   tttttacctg atactaaaag taaaacattc attcgcttga aaatttggac acagaacacc 3660
   aaaaaaaatc cataatctca tctctctttt tctgtctttt ccttcctttt ttccctttaa 3720
   aaacaagagt gaaaacctac cggttctccc tccaatttaa ttcctaaata taatcactgt 3780
   taacatcttg gacatttcct gtgtctaaac acacatactc actttttctt ttttttagca 3840
   aaaagtggat ttctgctaca tgtagtgttc cgcaacttcc tacatgttta caaaatcagt 3900
   acatttacat acgctgaatt cagtccttaa tggtattata ttttgtgaat ataacaaaat 3960
   ttatttaacc acttagacaa tctaagatat tctcagtttg ctgttatgag caatgctctt 4020
   cctttacata tacatatata tgtgtgtgtg tgtgtgtgtg tgtgtgtgtg tgtgtgtgtt 4080
   ttagtaggat agatttctag gagagggcga aatgtcatat gacatccaca tttacaattg 4140
   taataggaag tatcaaagtg ccccctaaaa aaaaaaattc ctcccattag tgtgtgagaa 4200
   agcctatttg ttcatatctt cacaaacact aaatattaga aatatttaca attgtgttca 4260
   agctaagaag tgaaaaatgg tatttcatat cttataattt ttgttgtgag attgaacata 4320
   tttcctatgt ttacatgtca cctgtatttc ttattctctg aactatacgt tatgaccttt 4380
   cacttatttt cctcatgggt tatgtgtagt ttgtgtagtt gtcttattga ttgttaggag 4440
   ctatttatat attaggaaca ttaatctcct gtcttatatg tatgtggcat tgattagttg 4500
   atcatttgtg agttcatgtc tgtatacaaa gattagagag gcagtaagag ggaaaactta 4560
   cctctttctt atcaaagttt gtaaatatat gtataacaga agagagagaa aacattaata 4620
   aatgctgaaa taaagtataa atttttactc cactacttca acataaacta caaaaggaga 4680
   gtgacttttc tttcatgctg acttccatat tccccatgcc taaaatagtg cctagcacag 4740
   aagaggtgct caatcagtgt ttgnnnnnnn nnnnnnnnnn nnnnnnnnnn nnnnnnnnnn 4800
   nnaaatgaag aatagaatct aggcagatac tctggaagag tgactgtgag tcattcatgg 4860
   tcttggtatg aattagtcaa atccagcttt ctccccttcc cactccccac tgttagtaga 4920
   ataatctgtt tattgagaga atagatttat aatttagaat aagaggggca gaagaggaga 4980
   ttttgaagga tggcacctga aggaggacta gcatggctga gatagtgaag tggaagtctt 5040
   ggaaagctaa agggtaagat gaaagtattt agccgtaggg ggaaaaagca ttgacaggtt 5100
   ggaaatgtaa aagtcagatt ctccttgctt tgaaattttg tacagggcag gttctactag 5160
   gtatgttaca atgcagaaaa aacatgaaat agttgagagg aatttggtga aatattatct 5220
   tcctggcttc ttttgagtga gcagattttt ttcagggcct gtaactataa taaatttgaa 5280
   acttctcatc ttttagtaac ttttttcact taagtttatg tggctgtggg caatggaatg 5340
   aaggtattga acttcctatt ccctgctggg tttccacaat tacaagtcaa tcatgactgg 5400
   ttattagaag actatttcaa ttagaaccac caagtcccat aatgtcatat tgtgtgttta 5460
   attattaagt aaagcagtct tctttttgtg ttttccataa ttagcacatt ccagaaagat 5520
   gaggatattt gctgtcttta tattcacgat ctactggcat ttgctgaatg gtaagacacc 5580
   aaatccttcc attaggttct atattttaaa tattttaacc atgagtttaa aactaaaatg 5640
   ataatttaaa atgcatgcaa ttttcttata gagagaatat tctcttcttt cttctacttt 5700
   acacaatgac aaagtctttc ttctgcttta cacaatgata aagctccctg tgtcattgtg 5760
   taaaaatata gagaatatag acaaattgaa agacacaaaa taatctatta cccatttccc 5820
   agggttaact actgaaaata tctggggaaa tggcctgtat gtatatattt atttgtttgt 5880
   tttcaacaag gccaggatct ttcaatcttt caatcttggt tgctctgtga catgcctttc 5940
   ctgatgagaa tactctaagg aagaattgta ggatacatgg aaaatgtcag ggtaacacag 6000
   tactggcacc accctgcggt cctttctgaa ctccatacca atgtacttct tgccagaaaa 6060
   ctgatcaaaa gtttagggaa gtaaaaagag atgttagaat ctaccattcc ctctatgtag 6120
   gaagcaaata ggtgtccagt caaaggacat tctggggatg tctacatgaa accaagtctc 6180
   ctggttgtaa gtactccatc tccatataat atttctacag taatatatgt ttataaattg 6240
   tgggggcaac ttgtttagct aattttatta ttctgttatt gggacactat gtctctgcat 6300
   gagacatagt gtcccaaaac atatttcaag cccattggat aaaatatgta tttagcaagt 6360
   tcttaaatat aatgataaca taactgacca gataaggtga tttttaaatg ctgtgccaac 6420
   tttataaatg ttttgaggaa ttttcccttt tctgaaggtt attcttcttt ctttttagca 6480
   tttactgtca cggttcccaa ggacctatat gtggtagagt atggcagcaa tatgacaatt 6540
   gaatgcaaat tcccagtaga aaaacaatta gacctgactt cactaattgt ctattgggaa 6600
   atggaggata agaacattat tcaatttgtg catggagagg aagacctgaa ggttcagcat 6660
   agtaactaca gacagagggc ccagctgttg aaggaccagc tctccctggg aaatgctgca 6720
   cttcggatca cagatgtgaa attgcaggat gcaggggttt accgctgcat gatcagctat 6780
   ggtggtgccg actacaagcg gattaccgtg aaagtcaatg gtaagaatta ttatagatga 6840
   gaggcctgat ctttatagaa aacatattct aagtgttgaa gacttttcat tcttataagt 6900
   ccatacttat tttcaaacag aatagcatag tctcttcatt cattcattca gttcatgaat 6960
   tcattcacgt aagtctccaa ttagcatttc ttgagcacct atatgatagt cattggaaat 7020
   ccagagacaa cacagagcca tgttctacgg tatgtacagt tttccaaaaa taattcctag 7080
   tctttacttt tttattataa atgtaataca tatacttgca aagaattcag atactatgga 7140
   agagatcaaa tgaattgcaa aagtgtccct cctcccttca ccactatctc ccatgagata 7200
   accaagagac actccaagag agtaaccatt atttgtgtgt ccctccagaa ccttttttat 7260
   tcaactacca tttttttatt ttattaggtc tgtcagtttt ccttttttga gcctctctat 7320
   atcaaatgct aataaatata ttcagatcaa accccactgt aaggttcata ttaaaaaaga 7380
   cttgaagtct ccctatgaag acaaaaaata atcatattaa gtgtaaaaga acttattctt 7440
   ccagtacagt ataaactata ctcactgggc atatattcat cttgaaaatc tatactgatg 7500
   ttgtcttggg gaattgaaga ggaactagga gtgtcaattc ctgggaactg acccacagtt 7560
   atgtcatcag gtcacttgag ttcgaagttt tgtgttagca ctagctaagt aaaggaaaac 7620
   acctctgctt tcattgttga gtttcataga attgagagct gaaaggatcc caggcaggag 7680
   cgactaatcc aaactcccac aaagaacaaa aatcccccag aggatcttct gttcatatat 7740
   ttcctgcagt ggcatccctg tcatatccca caatggcatc cctgccattt ggactcccct 7800
   tccatatcct gttgaaatta ctccctaata gtaagctgaa atctgcccct ctagttgtag 7860
   ttttggaatt atttcatttc catgatgacc ttttaatatt tgactagaat taaatcatct 7920
   ccccttggta tttccattcc tggactaact accatcaatc tgagggctaa caatacaagt 7980
   agaaaaagtc tacacttgtc attgatcact gatcaatgat taatcaatga tcactgataa 8040
   ttataaactc aaaaacaaaa tcatgtaggg attaagagaa atgtatcagt tttatgttgt 8100
   atttctggtc cctgattctg gctcaaataa tgctactatt gtcaagaaga tatcacttgt 8160
   aaagtagatt taattttcat tatattttac catgtgcttc tccattcacg gcatttcttg 8220
   agatgttgtg gtttatactt tcagtttttc tccagtccat cagcaaatat caggcatcta 8280
   ctgtgttcca agatattaaa gaaatcatca tgacttagcc tcatcaacag cattgctaga 8340
   tctgggatgg aaaagaagag tataatcctg gcagtcagga agaaggctgc ataaagtata 8400
   agtttctgct tccaaagaag atctctcatc agcctgtagg gagtgtatag ggaggggaca 8460
   gctgtccttg tagtagcaaa gggttttatt caggtcatct gggctccata atatcccttg 8520
   tgtatctgca gtctcctttg ccatggatca acacaatagg aaatcttccg gcactgatgg 8580
   tttttccaag ggggagttct tcagggagca aagcaaatga ccaaccaggt ttgaggacct 8640
   gatttgacaa ttccattttg tattttaaat tagttaattt gcattctagt cccaatccat 8700
   cttgtcattt gcagacagtg gttttggggt tgagttgagc tataccaaaa gtctgaacct 8760
   tctgcactta gaacnnnnaa ggcaaccacc aagcttcact tgcactgagg cagtgtctcc 8820
   aatggaaacg aggtagctgg cttgcagaag ctttccaact cagggaagta gaactcctga 8880
   gtcacctcca tatgcaaata atttcacagt actgctgttg aacttcactt cccatcacag 8940
   caaatgtgtg gtaacatagc tttgccacag gagtttactn nnnnccatgg gattttaaag 9000
   gtgaaacatt tcaaaactga aatttgaaag aatttagttt tggattcact caattatcat 9060
   gatcactttg ggtgttattg cacctttcat gtttgtgagt ttaaatacca gactctcagg 9120
   cctctaactt tcaattaaaa gtgtttttct ttaatcactg nnnnnnnnnn nnnnnnnnnn 9180
   nnnnnnnnnn nnnnnnnnnn nnnnnnnnnn nnnnnnnnnn nnnnnnnnnn nnnnnnnnnn 9240
   nnnnnnnnnn nnnnnnnnnn nnnnnnnnnn nnnnnnnnnn nnnnnnnnnn nnnctgtcat 9300
   tccccaccaa ccccatcttt ccccaaaatt cccagccctg tttnnnnngt ttttctttaa 9360
   tcactgaacc taatagtagg gaaaacgaaa tgttcattca gactttcagg accttcaatg 9420
   agatgaggca gctgaaagat caaagtagtt gcatagttat cccagtaaag ctatttggat 9480
   cgtatggacc agatcaactg ctgtcattcc ccaccaaccc catctttccc caaaattccc 9540
   agccctgttt aagtgttctc tgtagcattt atctctatct agtatattgt gtagcatatc 9600
   atatcatact tttctatttt gtttattgtc tctctcctcc tagaatataa actccacaag 9660
   cacagagatt tgggtctgtt ttttaatatt gttgtatccc cagggcttga cgtaaagcag 9720
   agtggtagta tgacaaaagc acacaaaaaa tatttgttga gtcaatgaat gaatgatttc 9780
   ctcaaatagg attagcctaa aatttggaaa catgaacaga cttggatata tgaaaatttn 9840
   nnnnnnnnnn nnnnnnnnnn nnnnnnnnnn nnnnttctaa agggtacact gaagcagcag 9900
   tagtaaaagg aggaggagga gaagcagctc tgctactact attatcgagt actactannn 9960
   nnnnnnnnnn nnnctacaac tatcgagtac tactacaatt agcacttgct tattctgtgt 10020
   gttaggtcct gtactgaaca ttctgcctaa attagttcat ttcctcctgg aaatgactct 10080
   gtggggtagg tgcttcatca tgtaagatga gtatttttca cacttcactg tctctgaaat 10140
   ctgagtgtgt ctttcaatga tggaatcttt gatttcatga taagtggtat tattcccatt 10200
   ttaaggatga ggaaactggg gtccaaagaa attaagtaat ttgcccaaat tcacctagcc 10260
   tcgtaaatga taaagctagt tctaaatcca agcagattgg ctctgaagtc tgggccctta 10320
   ataaccactt attgcctgta tttgcacctc tggtgtatgt atcaagttat atattggctt 10380
   caaaactatc atgacctttt cttgattttg attgttcaat attagtatag tgttctnnnn 10440
   nnnnnnnnnn nngggtctgc aaaaaatttt gattactcac ctcatctgta aaacatttta 10500
   aacttgtgtg tctgtgcagg cacatttgtg tgtaattata aaaaatttac tatctattaa 10560
   tatataggtt gtaccgtaag aaaaattgcc atttttgaag agncaaaaaa ggttgaatat 10620
   taccagtttc atctggttca acctaataga catttgtaca aaaacagaca ttttaagagg 10680
   atgaaataaa aatttaataa acaatatttt caatttttac taattgtgac gcttcactat 10740
   tgttagctaa tatgtcaagg catgatatac cttagggtgg aatttatcat taacaaaggt 10800
   ggatagtgtc aataatcttg aggtttgtgn ttttttatat aacactgtga ggtctaatta 10860
   agtacttaat tgtttacaac ctcatacagt cgccaataat aagtgtcact tctgctgttt 10920
   cctctgggtt gtgcttgaat tattagtatt atcttcaatc ctcagtttct ttgtggaaaa 10980
   ctttttaann nnnnnnnnnn nnnnnnaaga tggttagttt agtcaaaatt agataagaga 11040
   atctgaaaat ccataattac cccaaagcaa cccactcata agaactatta tttttgtgtt 11100
   ttggnnnnnn nnnnnnnnnn nntttccagt gtttccactg gtagtggttt cattgatgta 11160
   ggagtatcaa aacatcacta attatttatt tcagttttgt ttgatcctag ctgttttgtg 11220
   ttaactttga agaaattaca tcacagatct attgttgtcc ttggtaaagg aatggagagt 11280
   taaggctcta gatcattagt ggttatgctg tagtattagg agtaaaaaaa agattatatc 11340
   aacaaaataa gaacatgtta atgtacttgt aatagataaa catgaataaa gctcttatgc 11400
   tatatagatg cactgaacaa tctactagaa ttgtcagcaa acggtatctt aatcctnaaa 11460
   agggtcccaa accaatgatc taaaattgaa tcaaactttc ttccttgagc ataattattt 11520
   aagtgattta ttaaaatagc cagcatttaa aagcttaaaa tataagtatc ataatgtggt 11580
   atcctagata gatcccagaa cagagaaagg atattaggga aaaactggag gaatggaata 11640
   aattatgcag tttagttatt aataatgtac taatgtcctt agttatgacc attgtaccat 11700
   ggtaaagtaa gatactaaca atagaggaaa ttgggtaagg ggtatatgta actctatact 11760
   atctttgcaa tttttttgta aatttaaaac ttctaaaata aagaacaaat ttgaacatta 11820
   aaaagtgtcg ccaggaacat gtatcactgt ttacagatga aacagtatgt atttttatat 11880
   ctaatttctg atcattggct tcaaatcaga aaagtgaatg acacatcaag atcaggtttt 11940
   ctgtttacta aataaagtct aagaaaacaa agcataccag ctggagagat tcatgtttat 12000
   aaagacagat ttataacaac aaaataaaat atccaagaat aaatttaaga agaaataggg 12060
   cactatgtaa aaagtatagc actttactga gaaacatatg aaaacctgaa tacatggaga 12120
   gaggtatttt atatttgaat agaaagattg ctggtttaaa gataattctc tttaaatttt 12180
   ttttgttaga aatttaagag gtacaagagc agttttgtca cacggatata ttacatagtg 12240
   gtgaagtctg gggttttagt gtaaattaat ctttacattt tgtttgagcc caataaatgt 12300
   accaacatga tttttatagc aagatagtca ttcctattaa cccaaacttg tcccaacttt 12360
   gaactgaact gaggcagagc tagcaggtgt tccccactgc tgaggcatct gaacattaag 12420
   cgtatccctc tgagaaccag cctgcattga tcctctttct aatgtagaca gcatcaagct 12480
   atatatctag ttctgtgctc agcaaaagcc ctgacttctt tttgcttatg tcctagctcc 12540
   atacaacaaa atcaaccaaa gaattttggt tgtcgatcca gtcacctctg aacatgaact 12600
   aacatgtcag gctgagggct accccaaggc cgaagtcatt tggacaagca gtgaccatca 12660
   agtcctgagt ggtaagacca ccaccaccaa ttccaagaga gaggagaagc ttttaaatgt 12720
   gaccagcaca ctgagaatca acacaacagc taatgagatt ttctactgca tttttaggag 12780
   attagatcct gaggaaaacc atacagctga attggtcatc ccaggtaata ttctgaatgt 12840
   gtccattaaa atatgtctaa cactgtcccc tagcacctag tatgatgtct gcctatcata 12900
   gtcattcagt gtttgttgaa taaatgaatt aatgaataac attatattta caaaatgtat 12960
   cctaattcct cacttccatt catccaaatc atattgttac ttaataaaca ttcaccaaat 13020
   atttattgaa tatgcctttt gttccatgca ttgtagtact catttgacac acatagaata 13080
   ataagactca cgttcacact cttcaggaaa cagataaaaa acaaatgaac aaacaaaaaa 13140
   caggcaatcc aataccatgt gggaaatgct ttcataccat gtgggaaacc tgggggaata 13200
   cctgagagga atattcaatt caggccatgt ttcaggaatc caaatcctgg cacatcagag 13260
   ccgcctcctt cttactaggg tttctgtggc aggaaataaa tggaacgtat ttttctatct 13320
   tatgccaaac aggagggacc ctttctcccc tgtgcctctc ccaaggtagt ctacaatatt 13380
   tcaacgctag cagtctgttt agtgcacagg acatgaggct gtgtatccct gggcaaattg 13440
   ctacacttct gtgtgcttca ctttctctgt aggattataa cctactgagc aaggttattg 13500
   tgggggtcaa attagcaaca gtgtatgaaa atgatttgag accagtgcct gcacaaattc 13560
   aactattttt ttttatctca ctactctata gaagtaggta ggatgggaga cagagtctga 13620
   tgggaggctc agaatgtgaa agtaagtgag gtgagtgagc atgatatttc atataaacac 13680
   aaagatactc tgagaagagc ttctcacttn ccccgccccc aatagatgtt gacaggaaaa 13740
   tgccacgtac ttcagcaaaa acagctgaaa aattagacat aaaagtcaat caataggaaa 13800
   agataatcca ggatggtctt gtgaacagaa agaggaaaaa aaaagtttag aaaatgatgg 13860
   ggatgctctt actggggtat gagtcctcag gtattcaact ggctttcaga aaaagctaga 13920
   ctagtgggtt cctgccattt aaaagctgtt ttatgacaac ttacttgttg ggtggcctac 13980
   agtaactcac ttaactgtgc tgagtctgtt tcctnnnnnn nnnnnnnnnn nnnnnnnnnn 14040
   nnnnnnnnna tgcctaactc ataaagttgt tctgaaactg aaataaaaca tatatgaaca 14100
   ggcattgtaa actgtaagtt acggaaaaag ctggctgttg ttgtgtcttt aaagcttcac 14160
   ctgggtagtt agagatggat catgggtctc agtggagagc tgagccaggc aggagctgac 14220
   taagggtaag aggtgggaat tagcaatctc tgaacatctg tgtgccatgg gacccctttt 14280
   cctcctgcat ggtaccccag acaaggagcc tagtaagaga tactaatgac ttgttgtcca 14340
   gagatgttca aactgcagag aaagataaga caacaagcat tggcctccaa tcatgatgac 14400
   agatagagga ggtgggagct ccttagcagt gctggttggt tttccatgtt ctactgtggg 14460
   ccatctctgc catgtactgt aggctactaa cttctatatt aaaaaatgca agaggggnnn 14520
   nnnnnnnnnn nnnnnnnnnn nnnnnnnnnn nnnnnnnnnn nnnnnnnnnn nnnnnnnnnn 14580
   nnnnntcagg agtttgtaac cagcctggcc aacatggtga atctctgcct ctactaaaaa 14640
   tacaaaaatt agccagatgt ggtggcgtgc acccgtaatc ccagctacnn nnnnnnnnna 14700
   ggcacgnnnn nnnnnnnnnn nnnnnnnnnn nnnnnnnnnn nnnnnnnnnn nnnnnnnnnn 14760
   nnnnnnnagc atgggcaaca gagaaagact ttgcctcaaa aataaataaa aaataaataa 14820
   ataaataaat aaataannnn nnnnnnnnnn nnnnnnnnnn nnnnncattt aatactgagt 14880
   agccactcca tgtggtactg tgctaagcac attataaaat attagcctca caagaaatgt 14940
   attagcattt gtattttgta cactggttaa gtatcttgcc caagacctca aaactggtta 15000
   aggggcagca gaatttaacc ccagcgccac cttttcaaag tctgggcttc ttacacttct 15060
   ccatgctgtt cccattttaa cagatgtatc tcgccattcc agccactcaa actttggcat 15120
   ttaagaaaat tatcctaaag ctaaactaaa cttcaaggat gactattctc ctcatgaccc 15180
   cttcccatca aaattttatc tttagtcagt ttgtttttgt tttgttttgt ttttcagaac 15240
   tacctctggc gcttcctcca aatgaaagga ctcacttggt aattctggga gccatctttt 15300
   tactccttgg tgtagcactg acattcatct tctatttaag aaaaggtagt atttccttaa 15360
   ttgcagtggt ctccactggg gatgaggagg gggtgagaat tggatcgatc atggctgcaa 15420
   ggaaacctga cttaacctct gcagggtggt gcaaaggcat tccactattc aacagtaatt 15480
   atattgaagc tgcatgggat cactgggtga agatggggtg taaggggtga gggacaggag 15540
   aatgggtatg gatggaggta gaagatgcag tgtcatacaa tttttttcta tcatgaaaat 15600
   aaccacagac ttactgtaaa gaaggagcta aaatgcctgt cattttcagt tgcattttag 15660
   ttttgcatta gttgcaccca gctggtttct gggtactcta acnnnnnnnn nnnnnnnnnn 15720
   nnnnnnnnnn nnnnnnnnnn nccatagagt attttgtaaa attattggca gaggatgtac 15780
   ataatttgca tgtgttcctt tctccattta cctgtgggaa caattaaaat ccaggaaaat 15840
   gagtatattc aaataatttc ctcccattta tgatgattca gagtaaataa ttcctctgat 15900
   acttagagaa gtataccaag agatccagtg attgtataga gttgtctgat gttaaatagg 15960
   gaagtagaat atggaaggga ttccaatagt cgttgaaaaa ttccccacaa ccccttacat 16020
   gggggaaagt ggtgttaact gagatagtag agataagctg ttaccaaaaa ttatgttctt 16080
   aacaggattg agatagccag aatataagga tcaagtttca atgacagtaa gatcctgaga 16140
   tgcagttgat ttgcacaaag aaataattgt tgccagcttg cattttgaat atttctctgg 16200
   aaaaagagat tagttggcag tagaaatgaa tagaaatcaa tagatattaa aatacctcag 16260
   aatttgattc atctctggga aaagatgaaa aataaaagtg tatagtcctc aagaaaatct 16320
   gggatcaaaa gcatgggcct taccctattg aattaattaa cctcagaagt tgggaactgt 16380
   ggaataagga tgtccaccag acttcctagg gattacaaat gtttcataga acttgaaatt 16440
   taaacttggg tcactgtatg ggatgtagag ctgtgctata tggaaataaa aatgatttct 16500
   ttttctcaag ggagaatgat ggatatgaaa aaatgtggca ttcgagttac aaactcaaag 16560
   aagcaacgtg gtaagaatat cagaaggaat tgggaagtag aaggcaaagg aaacaaaaag 16620
   ctaaagcaat aacaaagaga aatccattag tcataatctc ctctcctttt aaagaatgct 16680
   ggttcccctt tgcctcacaa ctaatacaag aacttctcca ccatctcagg aagtttaggg 16740
   atggccttca agagtagaga gtagggagca gctctgtgga gagaggagag gagcagggaa 16800
   ggggaaggag tcagcttctc tttgctaatc tgttgccctg caccctagca gctccctgca 16860
   gcaggggaca aggttgactt aggtggatgg ataattaatt gattctaaaa tattgtgtgt 16920
   cagtattgta tattgtaata ctatgttaac tgcgccatgc acggtatctc atttaatccc 16980
   ccaccccttg ccattaccaa aaagagagag agaaaaatac tagaattatc ctcattttac 17040
   agtagagaaa acagagggtc aagaagataa agtaaagtgc ccaagaacac acaactgatc 17100
   acaaatatca agcttggggt ccattagcct aaccacagac ccttactctt aacccatctg 17160
   cttcaatcca ttttgctaca aatgtttaca ttatatgcag ggcagaaaag tctcatccag 17220
   tttattgaac taagaagaaa gttatattaa ggtgtctaat tttttttaat gtagttagaa 17280
   accaaactta acaatgagcc caagtttaaa gcagtctaat taacttgaca agctcaggca 17340
   agtttcattc tgtggcctgt agcatcatct gtgttgtaaa gctaagtagc aaatgttatt 17400
   tgnnnnnnnn nnnnnggtca tcctgggggg aaagtcatcc caatttgctc aagactgagg 17460
   ggtttttcag gatatcatgt aaggataatt gggtacaaat ataacctgct tctttctctc 17520
   atttcaaatt tatcatttat catctcagca actatgagtt atgtttttta ttagatttct 17580
   tgttactttt tccccagacc gctccccatg aaattaatat actattatca ctctccagat 17640
   acacaattgg aggagacgta atccagcatt ggaacttctg atcttcaagc agggattctc 17700
   agcctgtggt ttgggggttc gtcagggctg agcatgacca gaggaatgaa tgggcccgtg 17760
   ggatgcatgc agtatgggac ttaaaaggcc caagcactga aaatggaacc tggcgaaagc 17820
   agaggaggag aatgaagaaa aatggagttg aacagggagc gtggagggag accttgatac 17880
   tttcaaatgc ctgaggggct catcggtgca tgtgacaggg agaaaggata cttctgaaca 17940
   aggagcctcc aagcaaatca tccactgctc atcttaggaa aacgggttga gaatccctaa 18000
   tttgagggtc agttcctgca gaagtgccct ttgcctccac tcaatgcctc aatttgtttt 18060
   ctgcgtgact gagggtccca gtgttggaac agtatttatg tatgagattt tcctatttat 18120
   tttgagtctg tgaggtcttc ttgtcatggg agtgtggttg tgaatgattt cttttgaaga 18180
   tatattgtag tagatgttac aattttgtcg ccaaactaaa cttgatgctt aatgacttgc 18240
   tcacatctag taaaacatgg agtatttgta aggtgcttgg tctcctctat aactacaagt 18300
   acacattgga agcataaaga tcaaaccgtt gatttgtata ggatgtcacc tttatttaac 18360
   ccattaatac tctgattgac ttaatcttat tctcagacct caagtgtctg tgcagtatct 18420
   gttccattta aatatcagct ttataattat gtggtaccat acacacataa tctcctttca 18480
   tcgctgtaac caccctgttg tgatgaccac tattatttta cccattgtac agctgaggaa 18540
   gcaaacagat taagtaactt gccaaaacca gtaaatagca gagctcagac tgccacccac 18600
   tgtcctttta taatacaatt tacagctata ttttacttta agcaattcat ttattcaaaa 18660
   cccatttatt aagtgccctt gcaatatcaa tcactgtacc aggcattgaa tctacagatg 18720
   tgagcaagag aaagtacctg tcctcaagga gcttggagta taataaggag attaataaga 18780
   aaatatatta ttacaatcta gtccagtgtc atagcataag gatgatgtga ggagaaaagc 18840
   tgagcagtgt tgccaagagg aggaaatagg ccaatgtggt ctgggacagt tgaatgtatt 18900
   taaacatctt aataatcaaa gtaattttca tttacaaaga gaagtcagta cttaaaataa 18960
   ccctgaaaaa taacactgga attccttttc tagcattata tttatccctg atttgccttt 19020
   gccatacaat ctaatgcttg tttatatagt gtctgatatt gtttaacagt tctgtctttt 19080
   ctattcaatg ctattaattt taaattcata cctttccatg annnnnnnnn nnnnngatcc 19140
   catgggagat ggtttgaaaa tctccacttc atcctccaag ccattcaagt ttcctttcca 19200
   gaagcaactg ctactgcctt ttattcatat gttcttctaa agatagtcta catttggaaa 19260
   tgtatgttaa aagcatatat ttttaaattt ttttccctaa atagtaacac attatatgtc 19320
   tgctgtgcac tttgctattt ttatttattg tagtgtttct tatgtagcag atggaatgaa 19380
   tttgaagctc ccaaaggtca ggacacatgc cttctttgtt tctaagttat ctttcccata 19440
   gcttttcata atctttcata tgatttagta catgttaaat atgtgctaca tatacattta 19500
   gacaaccagc atttgttaag tatttgctct aggactgagt ttggatttat gtttgctcaa 19560
   aaggagaccc atgggctctc cagggtgcac tgagtcaatc tagtcctaaa aagcaatctt 19620
   attattaact ctgtatgaca gaatcatatc tggaactttt gttttctgct ttctgtcaag 19680
   tataaacttc actttgatgc tgtacttgca aaatcacatt ttctttctgg aaattccagt 19740
   agtgtacctt gactgctagt taccctgtgc cagaaaagcc tcattcgttg tgcttgaacc 19800
   ctttaatgcc accagctgtc atcactacac aggcctccta agaggcttcc tggaggtttt 19860
   gagattcaga tgccctgaga gatcccagag tttcctttcc ctcttggcca cattctggtg 19920
   tcagtgacaa ggaatacctt cgctttgcca cccgtcaagg ttgaagaaac agcgtctcca 19980
   acagagctcc ttgtgttatc tgtttgtaca tgtgcatttg tacagtaatt tgtgtgacag 20040
   tgttctttgt gtgaattaca ggcaagaact gtggctgagc aaggcacata gtctactcag 20100
   tctattccta actcctcctt ttggtgttgg atttgtaagg cactttatcc cttttgtctc 20160
   atgtttcatc gtaaatggca taggcagaga tgatatctaa ttctgcattt gattgtcact 20220
   ttttgtacct gcattaattt aataaaatat ccttatttat tttgttactt ggtacaccag 20280
   catgtccatt ttcttgttta ttttgtgttt cataaaatgc ttagtttaac atcccggtgg 20340
<210> 4
   <211> 20641
   <212> DNA
   <213> mus musculus
<400> 4 
gcctcatgcc aggctgcact tgcacgtcgc gggccagtct cctcgcctgc aggtaaggga 60

gcatcttctc gcggaatccg cttgcagggc actttaaaga gccagaatcc ctagaccttt 120

ttaggacgga gaagggaacc ggtttcctgg gaaagttaag aactcagaat ccgcagtttt 180

gtgtgtttat ggatcttgtg ggtaggtagc tgggtcagaa gagatgaatt aattggtcct 240

agcgcgactt gactgtttgc tagcaatgac tgggtctttc cacttgaagc atctccggag 300
 
gtcccttcct ctgtcgaggt ctaggatgct ggagcttaag attttcattc tatctgccca 360
gagaacctaa aggatttttg gaagaaaatg tcccaaacag ttcttagata cagtgaccta 420
ggctatgtta aagtttaggt tggggttgtc ataagaggtg aaagtacaat attctatctt 480
tttttaaaaa taaattatta caggtgcttt cctatgaatt acttagccag ttttttttta 540
attgctatat tccattgtgc tgggttatta aagggcatca ctctcccact gaggaatgca 600
caggttagta atagtgcctt actagctcag tcatccactg accatctgtg tatgtgactg 660
tctggaatat ggctaggtat ttcatttaga taaactctta tatgtataag tacttcatta 720
tgtttgagtg tctgaaaggc ctttgtgtag aaaaaaaaaa aacaacaacc tgagaataca 780
ttccagaaag tatccgcagc tgatgatgat agtgaccaag cggtgtttaa ggagaacttt 840
atacagggaa aatattcacc tttcccagct gagcatcata gataggaaga catgatagtg 900
gttatatgct gctctaagga gggatggaga agggatggat ttgagcacag ggttcaggat 960
catttgatag aatttgaaaa ctgggtaaat tttcactctt ttgagaaagg aagtttgcat 1020
ctgcttcccc tccccgcccc aggcactctt ccccccccca tccccccccg tgcccccccc 1080
ccccatggga agataaagaa gtggcgttcc aagatcttca tctggacttc ccctgggaca 1140
aacctatttc cccatcttga atgagcgtca cttctttact ttaaactctg ttctgtgaaa 1200
ggctctcagc tcttgtctgg gaatttgtga aaggttacag tttcttgccg gagggaggga 1260
gaaggtgacc ctggactctt agcccatgta agaggtgcag gtttagaggt caaaggccat 1320
gctgatttac atggtccctg aatttcccct tttgatctac tgctcacacc tgcaccctga 1380
gagccaggga ggggaggata tgatactgaa gaaggtgact atcagaccca acccatcacc 1440
tttcagactt tatactaagc tttaaagcag actcataagc aatagataac tgttccaact 1500
gagtctgcca tcaacgtagg aaaaattagg tacagtcttc agtggatatc atgtacctca 1560
ttgtgactgt ctagtactcg gcttggccat ttctcccagg gcctcattgc aacagtcact 1620
gtgtcctcac aggcctggat gtcatcctgc ctcatctgct tgggcagatg aactggagag 1680
atatgattaa taatgagtca tttgattttg ctctttttgt aaatgtcctt ggtttggttt 1740
ggtttgttta ttttttgttt tgttgttgtt gtttgttttt attttcatgc tcttgttgtt 1800
gtttgagaca gagtctcact atgtagccca ggctagcctc ttcccccagt ctctcagatg 1860
ctgttgcatc tgcccttttc taacatcaat gattcataat aatcagcaag aaggctatga 1920
ccttaacagt caaaataagt aatcaggtaa ttattgagag ctaaatattt atttaaacaa 1980
atcaagttgc atttttcaag aattgaatgg ggggatctaa aaagtaatgt ttgtgctgca 2040
caaagatgta gtggtatcag ttgccatagt ctttctccct agatccgcag atctccttag 2100
ggaaatctat aattcaagcc taaacttgga cctggtacac aactggtact cgcccaaaca 2160
ttggccaagc acacacatat ttgtgactct gaaaaaaggg acaacacata acagcaaagg 2220
 
cgctccgtgt taggggcctg ttaaatacaa gacttgggga aattatgcca taatcttaca 2280
ttttaaaaat ggctattaca tgtgtgagac aacgggggct ggagagatac ctcagaggta 2340
ctaggcacac atatggcaag cagatgtaca agcaggcaaa acattcttaa aaaacaaaaa 2400
caaaacccca aaacagattt ctttctatat tatttatatg gggttctgcc tgcgtgtaca 2460
cctgaagggc agaggagagc accaggtctc ataggtggtt ggttgtaagc taccatatgg 2520
ttgctaggaa ttaaactcag gactttggta agagcagcca gtgctcttaa cctctgagcc 2580
atccctccag tcccagacaa aacactctta cacataaaat aaatcaatct ttaaaagtgt 2640
agaggaaaat ggctgaacag aaactattga cagacatgct gagagtggtc accgtttctc 2700
cagcttggca ctggtggtgc tgtaggccat ctcagtgtct gtcaccttgg ggcggggcgg 2760
ggggcgggag ggggggaagg cggcagggca gggtcttggc agcatctcgg gcctctattt 2820
catcacatca gtaacagctc tccttcgtcc caagcaagct ttggcaacca aataaatatg 2880
ttagccaata ttaccaaacg gtccctagag gagaatgtgt gtaatgtgcc tacattcgtt 2940
tttataacaa caccaacaat atccaaccat aaaaataaac tgaaatggtc actctacctc 3000
agtgacaacg tctgagctcc gtttcagggt ccctcacaag aaattcgata ggtgtaatga 3060
tttatttttc ctaataagta aaatgattat tcactcattt gaaaattgga cacacacaca 3120
aaaaaatgaa gaaaaaattt gctatttcat ctctcctttt ctatctcttt ccctttaaaa 3180
aaagtataaa tgtgggctaa agagatggct cagcagttaa gagcactggc tgctcttccg 3240
gaggtactga gttcaaactg caccaaccac atggtggctc acaaccatct gtaatgggat 3300
ctgatgccct cttctggtgt gtctagagac agtgacagtg tactcacata cataaaataa 3360
gtaaaaaaaa aaaaaacaaa actttttttt aagtataaat gtgtaagaac ttacctgtct 3420
tccctataaa agccacaatt acctttgcag ataatcactg tcaggcagtg tcttcaagag 3480
aagtggggtt gttctacgac acaagaatat ttttgtttat atactaccct ctaagctcct 3540
tcctacttct ctgcaaactc atcccctgtg catatactga aatctatccg tcacggtatt 3600
ttgagtatac acagttatcc gtcacggtat tttgagtata cacagtttta ttagctaata 3660
catggggtat ttcaagtctg ggactgttgt tggctgtgtc atctctgtta catatgactt 3720
atattttcta agatgaattt ccaagacagg aatagagaga tcatacgaca tgcacagtta 3780
aagttttaag agattacaaa attgaccttt aagaagctat acaatttcac atcctttcca 3840
ggaaaggtca agaaatccct gtatcttcac taatgctacc tatgaggagt ctgtaatttt 3900
ggtagagcag aaggtccaaa tggtatcaca gatcctccat ggactttctt atcgagagcg 3960
ttctttccgc ttttattgat tatccttcga gctatgtgag actctcatcc gtcttcctct 4020
tgaccgatgt atagttttct cactgagttc agaggctagg atgcagaagc cgtcctcgtc 4080
 
agctttatac acctttcaga ttgactgtga atgagcacac aggtcagaga gacggtaagg 4140

gaagacctac tctcccatga acgttgtaaa catgcgaata acacagaagc agaaaatgta 4200

aactattaga ctggctggaa tgtagcgctc acctttgagc ccactccttt agacaccggt 4260

ttcatggaag aatggctcct gtttcccacc tgctgcttca gcagtgcccc aaatagtgcc 4320

agattcagag gagatactca gtgtttgccg agtgaattag gaaagtgttc agcggagggg 4380

acactagcat tcagacatcc agaccttaga gtaaccgggc cagatggaag tctccttcct 4440

tcccatagct gttaccactg tactgtcttt actggggaaa agatttatca tgtggaatag 4500

gtgcgaggca gaggtgagat tttaatgggg aggaagcatt gaaaagtgaa agtgaaaatt 4560

ggatgctctt tgctttgaag ctttgcctaa agcaggtttt agctttcaaa tacgtttcaa 4620

tgttgaaaga acgcatgata catatggagg ggggcctggg gggggtcctt ggctgagttt 4680

gaatgtacat taacaatctg gggggctaat aactcaatgt aaagctgctg atcccatcat 4740

actgacttct ttccacttgg ttctacatgg ctttgagtta caaaatgaaa gcattgaatt 4800

ttgaactgtt cagctgtgtt tccacacttg caaatcggtt gttggccagc cctcagaatt 4860

gcttcagtta cagctggctc gtctgctctt tccagactgg cttttagggc ttatgtatat 4920

atgagaagga cacatttact agtgtctcct tgctctgcta ttgaaattaa gcagacctct 4980

ctgtgtttcc cgttactaga tagttcccaa aacatgagga tatttgctgg cattatattc 5040

acagcctgct gtcacttgct acggggtaag tcaccaaatc ttttcagtgg gttctatatt 5100

ttcaatattt tagctatgaa ttaaaaatgg aagtaatttg tggggtgtgt atgtgtgtgt 5160

atatgtgtgt gtagaggggg gtctgtgtgt atgtgcagtt gctaggcaca cataaagcgt 5220

tcataggaca acctagagct tagtcctcac cttctacctt gtttgagaca aggtctctta 5280

tttgttgtac attgctgagt cctgtagttc ggctagctca gaacctcctg ggggctctcc 5340

tgtctccacc tcccagtcca ctgagattgt aggcacatgc tactgcacct ggcttctacc 5400

tggtctctgg ggatttgaac ttgggtccat gggctacaca gcaagtcgtt tacttactgg 5460

gcaatcactc catcccctaa gataattata aggaatatac cttgcttatc caaacacatt 5520

ctcattctcc tttgccataa ataagttact tggcaaatat attgtatgta tttttaataa 5580

ataaataaaa tcttaaaaat aaataaaatt atttgtgaag acaaaaaaaa taagttactt 5640

ggaaaggatg aaggaaaata ctggagcttt gggtgtggtt tagtagtaga acacttggct 5700

gatgtaaaaa aaaagcccta ggtgcaatcc caacaccaga aacaaatgaa ggaatgaaca 5760

acaaccgccc ccacccccca ggggatgaat ataaaaatat caggtaatac agaactaaca 5820

ggtgatccgt ttcctatgaa taactactga acattcccag ggaggtggcc cactgataat 5880

atatttttat ttattggttc cttttaaaca agactgggaa tatattatct agcttgcatc 5940

accaccacca ccccccaccc ccgccccatg aagttatttc aaagaagaat tttagtgttc 6000
 
atgtgattcc ctaaataaaa tgatagtaac cttttaccca ggttttcaga tgtgtttgga 6060

ggagttttct gtcttctgag ggctggtcct ctttcctttt cagcgtttac tatcacggct 6120

ccaaaggact tgtacgtggt ggagtatggc agcaacgtca cgatggagtg cagattccct 6180

gtagaacggg agctggacct gcttgcgtta gtggtgtact gggaaaagga agatgagcaa 6240

gtgattcagt ttgtggcagg agaggaggac cttaagcctc agcacagcaa cttcaggggg 6300

agagcctcgc tgccaaagga ccagcttttg aagggaaatg ctgcccttca gatcacagac 6360

gtcaagctgc aggacgcagg cgtttactgc tgcataatca gctacggtgg tgcggactac 6420

aagcgaatca cgctgaaagt caatggtaag aattaccctg gatggggaag gcttcatccg 6480

tatttaaaac agctccctaa tgttgagagc tcttcattct tgagagttcg cacgcacttc 6540

tcacagaaca acagcagcct gttcttctcg ctcgtttgtt cattcgttcg ttcacacact 6600

tcaccagtga aaaagcctag cactgtgtgt ttgatagtaa cttgagattc agtaccagat 6660

aatactcagc catgctttgc agtcagtacc atgatcttgc aaaggtgaaa tgccaggtgt 6720

ttgtttctta tcataaatgc aatatataat atattacata gatgtataga tataactgtg 6780

taacatgcaa taagatataa tatgcatata tttcatataa cataatgtat aatatataat 6840

gtataataat atatactaca atatatagtt atatgcatag ttatatattg catttatgat 6900

aaaaagcaaa cacctggcat ttcacttttg caagcttttt gaattacttg taaatatata 6960

tacatgcaaa catacataca cacacatgtt tttttacaag taatttgaat gtcatggaaa 7020

gaaatagaat cataaaaatg tccctcctcc ctaactacca tcttctaagc ataaatatac 7080

agtaactact atttgtacat ccctccatga ctttttgatt ggattactgt ttatatttaa 7140

tctatcaggc ttagcacatt ttctttcctt tgaatacctc catacaaaat tcaatgtgtg 7200

tttatatata tatgtatata tatagttata tcatatcata tatcatacaa agttttatat 7260

atgtatacat atataaacac acatatctac acatacatac acatttttta tatatataca 7320

caatatataa tgtatatgtg tgtgtgtgtg catatacctc tatatctatc tatctatcta 7380

tctatctatc tatctatcta tctatctatc tatagcttct actgtaaggg tcacttttta 7440

aaaaattaag gttaatctat gaaggatgag aagtgaagat cttaagtgta gaagaagccg 7500

ttcttccaca gagatggtac aggctacact cagcaggcat gcattcattt tcagggcctg 7560

catctctggg agtgctgagg aggaactatg agtgtaagtt cctgggtaac gagccacaga 7620

aatgtcatca ggtcccttga gttcacggtt ctgggttgga actaactaag ggaaggaaaa 7680

caccttgcta accacccctg ctttcactgt tgggttgcat agacctgaga gcttcactca 7740

tcgcaggcgt gcacacagcc agcccagttg cccaccagga ctggaaatcc ccaaggctct 7800

cctcttcaca tacctcccac agtggtgtcc ctcgaatcag gtccctttcg tggtctgcgg 7860
 
aaacttcacc ttgatggaca ctgagccgaa ccccgtcttc tccaggcaga cgttgcagat 7920

ggtctgcctg ctccttttcg gttttcataa taactttaaa actatttggc tagaatcaga 7980

tagtctcctt cgatcttttt actcctaaac tagtgtgaag ctcaatatta tacataggaa 8040

aaattcacat tcctcataag ggatcacggc agttattaac gcaatcttat gtagagatta 8100

ggaaaaatgt gtccattcga cagtttatta atgggtctgt gatggtgggt cgcttagagc 8160

ttatgaagat gccacttgcc ttatttcggt gcttggctct aacttccatt accttttact 8220

tacaattctt catttgtgac actcatgaaa ggagactcgg gatatggttg ttaatatctt 8280

gagcagctgt tagtacatca gcaaatatga ggctcctgtt tggttccaag atttttaaag 8340

acattgtgaa gctggaaggc catgagcagt caaagttgct ggacctgggg cactggaagg 8400

agctcaaggc gggcagtcag gaagaaggga gcagggtgta agcctctgtg acaagaggtc 8460

tctcatggct tgttaggagt aggtgactct ggtaaggagg aatttactct agattatgtg 8520

ctcagtgata tctctgatac caaagacaat cttttggagc gcacggcttc tccacggagg 8580

gacctgtgat gcggcaaagc tggtatccaa gcagtcttga aggcctgctt gactattttg 8640

ttttcttaca gaaatcacta cgtcgatatt ttactcccag ttcaccttgc catttgcatg 8700

cacgttagct ggtctaggcc aagagtctga accttggttg tggctcaaat ctgtaatctc 8760

agctatgtgg gaagctgagg caggagaatc gcgcaattag gacttgtctg agctacgaac 8820

atatagcagg ttcaagagca gcctgggcaa tttggcaaga tccgttctca aagacataca 8880

tacatacata tatttggcaa gatccgttct caaagacata catacataca tacatttggc 8940

aagatccgtt ctcaaagaca tacatacatg catgcatgca taaatacatg tataaatgaa 9000

tataaaatta aaataggaac aggactgagg acatgcccag tgatgtacag gaccctggtt 9060

tcagtttcta gcaccatggt cgctggcatg caaaagcttt ccatgtcaag gaagtagaac 9120

tcctgaattc tgtgctatgc aaatgacttc acagcaaggt tccttcccat cactgcaagt 9180

gtgtggtggt gaagcagccc tacagggagt tcactcgcca tggggtttta aaggtgaatt 9240

atttcaaaac tgaaatctga aagagtgtag ttttggatta actcagtttt tattatctct 9300

ttggataatg ttctaccttc aaaggtttac agaagaccct caggcatcag gcttttaatt 9360

gaaaaatatt cagctttaat cactgacctc aatatcagaa aagaatgaag tgtccattta 9420

gtagtcctcg gaccgtagat ggcattaagc agctgtaaga aacacgctca tcacatggtt 9480

caacaattaa ggattgccat ggtgcacccg atcccccagc tctgtttaaa ccttttctct 9540

gtcatctatg cctatctaag atgtctcagc atatcatgct tattgtctct ttccttctct 9600

tgaacacaag ctctaggggt aggggctttg gtcagtttta ttgttggacc tgttggcctt 9660

gacgtattat gaaggggcag tgtaataaga aaagaaaacc gtgtttgttg atggaatggt 9720

ttaaaaaaaa attagcttaa aatgtatgct atacgaacgg gcatgaatct gggagatttt 9780
 
agactgttcc ttgtgagccg taggaacttt cttgtttctg tgttcacagt cctccgcagc 9840

tgtgctcatg gtcctccaca tctgcctaac aacagcaggg gtagatgtta taattaacac 9900

tcacagaaca cctgatatgt gagaggttgt gccctgggct tcttgctcat atgaattcat 9960

ttcatcttta acataactct tcgggggagg cacttgccca tgaccatggc ttgttttctt 10020

tcctatcgac tatctttcaa atcggaatgt gtctttccct aggtgacatc tcagattcaa 10080

tgttacacag cactgctgtg cccattttca ggcattaaaa atgcgatccg cctaaattcc 10140

accccgataa aattatgcag ccaggttcgg aacccaacta gcccatctct aaaaatctat 10200

gcccttccta gcctcgcttt gttgcctgtc tttgtacctc tggagtgggt atcaaggaac 10260

agaatggctt taaacatgat ttgaactttt cctggttttt gatcacagca gtaacagagt 10320

gttctagaga taagccagag gtttgaaaac aaacagaact tgtctttgag gtgttataaa 10380

catgtgtgtg attataaagg atatatctgg tgccaatagg tatagcttat attataagaa 10440

atggccattg tgaagatcag aaggagataa ctcactgatt tcaggtggtt gattcaatct 10500

aacacatgat atattctgaa actgtacaaa aaagatattc aaaagaagaa acacatggaa 10560

caaagagtat atggaaattc tgacaactca gaatcagtgt atgttaagat gaagttcatg 10620

attaacaaat ggagatggtc gcagtcatcc tgaagcttat tttagccaat gctttatcac 10680

atccaactca atcttcgttt tttttttttc agatggatct tcccattttt aattaacctt 10740

atcatgatag cccctagtaa ggcatgtcct gacatttctt ttcttttttt ttatagctta 10800

tgtttctttt tttaaaaatt tttttactac atattttcct caattacatt tccaatgcta 10860

tcccaaaagt cccccatacc ctcctccccc actcccctac ccacccactc ccactttttg 10920

gccctggggt tcccctgtac tgggggatat aaagtctgcg tgtccaatgg gcctctcttt 10980

ccagtgatgg cctactaggc catcttttga tacatatgca gcttgagtca agagctccgg 11040

ggtactggtt agttcatagt gttgttccac ctatagggtt gcagatccct ttagttcctt 11100

gggtgctttc tctagcttct ccattgggag ccctgtgatc catccaatag ctgactgtga 11160

gcatccactt ctgtgtttgc taggtcccgg ccaagtctca caagagacag ctatttcagg 11220

gtcctttcag catatgcttg ctagtgtatg caatggtgtc atcgtttgga ggctaattat 11280

gggatggctc cctggatatg gcagtctcta aatggtccat ccttttgtct cagctccaaa 11340

ctttgtctct gtaactcctt tcaatcttcg tttttaatgt ccgaaaatga ctggtaacgt 11400

cactgcttac atttcccttt gctttgtgtt gaattgtgag ctttatcttt gatctggagt 11460

ttctttaaga agactttgta atgtgcttat ttttaaagat ggtgaattca attaaagata 11520

gacaagaggg acgggtctgt atttcatctg gtagagtgct tacctgccat tcatgaagtt 11580

ctggttcgat ccccggcaca gcatagaacc acatgcggtg gcaactcagt tgcctgtgac 11640
 
cccagcactc aggagctaga ggcaggttac ctttgactac acagcaagtt caaggctagc 11700
ctggtctaca tgcaactctt cctcaaaggg aaaacaaaac aaaaatagac aggagaattg 11760
gaaacatcca tagaaaccca aatatgaatc aatagactcc cctcttcctg tgtcagacat 11820
tcagactcag tgacagccgt caccgtttat gaggcatgac tgattttaca gatcctagag 11880
caagcgtgca caattctggt gtttcctgcc tgcgtcctca cacacacgct caccttaggg 11940
acggaggcag cggaggtcta gtcagtttca gacgaatcca ctcatcagct gctcaccatg 12000
gtcttctagg tgaagcagaa ctctccttcc atcagcaccc gtgaaaaaca aaagtggaaa 12060
gcagatattt cactccgaat gctttatcat tggcagtgat ttcaaattta aaccactaat 12120
ttcccagaag aattaggtca cgggccatgt ggcattagac cctattgtgg tgacattatt 12180
tcctgatgtg cctatttaat aatgtatagt ggtctcatct ttatttagtt ggtcttgaat 12240
gtgataaata caagactggg caagtgttca tcaaagctgt tacttgaaat tgtaattata 12300
cctattttgt tggtggtgtt ttttcttggt tcatggacaa ccaaacacat tacggctttg 12360
ctttgtctta gctactctgt gtcaagttcc tgtgaaaata ccgtattaca gtgctcttat 12420
tacatctttg gtaatgaagt agtcacctaa gcccctatcc tgggctctct atggctcact 12480
aaagaatcgt aagttggata atgattgctg aagaaataga tatgttttat ttacattgag 12540
gtagaaagac ctgtggtttc taggtaacag tttacttaac ttagttgcta catctagttt 12600
gcactcagta aacaccctgg tgtggttttt acaacaacaa ggttgttcct gaagcccaaa 12660
catggcccag atttgaatag aagacctgag gcagactaac actcactccc tgctgccaaa 12720
gtagcagagc cggggacata tttctccaat agccagcccg agttgatgct ctttgtaagc 12780
agacaccaca aagccacacg gctagcccta agatgggaga gccctgacct ctctttgctt 12840
ctgacctagc cccataccgc aaaatcaacc agagaatttc cgtggatcca gccacttctg 12900
agcatgaact aatatgtcag gccgagggtt atccagaagc tgaggtaatc tggacaaaca 12960
gtgaccacca acccgtgagt gggaagagaa gtgtcaccac ttcccggaca gaggggatgc 13020
ttctcaatgt gaccagcagt ctgagggtca acgccacagc gaatgatgtt ttctactgta 13080
cgttttggag atcacagcca gggcaaaacc acacagcgga gctgatcatc ccaggtgagt 13140
tgcctaactc gtccccggat tcctagcacg atggccatcc gccatagtca tttagcagta 13200
gttggccgag tgtatgaata catgaaactt acattggcta gacgtattct agttcctctt 13260
tctcttgcgt gcaagccata atgctacttg gtcaacactc agcaagaatt aagtgtttgc 13320
tcctgacaga cagtatgtta gtcattgagt attcgtggct ggataggaca taagttactc 13380
tgctccagga aacaggtgac agtcgcatgt aggagatgct cttgtaactt gggcaccacg 13440
ggcaacgtga gagcaagctt atgcttcagg aatgtaaacc tttgtcacat caaagctgcc 13500
ctcttccctc ccgggctgct gtagtagaaa gcaaatgcat tgcctttttt tgtatcttat 13560
 
attcaacagg aagaatcctt tcttcccata ctcctcttca gggcattccc tgatacttca 13620

actccaggaa gtctgcctgt gcatagaaca cgaggctgat gcctatgggc aaccgactag 13680

ctgggttgta actcaccagg ccagggcatt gtagggatag aatgagctgc acgaaaatga 13740

ttgtaggaaa atcgtttgag aattcagctg tctttaaaat tcatttacct tacagaaata 13800

gggccacagg agacagtttg gtgagaggct cagagcatta tatgaatatg aacccagagg 13860

tactcactct gaggagggtt gcttctgcct ccatcgacac ccctttccca atgtatgtca 13920

acagtacatt gatgtctaga gatagctctg tgaagtttag agaagatata taaaactaca 13980

ttaaaaagtc aaccaatatg aaaaatgaag cccaggatgg gcgtggagcc aaaaggagca 14040

cacaagtttg gaaaaatgta aaataaaaag gaagacactc taactagaat tggacttttc 14100

cataaaagct atattgttgg tttcctacca ttaaatgttt tatggggctg gagacgttgt 14160

agctcagttg ggagagtact tgcctagcat ccacaatgct ctgaccccag cgccatataa 14220

acgtatatag ggctcaccta tagtctaaac actttggtgg tggaagccgg agggtcagtt 14280

caaggtcatc ctcagcttca tagcaagttc aaggccagta tggactgact acaagagacc 14340

ctgcctcaaa gacaaaacaa gcaaacatca tcataataaa acagcaaaaa gctgttctct 14400

ggccatttct ttgtaggaca tcctggagta acttaaccac tctgagtgtc tcttctcatc 14460

tgtaacttgg gttcatagtg ggaccaaata ggagctgagt tacaacagga gatggaagtt 14520

agcaatctca gagcatccca gagccctggg acccattttt ctctttctca aaactccagg 14580

cagagaaaca gggagatgtt aatgactgga gtgcccaaga gtctcagaca tggaagaaac 14640

acaacccgca ctggtccaca tttgtgggag acagaagcgg cttagcagtg ccccgctttc 14700

ccatgttcgg gagttggcct tccacaccaa gtcagccatt actgtctata ttaatgagtg 14760

cacaaggaag ttactgcact aaggttacca tttaatattg cagtcacttt ctgtggcaac 14820

atgtcacagg cctggagttc ttctctgtgc tgctcccagt ttcttaggag atctgtcttc 14880

ttcttccaac ccttacctgg acattaactt tcagtgatta agattaccct aagaccaaac 14940

tatatttcag gagtcatcat tcccctgact atggcgcagc aaagccttac cttccatcag 15000

cttctcttta tccttttcca gaactgcctg caacacatcc tccacagaac aggactcact 15060

gggtgcttct gggatccatc ctgttgttcc tcattgtagt gtccacggtc ctcctcttct 15120

tgagaaaaca aggtatttcc tccattgctg tactgcctgc agggaagtgc atgagggtcc 15180

cttcaccatt gccttgagca ggcagtgcca gagatggtac caccctgaca agaacagacc 15240

cacatcacag ggcgtcctac actgcacgct agctgaacct caccaggaaa cactgacttg 15300

aaaggtgctg ccctccattc cctgggcttg ctttcagtta cacagagtaa gttttaacag 15360

tgactctggt gtactgagtc ttccttaagt cctgttcaca ttgttgctct gcgacatctc 15420
 
ctaccttgga gctcggtgtt ggaccctgtt gtaatatgaa gaggtatcag ttggacaagg 15480

gcgtagggaa gctaaaagac ttagctttca aggcgtgggg gagggggagg gcattgtgag 15540

gttccctgtt cagcggttca gagtaaagct gggcagcaca caagtttaga caacaaggaa 15600

gatgctctaa ctggggttgg aggtcaatgg tgcagggata ggtagcaatg gaggctaaag 15660

aagaaagcca taaggtttct ttctacagac tctgaagaca gagcactgtg gtgcctattg 15720

gtctcaggtg aactcaagtc ttgtgttcct tacaccacct attgagctgg gttctggata 15780

ccctaagtgg aaaaacactt ctgtctaaaa agagctgtgg catctttacc acaaagcact 15840

tttttttttt gcaagatttc tgaagacgaa atgacacagt ttttaaaata atttccatat 15900

atttatttca gtacttaccc ataagaatga ctgaaatcta ggaaaatgaa tatattcaaa 15960

taatgccttc ctattgtaaa gtgacttaga gtaaaccttt tcaccaaata ctttgaaatg 16020

cataccaagt gagccaatga tgcatggact tgtgtgaagc caaatgggga aatagaatat 16080

gggagggaac atcaatcatc agagaggaat tccccatcac ccttttggag ggcgcgtgta 16140

tgggaaaagt gttgtaactg gggcggtagc gaccagctgc ttccaaagaa taagccctga 16200

gcagaaccaa gagggttgcc agggtgtgaa gattaagctt cagagacaca tctggcttag 16260

ggctagccta ggacttgcac aggaccgcag ttgttgccta atggcatctg ggatgattgg 16320

ctgaggatga ggaaagcaga gagtcagtta ggtggtaagc tagctcagga tgtgggacgt 16380

ttctgaggga agggtggaag acaaagtaga cagtcctaaa aaccatctga gaatgaaagt 16440

atgtacccta aattgttgaa taattaactt tgcggcgtgg gatctggaat aaggaagttc 16500

acgggctaaa agcctggtga ttaggggatt gatcggtgtc tcctaaaaac cttaagttga 16560

aaatcgagtc cctgtgggtt gtagtagcat cttgctataa ggaattaaga ttgatttctt 16620

cttctttagt gagaatgcta gatgtggaga aatgtggcgt tgaagataca agctcaaaaa 16680

accgaaatgg taagtgtgag taacgaggga ggggcaagcc gagggaatga gtgggacaga 16740

gcagccaagc agggtcctgc aagggctgca gttccagcgc tctgtaggct gaggcagggg 16800

gatagggagt tccaggccag caaccacaca gccaaaacaa aacaaacaaa acaaaaaaca 16860

aagcaaaagg aagagcagaa aaagctactg ttaccaacaa gagacatact ttagccgtga 16920

tctcctctcc ttccaaagga agctgtttgt gggggctgct ggttcctctg cctaaggatc 16980

gccacatctg agaaaactct gggtttgctg ctgcggtgtc tgtgtggtag ccctgcaacc 17040

agggacgctg ttgacttatt tgaatgggca tttgattaag caaatgattc tgctgtgtgg 17100

ttagctgtta actgtttcat gtgtatgata tggcatttca tttcatcatc ctgcccccaa 17160

tcagataaat atgagaatca tctccattgt atagtaggga aaacagggct gagaagataa 17220

tgcgaggtgc ctaagaacac acagacttgc taccacagac gtccgttctt tgctaggtgc 17280

ttcaatctat gctgctacta atgtctgtat ttataagagt agaaaactct gcccgggcgt 17340
 
tggtggccca cgcctttaat cccagcactc gggaggcaga ggtagtcgga tttctgagtt 17400

tgaggccagc ctggtctaca gagtgagttc caggacagcc aggctacaca gagaaaccct 17460

gtctcgaaaa aaatcaaaaa agaaaaaaga acaagaaaac tctaactgaa ctatcttgta 17520

gtaaattaca gtgtgttcta attcatttta gtgttgttag aaaccaaaca gtaaggccag 17580

gcagaactaa agtggtttcg ctaacttggg cagacccaag caggttccct cctgtacatt 17640

accatttatt ctgctgagtc accctgagga accggcctgg cttgctcaag actgaggatt 17700

tctcagccat gaacactttc cattttaaaa ctagggcagt cccggacaag caaggatggt 17760

tagtcatcct tcatcatgta agggtagtca gacataggta gtatctgtct ctgtcttacg 17820

gcaacgttac tatctgctct agcaaacatc ccatttgttt atcttacata tcatacgttt 17880

cccccccaca tattccctgc ccctgtgaaa ttattatact atgatcactc tccagataca 17940

caattcgagg agacgtaagc agtgttgaac cctctgatcg tcgattggca gcttgtggtc 18000

tgtgaaagaa agggcccatg ggacatgagt ccaaagactc aagatggaac ctgagggaga 18060

gaaccaagaa agtgttggga gaggagcctg gaacaacgga cattttttcc agggagacac 18120

tgctaagcaa gttgcccatc agtcgtcttg ggaaatggat tgagggttcc tggcttagca 18180

gctggtcctt gcacagtgac cttttcctct gctcagtgcc gggatgagag atggagtcat 18240

gagtgttgaa gaataagtgc cttctattta ttttgagtct gtgtgttctc actttgggca 18300

tgtaattatg actggtgaat tctgacgaca tgatagatct taagatgtag tcaccaaact 18360

caactgctgc ttagcatcct ccgtaactac tgatacaagc agggaacaca gaggtcacct 18420

gcttggtttg acaggctctt gctgtctgac tcaaataatc tttatttttc agtcctcaag 18480

gctcttcgat agcagttgtt ctgtatcagc cttataggtg tcaggtatag cactcaacat 18540

ctcatctcat tacaatagca accctcatca ccatagcaac agctaacctc tgttatcctc 18600

acttcatagc caggaagctg agcgactaag tcacttgccc acagagtatc agctctcaga 18660

tttctgttct tcagccactg tcctttcagg atagaatttg tcgttaagaa attaatttaa 18720

aaactgatta ttgagtagca ttgtatatca atcacaacat gccttgtgca ctgtgctggc 18780

ctctgagcat aaagatgtac gccggagtac cggtcggaca tgtttatgtg tgttaaatac 18840

tcagagaaat gttcattaac aaggagcttg cattttagag acactggaaa gtaactccag 18900

ttcattgtct agcattacat ttacctcatt tgctatcctt gccatacagt ctcttgttct 18960

ccatgaagtg tcatgaatct tgttgaatag ttcttttatt ttttaaatgt ttctatttaa 19020

atgatattga catctgaggc gatagctcag ttggtaaaac cctttcctca caagtgtgaa 19080

accctgagtc ttatccctag aacccacata aaaaacagtt gcgtatgttt gtgcatgctt 19140

ttgatcccag cactagggag gcagaggcag gcagatcctg agctctcatt gaccacccag 19200
 
cctagcctac atggttagct ccaggcctac aggagctggc agagcctgaa aaacgatgcc 19260

tagacacaca cacacacaca cacacacaca cacacacaca cacacaccat gtactcatag 19320

acctaagtgc accctcctac acatgcacac acatacaatt caaacacaaa tcaacaggga 19380

attgtctcag aatggtcccc aagacaaaga agaagaaaaa caccaaacca gctctattcc 19440

ctcagcctat cctctctact ccttcctaga agcaactact attgtttttg tatataaatt 19500

tacccaacga cagttaatat gtagaatata tattaaagtg tctgtcaata tatattatct 19560

ctttctttct ttcttccttt ctttctttct ttctttcttt ctttctttct ttctttcttt 19620

ctttcttcct tccttccttc cttccttcct tccttccttc ctttctttct ttctttcttt 19680

ttttctgtct atctgtacct aaatggttgc tcactatgca ttttctgtgc tcttcgccct 19740

ttttatttaa tgtatggata tttatgctgc ttccagaatg gatctaaagc tctttgtttc 19800

taggttttct cccccatcct tctaggcatc tctcacactg tctaggccag acaccatgtc 19860

tgctgcctga atctgtagac accatttata aagcacgtac tcaccgagtt tgtatttggc 19920

ttgttctgtg tctgattaaa gggagaccat gagtccccag ggtacactga gttaccccag 19980

taccaagggg gagccttgtt tgtgtctcca tggcagaagc aggcctggag ccattttggt 20040

ttcttccttg acttctctca aacacagacg cctcacttgc tcattacagg ttctcctttg 20100

ggaatgtcag cattgctcct tgactgctgg ctgccctgga aggagcccat tagctctgtg 20160

tgagcccttg acagctactg cctctcctta ccacaggggc ctctaagata ctgttaccta 20220

gaggtcttga ggatctgtgt tctctggggg gaggaaagga ggaggaaccc agaactttct 20280

tacagttttc cttgttctgt cacatgtcaa gactgaagga acaggctggg ctacgtagtg 20340

agatcctgtc tcaaaggaaa gacgagcata gccgaacccc cggtggaacc ccctctgtta 20400

cctgttcaca caagcttatt gatgagtctc atgttaatgt cttgtttgta tgaagtttaa 20460

gaaaatatcg ggttgggcaa cacattctat ttattcattt tatttgaaat cttaatgcca 20520

tctcatggtg ttggattggt gtggcacttt attcttttgt gttgtgtata accataaatt 20580

ttattttgca tcagattgtc aatgtattgc attaatttaa taaatatttt tatttattaa 20640

a 20641
<210> 5
   <211> 16
   <212> DNA
   <213> Artificial Sequence
<220>
   <223> Oligonucleotide motif
<400> 5
   taattggctc tactgc 16
   <210> 6
   <211> 16
   <212> DNA
   <213> Artificial Sequence
<220>
   <223> Oligonucleotide motif
<400> 6
   tcgcataaga atgact 16
<210> 7
   <211> 16
   <212> DNA
   <213> Artificial Sequence
<220>
   <223> Oligonucleotide motif
<400> 7
   tgaacacaca gtcgca 16
<210> 8
   <211> 16
   <212> DNA
   <213> Artificial Sequence
<220>
   <223> Oligonucleotide motif
<400> 8
   ctgaacacac agtcgc 16
<210> 9
   <211> 16
   <212> DNA
   <213> Artificial Sequence
<220>
   <223> Oligonucleotide motif
<400> 9
   tctgaacaca cagtcg 16
<210> 10
   <211> 16
   <212> DNA
   <213> Artificial Sequence
<220>
   <223> Oligonucleotide motif
<400> 10
   ttctgaacac acagtc 16
<210> 11
   <211> 16
   <212> DNA
   <213> Artificial Sequence
<220>
   <223> Oligonucleotide motif
<400> 11
   acaagtcatg ttacta 16
<210> 12
   <211> 16
   <212> DNA
   <213> Artificial Sequence
<220>
   <223> Oligonucleotide motif
<400> 12
   acacaagtca tgttac 16
<210> 13
   <211> 16
   <212> DNA
   <213> Artificial Sequence
<220>
   <223> Oligonucleotide motif
<400> 13
   cttacttaga tgctgc 16
<210> 14
   <211> 16
   <212> DNA
   <213> Artificial Sequence
<220>
   <223> Oligonucleotide motif
<400> 14
   acttacttag atgctg 16
<210> 15
   <211> 16
   <212> DNA
   <213> Artificial Sequence
<220>
   <223> Oligonucleotide motif
<400> 15
   gacttactta gatgct 16
<210> 16
   <211> 16
   <212> DNA
   <213> Artificial Sequence
<220>
   <223> Oligonucleotide motif
<400> 16
   agacttactt agatgc 16
<210> 17
   <211> 16
   <212> DNA
   <213> Artificial Sequence
<220>
   <223> Oligonucleotide motif
<400> 17
   gcaggaagag acttac 16
<210> 18
   <211> 17
   <212> DNA
   <213> Artificial Sequence
<220>
   <223> Oligonucleotide motif
<400> 18
   aataaattcc gttcagg 17
<210> 19
   <211> 16
   <212> DNA
   <213> Artificial Sequence
<220>
   <223> Oligonucleotide motif
<400> 19
   gcaaataaat tccgtt 16
<210> 20
   <211> 16
   <212> DNA
   <213> Artificial Sequence
<220>
   <223> Oligonucleotide motif
<400> 20
   agcaaataaa ttccgt 16
<210> 21
   <211> 17
   <212> DNA
   <213> Artificial Sequence
<220>
   <223> Oligonucleotide motif
<400> 21
   cagagcaaat aaattcc 17
<210> 22
   <211> 18
   <212> DNA
   <213> Artificial Sequence
<220>
   <223> Oligonucleotide motif
<400> 22
   tggacagagc aaataaat 18
<210> 23
   <211> 16
   <212> DNA
   <213> Artificial Sequence
<220>
   <223> Oligonucleotide motif
<400> 23
   atggacagag caaata 16
<210> 24
   <211> 16
   <212> DNA
   <213> Artificial Sequence
<220>
   <223> Oligonucleotide motif
<400> 24
   cagaatggac agagca 16
<210> 25
   <211> 16
   <212> DNA
   <213> Artificial Sequence
<220>
   <223> Oligonucleotide motif
<400> 25
   ttctcagaat ggacag 16
<210> 26
   <211> 16
   <212> DNA
   <213> Artificial Sequence
<220>
   <223> Oligonucleotide motif
<400> 26
   ctgaactttg acatag 16
<210> 27
   <211> 18
   <212> DNA
   <213> Artificial Sequence
<220>
   <223> Oligonucleotide motif
<400> 27
   aagacaaacc cagactga 18
<210> 28
   <211> 18
   <212> DNA
   <213> Artificial Sequence
<220>
   <223> Oligonucleotide motif
<400> 28
   tataagacaa acccagac 18
<210> 29
   <211> 18
   <212> DNA
   <213> Artificial Sequence
<220>
   <223> Oligonucleotide motif
<400> 29
   ttataagaca aacccaga 18
<210> 30
   <211> 17
   <212> DNA
   <213> Artificial Sequence
<220>
   <223> Oligonucleotide motif
<400> 30
   tgttataaga caaaccc 17
<210> 31
   <211> 17
   <212> DNA
   <213> Artificial Sequence
<220>
   <223> Oligonucleotide motif
<400> 31
   tagaacaatg gtacttt 17
<210> 32
   <211> 16
   <212> DNA
   <213> Artificial Sequence
<220>
   <223> Oligonucleotide motif
<400> 32
   gtagaacaat ggtact 16
<210> 33
   <211> 16
   <212> DNA
   <213> Artificial Sequence
<220>
   <223> Oligonucleotide motif
<400> 33
   aggtagaaca atggta 16
<210> 34
   <211> 17
   <212> DNA
   <213> Artificial Sequence
<220>
   <223> Oligonucleotide motif
<400> 34
   aagaggtaga acaatgg 17
<210> 35
   <211> 16
   <212> DNA
   <213> Artificial Sequence
<220>
   <223> Oligonucleotide motif
<400> 35
   gcatccacag taaatt 16
<210> 36
   <211> 16
   <212> DNA
   <213> Artificial Sequence
<220>
   <223> Oligonucleotide motif
<400> 36
   gaaggttatt taattc 16
<210> 37
   <211> 16
   <212> DNA
   <213> Artificial Sequence
<220>
   <223> Oligonucleotide motif
<400> 37
   ctaatcgaat gcagca 16
<210> 38
   <211> 16
   <212> DNA
   <213> Artificial Sequence
<220>
   <223> Oligonucleotide motif
<400> 38
   tacccaatct aatcga 16
<210> 39
   <211> 16
   <212> DNA
   <213> Artificial Sequence
<220>
   <223> Oligonucleotide motif
<400> 39
   tagttaccca atctaa 16
<210> 40
   <211> 16
   <212> DNA
   <213> Artificial Sequence
<220>
   <223> Oligonucleotide motif
<400> 40
   catttagtta cccaat 16
<210> 41
   <211> 16
   <212> DNA
   <213> Artificial Sequence
<220>
   <223> Oligonucleotide motif
<400> 41
   tcatttagtt acccaa 16
<210> 42
   <211> 16
   <212> DNA
   <213> Artificial Sequence
<220>
   <223> Oligonucleotide motif
<400> 42
   ttcatttagt taccca 16
<210> 43
   <211> 18
   <212> DNA
   <213> Artificial Sequence
<220>
   <223> Oligonucleotide motif
<400> 43
   gaattaattt catttagt 18
<210> 44
   <211> 17
   <212> DNA
   <213> Artificial Sequence
<220>
   <223> Oligonucleotide motif
<400> 44
   cagtgaggaa ttaattt 17
<210> 45
   <211> 16
   <212> DNA
   <213> Artificial Sequence
<220>
   <223> Oligonucleotide motif
<400> 45
   ccaacagtga ggaatt 16
<210> 46
   <211> 16
   <212> DNA
   <213> Artificial Sequence
<220>
   <223> Oligonucleotide motif
<400> 46
   cccaacagtg aggaat 16
<210> 47
   <211> 17
   <212> DNA
   <213> Artificial Sequence
<220>
   <223> Oligonucleotide motif
<400> 47
   tatacccaac agtgagg 17
<210> 48
   <211> 17
   <212> DNA
   <213> Artificial Sequence
<220>
   <223> Oligonucleotide motif
<400> 48
   ttatacccaa cagtgag 17
<210> 49
   <211> 17
   <212> DNA
   <213> Artificial Sequence
<220>
   <223> Oligonucleotide motif
<400> 49
   tttataccca acagtga 17
<210> 50
   <211> 16
   <212> DNA
   <213> Artificial Sequence
<220>
   <223> Oligonucleotide motif
<400> 50
   cctttatacc caacag 16
<210> 51
   <211> 16
   <212> DNA
   <213> Artificial Sequence
<220>
   <223> Oligonucleotide motif
<400> 51
   taacctttat acccaa 16
<210> 52
   <211> 17
   <212> DNA
   <213> Artificial Sequence
<220>
   <223> Oligonucleotide motif
<400> 52
   aataaccttt ataccca 17
<210> 53
   <211> 16
   <212> DNA
   <213> Artificial Sequence
<220>
   <223> Oligonucleotide motif
<400> 53
   gtaaataacc tttata 16
<210> 54
   <211> 18
   <212> DNA
   <213> Artificial Sequence
<220>
   <223> Oligonucleotide motif
<400> 54
   actgtaaata acctttat 18
<210> 55
   <211> 16
   <212> DNA
   <213> Artificial Sequence
<220>
   <223> Oligonucleotide motif
<400> 55
   atatatatgc aatgag 16
<210> 56
   <211> 16
   <212> DNA
   <213> Artificial Sequence
<220>
   <223> Oligonucleotide motif
<400> 56
   agatatatat gcaatg 16
<210> 57
   <211> 16
   <212> DNA
   <213> Artificial Sequence
<220>
   <223> Oligonucleotide motif
<400> 57
   gagatatata tgcaat 16
<210> 58
   <211> 16
   <212> DNA
   <213> Artificial Sequence
<220>
   <223> Oligonucleotide motif
<400> 58
   ccagagatat atatgc 16
<210> 59
   <211> 17
   <212> DNA
   <213> Artificial Sequence
<220>
   <223> Oligonucleotide motif
<400> 59
   caatattcca gagatat 17
<210> 60
   <211> 17
   <212> DNA
   <213> Artificial Sequence
<220>
   <223> Oligonucleotide motif
<400> 60
   gcaatattcc agagata 17
<210> 61
   <211> 17
   <212> DNA
   <213> Artificial Sequence
<220>
   <223> Oligonucleotide motif
<400> 61
   agcaatattc cagagat 17
<210> 62
   <211> 16
   <212> DNA
   <213> Artificial Sequence
<220>
   <223> Oligonucleotide motif
<400> 62
   cagcaatatt ccagag 16
<210> 63
   <211> 17
   <212> DNA
   <213> Artificial Sequence
<220>
   <223> Oligonucleotide motif
<400> 63
   aatcagcaat attccag 17
<210> 64
   <211> 17
   <212> DNA
   <213> Artificial Sequence
<220>
   <223> Oligonucleotide motif
<400> 64
   acaatcagca atattcc 17
<210> 65
   <211> 19
   <212> DNA
   <213> Artificial Sequence
<220>
   <223> Oligonucleotide motif
<400> 65
   actaagtagt tacacttct 19
<210> 66
   <211> 17
   <212> DNA
   <213> Artificial Sequence
<220>
   <223> Oligonucleotide motif
<400> 66
   ctaagtagtt acacttc 17
<210> 67
   <211> 18
   <212> DNA
   <213> Artificial Sequence
<220>
   <223> Oligonucleotide motif
<400> 67
   gactaagtag ttacactt 18
<210> 68
   <211> 16
   <212> DNA
   <213> Artificial Sequence
<220>
   <223> Oligonucleotide motif
<400> 68
   tgactaagta gttaca 16
<210> 69
   <211> 17
   <212> DNA
   <213> Artificial Sequence
<220>
   <223> Oligonucleotide motif
<400> 69
   ctttgactaa gtagtta 17
<210> 70
   <211> 16
   <212> DNA
   <213> Artificial Sequence
<220>
   <223> Oligonucleotide motif
<400> 70
   ctctttgact aagtag 16
<210> 71
   <211> 16
   <212> DNA
   <213> Artificial Sequence
<220>
   <223> Oligonucleotide motif
<400> 71
   gctctttgac taagta 16
<210> 72
   <211> 17
   <212> DNA
   <213> Artificial Sequence
<220>
   <223> Oligonucleotide motif
<400> 72
   ccttaaatac tgttgac 17
<210> 73
   <211> 16
   <212> DNA
   <213> Artificial Sequence
<220>
   <223> Oligonucleotide motif
<400> 73
   cttaaatact gttgac 16
<210> 74
   <211> 16
   <212> DNA
   <213> Artificial Sequence
<220>
   <223> Oligonucleotide motif
<400> 74
   tccttaaata ctgttg 16
<210> 75
   <211> 17
   <212> DNA
   <213> Artificial Sequence
<220>
   <223> Oligonucleotide motif
<400> 75
   tctccttaaa tactgtt 17
<210> 76
   <211> 16
   <212> DNA
   <213> Artificial Sequence
<220>
   <223> Oligonucleotide motif
<400> 76
   tatcatagtt ctcctt 16
<210> 77
   <211> 16
   <212> DNA
   <213> Artificial Sequence
<220>
   <223> Oligonucleotide motif
<400> 77
   agtatcatag ttctcc 16
<210> 78
   <211> 16
   <212> DNA
   <213> Artificial Sequence
<220>
   <223> Oligonucleotide motif
<400> 78
   gagtatcata gttctc 16
<210> 79
   <211> 16
   <212> DNA
   <213> Artificial Sequence
<220>
   <223> Oligonucleotide motif
<400> 79
   agagtatcat agttct 16
<210> 80
   <211> 16
   <212> DNA
   <213> Artificial Sequence
<220>
   <223> Oligonucleotide motif
<400> 80
   cagagtatca tagttc 16
<210> 81
   <211> 16
   <212> DNA
   <213> Artificial Sequence
<220>
   <223> Oligonucleotide motif
<400> 81
   ttcagagtat catagt 16
<210> 82
   <211> 16
   <212> DNA
   <213> Artificial Sequence
<220>
   <223> Oligonucleotide motif
<400> 82
   cttcagagta tcatag 16
<210> 83
   <211> 17
   <212> DNA
   <213> Artificial Sequence
<220>
   <223> Oligonucleotide motif
<400> 83
   ttcttcagag tatcata 17
<210> 84
   <211> 17
   <212> DNA
   <213> Artificial Sequence
<220>
   <223> Oligonucleotide motif
<400> 84
   tttcttcaga gtatcat 17
<210> 85
   <211> 16
   <212> DNA
   <213> Artificial Sequence
<220>
   <223> Oligonucleotide motif
<400> 85
   gagaaaggct aagttt 16
<210> 86
   <211> 16
   <212> DNA
   <213> Artificial Sequence
<220>
   <223> Oligonucleotide motif
<400> 86
   gacactcttg tacatt 16
<210> 87
   <211> 17
   <212> DNA
   <213> Artificial Sequence
<220>
   <223> Oligonucleotide motif
<400> 87
   tgagacactc ttgtaca 17
<210> 88
   <211> 16
   <212> DNA
   <213> Artificial Sequence
<220>
   <223> Oligonucleotide motif
<400> 88
   tgagacactc ttgtac 16
<210> 89
   <211> 16
   <212> DNA
   <213> Artificial Sequence
<220>
   <223> Oligonucleotide motif
<400> 89
   ctttattaaa ctccat 16
<210> 90
   <211> 16
   <212> DNA
   <213> Artificial Sequence
<220>
   <223> Oligonucleotide motif
<400> 90
   accaaacttt attaaa 16
<210> 91
   <211> 16
   <212> DNA
   <213> Artificial Sequence
<220>
   <223> Oligonucleotide motif
<400> 91
   aaacctctac taagtg 16
<210> 92
   <211> 16
   <212> DNA
   <213> Artificial Sequence
<220>
   <223> Oligonucleotide motif
<400> 92
   agattaagac agttga 16
<210> 93
   <211> 17
   <212> DNA
   <213> Artificial Sequence
<220>
   <223> Oligonucleotide motif
<400> 93
   aagtaggagc aagaggc 17
<210> 94
   <211> 17
   <212> DNA
   <213> Artificial Sequence
<220>
   <223> Oligonucleotide motif
<400> 94
   aaagtaggag caagagg 17
<210> 95
   <211> 16
   <212> DNA
   <213> Artificial Sequence
<220>
   <223> Oligonucleotide motif
<400> 95
   gttaagcagc caggag 16
<210> 96
   <211> 16
   <212> DNA
   <213> Artificial Sequence
<220>
   <223> Oligonucleotide motif
<400> 96
   agggtaggat gggtag 16
<210> 97
   <211> 16
   <212> DNA
   <213> Artificial Sequence
<220>
   <223> Oligonucleotide motif
<400> 97
   aagggtagga tgggta 16
<210> 98
   <211> 16
   <212> DNA
   <213> Artificial Sequence
<220>
   <223> Oligonucleotide motif
<400> 98
   caagggtagg atgggt 16
<210> 99
   <211> 16
   <212> DNA
   <213> Artificial Sequence
<220>
   <223> Oligonucleotide motif
<400> 99
   ccaagggtag gatggg 16
<210> 100
   <211> 16
   <212> DNA
   <213> Artificial Sequence
<220>
   <223> Oligonucleotide motif
<400> 100
   tccaagggta ggatgg 16
<210> 101
   <211> 16
   <212> DNA
   <213> Artificial Sequence
<220>
   <223> Oligonucleotide motif
<400> 101
   cttccaaggg taggat 16
<210> 102
   <211> 17
   <212> DNA
   <213> Artificial Sequence
<220>
   <223> Oligonucleotide motif
<400> 102
   atcttccaag ggtagga 17
<210> 103
   <211> 17
   <212> DNA
   <213> Artificial Sequence
<220>
   <223> Oligonucleotide motif
<400> 103
   agaagtgatg gctcatt 17
<210> 104
   <211> 17
   <212> DNA
   <213> Artificial Sequence
<220>
   <223> Oligonucleotide motif
<400> 104
   aagaagtgat ggctcat 17
<210> 105
   <211> 17
   <212> DNA
   <213> Artificial Sequence
<220>
   <223> Oligonucleotide motif
<400> 105
   gaagaagtga tggctca 17
<210> 106
   <211> 17
   <212> DNA
   <213> Artificial Sequence
<220>
   <223> Oligonucleotide motif
<400> 106
   atgaaatgta aactggg 17
<210> 107
   <211> 18
   <212> DNA
   <213> Artificial Sequence
<220>
   <223> Oligonucleotide motif
<400> 107
   caatgaaatg taaactgg 18
<210> 108
   <211> 18
   <212> DNA
   <213> Artificial Sequence
<220>
   <223> Oligonucleotide motif
<400> 108
   gcaatgaaat gtaaactg 18
<210> 109
   <211> 18
   <212> DNA
   <213> Artificial Sequence
<220>
   <223> Oligonucleotide motif
<400> 109
   agcaatgaaa tgtaaact 18
<210> 110
   <211> 18
   <212> DNA
   <213> Artificial Sequence
<220>
   <223> Oligonucleotide motif
<400> 110
   gagcaatgaa atgtaaac 18
<210> 111
   <211> 17
   <212> DNA
   <213> Artificial Sequence
<220>
   <223> Oligonucleotide motif
<400> 111
   tgaattccca tatccga 17
<210> 112
   <211> 16
   <212> DNA
   <213> Artificial Sequence
<220>
   <223> Oligonucleotide motif
<400> 112
   agaattatga ccatat 16
<210> 113
   <211> 17
   <212> DNA
   <213> Artificial Sequence
<220>
   <223> Oligonucleotide motif
<400> 113
   aggtaagaat tatgacc 17
<210> 114
   <211> 18
   <212> DNA
   <213> Artificial Sequence
<220>
   <223> Oligonucleotide motif
<400> 114
   tcaggtaaga attatgac 18
<210> 115
   <211> 18
   <212> DNA
   <213> Artificial Sequence
<220>
   <223> Oligonucleotide motif
<400> 115
   cttcaggtaa gaattatg 18
<210> 116
   <211> 17
   <212> DNA
   <213> Artificial Sequence
<220>
   <223> Oligonucleotide motif
<400> 116
   tcttcaggta agaatta 17
<210> 117
   <211> 17
   <212> DNA
   <213> Artificial Sequence
<220>
   <223> Oligonucleotide motif
<400> 117
   cttcttcagg taagaat 17
<210> 118
   <211> 17
   <212> DNA
   <213> Artificial Sequence
<220>
   <223> Oligonucleotide motif
<400> 118
   tcttcttcag gtaagaa 17
<210> 119
   <211> 16
   <212> DNA
   <213> Artificial Sequence
<220>
   <223> Oligonucleotide motif
<400> 119
   tcttcttcag gtaaga 16
<210> 120
   <211> 17
   <212> DNA
   <213> Artificial Sequence
<220>
   <223> Oligonucleotide motif
<400> 120
   tggtctaaga gaagaag 17
<210> 121
   <211> 16
   <212> DNA
   <213> Artificial Sequence
<220>
   <223> Oligonucleotide motif
<400> 121
   gttggtctaa gagaag 16
<210> 122
   <211> 16
   <212> DNA
   <213> Artificial Sequence
<220>
   <223> Oligonucleotide motif
<400> 122
   agttggtcta agagaa 16
<210> 123
   <211> 17
   <212> DNA
   <213> Artificial Sequence
<220>
   <223> Oligonucleotide motif
<400> 123
   cagttggtct aagagaa 17
<210> 124
   <211> 18
   <212> DNA
   <213> Artificial Sequence
<220>
   <223> Oligonucleotide motif
<400> 124
   gcagttggtc taagagaa 18
<210> 125
   <211> 16
   <212> DNA
   <213> Artificial Sequence
<220>
   <223> Oligonucleotide motif
<400> 125
   cagttggtct aagaga 16
<210> 126
   <211> 17
   <212> DNA
   <213> Artificial Sequence
<220>
   <223> Oligonucleotide motif
<400> 126
   gcagttggtc taagaga 17
<210> 127
   <211> 16
   <212> DNA
   <213> Artificial Sequence
<220>
   <223> Oligonucleotide motif
<400> 127
   gcagttggtc taagag 16
<210> 128
   <211> 16
   <212> DNA
   <213> Artificial Sequence
<220>
   <223> Oligonucleotide motif
<400> 128
   ctcatatcag ggcagt 16
<210> 129
   <211> 17
   <212> DNA
   <213> Artificial Sequence
<220>
   <223> Oligonucleotide motif
<400> 129
   cacacatgtt ctttaac 17
<210> 130
   <211> 18
   <212> DNA
   <213> Artificial Sequence
<220>
   <223> Oligonucleotide motif
<400> 130
   taaatacaca catgttct 18
<210> 131
   <211> 18
   <212> DNA
   <213> Artificial Sequence
<220>
   <223> Oligonucleotide motif
<400> 131
   gtaaatacac acatgttc 18
<210> 132
   <211> 18
   <212> DNA
   <213> Artificial Sequence
<220>
   <223> Oligonucleotide motif
<400> 132
   tgtaaataca cacatgtt 18
<210> 133
   <211> 18
   <212> DNA
   <213> Artificial Sequence
<220>
   <223> Oligonucleotide motif
<400> 133
   gatcatgtaa atacacac 18
<210> 134
   <211> 18
   <212> DNA
   <213> Artificial Sequence
<220>
   <223> Oligonucleotide motif
<400> 134
   agatcatgta aatacaca 18
<210> 135
   <211> 20
   <212> DNA
   <213> Artificial Sequence
<220>
   <223> Oligonucleotide motif
<400> 135
   caaagatcat gtaaatacac 20
<210> 136
   <211> 20
   <212> DNA
   <213> Artificial Sequence
<220>
   <223> Oligonucleotide motif
<400> 136
   acaaagatca tgtaaataca 20
<210> 137
   <211> 18
   <212> DNA
   <213> Artificial Sequence
<220>
   <223> Oligonucleotide motif
<400> 137
   gaatacaaag atcatgta 18
<210> 138
   <211> 18
   <212> DNA
   <213> Artificial Sequence
<220>
   <223> Oligonucleotide motif
<400> 138
   agaatacaaa gatcatgt 18
<210> 139
   <211> 18
   <212> DNA
   <213> Artificial Sequence
<220>
   <223> Oligonucleotide motif
<400> 139
   cagaatacaa agatcatg 18
<210> 140
   <211> 17
   <212> DNA
   <213> Artificial Sequence
<220>
   <223> Oligonucleotide motif
<400> 140
   gcagaataca aagatca 17
<210> 141
   <211> 17
   <212> DNA
   <213> Artificial Sequence
<220>
   <223> Oligonucleotide motif
<400> 141
   aggcagaata caaagat 17
<210> 142
   <211> 17
   <212> DNA
   <213> Artificial Sequence
<220>
   <223> Oligonucleotide motif
<400> 142
   aaggcagaat acaaaga 17
<210> 143
   <211> 16
   <212> DNA
   <213> Artificial Sequence
<220>
   <223> Oligonucleotide motif
<400> 143
   attagtgagg gacgaa 16
<210> 144
   <211> 16
   <212> DNA
   <213> Artificial Sequence
<220>
   <223> Oligonucleotide motif
<400> 144
   cattagtgag ggacga 16
<210> 145
   <211> 16
   <212> DNA
   <213> Artificial Sequence
<220>
   <223> Oligonucleotide motif
<400> 145
   gagggtgatg gattag 16
<210> 146
   <211> 16
   <212> DNA
   <213> Artificial Sequence
<220>
   <223> Oligonucleotide motif
<400> 146
   ttaggagtaa taaagg 16
<210> 147
   <211> 16
   <212> DNA
   <213> Artificial Sequence
<220>
   <223> Oligonucleotide motif
<400> 147
   ttaatgaatt tggttg 16
<210> 148
   <211> 16
   <212> DNA
   <213> Artificial Sequence
<220>
   <223> Oligonucleotide motif
<400> 148
   ctttaatgaa tttggt 16
<210> 149
   <211> 16
   <212> DNA
   <213> Artificial Sequence
<220>
   <223> Oligonucleotide motif
<400> 149
   catggattac aactaa 16
<210> 150
   <211> 16
   <212> DNA
   <213> Artificial Sequence
<220>
   <223> Oligonucleotide motif
<400> 150
   tcatggatta caacta 16
<210> 151
   <211> 16
   <212> DNA
   <213> Artificial Sequence
<220>
   <223> Oligonucleotide motif
<400> 151
   gtcatggatt acaact 16
<210> 152
   <211> 16
   <212> DNA
   <213> Artificial Sequence
<220>
   <223> Oligonucleotide motif
<400> 152
   cattaaatct agtcat 16
<210> 153
   <211> 17
   <212> DNA
   <213> Artificial Sequence
<220>
   <223> Oligonucleotide motif
<400> 153
   gacattaaat ctagtca 17
<210> 154
   <211> 16
   <212> DNA
   <213> Artificial Sequence
<220>
   <223> Oligonucleotide motif
<400> 154
   agggacatta aatcta 16
<210> 155
   <211> 16
   <212> DNA
   <213> Artificial Sequence
<220>
   <223> Oligonucleotide motif
<400> 155
   caaagcatta taacca 16
<210> 156
   <211> 16
   <212> DNA
   <213> Artificial Sequence
<220>
   <223> Oligonucleotide motif
<400> 156
   acttactagg cagaag 16
<210> 157
   <211> 16
   <212> DNA
   <213> Artificial Sequence
<220>
   <223> Oligonucleotide motif
<400> 157
   cagagttaac tgtaca 16
<210> 158
   <211> 16
   <212> DNA
   <213> Artificial Sequence
<220>
   <223> Oligonucleotide motif
<400> 158
   ccagagttaa ctgtac 16
<210> 159
   <211> 16
   <212> DNA
   <213> Artificial Sequence
<220>
   <223> Oligonucleotide motif
<400> 159
   gccagagtta actgta 16
<210> 160
   <211> 16
   <212> DNA
   <213> Artificial Sequence
<220>
   <223> Oligonucleotide motif
<400> 160
   tgggccagag ttaact 16
<210> 161
   <211> 16
   <212> DNA
   <213> Artificial Sequence
<220>
   <223> Oligonucleotide motif
<400> 161
   cagcatctat cagact 16
<210> 162
   <211> 18
   <212> DNA
   <213> Artificial Sequence
<220>
   <223> Oligonucleotide motif
<400> 162
   tgaaataaca tgagtcat 18
<210> 163
   <211> 17
   <212> DNA
   <213> Artificial Sequence
<220>
   <223> Oligonucleotide motif
<400> 163
   gtgaaataac atgagtc 17
<210> 164
   <211> 16
   <212> DNA
   <213> Artificial Sequence
<220>
   <223> Oligonucleotide motif
<400> 164
   tctgtttatg tcactg 16
<210> 165
   <211> 16
   <212> DNA
   <213> Artificial Sequence
<220>
   <223> Oligonucleotide motif
<400> 165
   gtctgtttat gtcact 16
<210> 166
   <211> 16
   <212> DNA
   <213> Artificial Sequence
<220>
   <223> Oligonucleotide motif
<400> 166
   tggtctgttt atgtca 16
<210> 167
   <211> 16
   <212> DNA
   <213> Artificial Sequence
<220>
   <223> Oligonucleotide motif
<400> 167
   ttggtctgtt tatgtc 16
<210> 168
   <211> 16
   <212> DNA
   <213> Artificial Sequence
<220>
   <223> Oligonucleotide motif
<400> 168
   tcacccattg tttaaa 16
<210> 169
   <211> 16
   <212> DNA
   <213> Artificial Sequence
<220>
   <223> Oligonucleotide motif
<400> 169
   ttcagcaaat attcgt 16
<210> 170
   <211> 17
   <212> DNA
   <213> Artificial Sequence
<220>
   <223> Oligonucleotide motif
<400> 170
   gtgtgttcag caaatat 17
<210> 171
   <211> 16
   <212> DNA
   <213> Artificial Sequence
<220>
   <223> Oligonucleotide motif
<400> 171
   tctattgtta ggtatc 16
<210> 172
   <211> 16
   <212> DNA
   <213> Artificial Sequence
<220>
   <223> Oligonucleotide motif
<400> 172
   attgcccatc ttactg 16
<210> 173
   <211> 16
   <212> DNA
   <213> Artificial Sequence
<220>
   <223> Oligonucleotide motif
<400> 173
   tattgcccat cttact 16
<210> 174
   <211> 16
   <212> DNA
   <213> Artificial Sequence
<220>
   <223> Oligonucleotide motif
<400> 174
   aaatattgcc catctt 16
<210> 175
   <211> 16
   <212> DNA
   <213> Artificial Sequence
<220>
   <223> Oligonucleotide motif
<400> 175
   ataaccttat cataca 16
<210> 176
   <211> 16
   <212> DNA
   <213> Artificial Sequence
<220>
   <223> Oligonucleotide motif
<400> 176
   tataacctta tcatac 16
<210> 177
   <211> 16
   <212> DNA
   <213> Artificial Sequence
<220>
   <223> Oligonucleotide motif
<400> 177
   ttataacctt atcata 16
<210> 178
   <211> 16
   <212> DNA
   <213> Artificial Sequence
<220>
   <223> Oligonucleotide motif
<400> 178
   tttataacct tatcat 16
<210> 179
   <211> 16
   <212> DNA
   <213> Artificial Sequence
<220>
   <223> Oligonucleotide motif
<400> 179
   actgctattg ctatct 16
<210> 180
   <211> 16
   <212> DNA
   <213> Artificial Sequence
<220>
   <223> Oligonucleotide motif
<400> 180
   aggactgcta ttgcta 16
<210> 181
   <211> 16
   <212> DNA
   <213> Artificial Sequence
<220>
   <223> Oligonucleotide motif
<400> 181
   gaggactgct attgct 16
<210> 182
   <211> 16
   <212> DNA
   <213> Artificial Sequence
<220>
   <223> Oligonucleotide motif
<400> 182
   acgtagaata ataaca 16
<210> 183
   <211> 16
   <212> DNA
   <213> Artificial Sequence
<220>
   <223> Oligonucleotide motif
<400> 183
   ccaagtgata taatgg 16
<210> 184
   <211> 16
   <212> DNA
   <213> Artificial Sequence
<220>
   <223> Oligonucleotide motif
<400> 184
   ttagcagacc aagtga 16
<210> 185
   <211> 16
   <212> DNA
   <213> Artificial Sequence
<220>
   <223> Oligonucleotide motif
<400> 185
   gtttagcaga ccaagt 16
<210> 186
   <211> 16
   <212> DNA
   <213> Artificial Sequence
<220>
   <223> Oligonucleotide motif
<400> 186
   tgacagtgat tatatt 16
<210> 187
   <211> 16
   <212> DNA
   <213> Artificial Sequence
<220>
   <223> Oligonucleotide motif
<400> 187
   tgtccaagat attgac 16
<210> 188
   <211> 16
   <212> DNA
   <213> Artificial Sequence
<220>
   <223> Oligonucleotide motif
<400> 188
   gaatatccta gattgt 16
<210> 189
   <211> 16
   <212> DNA
   <213> Artificial Sequence
<220>
   <223> Oligonucleotide motif
<400> 189
   caaactgaga atatcc 16
<210> 190
   <211> 16
   <212> DNA
   <213> Artificial Sequence
<220>
   <223> Oligonucleotide motif
<400> 190
   gcaaactgag aatatc 16
<210> 191
   <211> 16
   <212> DNA
   <213> Artificial Sequence
<220>
   <223> Oligonucleotide motif
<400> 191
   tcctattaca atcgta 16
<210> 192
   <211> 16
   <212> DNA
   <213> Artificial Sequence
<220>
   <223> Oligonucleotide motif
<400> 192
   ttcctattac aatcgt 16
<210> 193
   <211> 16
   <212> DNA
   <213> Artificial Sequence
<220>
   <223> Oligonucleotide motif
<400> 193
   actaatggga ggattt 16
<210> 194
   <211> 16
   <212> DNA
   <213> Artificial Sequence
<220>
   <223> Oligonucleotide motif
<400> 194
   tagttcagag aataag 16
<210> 195
   <211> 16
   <212> DNA
   <213> Artificial Sequence
<220>
   <223> Oligonucleotide motif
<400> 195
   taacatatag ttcaga 16
<210> 196
   <211> 16
   <212> DNA
   <213> Artificial Sequence
<220>
   <223> Oligonucleotide motif
<400> 196
   ataacatata gttcag 16
<210> 197
   <211> 16
   <212> DNA
   <213> Artificial Sequence
<220>
   <223> Oligonucleotide motif
<400> 197
   cataacatat agttca 16
<210> 198
   <211> 16
   <212> DNA
   <213> Artificial Sequence
<220>
   <223> Oligonucleotide motif
<400> 198
   tcataacata tagttc 16
<210> 199
   <211> 16
   <212> DNA
   <213> Artificial Sequence
<220>
   <223> Oligonucleotide motif
<400> 199
   tagctcctaa caatca 16
<210> 200
   <211> 16
   <212> DNA
   <213> Artificial Sequence
<220>
   <223> Oligonucleotide motif
<400> 200
   ctccaatctt tgtata 16
<210> 201
   <211> 16
   <212> DNA
   <213> Artificial Sequence
<220>
   <223> Oligonucleotide motif
<400> 201
   tctccaatct ttgtat 16
<210> 202
   <211> 16
   <212> DNA
   <213> Artificial Sequence
<220>
   <223> Oligonucleotide motif
<400> 202
   tctatttcag ccaatc 16
<210> 203
   <211> 16
   <212> DNA
   <213> Artificial Sequence
<220>
   <223> Oligonucleotide motif
<400> 203
   cggaagtcag agtgaa 16
<210> 204
   <211> 16
   <212> DNA
   <213> Artificial Sequence
<220>
   <223> Oligonucleotide motif
<400> 204
   ttaagcatga ggaata 16
<210> 205
   <211> 16
   <212> DNA
   <213> Artificial Sequence
<220>
   <223> Oligonucleotide motif
<400> 205
   tgattgagca cctctt 16
<210> 206
   <211> 16
   <212> DNA
   <213> Artificial Sequence
<220>
   <223> Oligonucleotide motif
<400> 206
   gactaattat ttcgtt 16
<210> 207
   <211> 16
   <212> DNA
   <213> Artificial Sequence
<220>
   <223> Oligonucleotide motif
<400> 207
   tgactaatta tttcgt 16
<210> 208
   <211> 16
   <212> DNA
   <213> Artificial Sequence
<220>
   <223> Oligonucleotide motif
<400> 208
   gtgactaatt atttcg 16
<210> 209
   <211> 16
   <212> DNA
   <213> Artificial Sequence
<220>
   <223> Oligonucleotide motif
<400> 209
   ctgcttgaaa tgtgac 16
<210> 210
   <211> 16
   <212> DNA
   <213> Artificial Sequence
<220>
   <223> Oligonucleotide motif
<400> 210
   cctgcttgaa atgtga 16
<210> 211
   <211> 16
   <212> DNA
   <213> Artificial Sequence
<220>
   <223> Oligonucleotide motif
<400> 211
   atcctgcttg aaatgt 16
<210> 212
   <211> 16
   <212> DNA
   <213> Artificial Sequence
<220>
   <223> Oligonucleotide motif
<400> 212
   attataaatc tattct 16
<210> 213
   <211> 16
   <212> DNA
   <213> Artificial Sequence
<220>
   <223> Oligonucleotide motif
<400> 213
   gctaaatact ttcatc 16
<210> 214
   <211> 16
   <212> DNA
   <213> Artificial Sequence
<220>
   <223> Oligonucleotide motif
<400> 214
   cattgtaaca taccta 16
<210> 215
   <211> 16
   <212> DNA
   <213> Artificial Sequence
<220>
   <223> Oligonucleotide motif
<400> 215
   gcattgtaac atacct 16
<210> 216
   <211> 16
   <212> DNA
   <213> Artificial Sequence
<220>
   <223> Oligonucleotide motif
<400> 216
   taatattgca ccaaat 16
<210> 217
   <211> 16
   <212> DNA
   <213> Artificial Sequence
<220>
   <223> Oligonucleotide motif
<400> 217
   gataatattg caccaa 16
<210> 218
   <211> 16
   <212> DNA
   <213> Artificial Sequence
<220>
   <223> Oligonucleotide motif
<400> 218
   agataatatt gcacca 16
<210> 219
   <211> 16
   <212> DNA
   <213> Artificial Sequence
<220>
   <223> Oligonucleotide motif
<400> 219
   gccaagaaga taatat 16
<210> 220
   <211> 16
   <212> DNA
   <213> Artificial Sequence
<220>
   <223> Oligonucleotide motif
<400> 220
   cacagccaca taaact 16
<210> 221
   <211> 16
   <212> DNA
   <213> Artificial Sequence
<220>
   <223> Oligonucleotide motif
<400> 221
   ttgtaattgt ggaaac 16
<210> 222
   <211> 16
   <212> DNA
   <213> Artificial Sequence
<220>
   <223> Oligonucleotide motif
<400> 222
   tgacttgtaa ttgtgg 16
<210> 223
   <211> 16
   <212> DNA
   <213> Artificial Sequence
<220>
   <223> Oligonucleotide motif
<400> 223
   tctaactgaa atagtc 16
<210> 224
   <211> 16
   <212> DNA
   <213> Artificial Sequence
<220>
   <223> Oligonucleotide motif
<400> 224
   gtggttctaa ctgaaa 16
<210> 225
   <211> 16
   <212> DNA
   <213> Artificial Sequence
<220>
   <223> Oligonucleotide motif
<400> 225
   caatatggga cttggt 16
<210> 226
   <211> 16
   <212> DNA
   <213> Artificial Sequence
<220>
   <223> Oligonucleotide motif
<400> 226
   atgacaatat gggact 16
<210> 227
   <211> 16
   <212> DNA
   <213> Artificial Sequence
<220>
   <223> Oligonucleotide motif
<400> 227
   tatgacaata tgggac 16
<210> 228
   <211> 16
   <212> DNA
   <213> Artificial Sequence
<220>
   <223> Oligonucleotide motif
<400> 228
   atatgacaat atggga 16
<210> 229
   <211> 16
   <212> DNA
   <213> Artificial Sequence
<220>
   <223> Oligonucleotide motif
<400> 229
   cttcacttaa taatta 16
<210> 230
   <211> 16
   <212> DNA
   <213> Artificial Sequence
<220>
   <223> Oligonucleotide motif
<400> 230
   ctgcttcact taataa 16
<210> 231
   <211> 16
   <212> DNA
   <213> Artificial Sequence
<220>
   <223> Oligonucleotide motif
<400> 231
   aagactgctt cactta 16
<210> 232
   <211> 16
   <212> DNA
   <213> Artificial Sequence
<220>
   <223> Oligonucleotide motif
<400> 232
   gaatgcccta attatg 16
<210> 233
   <211> 16
   <212> DNA
   <213> Artificial Sequence
<220>
   <223> Oligonucleotide motif
<400> 233
   tggaatgccc taatta 16
<210> 234
   <211> 16
   <212> DNA
   <213> Artificial Sequence
<220>
   <223> Oligonucleotide motif
<400> 234
   gcaaatgcca gtaggt 16
<210> 235
   <211> 16
   <212> DNA
   <213> Artificial Sequence
<220>
   <223> Oligonucleotide motif
<400> 235
   ctaatggaag gatttg 16
<210> 236
   <211> 16
   <212> DNA
   <213> Artificial Sequence
<220>
   <223> Oligonucleotide motif
<400> 236
   aatatagaac ctaatg 16
<210> 237
   <211> 16
   <212> DNA
   <213> Artificial Sequence
<220>
   <223> Oligonucleotide motif
<400> 237
   gaaagaatag aatgtt 16
<210> 238
   <211> 16
   <212> DNA
   <213> Artificial Sequence
<220>
   <223> Oligonucleotide motif
<400> 238
   atgggtaata gattat 16
<210> 239
   <211> 16
   <212> DNA
   <213> Artificial Sequence
<220>
   <223> Oligonucleotide motif
<400> 239
   gaaagagcac agggtg 16
<210> 240
   <211> 16
   <212> DNA
   <213> Artificial Sequence
<220>
   <223> Oligonucleotide motif
<400> 240
   ctacatagag ggaatg 16
<210> 241
   <211> 16
   <212> DNA
   <213> Artificial Sequence
<220>
   <223> Oligonucleotide motif
<400> 241
   gcttcctaca tagagg 16
<210> 242
   <211> 16
   <212> DNA
   <213> Artificial Sequence
<220>
   <223> Oligonucleotide motif
<400> 242
   tgcttcctac atagag 16
<210> 243
   <211> 16
   <212> DNA
   <213> Artificial Sequence
<220>
   <223> Oligonucleotide motif
<400> 243
   tgggcttgaa atatgt 16
<210> 244
   <211> 16
   <212> DNA
   <213> Artificial Sequence
<220>
   <223> Oligonucleotide motif
<400> 244
   cattatattt aagaac 16
<210> 245
   <211> 16
   <212> DNA
   <213> Artificial Sequence
<220>
   <223> Oligonucleotide motif
<400> 245
   tcggttatgt tatcat 16
<210> 246
   <211> 16
   <212> DNA
   <213> Artificial Sequence
<220>
   <223> Oligonucleotide motif
<400> 246
   cactttatct ggtcgg 16
<210> 247
   <211> 16
   <212> DNA
   <213> Artificial Sequence
<220>
   <223> Oligonucleotide motif
<400> 247
   aaattggcac agcgtt 16
<210> 248
   <211> 16
   <212> DNA
   <213> Artificial Sequence
<220>
   <223> Oligonucleotide motif
<400> 248
   accgtgacag taaatg 16
<210> 249
   <211> 17
   <212> DNA
   <213> Artificial Sequence
<220>
   <223> Oligonucleotide motif
<400> 249
   tgggaaccgt gacagta 17
<210> 250
   <211> 16
   <212> DNA
   <213> Artificial Sequence
<220>
   <223> Oligonucleotide motif
<400> 250
   ccacatatag gtcctt 16
<210> 251
   <211> 16
   <212> DNA
   <213> Artificial Sequence
<220>
   <223> Oligonucleotide motif
<400> 251
   catattgcta ccatac 16
<210> 252
   <211> 16
   <212> DNA
   <213> Artificial Sequence
<220>
   <223> Oligonucleotide motif
<400> 252
   tcatattgct accata 16
<210> 253
   <211> 16
   <212> DNA
   <213> Artificial Sequence
<220>
   <223> Oligonucleotide motif
<400> 253
   caattgtcat attgct 16
<210> 254
   <211> 18
   <212> DNA
   <213> Artificial Sequence
<220>
   <223> Oligonucleotide motif
<400> 254
   cattcaattg tcatattg 18
<210> 255
   <211> 17
   <212> DNA
   <213> Artificial Sequence
<220>
   <223> Oligonucleotide motif
<400> 255
   tttctactgg gaatttg 17
<210> 256
   <211> 16
   <212> DNA
   <213> Artificial Sequence
<220>
   <223> Oligonucleotide motif
<400> 256
   caattagtgc agccag 16
<210> 257
   <211> 17
   <212> DNA
   <213> Artificial Sequence
<220>
   <223> Oligonucleotide motif
<400> 257
   gaataatgtt cttatcc 17
<210> 258
   <211> 20
   <212> DNA
   <213> Artificial Sequence
<220>
   <223> Oligonucleotide motif
<400> 258
   cacaaattga ataatgttct 20
<210> 259
   <211> 19
   <212> DNA
   <213> Artificial Sequence
<220>
   <223> Oligonucleotide motif
<400> 259
   catgcacaaa ttgaataat 19
<210> 260
   <211> 17
   <212> DNA
   <213> Artificial Sequence
<220>
   <223> Oligonucleotide motif
<400> 260
   atcctgcaat ttcacat 17
<210> 261
   <211> 16
   <212> DNA
   <213> Artificial Sequence
<220>
   <223> Oligonucleotide motif
<400> 261
   ccaccatagc tgatca 16
<210> 262
   <211> 17
   <212> DNA
   <213> Artificial Sequence
<220>
   <223> Oligonucleotide motif
<400> 262
   accaccatag ctgatca 17
<210> 263
   <211> 17
   <212> DNA
   <213> Artificial Sequence
<220>
   <223> Oligonucleotide motif
<400> 263
   caccaccata gctgatc 17
<210> 264
   <211> 16
   <212> DNA
   <213> Artificial Sequence
<220>
   <223> Oligonucleotide motif
<400> 264
   tagtcggcac caccat 16
<210> 265
   <211> 17
   <212> DNA
   <213> Artificial Sequence
<220>
   <223> Oligonucleotide motif
<400> 265
   cttgtagtcg gcaccac 17
<210> 266
   <211> 16
   <212> DNA
   <213> Artificial Sequence
<220>
   <223> Oligonucleotide motif
<400> 266
   cttgtagtcg gcacca 16
<210> 267
   <211> 16
   <212> DNA
   <213> Artificial Sequence
<220>
   <223> Oligonucleotide motif
<400> 267
   cgcttgtagt cggcac 16
<210> 268
   <211> 16
   <212> DNA
   <213> Artificial Sequence
<220>
   <223> Oligonucleotide motif
<400> 268
   tcaataaaga tcaggc 16
<210> 269
   <211> 16
   <212> DNA
   <213> Artificial Sequence
<220>
   <223> Oligonucleotide motif
<400> 269
   tggacttaca agaatg 16
<210> 270
   <211> 16
   <212> DNA
   <213> Artificial Sequence
<220>
   <223> Oligonucleotide motif
<400> 270
   atggacttac aagaat 16
<210> 271
   <211> 16
   <212> DNA
   <213> Artificial Sequence
<220>
   <223> Oligonucleotide motif
<400> 271
   gctcaagaaa ttggat 16
<210> 272
   <211> 16
   <212> DNA
   <213> Artificial Sequence
<220>
   <223> Oligonucleotide motif
<400> 272
   tactgtagaa catggc 16
<210> 273
   <211> 16
   <212> DNA
   <213> Artificial Sequence
<220>
   <223> Oligonucleotide motif
<400> 273
   gcaattcatt tgatct 16
<210> 274
   <211> 18
   <212> DNA
   <213> Artificial Sequence
<220>
   <223> Oligonucleotide motif
<400> 274
   tgaagggagg agggacac 18
<210> 275
   <211> 17
   <212> DNA
   <213> Artificial Sequence
<220>
   <223> Oligonucleotide motif
<400> 275
   agtggtgaag ggaggag 17
<210> 276
   <211> 18
   <212> DNA
   <213> Artificial Sequence
<220>
   <223> Oligonucleotide motif
<400> 276
   tagtggtgaa gggaggag 18
<210> 277
   <211> 19
   <212> DNA
   <213> Artificial Sequence
<220>
   <223> Oligonucleotide motif
<400> 277
   atagtggtga agggaggag 19
<210> 278
   <211> 17
   <212> DNA
   <213> Artificial Sequence
<220>
   <223> Oligonucleotide motif
<400> 278
   tagtggtgaa gggagga 17
<210> 279
   <211> 18
   <212> DNA
   <213> Artificial Sequence
<220>
   <223> Oligonucleotide motif
<400> 279
   atagtggtga agggagga 18
<210> 280
   <211> 16
   <212> DNA
   <213> Artificial Sequence
<220>
   <223> Oligonucleotide motif
<400> 280
   tagtggtgaa gggagg 16
<210> 281
   <211> 17
   <212> DNA
   <213> Artificial Sequence
<220>
   <223> Oligonucleotide motif
<400> 281
   atagtggtga agggagg 17
<210> 282
   <211> 18
   <212> DNA
   <213> Artificial Sequence
<220>
   <223> Oligonucleotide motif
<400> 282
   gatagtggtg aagggagg 18
<210> 283
   <211> 16
   <212> DNA
   <213> Artificial Sequence
<220>
   <223> Oligonucleotide motif
<400> 283
   atagtggtga agggag 16
<210> 284
   <211> 17
   <212> DNA
   <213> Artificial Sequence
<220>
   <223> Oligonucleotide motif
<400> 284
   gatagtggtg aagggag 17
<210> 285
   <211> 16
   <212> DNA
   <213> Artificial Sequence
<220>
   <223> Oligonucleotide motif
<400> 285
   gagatagtgg tgaagg 16
<210> 286
   <211> 16
   <212> DNA
   <213> Artificial Sequence
<220>
   <223> Oligonucleotide motif
<400> 286
   catgggagat agtggt 16
<210> 287
   <211> 18
   <212> DNA
   <213> Artificial Sequence
<220>
   <223> Oligonucleotide motif
<400> 287
   acaaataatg gttactct 18
<210> 288
   <211> 18
   <212> DNA
   <213> Artificial Sequence
<220>
   <223> Oligonucleotide motif
<400> 288
   acacacaaat aatggtta 18
<210> 289
   <211> 18
   <212> DNA
   <213> Artificial Sequence
<220>
   <223> Oligonucleotide motif
<400> 289
   gagggacaca caaataat 18
<210> 290
   <211> 16
   <212> DNA
   <213> Artificial Sequence
<220>
   <223> Oligonucleotide motif
<400> 290
   atatagagag gctcaa 16
<210> 291
   <211> 16
   <212> DNA
   <213> Artificial Sequence
<220>
   <223> Oligonucleotide motif
<400> 291
   ttgatataga gaggct 16
<210> 292
   <211> 16
   <212> DNA
   <213> Artificial Sequence
<220>
   <223> Oligonucleotide motif
<400> 292
   gcatttgata tagaga 16
<210> 293
   <211> 16
   <212> DNA
   <213> Artificial Sequence
<220>
   <223> Oligonucleotide motif
<400> 293
   tttgcatttg atatag 16
<210> 294
   <211> 16
   <212> DNA
   <213> Artificial Sequence
<220>
   <223> Oligonucleotide motif
<400> 294
   ctggaagaat aggttc 16
<210> 295
   <211> 16
   <212> DNA
   <213> Artificial Sequence
<220>
   <223> Oligonucleotide motif
<400> 295
   actggaagaa taggtt 16
<210> 296
   <211> 16
   <212> DNA
   <213> Artificial Sequence
<220>
   <223> Oligonucleotide motif
<400> 296
   tactggaaga ataggt 16
<210> 297
   <211> 16
   <212> DNA
   <213> Artificial Sequence
<220>
   <223> Oligonucleotide motif
<400> 297
   tggcttatcc tgtact 16
<210> 298
   <211> 16
   <212> DNA
   <213> Artificial Sequence
<220>
   <223> Oligonucleotide motif
<400> 298
   atggcttatc ctgtac 16
<210> 299
   <211> 16
   <212> DNA
   <213> Artificial Sequence
<220>
   <223> Oligonucleotide motif
<400> 299
   tatggcttat cctgta 16
<210> 300
   <211> 16
   <212> DNA
   <213> Artificial Sequence
<220>
   <223> Oligonucleotide motif
<400> 300
   gtatggctta tcctgt 16
<210> 301
   <211> 17
   <212> DNA
   <213> Artificial Sequence
<220>
   <223> Oligonucleotide motif
<400> 301
   atgaatatat gcccagt 17
<210> 302
   <211> 16
   <212> DNA
   <213> Artificial Sequence
<220>
   <223> Oligonucleotide motif
<400> 302
   gatgaatata tgccca 16
<210> 303
   <211> 17
   <212> DNA
   <213> Artificial Sequence
<220>
   <223> Oligonucleotide motif
<400> 303
   caagatgaat atatgcc 17
<210> 304
   <211> 17
   <212> DNA
   <213> Artificial Sequence
<220>
   <223> Oligonucleotide motif
<400> 304
   gacaacatca gtataga 17
<210> 305
   <211> 16
   <212> DNA
   <213> Artificial Sequence
<220>
   <223> Oligonucleotide motif
<400> 305
   caagacaaca tcagta 16
<210> 306
   <211> 16
   <212> DNA
   <213> Artificial Sequence
<220>
   <223> Oligonucleotide motif
<400> 306
   cactcctagt tccttt 16
<210> 307
   <211> 16
   <212> DNA
   <213> Artificial Sequence
<220>
   <223> Oligonucleotide motif
<400> 307
   aacactccta gttcct 16
<210> 308
   <211> 16
   <212> DNA
   <213> Artificial Sequence
<220>
   <223> Oligonucleotide motif
<400> 308
   taacactcct agttcc 16
<210> 309
   <211> 16
   <212> DNA
   <213> Artificial Sequence
<220>
   <223> Oligonucleotide motif
<400> 309
   ctaacactcc tagttc 16
<210> 310
   <211> 16
   <212> DNA
   <213> Artificial Sequence
<220>
   <223> Oligonucleotide motif
<400> 310
   tgataacata actgtg 16
<210> 311
   <211> 16
   <212> DNA
   <213> Artificial Sequence
<220>
   <223> Oligonucleotide motif
<400> 311
   ctgataacat aactgt 16
<210> 312
   <211> 16
   <212> DNA
   <213> Artificial Sequence
<220>
   <223> Oligonucleotide motif
<400> 312
   tttgaactca agtgac 16
<210> 313
   <211> 16
   <212> DNA
   <213> Artificial Sequence
<220>
   <223> Oligonucleotide motif
<400> 313
   tcctttactt agctag 16
<210> 314
   <211> 16
   <212> DNA
   <213> Artificial Sequence
<220>
   <223> Oligonucleotide motif
<400> 314
   gagtttggat tagctg 16
<210> 315
   <211> 16
   <212> DNA
   <213> Artificial Sequence
<220>
   <223> Oligonucleotide motif
<400> 315
   tgggatatga caggga 16
<210> 316
   <211> 16
   <212> DNA
   <213> Artificial Sequence
<220>
   <223> Oligonucleotide motif
<400> 316
   tgtgggatat gacagg 16
<210> 317
   <211> 16
   <212> DNA
   <213> Artificial Sequence
<220>
   <223> Oligonucleotide motif
<400> 317
   atatggaagg gatatc 16
<210> 318
   <211> 16
   <212> DNA
   <213> Artificial Sequence
<220>
   <223> Oligonucleotide motif
<400> 318
   acaggatatg gaaggg 16
<210> 319
   <211> 17
   <212> DNA
   <213> Artificial Sequence
<220>
   <223> Oligonucleotide motif
<400> 319
   atttcaacag gatatgg 17
<210> 320
   <211> 16
   <212> DNA
   <213> Artificial Sequence
<220>
   <223> Oligonucleotide motif
<400> 320
   gagtaatttc aacagg 16
<210> 321
   <211> 17
   <212> DNA
   <213> Artificial Sequence
<220>
   <223> Oligonucleotide motif
<400> 321
   agggagtaat ttcaaca 17
<210> 322
   <211> 17
   <212> DNA
   <213> Artificial Sequence
<220>
   <223> Oligonucleotide motif
<400> 322
   attagggagt aatttca 17
<210> 323
   <211> 16
   <212> DNA
   <213> Artificial Sequence
<220>
   <223> Oligonucleotide motif
<400> 323
   cttactatta gggagt 16
<210> 324
   <211> 16
   <212> DNA
   <213> Artificial Sequence
<220>
   <223> Oligonucleotide motif
<400> 324
   cagcttacta ttaggg 16
<210> 325
   <211> 16
   <212> DNA
   <213> Artificial Sequence
<220>
   <223> Oligonucleotide motif
<400> 325
   tcagcttact attagg 16
<210> 326
   <211> 18
   <212> DNA
   <213> Artificial Sequence
<220>
   <223> Oligonucleotide motif
<400> 326
   atttcagctt actattag 18
<210> 327
   <211> 16
   <212> DNA
   <213> Artificial Sequence
<220>
   <223> Oligonucleotide motif
<400> 327
   ttcagcttac tattag 16
<210> 328
   <211> 16
   <212> DNA
   <213> Artificial Sequence
<220>
   <223> Oligonucleotide motif
<400> 328
   cagatttcag cttact 16
<210> 329
   <211> 16
   <212> DNA
   <213> Artificial Sequence
<220>
   <223> Oligonucleotide motif
<400> 329
   gactacaact agaggg 16
<210> 330
   <211> 16
   <212> DNA
   <213> Artificial Sequence
<220>
   <223> Oligonucleotide motif
<400> 330
   agactacaac tagagg 16
<210> 331
   <211> 16
   <212> DNA
   <213> Artificial Sequence
<220>
   <223> Oligonucleotide motif
<400> 331
   aagactacaa ctagag 16
<210> 332
   <211> 20
   <212> DNA
   <213> Artificial Sequence
<220>
   <223> Oligonucleotide motif
<400> 332
   atgatttaat tctagtcaaa 20
<210> 333
   <211> 16
   <212> DNA
   <213> Artificial Sequence
<220>
   <223> Oligonucleotide motif
<400> 333
   tttaattcta gtcaaa 16
<210> 334
   <211> 16
   <212> DNA
   <213> Artificial Sequence
<220>
   <223> Oligonucleotide motif
<400> 334
   gatttaattc tagtca 16
<210> 335
   <211> 18
   <212> DNA
   <213> Artificial Sequence
<220>
   <223> Oligonucleotide motif
<400> 335
   atgatttaat tctagtca 18
<210> 336
   <211> 19
   <212> DNA
   <213> Artificial Sequence
<220>
   <223> Oligonucleotide motif
<400> 336
   gatgatttaa ttctagtca 19
<210> 337
   <211> 16
   <212> DNA
   <213> Artificial Sequence
<220>
   <223> Oligonucleotide motif
<400> 337
   gatttaattc tagtca 16
<210> 338
   <211> 18
   <212> DNA
   <213> Artificial Sequence
<220>
   <223> Oligonucleotide motif
<400> 338
   gatgatttaa ttctagtc 18
<210> 339
   <211> 16
   <212> DNA
   <213> Artificial Sequence
<220>
   <223> Oligonucleotide motif
<400> 339
   tgatttaatt ctagtc 16
<210> 340
   <211> 17
   <212> DNA
   <213> Artificial Sequence
<220>
   <223> Oligonucleotide motif
<400> 340
   gagatgattt aattcta 17
<210> 341
   <211> 16
   <212> DNA
   <213> Artificial Sequence
<220>
   <223> Oligonucleotide motif
<400> 341
   gagatgattt aattct 16
<210> 342
   <211> 16
   <212> DNA
   <213> Artificial Sequence
<220>
   <223> Oligonucleotide motif
<400> 342
   cagattgatg gtagtt 16
<210> 343
   <211> 16
   <212> DNA
   <213> Artificial Sequence
<220>
   <223> Oligonucleotide motif
<400> 343
   ctcagattga tggtag 16
<210> 344
   <211> 16
   <212> DNA
   <213> Artificial Sequence
<220>
   <223> Oligonucleotide motif
<400> 344
   gttagccctc agattg 16
<210> 345
   <211> 16
   <212> DNA
   <213> Artificial Sequence
<220>
   <223> Oligonucleotide motif
<400> 345
   tgtattgtta gccctc 16
<210> 346
   <211> 16
   <212> DNA
   <213> Artificial Sequence
<220>
   <223> Oligonucleotide motif
<400> 346
   acttgtattg ttagcc 16
<210> 347
   <211> 16
   <212> DNA
   <213> Artificial Sequence
<220>
   <223> Oligonucleotide motif
<400> 347
   agccagtatc agggac 16
<210> 348
   <211> 16
   <212> DNA
   <213> Artificial Sequence
<220>
   <223> Oligonucleotide motif
<400> 348
   ttgacaatag tggcat 16
<210> 349
   <211> 16
   <212> DNA
   <213> Artificial Sequence
<220>
   <223> Oligonucleotide motif
<400> 349
   acaagtggta tcttct 16
<210> 350
   <211> 16
   <212> DNA
   <213> Artificial Sequence
<220>
   <223> Oligonucleotide motif
<400> 350
   aatctacttt acaagt 16
<210> 351
   <211> 18
   <212> DNA
   <213> Artificial Sequence
<220>
   <223> Oligonucleotide motif
<400> 351
   cacagtagat gcctgata 18
<210> 352
   <211> 16
   <212> DNA
   <213> Artificial Sequence
<220>
   <223> Oligonucleotide motif
<400> 352
   gaacacagta gatgcc 16
<210> 353
   <211> 17
   <212> DNA
   <213> Artificial Sequence
<220>
   <223> Oligonucleotide motif
<400> 353
   cttggaacac agtagat 17
<210> 354
   <211> 16
   <212> DNA
   <213> Artificial Sequence
<220>
   <223> Oligonucleotide motif
<400> 354
   atatcttgga acacag 16
<210> 355
   <211> 18
   <212> DNA
   <213> Artificial Sequence
<220>
   <223> Oligonucleotide motif
<400> 355
   tctttaatat cttggaac 18
<210> 356
   <211> 19
   <212> DNA
   <213> Artificial Sequence
<220>
   <223> Oligonucleotide motif
<400> 356
   tgatttcttt aatatcttg 19
<210> 357
   <211> 19
   <212> DNA
   <213> Artificial Sequence
<220>
   <223> Oligonucleotide motif
<400> 357
   tgatgatttc tttaatatc 19
<210> 358
   <211> 16
   <212> DNA
   <213> Artificial Sequence
<220>
   <223> Oligonucleotide motif
<400> 358
   aggctaagtc atgatg 16
<210> 359
   <211> 16
   <212> DNA
   <213> Artificial Sequence
<220>
   <223> Oligonucleotide motif
<400> 359
   ttgatgaggc taagtc 16
<210> 360
   <211> 16
   <212> DNA
   <213> Artificial Sequence
<220>
   <223> Oligonucleotide motif
<400> 360
   ccaggattat actctt 16
<210> 361
   <211> 16
   <212> DNA
   <213> Artificial Sequence
<220>
   <223> Oligonucleotide motif
<400> 361
   gccaggatta tactct 16
<210> 362
   <211> 16
   <212> DNA
   <213> Artificial Sequence
<220>
   <223> Oligonucleotide motif
<400> 362
   ctgccaggat tatact 16
<210> 363
   <211> 19
   <212> DNA
   <213> Artificial Sequence
<220>
   <223> Oligonucleotide motif
<400> 363
   cagaaactta tactttatg 19
<210> 364
   <211> 17
   <212> DNA
   <213> Artificial Sequence
<220>
   <223> Oligonucleotide motif
<400> 364
   aagcagaaac ttatact 17
<210> 365
   <211> 18
   <212> DNA
   <213> Artificial Sequence
<220>
   <223> Oligonucleotide motif
<400> 365
   gaagcagaaa cttatact 18
<210> 366
   <211> 20
   <212> DNA
   <213> Artificial Sequence
<220>
   <223> Oligonucleotide motif
<400> 366
   tggaagcaga aacttatact 20
<210> 367
   <211> 19
   <212> DNA
   <213> Artificial Sequence
<220>
   <223> Oligonucleotide motif
<400> 367
   tggaagcaga aacttatac 19
<210> 368
   <211> 16
   <212> DNA
   <213> Artificial Sequence
<220>
   <223> Oligonucleotide motif
<400> 368
   aagcagaaac ttatac 16
<210> 369
   <211> 18
   <212> DNA
   <213> Artificial Sequence
<220>
   <223> Oligonucleotide motif
<400> 369
   tggaagcaga aacttata 18
<210> 370
   <211> 16
   <212> DNA
   <213> Artificial Sequence
<220>
   <223> Oligonucleotide motif
<400> 370
   aagggatatt atggag 16
<210> 371
   <211> 16
   <212> DNA
   <213> Artificial Sequence
<220>
   <223> Oligonucleotide motif
<400> 371
   tgccggaaga tttcct 16
<210> 372
   <211> 16
   <212> DNA
   <213> Artificial Sequence
<220>
   <223> Oligonucleotide motif
<400> 372
   atggattggg agtaga 16
<210> 373
   <211> 16
   <212> DNA
   <213> Artificial Sequence
<220>
   <223> Oligonucleotide motif
<400> 373
   agatggattg ggagta 16
<210> 374
   <211> 16
   <212> DNA
   <213> Artificial Sequence
<220>
   <223> Oligonucleotide motif
<400> 374
   aagatggatt gggagt 16
<210> 375
   <211> 16
   <212> DNA
   <213> Artificial Sequence
<220>
   <223> Oligonucleotide motif
<400> 375
   acaagatgga ttggga 16
<210> 376
   <211> 16
   <212> DNA
   <213> Artificial Sequence
<220>
   <223> Oligonucleotide motif
<400> 376
   agaaggttca gacttt 16
<210> 377
   <211> 16
   <212> DNA
   <213> Artificial Sequence
<220>
   <223> Oligonucleotide motif
<400> 377
   gcagaaggtt cagact 16
<210> 378
   <211> 16
   <212> DNA
   <213> Artificial Sequence
<220>
   <223> Oligonucleotide motif
<400> 378
   tgcagaaggt tcagac 16
<210> 379
   <211> 16
   <212> DNA
   <213> Artificial Sequence
<220>
   <223> Oligonucleotide motif
<400> 379
   agtgcagaag gttcag 16
<210> 380
   <211> 16
   <212> DNA
   <213> Artificial Sequence
<220>
   <223> Oligonucleotide motif
<400> 380
   aagtgcagaa ggttca 16
<210> 381
   <211> 16
   <212> DNA
   <213> Artificial Sequence
<220>
   <223> Oligonucleotide motif
<400> 381
   taagtgcaga aggttc 16
<210> 382
   <211> 16
   <212> DNA
   <213> Artificial Sequence
<220>
   <223> Oligonucleotide motif
<400> 382
   tctaagtgca gaaggt 16
<210> 383
   <211> 17
   <212> DNA
   <213> Artificial Sequence
<220>
   <223> Oligonucleotide motif
<400> 383
   ctcaggagtt ctacttc 17
<210> 384
   <211> 16
   <212> DNA
   <213> Artificial Sequence
<220>
   <223> Oligonucleotide motif
<400> 384
   ctcaggagtt ctactt 16
<210> 385
   <211> 18
   <212> DNA
   <213> Artificial Sequence
<220>
   <223> Oligonucleotide motif
<400> 385
   atggaggtga ctcaggag 18
<210> 386
   <211> 17
   <212> DNA
   <213> Artificial Sequence
<220>
   <223> Oligonucleotide motif
<400> 386
   atggaggtga ctcagga 17
<210> 387
   <211> 16
   <212> DNA
   <213> Artificial Sequence
<220>
   <223> Oligonucleotide motif
<400> 387
   atggaggtga ctcagg 16
<210> 388
   <211> 17
   <212> DNA
   <213> Artificial Sequence
<220>
   <223> Oligonucleotide motif
<400> 388
   tatggaggtg actcagg 17
<210> 389
   <211> 18
   <212> DNA
   <213> Artificial Sequence
<220>
   <223> Oligonucleotide motif
<400> 389
   atatggaggt gactcagg 18
<210> 390
   <211> 16
   <212> DNA
   <213> Artificial Sequence
<220>
   <223> Oligonucleotide motif
<400> 390
   tatggaggtg actcag 16
<210> 391
   <211> 17
   <212> DNA
   <213> Artificial Sequence
<220>
   <223> Oligonucleotide motif
<400> 391
   atatggaggt gactcag 17
<210> 392
   <211> 18
   <212> DNA
   <213> Artificial Sequence
<220>
   <223> Oligonucleotide motif
<400> 392
   catatggagg tgactcag 18
<210> 393
   <211> 16
   <212> DNA
   <213> Artificial Sequence
<220>
   <223> Oligonucleotide motif
<400> 393
   atatggaggt gactca 16
<210> 394
   <211> 17
   <212> DNA
   <213> Artificial Sequence
<220>
   <223> Oligonucleotide motif
<400> 394
   catatggagg tgactca 17
<210> 395
   <211> 16
   <212> DNA
   <213> Artificial Sequence
<220>
   <223> Oligonucleotide motif
<400> 395
   catatggagg tgactc 16
<210> 396
   <211> 17
   <212> DNA
   <213> Artificial Sequence
<220>
   <223> Oligonucleotide motif
<400> 396
   gcatatggag gtgactc 17
<210> 397
   <211> 18
   <212> DNA
   <213> Artificial Sequence
<220>
   <223> Oligonucleotide motif
<400> 397
   tgcatatgga ggtgactc 18
<210> 398
   <211> 19
   <212> DNA
   <213> Artificial Sequence
<220>
   <223> Oligonucleotide motif
<400> 398
   ttgcatatgg aggtgactc 19
<210> 399
   <211> 20
   <212> DNA
   <213> Artificial Sequence
<220>
   <223> Oligonucleotide motif
<400> 399
   tttgcatatg gaggtgactc 20
<210> 400
   <211> 16
   <212> DNA
   <213> Artificial Sequence
<220>
   <223> Oligonucleotide motif
<400> 400
   gcatatggag gtgact 16
<210> 401
   <211> 17
   <212> DNA
   <213> Artificial Sequence
<220>
   <223> Oligonucleotide motif
<400> 401
   tgcatatgga ggtgact 17
<210> 402
   <211> 18
   <212> DNA
   <213> Artificial Sequence
<220>
   <223> Oligonucleotide motif
<400> 402
   ttgcatatgg aggtgact 18
<210> 403
   <211> 19
   <212> DNA
   <213> Artificial Sequence
<220>
   <223> Oligonucleotide motif
<400> 403
   tttgcatatg gaggtgact 19
<210> 404
   <211> 16
   <212> DNA
   <213> Artificial Sequence
<220>
   <223> Oligonucleotide motif
<400> 404
   tgcatatgga ggtgac 16
<210> 405
   <211> 17
   <212> DNA
   <213> Artificial Sequence
<220>
   <223> Oligonucleotide motif
<400> 405
   ttgcatatgg aggtgac 17
<210> 406
   <211> 18
   <212> DNA
   <213> Artificial Sequence
<220>
   <223> Oligonucleotide motif
<400> 406
   tttgcatatg gaggtgac 18
<210> 407
   <211> 17
   <212> DNA
   <213> Artificial Sequence
<220>
   <223> Oligonucleotide motif
<400> 407
   tttgcatatg gaggtga 17
<210> 408
   <211> 16
   <212> DNA
   <213> Artificial Sequence
<220>
   <223> Oligonucleotide motif
<400> 408
   tttgcatatg gaggtg 16
<210> 409
   <211> 17
   <212> DNA
   <213> Artificial Sequence
<220>
   <223> Oligonucleotide motif
<400> 409
   aagtgaagtt caacagc 17
<210> 410
   <211> 16
   <212> DNA
   <213> Artificial Sequence
<220>
   <223> Oligonucleotide motif
<400> 410
   tgggaagtga agttca 16
<210> 411
   <211> 16
   <212> DNA
   <213> Artificial Sequence
<220>
   <223> Oligonucleotide motif
<400> 411
   atgggaagtg aagttc 16
<210> 412
   <211> 16
   <212> DNA
   <213> Artificial Sequence
<220>
   <223> Oligonucleotide motif
<400> 412
   gatgggaagt gaagtt 16
<210> 413
   <211> 17
   <212> DNA
   <213> Artificial Sequence
<220>
   <223> Oligonucleotide motif
<400> 413
   ctgtgatggg aagtgaa 17
<210> 414
   <211> 16
   <212> DNA
   <213> Artificial Sequence
<220>
   <223> Oligonucleotide motif
<400> 414
   attgagtgaa tccaaa 16
<210> 415
   <211> 16
   <212> DNA
   <213> Artificial Sequence
<220>
   <223> Oligonucleotide motif
<400> 415
   aattgagtga atccaa 16
<210> 416
   <211> 17
   <212> DNA
   <213> Artificial Sequence
<220>
   <223> Oligonucleotide motif
<400> 416
   gataattgag tgaatcc 17
<210> 417
   <211> 16
   <212> DNA
   <213> Artificial Sequence
<220>
   <223> Oligonucleotide motif
<400> 417
   gtgataattg agtgaa 16
<210> 418
   <211> 16
   <212> DNA
   <213> Artificial Sequence
<220>
   <223> Oligonucleotide motif
<400> 418
   aagaaaggtg caataa 16
<210> 419
   <211> 16
   <212> DNA
   <213> Artificial Sequence
<220>
   <223> Oligonucleotide motif
<400> 419
   caagaaaggt gcaata 16
<210> 420
   <211> 16
   <212> DNA
   <213> Artificial Sequence
<220>
   <223> Oligonucleotide motif
<400> 420
   acaagaaagg tgcaat 16
<210> 421
   <211> 16
   <212> DNA
   <213> Artificial Sequence
<220>
   <223> Oligonucleotide motif
<400> 421
   atttaaactc acaaac 16
<210> 422
   <211> 16
   <212> DNA
   <213> Artificial Sequence
<220>
   <223> Oligonucleotide motif
<400> 422
   ctgttaggtt cagcga 16
<210> 423
   <211> 17
   <212> DNA
   <213> Artificial Sequence
<220>
   <223> Oligonucleotide motif
<400> 423
   tctgaatgaa catttcg 17
<210> 424
   <211> 16
   <212> DNA
   <213> Artificial Sequence
<220>
   <223> Oligonucleotide motif
<400> 424
   ctcattgaag gttctg 16
<210> 425
   <211> 16
   <212> DNA
   <213> Artificial Sequence
<220>
   <223> Oligonucleotide motif
<400> 425
   ctaatctcat tgaagg 16
<210> 426
   <211> 16
   <212> DNA
   <213> Artificial Sequence
<220>
   <223> Oligonucleotide motif
<400> 426
   cctaatctca ttgaag 16
<210> 427
   <211> 16
   <212> DNA
   <213> Artificial Sequence
<220>
   <223> Oligonucleotide motif
<400> 427
   actttgatct ttcagc 16
<210> 428
   <211> 16
   <212> DNA
   <213> Artificial Sequence
<220>
   <223> Oligonucleotide motif
<400> 428
   actatgcaac actttg 16
<210> 429
   <211> 16
   <212> DNA
   <213> Artificial Sequence
<220>
   <223> Oligonucleotide motif
<400> 429
   caaatagctt tatcgg 16
<210> 430
   <211> 16
   <212> DNA
   <213> Artificial Sequence
<220>
   <223> Oligonucleotide motif
<400> 430
   ccaaatagct ttatcg 16
<210> 431
   <211> 16
   <212> DNA
   <213> Artificial Sequence
<220>
   <223> Oligonucleotide motif
<400> 431
   tccaaatagc tttatc 16
<210> 432
   <211> 16
   <212> DNA
   <213> Artificial Sequence
<220>
   <223> Oligonucleotide motif
<400> 432
   gatccaaata gcttta 16
<210> 433
   <211> 16
   <212> DNA
   <213> Artificial Sequence
<220>
   <223> Oligonucleotide motif
<400> 433
   atgatccaaa tagctt 16
<210> 434
   <211> 16
   <212> DNA
   <213> Artificial Sequence
<220>
   <223> Oligonucleotide motif
<400> 434
   tatgatccaa atagct 16
<210> 435
   <211> 17
   <212> DNA
   <213> Artificial Sequence
<220>
   <223> Oligonucleotide motif
<400> 435
   taaacagggc tgggaat 17
<210> 436
   <211> 16
   <212> DNA
   <213> Artificial Sequence
<220>
   <223> Oligonucleotide motif
<400> 436
   acttaaacag ggctgg 16
<210> 437
   <211> 16
   <212> DNA
   <213> Artificial Sequence
<220>
   <223> Oligonucleotide motif
<400> 437
   acacttaaac agggct 16
<210> 438
   <211> 16
   <212> DNA
   <213> Artificial Sequence
<220>
   <223> Oligonucleotide motif
<400> 438
   gaacacttaa acaggg 16
<210> 439
   <211> 17
   <212> DNA
   <213> Artificial Sequence
<220>
   <223> Oligonucleotide motif
<400> 439
   agagaacact taaacag 17
<210> 440
   <211> 16
   <212> DNA
   <213> Artificial Sequence
<220>
   <223> Oligonucleotide motif
<400> 440
   ctacagagaa cactta 16
<210> 441
   <211> 17
   <212> DNA
   <213> Artificial Sequence
<220>
   <223> Oligonucleotide motif
<400> 441
   atgctacaga gaacact 17
<210> 442
   <211> 19
   <212> DNA
   <213> Artificial Sequence
<220>
   <223> Oligonucleotide motif
<400> 442
   ataaatgcta cagagaaca 19
<210> 443
   <211> 18
   <212> DNA
   <213> Artificial Sequence
<220>
   <223> Oligonucleotide motif
<400> 443
   agataaatgc tacagaga 18
<210> 444
   <211> 17
   <212> DNA
   <213> Artificial Sequence
<220>
   <223> Oligonucleotide motif
<400> 444
   tagagataaa tgctaca 17
<210> 445
   <211> 18
   <212> DNA
   <213> Artificial Sequence
<220>
   <223> Oligonucleotide motif
<400> 445
   tagatagaga taaatgct 18
<210> 446
   <211> 18
   <212> DNA
   <213> Artificial Sequence
<220>
   <223> Oligonucleotide motif
<400> 446
   caatatacta gatagaga 18
<210> 447
   <211> 19
   <212> DNA
   <213> Artificial Sequence
<220>
   <223> Oligonucleotide motif
<400> 447
   tacacaatat actagatag 19
<210> 448
   <211> 16
   <212> DNA
   <213> Artificial Sequence
<220>
   <223> Oligonucleotide motif
<400> 448
   ctacacaata tactag 16
<210> 449
   <211> 16
   <212> DNA
   <213> Artificial Sequence
<220>
   <223> Oligonucleotide motif
<400> 449
   gctacacaat atacta 16
<210> 450
   <211> 18
   <212> DNA
   <213> Artificial Sequence
<220>
   <223> Oligonucleotide motif
<400> 450
   atatgctaca caatatac 18
<210> 451
   <211> 16
   <212> DNA
   <213> Artificial Sequence
<220>
   <223> Oligonucleotide motif
<400> 451
   tgatatgcta cacaat 16
<210> 452
   <211> 17
   <212> DNA
   <213> Artificial Sequence
<220>
   <223> Oligonucleotide motif
<400> 452
   atgatatgat atgctac 17
<210> 453
   <211> 18
   <212> DNA
   <213> Artificial Sequence
<220>
   <223> Oligonucleotide motif
<400> 453
   gaggagagag acaataaa 18
<210> 454
   <211> 17
   <212> DNA
   <213> Artificial Sequence
<220>
   <223> Oligonucleotide motif
<400> 454
   ctaggaggag agagaca 17
<210> 455
   <211> 17
   <212> DNA
   <213> Artificial Sequence
<220>
   <223> Oligonucleotide motif
<400> 455
   tattctagga ggagaga 17
<210> 456
   <211> 17
   <212> DNA
   <213> Artificial Sequence
<220>
   <223> Oligonucleotide motif
<400> 456
   ttatattcta ggaggag 17
<210> 457
   <211> 16
   <212> DNA
   <213> Artificial Sequence
<220>
   <223> Oligonucleotide motif
<400> 457
   gtttatattc taggag 16
<210> 458
   <211> 18
   <212> DNA
   <213> Artificial Sequence
<220>
   <223> Oligonucleotide motif
<400> 458
   tggagtttat attctagg 18
<210> 459
   <211> 16
   <212> DNA
   <213> Artificial Sequence
<220>
   <223> Oligonucleotide motif
<400> 459
   cgtaccacca ctctgc 16
<210> 460
   <211> 18
   <212> DNA
   <213> Artificial Sequence
<220>
   <223> Oligonucleotide motif
<400> 460
   tgaggaaatc attcattc 18
<210> 461
   <211> 18
   <212> DNA
   <213> Artificial Sequence
<220>
   <223> Oligonucleotide motif
<400> 461
   tttgaggaaa tcattcat 18
<210> 462
   <211> 16
   <212> DNA
   <213> Artificial Sequence
<220>
   <223> Oligonucleotide motif
<400> 462
   aggctaatcc tatttg 16
<210> 463
   <211> 16
   <212> DNA
   <213> Artificial Sequence
<220>
   <223> Oligonucleotide motif
<400> 463
   tttaggctaa tcctat 16
<210> 464
   <211> 16
   <212> DNA
   <213> Artificial Sequence
<220>
   <223> Oligonucleotide motif
<400> 464
   tgctccagtg taccct 16
<210> 465
   <211> 16
   <212> DNA
   <213> Artificial Sequence
<220>
   <223> Oligonucleotide motif
<400> 465
   tagtagtact cgatag 16
<210> 466
   <211> 18
   <212> DNA
   <213> Artificial Sequence
<220>
   <223> Oligonucleotide motif
<400> 466
   ctaattgtag tagtactc 18
<210> 467
   <211> 16
   <212> DNA
   <213> Artificial Sequence
<220>
   <223> Oligonucleotide motif
<400> 467
   tgctaattgt agtagt 16
<210> 468
   <211> 16
   <212> DNA
   <213> Artificial Sequence
<220>
   <223> Oligonucleotide motif
<400> 468
   agtgctaatt gtagta 16
<210> 469
   <211> 16
   <212> DNA
   <213> Artificial Sequence
<220>
   <223> Oligonucleotide motif
<400> 469
   gcaagtgcta attgta 16
<210> 470
   <211> 19
   <212> DNA
   <213> Artificial Sequence
<220>
   <223> Oligonucleotide motif
<400> 470
   gaggaaatga actaattta 19
<210> 471
   <211> 17
   <212> DNA
   <213> Artificial Sequence
<220>
   <223> Oligonucleotide motif
<400> 471
   caggaggaaa tgaacta 17
<210> 472
   <211> 16
   <212> DNA
   <213> Artificial Sequence
<220>
   <223> Oligonucleotide motif
<400> 472
   ccctagagtc atttcc 16
<210> 473
   <211> 17
   <212> DNA
   <213> Artificial Sequence
<220>
   <223> Oligonucleotide motif
<400> 473
   atcttacatg atgaagc 17
<210> 474
   <211> 18
   <212> DNA
   <213> Artificial Sequence
<220>
   <223> Oligonucleotide motif
<400> 474
   gacacactca gatttcag 18
<210> 475
   <211> 19
   <212> DNA
   <213> Artificial Sequence
<220>
   <223> Oligonucleotide motif
<400> 475
   agacacactc agatttcag 19
<210> 476
   <211> 20
   <212> DNA
   <213> Artificial Sequence
<220>
   <223> Oligonucleotide motif
<400> 476
   aagacacact cagatttcag 20
<210> 477
   <211> 18
   <212> DNA
   <213> Artificial Sequence
<220>
   <223> Oligonucleotide motif
<400> 477
   agacacactc agatttca 18
<210> 478
   <211> 19
   <212> DNA
   <213> Artificial Sequence
<220>
   <223> Oligonucleotide motif
<400> 478
   aagacacact cagatttca 19
<210> 479
   <211> 20
   <212> DNA
   <213> Artificial Sequence
<220>
   <223> Oligonucleotide motif
<400> 479
   aaagacacac tcagatttca 20
<210> 480
   <211> 20
   <212> DNA
   <213> Artificial Sequence
<220>
   <223> Oligonucleotide motif
<400> 480
   gaaagacaca ctcagatttc 20
<210> 481
   <211> 18
   <212> DNA
   <213> Artificial Sequence
<220>
   <223> Oligonucleotide motif
<400> 481
   aagacacact cagatttc 18
<210> 482
   <211> 19
   <212> DNA
   <213> Artificial Sequence
<220>
   <223> Oligonucleotide motif
<400> 482
   aaagacacac tcagatttc 19
<210> 483
   <211> 20
   <212> DNA
   <213> Artificial Sequence
<220>
   <223> Oligonucleotide motif
<400> 483
   tgaaagacac actcagattt 20
<210> 484
   <211> 19
   <212> DNA
   <213> Artificial Sequence
<220>
   <223> Oligonucleotide motif
<400> 484
   tgaaagacac actcagatt 19
<210> 485
   <211> 18
   <212> DNA
   <213> Artificial Sequence
<220>
   <223> Oligonucleotide motif
<400> 485
   tgaaagacac actcagat 18
<210> 486
   <211> 17
   <212> DNA
   <213> Artificial Sequence
<220>
   <223> Oligonucleotide motif
<400> 486
   attgaaagac acactca 17
<210> 487
   <211> 17
   <212> DNA
   <213> Artificial Sequence
<220>
   <223> Oligonucleotide motif
<400> 487
   tcattgaaag acacact 17
<210> 488
   <211> 16
   <212> DNA
   <213> Artificial Sequence
<220>
   <223> Oligonucleotide motif
<400> 488
   ttccatcatt gaaaga 16
<210> 489
   <211> 17
   <212> DNA
   <213> Artificial Sequence
<220>
   <223> Oligonucleotide motif
<400> 489
   ataataccac ttatcat 17
<210> 490
   <211> 18
   <212> DNA
   <213> Artificial Sequence
<220>
   <223> Oligonucleotide motif
<400> 490
   ttacttaatt tctttgga 18
<210> 491
   <211> 18
   <212> DNA
   <213> Artificial Sequence
<220>
   <223> Oligonucleotide motif
<400> 491
   ttagaactag ctttatca 18
<210> 492
   <211> 16
   <212> DNA
   <213> Artificial Sequence
<220>
   <223> Oligonucleotide motif
<400> 492
   gaggtacaaa tatagg 16
<210> 493
   <211> 16
   <212> DNA
   <213> Artificial Sequence
<220>
   <223> Oligonucleotide motif
<400> 493
   cttatgatac aactta 16
<210> 494
   <211> 16
   <212> DNA
   <213> Artificial Sequence
<220>
   <223> Oligonucleotide motif
<400> 494
   tcttatgata caactt 16
<210> 495
   <211> 16
   <212> DNA
   <213> Artificial Sequence
<220>
   <223> Oligonucleotide motif
<400> 495
   ttcttatgat acaact 16
<210> 496
   <211> 16
   <212> DNA
   <213> Artificial Sequence
<220>
   <223> Oligonucleotide motif
<400> 496
   cagtttctta tgatac 16
<210> 497
   <211> 16
   <212> DNA
   <213> Artificial Sequence
<220>
   <223> Oligonucleotide motif
<400> 497
   gcagtttctt atgata 16
<210> 498
   <211> 19
   <212> DNA
   <213> Artificial Sequence
<220>
   <223> Oligonucleotide motif
<400> 498
   tacaaatgtc tattaggtt 19
<210> 499
   <211> 18
   <212> DNA
   <213> Artificial Sequence
<220>
   <223> Oligonucleotide motif
<400> 499
   tgtacaaatg tctattag 18
<210> 500
   <211> 16
   <212> DNA
   <213> Artificial Sequence
<220>
   <223> Oligonucleotide motif
<400> 500
   agcatcacaa ttagta 16
<210> 501
   <211> 16
   <212> DNA
   <213> Artificial Sequence
<220>
   <223> Oligonucleotide motif
<400> 501
   ctaatgatag tgaagc 16
<210> 502
   <211> 16
   <212> DNA
   <213> Artificial Sequence
<220>
   <223> Oligonucleotide motif
<400> 502
   agctaatgat agtgaa 16
<210> 503
   <211> 16
   <212> DNA
   <213> Artificial Sequence
<220>
   <223> Oligonucleotide motif
<400> 503
   atgccttgac atatta 16
<210> 504
   <211> 17
   <212> DNA
   <213> Artificial Sequence
<220>
   <223> Oligonucleotide motif
<400> 504
   ctcaagatta ttgacac 17
<210> 505
   <211> 16
   <212> DNA
   <213> Artificial Sequence
<220>
   <223> Oligonucleotide motif
<400> 505
   acctcaagat tattga 16
<210> 506
   <211> 16
   <212> DNA
   <213> Artificial Sequence
<220>
   <223> Oligonucleotide motif
<400> 506
   aacctcaaga ttattg 16
<210> 507
   <211> 19
   <212> DNA
   <213> Artificial Sequence
<220>
   <223> Oligonucleotide motif
<400> 507
   cacaaacctc aagattatt 19
<210> 508
   <211> 16
   <212> DNA
   <213> Artificial Sequence
<220>
   <223> Oligonucleotide motif
<400> 508
   gtacttaatt agacct 16
<210> 509
   <211> 16
   <212> DNA
   <213> Artificial Sequence
<220>
   <223> Oligonucleotide motif
<400> 509
   agtacttaat tagacc 16
<210> 510
   <211> 16
   <212> DNA
   <213> Artificial Sequence
<220>
   <223> Oligonucleotide motif
<400> 510
   gtatgaggtg gtaaac 16
<210> 511
   <211> 17
   <212> DNA
   <213> Artificial Sequence
<220>
   <223> Oligonucleotide motif
<400> 511
   aggaaacagc agaagtg 17
<210> 512
   <211> 16
   <212> DNA
   <213> Artificial Sequence
<220>
   <223> Oligonucleotide motif
<400> 512
   gcacaaccca gaggaa 16
<210> 513
   <211> 16
   <212> DNA
   <213> Artificial Sequence
<220>
   <223> Oligonucleotide motif
<400> 513
   caagcacaac ccagag 16
<210> 514
   <211> 16
   <212> DNA
   <213> Artificial Sequence
<220>
   <223> Oligonucleotide motif
<400> 514
   ttcaagcaca acccag 16
<210> 515
   <211> 16
   <212> DNA
   <213> Artificial Sequence
<220>
   <223> Oligonucleotide motif
<400> 515
   aattcaagca caaccc 16
<210> 516
   <211> 19
   <212> DNA
   <213> Artificial Sequence
<220>
   <223> Oligonucleotide motif
<400> 516
   taataattca agcacaacc 19
<210> 517
   <211> 17
   <212> DNA
   <213> Artificial Sequence
<220>
   <223> Oligonucleotide motif
<400> 517
   actaataatt caagcac 17
<210> 518
   <211> 20
   <212> DNA
   <213> Artificial Sequence
<220>
   <223> Oligonucleotide motif
<400> 518
   ataatactaa taattcaagc 20
<210> 519
   <211> 16
   <212> DNA
   <213> Artificial Sequence
<220>
   <223> Oligonucleotide motif
<400> 519
   tagatttgtg aggtaa 16
<210> 520
   <211> 16
   <212> DNA
   <213> Artificial Sequence
<220>
   <223> Oligonucleotide motif
<400> 520
   agccttaatt ctccat 16
<210> 521
   <211> 17
   <212> DNA
   <213> Artificial Sequence
<220>
   <223> Oligonucleotide motif
<400> 521
   aatgatctag agcctta 17
<210> 522
   <211> 16
   <212> DNA
   <213> Artificial Sequence
<220>
   <223> Oligonucleotide motif
<400> 522
   ctaatgatct agagcc 16
<210> 523
   <211> 16
   <212> DNA
   <213> Artificial Sequence
<220>
   <223> Oligonucleotide motif
<400> 523
   actaatgatc tagagc 16
<210> 524
   <211> 18
   <212> DNA
   <213> Artificial Sequence
<220>
   <223> Oligonucleotide motif
<400> 524
   cattaacatg ttcttatt 18
<210> 525
   <211> 20
   <212> DNA
   <213> Artificial Sequence
<220>
   <223> Oligonucleotide motif
<400> 525
   acaagtacat taacatgttc 20
<210> 526
   <211> 19
   <212> DNA
   <213> Artificial Sequence
<220>
   <223> Oligonucleotide motif
<400> 526
   ttacaagtac attaacatg 19
<210> 527
   <211> 17
   <212> DNA
   <213> Artificial Sequence
<220>
   <223> Oligonucleotide motif
<400> 527
   gctttattca tgtttat 17
<210> 528
   <211> 16
   <212> DNA
   <213> Artificial Sequence
<220>
   <223> Oligonucleotide motif
<400> 528
   gctttattca tgttta 16
<210> 529
   <211> 18
   <212> DNA
   <213> Artificial Sequence
<220>
   <223> Oligonucleotide motif
<400> 529
   agagctttat tcatgttt 18
<210> 530
   <211> 18
   <212> DNA
   <213> Artificial Sequence
<220>
   <223> Oligonucleotide motif
<400> 530
   ataagagctt tattcatg 18
<210> 531
   <211> 17
   <212> DNA
   <213> Artificial Sequence
<220>
   <223> Oligonucleotide motif
<400> 531
   cataagagct ttattca 17
<210> 532
   <211> 16
   <212> DNA
   <213> Artificial Sequence
<220>
   <223> Oligonucleotide motif
<400> 532
   agcataagag ctttat 16
<210> 533
   <211> 16
   <212> DNA
   <213> Artificial Sequence
<220>
   <223> Oligonucleotide motif
<400> 533
   tagattgttt agtgca 16
<210> 534
   <211> 16
   <212> DNA
   <213> Artificial Sequence
<220>
   <223> Oligonucleotide motif
<400> 534
   gtagattgtt tagtgc 16
<210> 535
   <211> 17
   <212> DNA
   <213> Artificial Sequence
<220>
   <223> Oligonucleotide motif
<400> 535
   gacaattcta gtagatt 17
<210> 536
   <211> 16
   <212> DNA
   <213> Artificial Sequence
<220>
   <223> Oligonucleotide motif
<400> 536
   ctgacaattc tagtag 16
<210> 537
   <211> 16
   <212> DNA
   <213> Artificial Sequence
<220>
   <223> Oligonucleotide motif
<400> 537
   gctgacaatt ctagta 16
<210> 538
   <211> 16
   <212> DNA
   <213> Artificial Sequence
<220>
   <223> Oligonucleotide motif
<400> 538
   aggattaaga tacgta 16
<210> 539
   <211> 16
   <212> DNA
   <213> Artificial Sequence
<220>
   <223> Oligonucleotide motif
<400> 539
   caggattaag atacgt 16
<210> 540
   <211> 16
   <212> DNA
   <213> Artificial Sequence
<220>
   <223> Oligonucleotide motif
<400> 540
   tcaggattaa gatacg 16
<210> 541
   <211> 16
   <212> DNA
   <213> Artificial Sequence
<220>
   <223> Oligonucleotide motif
<400> a41
   ttcaggatta agatac 16
<210> 542
   <211> 18
   <212> DNA
   <213> Artificial Sequence
<220>
   <223> Oligonucleotide motif
<400> 542
   aggaagaaag tttgattc 18
<210> 543
   <211> 18
   <212> DNA
   <213> Artificial Sequence
<220>
   <223> Oligonucleotide motif
<400> 543
   tcaaggaaga aagtttga 18
<210> 544
   <211> 18
   <212> DNA
   <213> Artificial Sequence
<220>
   <223> Oligonucleotide motif
<400> 544
   ctcaaggaag aaagtttg 18
<210> 545
   <211> 17
   <212> DNA
   <213> Artificial Sequence
<220>
   <223> Oligonucleotide motif
<400> 545
   tgctcaagga agaaagt 17
<210> 546
   <211> 18
   <212> DNA
   <213> Artificial Sequence
<220>
   <223> Oligonucleotide motif
<400> 546
   aattatgctc aaggaaga 18
<210> 547
   <211> 18
   <212> DNA
   <213> Artificial Sequence
<220>
   <223> Oligonucleotide motif
<400> 547
   taggatacca cattatga 18
<210> 548
   <211> 20
   <212> DNA
   <213> Artificial Sequence
<220>
   <223> Oligonucleotide motif
<400> 548
   cataatttat tccattcctc 20
<210> 549
   <211> 17
   <212> DNA
   <213> Artificial Sequence
<220>
   <223> Oligonucleotide motif
<400> 549
   tgcataattt attccat 17
<210> 550
   <211> 17
   <212> DNA
   <213> Artificial Sequence
<220>
   <223> Oligonucleotide motif
<400> 550
   actgcataat ttattcc 17
<210> 551
   <211> 19
   <212> DNA
   <213> Artificial Sequence
<220>
   <223> Oligonucleotide motif
<400> 551
   ctaaactgca taatttatt 19
<210> 552
   <211> 16
   <212> DNA
   <213> Artificial Sequence
<220>
   <223> Oligonucleotide motif
<400> 552
   ataactaaac tgcata 16
<210> 553
   <211> 19
   <212> DNA
   <213> Artificial Sequence
<220>
   <223> Oligonucleotide motif
<400> 553
   ttattaataa ctaaactgc 19
<210> 554
   <211> 19
   <212> DNA
   <213> Artificial Sequence
<220>
   <223> Oligonucleotide motif
<400> 554
   tagtacatta ttaataact 19
<210> 555
   <211> 16
   <212> DNA
   <213> Artificial Sequence
<220>
   <223> Oligonucleotide motif
<400> 555
   cataactaag gacgtt 16
<210> 556
   <211> 16
   <212> DNA
   <213> Artificial Sequence
<220>
   <223> Oligonucleotide motif
<400> 556
   tcataactaa ggacgt 16
<210> 557
   <211> 16
   <212> DNA
   <213> Artificial Sequence
<220>
   <223> Oligonucleotide motif
<400> 557
   cgtcataact aaggac 16
<210> 558
   <211> 16
   <212> DNA
   <213> Artificial Sequence
<220>
   <223> Oligonucleotide motif
<400> 558
   tcgtcataac taagga 16
<210> 559
   <211> 16
   <212> DNA
   <213> Artificial Sequence
<220>
   <223> Oligonucleotide motif
<400> 559
   atcgtcataa ctaagg 16
<210> 560
   <211> 16
   <212> DNA
   <213> Artificial Sequence
<220>
   <223> Oligonucleotide motif
<400> 560
   gttagtatct tacatt 16
<210> 561
   <211> 16
   <212> DNA
   <213> Artificial Sequence
<220>
   <223> Oligonucleotide motif
<400> 561
   ctctattgtt agtatc 16
<210> 562
   <211> 16
   <212> DNA
   <213> Artificial Sequence
<220>
   <223> Oligonucleotide motif
<400> 562
   agtatagagt tactgt 16
<210> 563
   <211> 16
   <212> DNA
   <213> Artificial Sequence
<220>
   <223> Oligonucleotide motif
<400> 563
   ttcctggtga tacttt 16
<210> 564
   <211> 16
   <212> DNA
   <213> Artificial Sequence
<220>
   <223> Oligonucleotide motif
<400> 564
   gttcctggtg atactt 16
<210> 565
   <211> 16
   <212> DNA
   <213> Artificial Sequence
<220>
   <223> Oligonucleotide motif
<400> 565
   tgttcctggt gatact 16
<210> 566
   <211> 18
   <212> DNA
   <213> Artificial Sequence
<220>
   <223> Oligonucleotide motif
<400> 566
   ataaacatga atctctcc 18
<210> 567
   <211> 19
   <212> DNA
   <213> Artificial Sequence
<220>
   <223> Oligonucleotide motif
<400> 567
   ctttataaac atgaatctc 19
<210> 568
   <211> 17
   <212> DNA
   <213> Artificial Sequence
<220>
   <223> Oligonucleotide motif
<400> 568
   ctgtctttat aaacatg 17
<210> 569
   <211> 18
   <212> DNA
   <213> Artificial Sequence
<220>
   <223> Oligonucleotide motif
<400> 569
   ttgttataaa tctgtctt 18
<210> 570
   <211> 19
   <212> DNA
   <213> Artificial Sequence
<220>
   <223> Oligonucleotide motif
<400> 570
   ttaaatttat tcttggata 19
<210> 571
   <211> 18
   <212> DNA
   <213> Artificial Sequence
<220>
   <223> Oligonucleotide motif
<400> 571
   cttaaattta ttcttgga 18
<210> 572
   <211> 19
   <212> DNA
   <213> Artificial Sequence
<220>
   <223> Oligonucleotide motif
<400> 572
   cttcttaaat ttattcttg 19
<210> 573
   <211> 17
   <212> DNA
   <213> Artificial Sequence
<220>
   <223> Oligonucleotide motif
<400> 573
   tatgtttctc agtaaag 17
<210> 574
   <211> 17
   <212> DNA
   <213> Artificial Sequence
<220>
   <223> Oligonucleotide motif
<400> 574
   gaattatctt taaacca 17
<210> 575
   <211> 16
   <212> DNA
   <213> Artificial Sequence
<220>
   <223> Oligonucleotide motif
<400> 575
   cccttaaatt tctaca 16
<210> 576
   <211> 16
   <212> DNA
   <213> Artificial Sequence
<220>
   <223> Oligonucleotide motif
<400> 576
   acactgctct tgtacc 16
<210> 577
   <211> 16
   <212> DNA
   <213> Artificial Sequence
<220>
   <223> Oligonucleotide motif
<400> 577
   tgacaacact gctctt 16
<210> 578
   <211> 16
   <212> DNA
   <213> Artificial Sequence
<220>
   <223> Oligonucleotide motif
<400> 578
   tacatttatt gggctc 16
<210> 579
   <211> 16
   <212> DNA
   <213> Artificial Sequence
<220>
   <223> Oligonucleotide motif
<400> 579
   gtacatttat tgggct 16
<210> 580
   <211> 16
   <212> DNA
   <213> Artificial Sequence
<220>
   <223> Oligonucleotide motif
<400> 580
   ttggtacatt tattgg 16
<210> 581
   <211> 17
   <212> DNA
   <213> Artificial Sequence
<220>
   <223> Oligonucleotide motif
<400> 581
   catgttggta catttat 17
<210> 582
   <211> 16
   <212> DNA
   <213> Artificial Sequence
<220>
   <223> Oligonucleotide motif
<400> 582
   aatcatgttg gtacat 16
<210> 583
   <211> 16
   <212> DNA
   <213> Artificial Sequence
<220>
   <223> Oligonucleotide motif
<400> 583
   aaatcatgtt ggtaca 16
<210> 584
   <211> 16
   <212> DNA
   <213> Artificial Sequence
<220>
   <223> Oligonucleotide motif
<400> 584
   gacaagtttg gattaa 16
<210> 585
   <211> 16
   <212> DNA
   <213> Artificial Sequence
<220>
   <223> Oligonucleotide motif
<400> 585
   aatgttcaga tgcctc 16
<210> 586
   <211> 16
   <212> DNA
   <213> Artificial Sequence
<220>
   <223> Oligonucleotide motif
<400> 586
   gcttaatgtt cagatg 16
<210> 587
   <211> 16
   <212> DNA
   <213> Artificial Sequence
<220>
   <223> Oligonucleotide motif
<400> 587
   cgtacatagc ttgatg 16
<210> 588
   <211> 16
   <212> DNA
   <213> Artificial Sequence
<220>
   <223> Oligonucleotide motif
<400> 588
   gtgaggaatt aggata 16
<210> 589
   <211> 16
   <212> DNA
   <213> Artificial Sequence
<220>
   <223> Oligonucleotide motif
<400> 589
   gtaacaatat ggtttg 16
<210> 590
   <211> 16
   <212> DNA
   <213> Artificial Sequence
<220>
   <223> Oligonucleotide motif
<400> 590
   gaaatattgt agacta 16
<210> 591
   <211> 16
   <212> DNA
   <213> Artificial Sequence
<220>
   <223> Oligonucleotide motif
<400> 591
   ttgaaatatt gtagac 16
<210> 592
   <211> 16
   <212> DNA
   <213> Artificial Sequence
<220>
   <223> Oligonucleotide motif
<400> 592
   aagtctagta atttgc 16
<210> 593
   <211> 16
   <212> DNA
   <213> Artificial Sequence
<220>
   <223> Oligonucleotide motif
<400> 593
   gctcagtaga ttataa 16
<210> 594
   <211> 16
   <212> DNA
   <213> Artificial Sequence
<220>
   <223> Oligonucleotide motif
<400> 594
   catacactgt tgctaa 16
<210> 595
   <211> 16
   <212> DNA
   <213> Artificial Sequence
<220>
   <223> Oligonucleotide motif
<400> 595
   atggtctcaa atcatt 16
<210> 596
   <211> 16
   <212> DNA
   <213> Artificial Sequence
<220>
   <223> Oligonucleotide motif
<400> 596
   caatggtctc aaatca 16
<210> 597
   <211> 16
   <212> DNA
   <213> Artificial Sequence
<220>
   <223> Oligonucleotide motif
<400> 597
   ttcctattga ttgact 16
<210> 598
   <211> 16
   <212> DNA
   <213> Artificial Sequence
<220>
   <223> Oligonucleotide motif
<400> 598
   tttctgttca caacac 16
<210> 599
   <211> 16
   <212> DNA
   <213> Artificial Sequence
<220>
   <223> Oligonucleotide motif
<400> 599
   aggaacccac taatct 16
<210> 600
   <211> 16
   <212> DNA
   <213> Artificial Sequence
<220>
   <223> Oligonucleotide motif
<400> 600
   taaatggcag gaaccc 16
<210> 601
   <211> 16
   <212> DNA
   <213> Artificial Sequence
<220>
   <223> Oligonucleotide motif
<400> 601
   gtaaatggca ggaacc 16
<210> 602
   <211> 16
   <212> DNA
   <213> Artificial Sequence
<220>
   <223> Oligonucleotide motif
<400> 602
   ttgtaaatgg caggaa 16
<210> 603
   <211> 16
   <212> DNA
   <213> Artificial Sequence
<220>
   <223> Oligonucleotide motif
<400> 603
   ttatgagtta ggcatg 16
<210> 604
   <211> 16
   <212> DNA
   <213> Artificial Sequence
<220>
   <223> Oligonucleotide motif
<400> 604
   ccaggtgaaa ctttaa 16
<210> 605
   <211> 16
   <212> DNA
   <213> Artificial Sequence
<220>
   <223> Oligonucleotide motif
<400> 605
   cccttagtca gctcct 16
<210> 606
   <211> 16
   <212> DNA
   <213> Artificial Sequence
<220>
   <223> Oligonucleotide motif
<400> 606
   acccttagtc agctcc 16
<210> 607
   <211> 16
   <212> DNA
   <213> Artificial Sequence
<220>
   <223> Oligonucleotide motif
<400> 607
   cacccttagt cagctc 16
<210> 608
   <211> 16
   <212> DNA
   <213> Artificial Sequence
<220>
   <223> Oligonucleotide motif
<400> 608
   tctcttacta ggctcc 16
<210> 609
   <211> 16
   <212> DNA
   <213> Artificial Sequence
<220>
   <223> Oligonucleotide motif
<400> 609
   cctatctgtc atcatg 16
<210> 610
   <211> 16
   <212> DNA
   <213> Artificial Sequence
<220>
   <223> Oligonucleotide motif
<400> 610
   tcctatctgt catcat 16
<210> 611
   <211> 16
   <212> DNA
   <213> Artificial Sequence
<220>
   <223> Oligonucleotide motif
<400> 611
   gagaagtgtg agaagc 16
<210> 612
   <211> 16
   <212> DNA
   <213> Artificial Sequence
<220>
   <223> Oligonucleotide motif
<400> 612
   catccttgaa gtttag 16
<210> 613
   <211> 16
   <212> DNA
   <213> Artificial Sequence
<220>
   <223> Oligonucleotide motif
<400> 613
   taataagatg gctccc 16
<210> 614
   <211> 16
   <212> DNA
   <213> Artificial Sequence
<220>
   <223> Oligonucleotide motif
<400> 614
   caaggcataa taagat 16
<210> 615
   <211> 16
   <212> DNA
   <213> Artificial Sequence
<220>
   <223> Oligonucleotide motif
<400> 615
   ccaaggcata ataaga 16
<210> 616
   <211> 16
   <212> DNA
   <213> Artificial Sequence
<220>
   <223> Oligonucleotide motif
<400> 616
   tgatccaatt ctcacc 16
<210> 617
   <211> 16
   <212> DNA
   <213> Artificial Sequence
<220>
   <223> Oligonucleotide motif
<400> 617
   atgatccaat tctcac 16
<210> 618
   <211> 16
   <212> DNA
   <213> Artificial Sequence
<220>
   <223> Oligonucleotide motif
<400> 618
   cgcttcatct tcaccc 16
<210> 619
   <211> 16
   <212> DNA
   <213> Artificial Sequence
<220>
   <223> Oligonucleotide motif
<400> 619
   tatgacactg catctt 16
<210> 620
   <211> 16
   <212> DNA
   <213> Artificial Sequence
<220>
   <223> Oligonucleotide motif
<400> 620
   gtatgacact gcatct 16
<210> 621
   <211> 16
   <212> DNA
   <213> Artificial Sequence
<220>
   <223> Oligonucleotide motif
<400> 621
   tgtatgacac tgcatc 16
<210> 622
   <211> 16
   <212> DNA
   <213> Artificial Sequence
<220>
   <223> Oligonucleotide motif
<400> 622
   ttctcttctg taagtc 16
<210> 623
   <211> 16
   <212> DNA
   <213> Artificial Sequence
<220>
   <223> Oligonucleotide motif
<400> 623
   ttctacagag gaacta 16
<210> 624
   <211> 16
   <212> DNA
   <213> Artificial Sequence
<220>
   <223> Oligonucleotide motif
<400> 624
   actacagttc tacaga 16
<210> 625
   <211> 16
   <212> DNA
   <213> Artificial Sequence
<220>
   <223> Oligonucleotide motif
<400> 625
   ttcccacagg taaatg 16
<210> 626
   <211> 20
   <212> DNA
   <213> Artificial Sequence
<220>
   <223> Oligonucleotide motif
<400> 626
   attatttgaa tatactcatt 20
<210> 627
   <211> 17
   <212> DNA
   <213> Artificial Sequence
<220>
   <223> Oligonucleotide motif
<400> 627
   tgggaggaaa ttatttg 17
<210> 628
   <211> 16
   <212> DNA
   <213> Artificial Sequence
<220>
   <223> Oligonucleotide motif
<400> 628
   tgactcatct taaatg 16
<210> 629
   <211> 16
   <212> DNA
   <213> Artificial Sequence
<220>
   <223> Oligonucleotide motif
<400> 629
   ctgactcatc ttaaat 16
<210> 630
   <211> 16
   <212> DNA
   <213> Artificial Sequence
<220>
   <223> Oligonucleotide motif
<400> 630
   tttactctga ctcatc 16
<210> 631
   <211> 16
   <212> DNA
   <213> Artificial Sequence
<220>
   <223> Oligonucleotide motif
<400> 631
   tattggagga attatt 16
<210> 632
   <211> 16
   <212> DNA
   <213> Artificial Sequence
<220>
   <223> Oligonucleotide motif
<400> 632
   gtattggagg aattat 16
<210> 633
   <211> 20
   <212> DNA
   <213> Artificial Sequence
<220>
   <223> Oligonucleotide motif
<400> 633
   tggtatactt ctctaagtat 20
<210> 634
   <211> 16
   <212> DNA
   <213> Artificial Sequence
<220>
   <223> Oligonucleotide motif
<400> 634
   gatctcttgg tatact 16
<210> 635
   <211> 16
   <212> DNA
   <213> Artificial Sequence
<220>
   <223> Oligonucleotide motif
<400> 635
   cagacaactc tatacc 16
<210> 636
   <211> 17
   <212> DNA
   <213> Artificial Sequence
<220>
   <223> Oligonucleotide motif
<400> 636
   aacatcagac aactcta 17
<210> 637
   <211> 18
   <212> DNA
   <213> Artificial Sequence
<220>
   <223> Oligonucleotide motif
<400> 637
   tttaacatca gacaactc 18
<210> 638
   <211> 16
   <212> DNA
   <213> Artificial Sequence
<220>
   <223> Oligonucleotide motif
<400> 638
   taacatcaga caactc 16
<210> 639
   <211> 16
   <212> DNA
   <213> Artificial Sequence
<220>
   <223> Oligonucleotide motif
<400> 639
   atttaacatc agacaa 16
<210> 640
   <211> 17
   <212> DNA
   <213> Artificial Sequence
<220>
   <223> Oligonucleotide motif
<400> 640
   cctatttaac atcagac 17
<210> 641
   <211> 16
   <212> DNA
   <213> Artificial Sequence
<220>
   <223> Oligonucleotide motif
<400> 641
   tccctattta acatca 16
<210> 642
   <211> 17
   <212> DNA
   <213> Artificial Sequence
<220>
   <223> Oligonucleotide motif
<400> 642
   tcaacgacta ttggaat 17
<210> 643
   <211> 16
   <212> DNA
   <213> Artificial Sequence
<220>
   <223> Oligonucleotide motif
<400> 643
   cttatattct ggctat 16
<210> 644
   <211> 16
   <212> DNA
   <213> Artificial Sequence
<220>
   <223> Oligonucleotide motif
<400> 644
   atccttatat tctggc 16
<210> 645
   <211> 16
   <212> DNA
   <213> Artificial Sequence
<220>
   <223> Oligonucleotide motif
<400> 645
   gatccttata ttctgg 16
<210> 646
   <211> 16
   <212> DNA
   <213> Artificial Sequence
<220>
   <223> Oligonucleotide motif
<400> 646
   tgatccttat attctg 16
<210> 647
   <211> 16
   <212> DNA
   <213> Artificial Sequence
<220>
   <223> Oligonucleotide motif
<400> 647
   attgaaactt gatcct 16
<210> 648
   <211> 16
   <212> DNA
   <213> Artificial Sequence
<220>
   <223> Oligonucleotide motif
<400> 648
   actgtcattg aaactt 16
<210> 649
   <211> 16
   <212> DNA
   <213> Artificial Sequence
<220>
   <223> Oligonucleotide motif
<400> 649
   tcttactgtc attgaa 16
<210> 650
   <211> 17
   <212> DNA
   <213> Artificial Sequence
<220>
   <223> Oligonucleotide motif
<400> 650
   aggatcttac tgtcatt 17
<210> 651
   <211> 16
   <212> DNA
   <213> Artificial Sequence
<220>
   <223> Oligonucleotide motif
<400> 651
   gcaaatcaac tccatc 16
<210> 652
   <211> 16
   <212> DNA
   <213> Artificial Sequence
<220>
   <223> Oligonucleotide motif
<400> 652
   gtgcaaatca actcca 16
<210> 653
   <211> 17
   <212> DNA
   <213> Artificial Sequence
<220>
   <223> Oligonucleotide motif
<400> 653
   caattatttc tttgtgc 17
<210> 654
   <211> 18
   <212> DNA
   <213> Artificial Sequence
<220>
   <223> Oligonucleotide motif
<400> 654
   tggcaacaat tatttctt 18
<210> 655
   <211> 16
   <212> DNA
   <213> Artificial Sequence
<220>
   <223> Oligonucleotide motif
<400> 655
   gctggcaaca attatt 16
<210> 656
   <211> 16
   <212> DNA
   <213> Artificial Sequence
<220>
   <223> Oligonucleotide motif
<400> 656
   atccatttct actgcc 16
<210> 657
   <211> 19
   <212> DNA
   <213> Artificial Sequence
<220>
   <223> Oligonucleotide motif
<400> 657
   taatatctat tgatttcta 19
<210> 658
   <211> 16
   <212> DNA
   <213> Artificial Sequence
<220>
   <223> Oligonucleotide motif
<400> 658
   tcaatagtgt agggca 16
<210> 659
   <211> 16
   <212> DNA
   <213> Artificial Sequence
<220>
   <223> Oligonucleotide motif
<400> 659
   ttcaatagtg tagggc 16
<210> 660
   <211> 19
   <212> DNA
   <213> Artificial Sequence
<220>
   <223> Oligonucleotide motif
<400> 660
   aggttaatta attcaatag 19
<210> 661
   <211> 16
   <212> DNA
   <213> Artificial Sequence
<220>
   <223> Oligonucleotide motif
<400> 661
   catttgtaat ccctag 16
<210> 662
   <211> 16
   <212> DNA
   <213> Artificial Sequence
<220>
   <223> Oligonucleotide motif
<400> 662
   acatttgtaa tcccta 16
<210> 663
   <211> 16
   <212> DNA
   <213> Artificial Sequence
<220>
   <223> Oligonucleotide motif
<400> 663
   aacatttgta atccct 16
<210> 664
   <211> 16
   <212> DNA
   <213> Artificial Sequence
<220>
   <223> Oligonucleotide motif
<400> 664
   taaatttcaa gttctg 16
<210> 665
   <211> 18
   <212> DNA
   <213> Artificial Sequence
<220>
   <223> Oligonucleotide motif
<400> 665
   gtttaaattt caagttct 18
<210> 666
   <211> 18
   <212> DNA
   <213> Artificial Sequence
<220>
   <223> Oligonucleotide motif
<400> 666
   ccaagtttaa atttcaag 18
<210> 667
   <211> 17
   <212> DNA
   <213> Artificial Sequence
<220>
   <223> Oligonucleotide motif
<400> 667
   acccaagttt aaatttc 17
<210> 668
   <211> 19
   <212> DNA
   <213> Artificial Sequence
<220>
   <223> Oligonucleotide motif
<400> 668
   catacagtga cccaagttt 19
<210> 669
   <211> 16
   <212> DNA
   <213> Artificial Sequence
<220>
   <223> Oligonucleotide motif
<400> 669
   acatcccata cagtga 16
<210> 670
   <211> 16
   <212> DNA
   <213> Artificial Sequence
<220>
   <223> Oligonucleotide motif
<400> 670
   agcacagctc tacatc 16
<210> 671
   <211> 16
   <212> DNA
   <213> Artificial Sequence
<220>
   <223> Oligonucleotide motif
<400> 671
   atatagcaca gctcta 16
<210> 672
   <211> 16
   <212> DNA
   <213> Artificial Sequence
<220>
   <223> Oligonucleotide motif
<400> 672
   tccatatagc acagct 16
<210> 673
   <211> 16
   <212> DNA
   <213> Artificial Sequence
<220>
   <223> Oligonucleotide motif
<400> 673
   atttccatat agcaca 16
<210> 674
   <211> 17
   <212> DNA
   <213> Artificial Sequence
<220>
   <223> Oligonucleotide motif
<400> 674
   tttatttcca tatagca 17
<210> 675
   <211> 16
   <212> DNA
   <213> Artificial Sequence
<220>
   <223> Oligonucleotide motif
<400> 675
   tttatttcca tatagc 16
<210> 676
   <211> 17
   <212> DNA
   <213> Artificial Sequence
<220>
   <223> Oligonucleotide motif
<400> 676
   aaggagagga gattatg 17
<210> 677
   <211> 16
   <212> DNA
   <213> Artificial Sequence
<220>
   <223> Oligonucleotide motif
<400> 677
   agttcttgtg ttagct 16
<210> 678
   <211> 16
   <212> DNA
   <213> Artificial Sequence
<220>
   <223> Oligonucleotide motif
<400> 678
   gagttcttgt gttagc 16
<210> 679
   <211> 17
   <212> DNA
   <213> Artificial Sequence
<220>
   <223> Oligonucleotide motif
<400> 679
   attaattatc catccac 17
<210> 680
   <211> 18
   <212> DNA
   <213> Artificial Sequence
<220>
   <223> Oligonucleotide motif
<400> 680
   atcaattaat tatccatc 18
<210> 681
   <211> 18
   <212> DNA
   <213> Artificial Sequence
<220>
   <223> Oligonucleotide motif
<400> 681
   agaatcaatt aattatcc 18
<210> 682
   <211> 16
   <212> DNA
   <213> Artificial Sequence
<220>
   <223> Oligonucleotide motif
<400> 682
   tgagataccg tgcatg 16
<210> 683
   <211> 16
   <212> DNA
   <213> Artificial Sequence
<220>
   <223> Oligonucleotide motif
<400> 683
   aatgagatac cgtgca 16
<210> 684
   <211> 16
   <212> DNA
   <213> Artificial Sequence
<220>
   <223> Oligonucleotide motif
<400> 684
   ctgtggttag gctaat 16
<210> 685
   <211> 20
   <212> DNA
   <213> Artificial Sequence
<220>
   <223> Oligonucleotide motif
<400> 685
   aagagtaagg gtctgtggtt 20
<210> 686
   <211> 16
   <212> DNA
   <213> Artificial Sequence
<220>
   <223> Oligonucleotide motif
<400> 686
   gatgggttaa gagtaa 16
<210> 687
   <211> 16
   <212> DNA
   <213> Artificial Sequence
<220>
   <223> Oligonucleotide motif
<400> 687
   agcagatggg ttaaga 16
<210> 688
   <211> 16
   <212> DNA
   <213> Artificial Sequence
<220>
   <223> Oligonucleotide motif
<400> 688
   tgtaaacatt tgtagc 16
<210> 689
   <211> 16
   <212> DNA
   <213> Artificial Sequence
<220>
   <223> Oligonucleotide motif
<400> 689
   cctgcttata aatgta 16
<210> 690
   <211> 16
   <212> DNA
   <213> Artificial Sequence
<220>
   <223> Oligonucleotide motif
<400> 690
   tgccctgctt ataaat 16
<210> 691
   <211> 16
   <212> DNA
   <213> Artificial Sequence
<220>
   <223> Oligonucleotide motif
<400> 691
   tcttcttagt tcaata 16
<210> 692
   <211> 16
   <212> DNA
   <213> Artificial Sequence
<220>
   <223> Oligonucleotide motif
<400> 692
   tggtttctaa ctacat 16
<210> 693
   <211> 17
   <212> DNA
   <213> Artificial Sequence
<220>
   <223> Oligonucleotide motif
<400> 693
   agtttggttt ctaacta 17
<210> 694
   <211> 16
   <212> DNA
   <213> Artificial Sequence
<220>
   <223> Oligonucleotide motif
<400> 694
   gaatgaaact tgcctg 16
<210> 695
   <211> 16
   <212> DNA
   <213> Artificial Sequence
<220>
   <223> Oligonucleotide motif
<400> 695
   attatcctta catgat 16
<210> 696
   <211> 16
   <212> DNA
   <213> Artificial Sequence
<220>
   <223> Oligonucleotide motif
<400> 696
   gtacccaatt atcctt 16
<210> 697
   <211> 16
   <212> DNA
   <213> Artificial Sequence
<220>
   <223> Oligonucleotide motif
<400> 697
   tgtacccaat tatcct 16
<210> 698
   <211> 16
   <212> DNA
   <213> Artificial Sequence
<220>
   <223> Oligonucleotide motif
<400> 698
   ttgtacccaa ttatcc 16
<210> 699
   <211> 16
   <212> DNA
   <213> Artificial Sequence
<220>
   <223> Oligonucleotide motif
<400> 699
   tttgtaccca attatc 16
<210> 700
   <211> 16
   <212> DNA
   <213> Artificial Sequence
<220>
   <223> Oligonucleotide motif
<400> 700
   agcagcaggt tatatt 16
<210> 701
   <211> 16
   <212> DNA
   <213> Artificial Sequence
<220>
   <223> Oligonucleotide motif
<400> 701
   tgggaagtgg tctggg 16
<210> 702
   <211> 16
   <212> DNA
   <213> Artificial Sequence
<220>
   <223> Oligonucleotide motif
<400> 702
   ctggagagtg ataata 16
<210> 703
   <211> 16
   <212> DNA
   <213> Artificial Sequence
<220>
   <223> Oligonucleotide motif
<400> 703
   aatgctggat tacgtc 16
<210> 704
   <211> 16
   <212> DNA
   <213> Artificial Sequence
<220>
   <223> Oligonucleotide motif
<400> 704
   caatgctgga ttacgt 16
<210> 705
   <211> 16
   <212> DNA
   <213> Artificial Sequence
<220>
   <223> Oligonucleotide motif
<400> 705
   ttgttcagaa gtatcc 16
<210> 706
   <211> 16
   <212> DNA
   <213> Artificial Sequence
<220>
   <223> Oligonucleotide motif
<400> 706
   gatgatttgc ttggag 16
<210> 707
   <211> 16
   <212> DNA
   <213> Artificial Sequence
<220>
   <223> Oligonucleotide motif
<400> 707
   gaaatcattc acaacc 16
<210> 708
   <211> 16
   <212> DNA
   <213> Artificial Sequence
<220>
   <223> Oligonucleotide motif
<400> 708
   ttgtaacatc tactac 16
<210> 709
   <211> 16
   <212> DNA
   <213> Artificial Sequence
<220>
   <223> Oligonucleotide motif
<400> 709
   cattaagcag caagtt 16
<210> 710
   <211> 16
   <212> DNA
   <213> Artificial Sequence
<220>
   <223> Oligonucleotide motif
<400> 710
   ttactagatg tgagca 16
<210> 711
   <211> 16
   <212> DNA
   <213> Artificial Sequence
<220>
   <223> Oligonucleotide motif
<400> 711
   tttactagat gtgagc 16
<210> 712
   <211> 16
   <212> DNA
   <213> Artificial Sequence
<220>
   <223> Oligonucleotide motif
<400> 712
   gaccaagcac cttaca 16
<210> 713
   <211> 16
   <212> DNA
   <213> Artificial Sequence
<220>
   <223> Oligonucleotide motif
<400> 713
   agaccaagca ccttac 16
<210> 714
   <211> 16
   <212> DNA
   <213> Artificial Sequence
<220>
   <223> Oligonucleotide motif
<400> 714
   atgggttaaa taaagg 16
<210> 715
   <211> 16
   <212> DNA
   <213> Artificial Sequence
<220>
   <223> Oligonucleotide motif
<400> 715
   tcaaccagag tattaa 16
<210> 716
   <211> 16
   <212> DNA
   <213> Artificial Sequence
<220>
   <223> Oligonucleotide motif
<400> 716
   gtcaaccaga gtatta 16
<210> 717
   <211> 16
   <212> DNA
   <213> Artificial Sequence
<220>
   <223> Oligonucleotide motif
<400> 717
   attgtaaagc tgatat 16
<210> 718
   <211> 16
   <212> DNA
   <213> Artificial Sequence
<220>
   <223> Oligonucleotide motif
<400> 718
   cacataattg taaagc 16
<210> 719
   <211> 16
   <212> DNA
   <213> Artificial Sequence
<220>
   <223> Oligonucleotide motif
<400> 719
   gaggtctgct atttac 16
<210> 720
   <211> 16
   <212> DNA
   <213> Artificial Sequence
<220>
   <223> Oligonucleotide motif
<400> 720
   tgtagattca atgcct 16
<210> 721
   <211> 16
   <212> DNA
   <213> Artificial Sequence
<220>
   <223> Oligonucleotide motif
<400> 721
   cctcattata ctatga 16
<210> 722
   <211> 16
   <212> DNA
   <213> Artificial Sequence
<220>
   <223> Oligonucleotide motif
<400> 722
   ccttatgcta tgacac 16
<210> 723
   <211> 16
   <212> DNA
   <213> Artificial Sequence
<220>
   <223> Oligonucleotide motif
<400> 723
   tccttatgct atgaca 16
<210> 724
   <211> 16
   <212> DNA
   <213> Artificial Sequence
<220>
   <223> Oligonucleotide motif
<400> 724
   aagatgttta agtata 16
<210> 725
   <211> 16
   <212> DNA
   <213> Artificial Sequence
<220>
   <223> Oligonucleotide motif
<400> 725
   ctgattatta agatgt 16
<210> 726
   <211> 16
   <212> DNA
   <213> Artificial Sequence
<220>
   <223> Oligonucleotide motif
<400> 726
   tggaaaggta tgaatt 16
<210> 727
   <211> 16
   <212> DNA
   <213> Artificial Sequence
<220>
   <223> Oligonucleotide motif
<400> 727
   acttgaatgg cttgga 16
<210> 728
   <211> 16
   <212> DNA
   <213> Artificial Sequence
<220>
   <223> Oligonucleotide motif
<400> 728
   aacttgaatg gcttgg 16
<210> 729
   <211> 16
   <212> DNA
   <213> Artificial Sequence
<220>
   <223> Oligonucleotide motif
<400> 729
   caatgtgtta ctattt 16
<210> 730
   <211> 16
   <212> DNA
   <213> Artificial Sequence
<220>
   <223> Oligonucleotide motif
<400> 730
   acaatgtgtt actatt 16
<210> 731
   <211> 16
   <212> DNA
   <213> Artificial Sequence
<220>
   <223> Oligonucleotide motif
<400> 731
   catctgctat ataaga 16
<210> 732
   <211> 16
   <212> DNA
   <213> Artificial Sequence
<220>
   <223> Oligonucleotide motif
<400> 732
   cctagagcaa atactt 16
<210> 733
   <211> 16
   <212> DNA
   <213> Artificial Sequence
<220>
   <223> Oligonucleotide motif
<400> 733
   cagagttaat aataag 16
<210> 734
   <211> 16
   <212> DNA
   <213> Artificial Sequence
<220>
   <223> Oligonucleotide motif
<400> 734
   gttcaagcac aacgaa 16
<210> 735
   <211> 16
   <212> DNA
   <213> Artificial Sequence
<220>
   <223> Oligonucleotide motif
<400> 735
   agggttcaag cacaac 16
<210> 736
   <211> 16
   <212> DNA
   <213> Artificial Sequence
<220>
   <223> Oligonucleotide motif
<400> 736
   tgttggagac actgtt 16
<210> 737
   <211> 16
   <212> DNA
   <213> Artificial Sequence
<220>
   <223> Oligonucleotide motif
<400> 737
   aaggaggagt taggac 16
<210> 738
   <211> 16
   <212> DNA
   <213> Artificial Sequence
<220>
   <223> Oligonucleotide motif
<400> 738
   ctatgccatt tacgat 16
<210> 739
   <211> 16
   <212> DNA
   <213> Artificial Sequence
<220>
   <223> Oligonucleotide motif
<400> 739
   tcaaatgcag aattag 16
<210> 740
   <211> 16
   <212> DNA
   <213> Artificial Sequence
<220>
   <223> Oligonucleotide motif
<400> 740
   agtgacaatc aaatgc 16
<210> 741
   <211> 16
   <212> DNA
   <213> Artificial Sequence
<220>
   <223> Oligonucleotide motif
<400> 741
   aagtgacaat caaatg 16
<210> 742
   <211> 16
   <212> DNA
   <213> Artificial Sequence
<220>
   <223> Oligonucleotide motif
<400> 742
   gtgtaccaag taacaa 16
<210> 743
   <211> 16
   <212> DNA
   <213> Artificial Sequence
<220>
   <223> Oligonucleotide motif
<400> 743
   tgggatgtta aactga 16
<210> 744
   <211> 16
   <212> DNA
   <213> Artificial Sequence
<220>
   <223> Oligonucleotide motif
<400> 744
   agtttacatt ttctgc 16
<210> 745
   <211> 16
   <212> DNA
   <213> Artificial Sequence
<220>
   <223> Oligonucleotide motif
<400> 745
   tatgtgaaga ggagag 16
<210> 746
   <211> 16
   <212> DNA
   <213> Artificial Sequence
<220>
   <223> Oligonucleotide motif
<400> 746
   cacctttaaa acccca 16
<210> 747
   <211> 16
   <212> DNA
   <213> Artificial Sequence
<220>
   <223> Oligonucleotide motif
<400> 747
   tcctttataa tcacac 16
<210> 748
   <211> 16
   <212> DNA
   <213> Artificial Sequence
<220>
   <223> Oligonucleotide motif
<400> 748
   acggtatttt cacagg 16
<210> 749
   <211> 16
   <212> DNA
   <213> Artificial Sequence
<220>
   <223> Oligonucleotide motif
<400> 749
   gacactacaa tgagga 16
<210> 750
   <211> 16
   <212> DNA
   <213> Artificial Sequence
<220>
   <223> Oligonucleotide motif
<400> 750
   tggtttttag gactgt 16
<210> 751
   <211> 16
   <212> DNA
   <213> Artificial Sequence
<220>
   <223> Oligonucleotide motif
<400> 751
   cgacaaattc tatcct 16
<210> 752
   <211> 16
   <212> DNA
   <213> Artificial Sequence
<220>
   <223> Oligonucleotide motif
<400> 752
   tgatatacaa tgctac 16
<210> 753
   <211> 16
   <212> DNA
   <213> Artificial Sequence
<220>
   <223> Oligonucleotide motif
<400> 753
   tcgttgggta aattta 16
<210> 754
   <211> 16
   <212> DNA
   <213> Artificial Sequence
<220>
   <223> Oligonucleotide motif
<400> 754
   tgctttataa atggtg 16
<210> 755
   <211> 18
   <212> DNA
   <213> Artificial Sequence
<220>
   <223> Oligonucleotide motif
<400> 755
   caagtttaca ttttctgc 18
<210> 756
   <211> 18
   <212> DNA
   <213> Artificial Sequence
<220>
   <223> Oligonucleotide motif
<400> 756
   catatgtgaa gaggagag 18
<210> 757
   <211> 16
   <212> DNA
   <213> Artificial Sequence
<220>
   <223> Oligonucleotide motif
<400> 757
   cacctttaaa acccca 16
<210> 758
   <211> 18
   <212> DNA
   <213> Artificial Sequence
<220>
   <223> Oligonucleotide motif
<400> 758
   catcctttat aatcacac 18
<210> 759
   <211> 18
   <212> DNA
   <213> Artificial Sequence
<220>
   <223> Oligonucleotide motif
<400> 759
   caacggtatt ttcacagg 18
<210> 760
   <211> 18
   <212> DNA
   <213> Artificial Sequence
<220>
   <223> Oligonucleotide motif
<400> 760
   cagacactac aatgagga 18
<210> 761
   <211> 18
   <212> DNA
   <213> Artificial Sequence
<220>
   <223> Oligonucleotide motif
<400> 761
   catggttttt aggactgt 18
<210> 762
   <211> 18
   <212> DNA
   <213> Artificial Sequence
<220>
   <223> Oligonucleotide motif
<400> 762
   cacgacaaat tctatcct 18
<210> 763
   <211> 18
   <212> DNA
   <213> Artificial Sequence
<220>
   <223> Oligonucleotide motif
<400> 763
   catgatatac aatgctac 18
<210> 764
   <211> 18
   <212> DNA
   <213> Artificial Sequence
<220>
   <223> Oligonucleotide motif
<400> 764
   catcgttggg taaattta 18
<210> 765
   <211> 18
   <212> DNA
   <213> Artificial Sequence
<220>
   <223> Oligonucleotide motif
<400> 765
   catgctttat aaatggtg 18
<210> 766
   <211> 20
   <212> DNA
   <213> Artificial Sequence
<220>
   <223> Oligonucleotide motif
<400> 766
   caacaaataa tggttactct 20
<210> 767
   <211> 18
   <212> DNA
   <213> Artificial Sequence
<220>
   <223> Oligonucleotide motif
<400> 767
   cacagattga tggtagtt 18
<210> 768
   <211> 19
   <212> DNA
   <213> Artificial Sequence
<220>
   <223> Oligonucleotide motif
<400> 768
   cacctattta acatcagac 19
<210> 769
   <211> 20
   <212> DNA
   <213> Artificial Sequence
<220>
   <223> Oligonucleotide motif
<400> 769
   cactaattgt agtagtactc 20
<210> 770
   <211> 20
   <212> DNA
   <213> Artificial Sequence
<220>
   <223> Oligonucleotide motif
<400> 770
   caataaacat gaatctctcc 20
<210> 771
   <211> 17
   <212> DNA
   <213> Artificial Sequence
<220>
   <223> Oligonucleotide motif
<400> 771
   tgatttaatt ctagtca 17
<210> 772
   <211> 16
   <212> DNA
   <213> artificial sequence
<220>
   <223> Target sequence
<400> 772
   gcagtagagc caatta 16
<210> 773
   <211> 10
   <212> PRT
   <213> artificial
<220>
   <223> HBV epitope
<400> 773 
Phe Leu Pro Ser Asp Phe Phe Pro Ser Val
1               5                   10
<210> 774
   <211> 15
   <212> PRT
   <213> Artificial
<220>
   <223> HBV epitope
<400> 774 
Gly Arg Glu Thr Val Leu Glu Tyr Leu Val Ser Phe Gly Val Trp
1               5                   10                  15
<210> 775
   <211> 15
   <212> PRT
   <213> Artificial
<220>
   <223> HBV epitope
<400> 775 
Thr Thr Phe His Gln Thr Leu Gln Asp Pro Arg Val Arg Gly Leu
1               5                   10                  15
<210> 776
   <211> 16
   <212> PRT
   <213> Artificial
<220>
   <223> HBV epitope
<400> 776 
Gln Ala Gly Phe Phe Leu Leu Thr Arg Ile Leu Thr Ile Pro Gln Ser
1               5                   10                  15
<210> 777
   <211> 9
   <212> PRT
   <213> Artificial
<220>
   <223> HBV epitope
<400> 777 
Phe Leu Leu Thr Arg Ile Leu Thr Ile
1               5
<210> 778
   <211> 15
   <212> PRT
   <213> Artificial
<220>
   <223> HBV epitope
<400> 778 
Thr Ser Leu Asn Phe Leu Gly Gly Thr Thr Val Cys Leu Gly Gln
1               5                   10                  15
<210> 779
   <211> 9
   <212> PRT
   <213> Artificial
<220>
   <223> HBV epitope
<400> 779 
Phe Leu Gly Gly Thr Thr Val Cys Leu
1               5
<210> 780
   <211> 9
   <212> PRT
   <213> Artificial
<220>
   <223> HBV epitope
<400> 780 
Trp Leu Ser Leu Leu Val Pro Phe Val
1               5
<210> 781
   <211> 21
   <212> PRT
   <213> Artificial
<220>
   <223> HBV epitope
<400> 781 
Ser Leu Leu Val Pro Phe Val Gln Trp Phe Val Gly Leu Ser Pro Thr
1               5                   10                  15

Val Trp Leu Ser Val
            20
<210> 782
   <211> 10
   <212> PRT
   <213> Artificial
<220>
   <223> HBV epitope
<400> 782 
Gly Leu Ser Pro Thr Val Trp Leu Ser Val
1               5                   10
<210> 783
   <211> 10
   <212> PRT
   <213> Artificial
<220>
   <223> HBV epitope
<400> 783 
Ser Ile Leu Ser Pro Phe Leu Pro Leu Leu
1               5                   10
<210> 784
   <211> 19
   <212> DNA
   <213> Artificial
<220>
   <223> Primer
<400> 784
   ctgtgccttg ggtggcttt 19
<210> 785
   <211> 24
   <212> DNA
   <213> Artificial
<220>
   <223> Primer
<400> 785
   aaggaaagaa gtcagaaggc aaaa 24
<210> 786
   <211> 25
   <212> DNA
   <213> Artificial
<220>
   <223> primer
<400> 786
   ttctttataa gggtcgatgt ccatg 25